(19) Europäisches Patentamt
European Patent Office
Office européen des brevets

(11) EP 3 851 533 A1

(12) EUROPEAN PATENT APPLICATION
published in accordance with Art. 153(4) EPC

(43) Date of publication:
**21.07.2021 Bulletin 2021/29**

(21) Application number: **19873006.1**

(22) Date of filing: **30.09.2019**

(51) Int Cl.:
***C12N 15/115*** *(2010.01)* ***A61K 47/56*** *(2017.01)*
***A61K 31/713*** *(2006.01)* ***C07H 21/04*** *(2006.01)*

(86) International application number:
**PCT/CN2019/109466**

(87) International publication number:
**WO 2020/078216 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority:
**19.10.2018 CN 201811224511**
**22.10.2018 CN 201811230635**
**23.10.2018 CN 201811237274**
**29.10.2018 CN 201811270171**
**29.10.2018 CN 201811270120**

(71) Applicant: **Bai Yao Zhi Da (Beijing) Nanobio Technology Co., Ltd.**
**Beijing 100080 (CN)**

(72) Inventors:
- **WANG, Liyuan**
  **Beijing 100080 (CN)**
- **WANG, Meng**
  **Beijing 100080 (CN)**

(74) Representative: **Biggi, Cristina**
**Bugnion S.p.A.**
**Viale Lancetti 17**
**20158 Milano (IT)**

# (54) NUCLEIC ACID NANOCARRIER MEDICINE AND PREPARATION METHOD THEREFOR

(57) Disclosed are a nucleic acid nanocarrier drug and a preparation method thereof. The drug includes nucleic acid nanoparticles and a drug ingredient, the drug ingredient is loaded on the nucleic acid nanoparticles, and the drug ingredient is dihydroartemisinin, cisplatin, oxaliplatin, flavone or vincristine; the nucleic acid nanoparticles include a nucleic acid structureal domain, the nucleic acid structureal domain includes a sequence a, a sequence b, and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1. The nucleic acid nanocarrier drug provided by the disclosure may have a better targeting property after the nucleic acid structureal domain thereof is modified by a target head, may deliver the drug ingredient stably, and has high reliability.

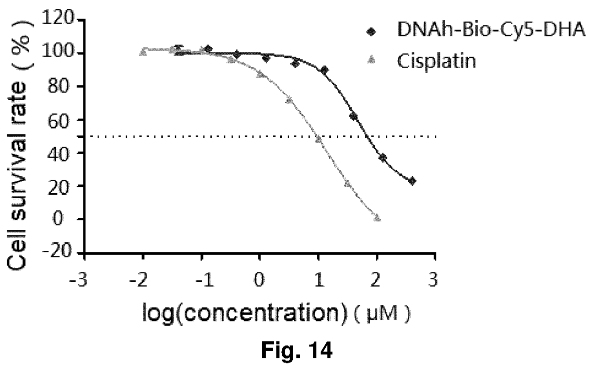


Fig. 14

EP 3 851 533 A1

## Description

### Technical Field

The disclosure relates to the field of medicines, and in particular to a nucleic acid nanocarrier drug and a preparation method thereof.

### Background

Dihydroartemisinin (DHA for short) is a derivative of artemisinin, and has significant anti-tumor activity, a main mechanism of action is as follows: the dihydroartemisinin may cause iron deficiency in tumor cells, reduce iron absorption, and interfere an existing balance of an iron element in cells so as to kill the tumor cells. The dihydroartemisinin may be mainly used to treat cancers such as a liver cancer, an ovarian cancer, a lung cancer, a pancreatic cancer, osteosarcoma and cerebral medulloblastoma.

Vincristine (Oncovin, VCR, molecular formula: $C_{46}H_{56}N_4O_{10}$, molecular weight: 824.96, and CAS number: 57-22-7) is a bi-indole alkaloid, and is a natural plant anti-tumor drug. It is mainly used to treat acute lymphocytic leukemia, and may also be used to treat chronic lymphocytic leukemia, Hodgkin's lymphoma, lymphosarcoma, Ewing sarcoma, neuroblastoma, reticulosarcoma, small cell carcinoma, digestive tract cancer, melanoma and multiple myeloma and a breast cancer. It has an effect of stopping cell division (mitosis) in a metaphase.

Cisplatin (referred to as DDP, CSA: 15773-27-1, molecular formula: $Cl_2H_4N_2PtI$, and molecular weight: 298.029) is orange-yellow or yellow crystalline powder, slightly soluble in water, freely soluble in methyl formamide. It may be gradually transformed into trans or hydrolyzed in aqueous solution. It is clinically used for various solid tumors such as an ovarian cancer, a prostate cancer, a testicular cancer, a lung cancer, a nasopharyngeal cancer, an esophageal cancer, malignant lymphoma, head and neck squamous cell carcinoma, a thyroid cancer and osteosarcoma.

Oxaliplatin is a third-generation platinum anticancer drug, and is a platinum compound of diaminocyclohexane, namely, an amino group of the cisplatin is substituted by a 1,2-diaminocyclohexane group. It has the same effect as other platinum drugs, they all use a DNA as a target action site, platinum atoms form a cross-linkage with the DNA to antagonize replication and transcription thereof. It has a better curative effect on the colorectal cancer and the ovarian cancer, and has a certain curative effect on a gastric cancer, non-Hodgkin's lymphoma, non-small cell carcinoma, and head and neck tumors.

Flavone refers to a series of compounds composed of two benzene rings (A- and B-cycles) with phenolic hydroxyl groups connected to each other through three central carbon atoms, and a basic mother nucleus thereof is 2-phenylchromone. In structures of flavonoids compounds, functional groups such as a phenolic hydroxyl, a methoxy, a methyl, and an isopentenyl are often linked. In addition, it is often combined with sugar to form a glycoside. Effects of the flavone are various. It is a very strong antioxidant which may effectively remove oxygen free radicals in vivo. For example, anthocyanins may inhibit the overflow of oily peroxides in all phases. This ability to prevent oxidation is more than ten times of a vitamin E, such an antioxidation effect may prevent cell degeneration, senescence, and may prevent cancer incidence. In addition, it also has an effect of improving blood circulation and the like.

At present, anti-tumor antibiotics, including the dihydroartemisinin, vincristine, cisplatin, oxaliplatin, and flavone, must use large doses of chemotherapeutics in order to achieve an effective treatment level at a tumor site, but the large doses of systemic administration may damage healthy normal cells, and cause adverse reactions in a series of tissues and organs. These adverse reactions include suppression of an immune system (myelosuppression), inflammation and ulcers of mucous membranes of a digestive tract (mucositis), hair loss (alopecia), and organ-specific toxicity, such as cardiotoxicity and neurotoxicity. In order to avoid the occurrence of these adverse reactions, it is necessary to replace the traditional systemic administration with a local tumor administration mode to achieve effects of increasing the local drug concentration of the tumor and reducing the systemic drug concentration. Therefore, how to achieve such local drug delivery and controlled release in vitro becomes a focus of a cancer chemotherapy research.

In order to alleviate side effects caused by poor targeting of active ingredients of a drug, a drug delivery carrier is generated at the right moment, and a function thereof is mainly to carry the active ingredients of the drug, and deliver the active ingredients into blood or tissue cells so as to treat diseases. There are already a variety of methods to achieve the targeted delivery of the different drugs. It may be achieved with instruments or devices, such as a gene gun, and an electroporator. These methods do not need to use a gene carrier, but the transfection efficiency is generally very low, the step is complicated, and the damage to a tissue is relatively large. It is also mediated by viral carriers, such as an adenovirus, and a lentivirus. Although the viral carriers have the higher transfection activity in vitro, the immunogenicity and disadvantages thereof which may easily cause mutations bring huge safety risks to in vivo delivery. There are also non-viral carriers, especially a biodegradable polymer material, to achieve the targeted delivery of the drug. A main advantage of the non-viral carriers is that the immunogenicity and many inflammatory reactions brought by the viral carriers may be greatly reduced under a condition of guaranteeing the expected transfection activity.

In the above multiple targeted delivery modes, more researches are focused on the field of the non-viral carriers at present, and it is generally a plurality of the following carrier designs: (a) a cationic liposome; and (b) a polycationic gene carrier. At present, the more researches focus on modification of the polycationic gene carrier and the cationic liposome, so that it is suitable for the targeted delivery of the genetic substances. The cationic liposome has the higher transfection activity in vivo and in vitro. However, because normal distribution thereof in vivo is affected by positive charge on a surface, at the same time, the cationic liposome may cause the immunogenicity and the inflammatory responses in animal experiments. The development of the polycationic gene carrier is relatively mature at present, but it is difficult to guarantee that a targeting group is on a surface of a structure in a structural design, and there is an own design contradiction between the toxicity and the transfection activity, at the same time, connection thereof is difficult to achieve non-toxic degradation in vivo.

Therefore, how to improve the delivery reliability of an existing small molecular drug of the dihydroartemisinin, vincristine, cisplatin, oxaliplatin, and flavone is one of the difficulties in solving a limited clinical application of the existing drug.

## Summary

A main purpose of the disclosure is to provide a nucleic acid nanocarrier drug and a preparation method thereof, as to improve delivery reliability of drug ingredients of dihydroartemisinin, vincristine, cisplatin, oxaliplatin, and flavone.

In order to achieve the above purpose, according to one aspect of the disclosure, a nucleic acid nanocarrier drug is provided, and it includes nucleic acid nanoparticles and a drug ingredient, the drug ingredient is loaded on the nucleic acid nanoparticles, and the drug ingredient is the dihydroartemisinin, cisplatin, oxaliplatin, flavone or vincristine; and the nucleic acid nanoparticles includes a nucleic acid structureal domain, the nucleic acid structureal domain includes a sequence a, a sequence b, and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein, the sequence a1 is SEQ ID NO:1: 5'-CCAGCGUUCC-3' or SEQ ID NO:2: 5'-CCAGCGTTCC-3'; the sequence b1 is SEQ ID NO:3: 5'-GGUUCGCCG-3' or SEQ ID NO:4: 5'-GGTTCGCCG-3'; and the sequence c1 is SEQ ID NO:5: 5'-CGGCCAUAGCGG-3' or SEQ ID NO:6: 5'-CGGCCATAGCGG-3'.

Further, when the sequence a1 is the SEQ ID NO:1, the sequence b1 is the SEQ ID NO:3, and the sequence c1 is the SEQ ID NO:5, at least one sequence of the sequence a, the sequence b and the sequence c includes a sequence by insertion, deletion or substitution of at least one base in the sequence a, the sequence b and/or the sequence c.

Further, the insertion, deletion or substitution of at least one base is occurred:

(1) at 1, 2, 4 or 5-th base starting from a 5 '-end of the sequence shown in the SEQ ID NO:1 or the SEQ ID NO:2; and/or

(2) between 8-th and 10-th bases starting from the 5 '-end of the sequence shown in the SEQ ID NO:1 or the SEQ ID NO:2; and/or

(3) between 1-th and 3-th bases starting from a 5 '-end of the sequence shown in the SEQ ID NO:3 or the SEQ ID NO:4; and/or

(4) between 6-th and 9-th bases starting from the 5 '-end of the sequence shown in the SEQ ID NO:3 or the SEQ ID NO:4; and/or

(5) between 1-th and 4-th bases starting from a 5 '-end of the sequence shown in the SEQ ID NO:5 or the SEQ ID NO:6; and/or

(6) between 9-th and 12-th bases starting from the 5 '-end of the sequence shown in the SEQ ID NO:5 or the SEQ ID NO:6

Further, the sequence a, the sequence b and the sequence c are self-assembled into a structure shown in Formula (1):

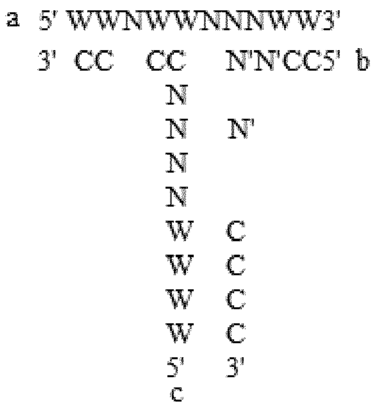


Formula (1),

Herein, W-C represents Watson-Crick pairing, N and N' represent a non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

Further, the sequence a, the sequence b and the sequence c are any one of the following groups: (1) sequence a: 5'-GGAGCGUUGG-3', sequence b: 5'-CCUUCGCCG-3', sequence c: 5'-CGGCCAUAGCCC-3'; (2) sequence a: 5'-GCAGCGUUCG-3', sequence b: 5'-CGUUCGCCG-3', sequence c: 5'-CGGCCAUAGCGC-3'; (3) sequence a: 5'-CGAGCGUUGC-3', sequence b: 5'-GCUUCGCCG-3', sequence c: 5'-CGGCCAUAGCCG-3'; (4) sequence a: 5'-GGAGCGUUGG-3', sequence b: 5'-CCUUCGGGG-3', sequence c: 5'-CCCCCAUAGCCC-3'; (5) sequence a: 5'-GCAGCGUUCG-3', sequence b: 5'-CGUUCGGCG-3', sequence c: 5'-CGCCCAUAGCGC-3'; (6) sequence a: 5'-GCAGCGUUCG-3', sequence b: 5'-CGUUCGGCC-3', sequence c: 5'-GGCCCAUAGCGC-3'; (7) sequence a: 5'-CGAGCGUUGC-3', sequence b: 5'-GCUUCGGCG-3', sequence c: 5'-CGCCCAUAGCCG-3'; (8) sequence a: 5'-GGAGCGTTGG-3', sequence b: 5'-CCTTCGCCG-3', sequence c: 5'-CGGCCATAGCCC-3'; (9) sequence a: 5'-GCAGCGTTCG-3', sequence b: 5'-CGTTCGCCG-3', sequence c: 5'-CGGCCATAGCGC-3'; (10) sequence a: 5'-CGAGCGTTGC-3', sequence b: 5'-GCTTCGCCG-3', sequence c: 5'-CGGCCATAGCCG-3'; (11) sequence a: 5'-GGAGCGTTGG-3', sequence b: 5'-CCTTCGGGG-3', sequence c: 5'-CCCCCATAGCCC-3'; (12) sequence a: 5'-GCAGCGTTCG-3', sequence b: 5'-CGTTCGGCG-3', sequence c: 5'-CGCCCATAGCGC-3'; (13) sequence a: 5'-GCAGCGTTCG-3', sequence b: 5'-CGTTCGGCC-3', sequence c: 5'-GGCCCATAGCGC-3'; (14) sequence a: 5'-CGAGCGTTGC-3', sequence b: 5'-GCTTCGGCG-3', sequence c: 5'-CGCCCATAGCCG-3'; and (15) sequence a: 5'-CGAGCGTTCC -3', sequence b: 5'-GGTTCGCCG -3', sequence c: 5'-CGGCCATAGCCG-3'.

Further, in the nucleic acid structureal domain, a first extension fragment is further includes, the first extension fragment is an extension fragment of Watson-Crick pairing, and the first extension fragment is positioned at the 5'-end and/or the 3'-end of any one sequence of the sequence a, the sequence b or the sequence c; preferably, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; and (9): b-strand 3'-end: 5'-CCA-3', c-strand 5'-end: 5'-TGG-3'.

Further, the nucleic acid structureal domain further includes a second extension fragment, the second extension fragment is positioned at the 5'-end and/or the 3'-end of any one sequence of the sequence a, the sequence b or the sequence c, and the second extension fragment is an extension fragment of Watson-Crick pairing; preferably, the second extension fragment is an extension sequence of a CG base pair; more preferably, the second extension fragment is an extension sequence of 1-10 CG base pairs.

Further, the nucleic acid structureal domain further includes at least one group of the following second extension fragments: a first group: a-strand 5'-end: 5'-CGCGCG-3', c-strand 3'-end: 5'-CGCGCG-3'; a second group: a-strand 3'-end: 5'-CGCCGC-3', b-strand 5'-end: 5'-GCGGCG-3'; and a third group: b-strand 3'-end: 5'-GGCGGC-3', c-strand 5'-end: 5'-GCCGCC-3'.

Further, the second extension fragment is an extension sequence containing both CG base pair and AT/AU base pair, and preferably the second extension fragment is an extension sequence of 2-50 base pairs.

Further, the second extension fragment is an extension sequence in which sequences of 2-8 continuous CG base pairs and sequences of 2-8 continuous AT/AU base pairs are alternately arranged; or the second extension fragment

is an extension sequence in which a sequence of 1 CG base pair and a sequence of 1 AT/AU base pair are alternately arranged.

Further, a base, a ribose and a phosphate in the sequence a, the sequence b and the sequence c have at least one modifiable site, and any one of the modifiable sites is modified by any one of the following modificationadaptors: -F, a methyl, an amino, a disulfide, a carbonyl, a carboxyl, a sulfhydryl and a formyl; and preferably, the base C or U in the sequence a, the sequence b and the sequence c has 2'-F modification.

Further, the drug ingredient is loaded on the nucleic acid nanoparticles in a physical linkage mode and/or a covalent linkage mode, and a molar ratio between the drug ingredient and the nucleic acid nanoparticles is 2-300:1, preferably 10-50:1, and more preferably 15-25:1.

Further, the nucleic acid nanoparticles further include a bioactive substance, the bioactive substance is linked with the nucleic acid structureal domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenols, a lecithin, and a small molecular drug except the dihydroartemisinin, the cisplatin, the oxaliplatin, the flavone and the vincristine.

Further, a relative molecular weight of the nucleic acid structureal domain is marked as N1, and a total relative molecular weight of the drug ingredient and the bioactive substance is marked as N2, $N1/N2 \geq 1:1$.

Further, the bioactive substance is one or more of the target head, the fluorescein and the miRNA, herein the target head is positioned on any one sequence of the sequence a, the sequence b and the sequence c, preferably the 5'-end or the 3'-end of any one sequence of the sequence a, the sequence b and the sequence c, or inserted between GC bonds of the nucleic acid structureal domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is a folic acid or a biotin, the fluorescein is any one or more of FAM, CY5 and CY3, and the anti-miRNA is anti-miR-21.

Further, the small molecular drug except the dihydroartemisinin, the cisplatin, the oxaliplatin, the flavone and the vincristine is a drug containing any one or more of the following groups: an amino group, a hydroxyl group, a carboxyl group, a mercapto group, a benzene ring group and an acetamido group.

Further, the protein is one or more of SOD, survivin, hTERT, EGFR and PSMA; the vitamin is L-VC and/or esterified VC; and the phenols are tea polyphenols and/or grape polyphenols.

Further, a particle size of the nucleic acid nanoparticles is 1-100 nm, preferably 5-50 nm; more preferably 10-30 nm; and further preferably 10-15 nm.

According to another aspect of the disclosure, a method for preparing a nucleic acid nanocarrier drug is further provided, and it includes the following operations: providing the above nucleic acid nanoparticles; and loading the drug ingredient on the nucleic acid nanoparticles in a physical linkage mode and/or a covalent linkage mode, to obtain the nucleic acid nanocarrier drug, wherein the drug ingredient is dihydroartemisinin, cisplatin, oxaliplatin, flavone or vincristine.

Further, the step of loading the drug ingredient in the physical linkage mode includes: mixing and stirring the drug ingredient, the nucleic acid nanoparticles and a first solvent, to obtain a premixed system; and precipitating the premixed system, to obtain the nucleic acid nanocarrier drug; preferably, the first solvent is selected from one or more of DCM, DCC, DMAP, Py, DMSO, PBS and glacial acetic acid; preferably, the step of precipitating the premixed system, to obtain the nucleic acid nanocarrier drug includes: precipitating the premixed system, to obtain a precipitation; and washing the precipitation to remove impurities, as to obtain the nucleic acid nanocarrier drug; more preferably, mixing the premixed system with absolute ethyl alcohol, and precipitating at a temperature condition lower than 10 DEG C, to obtain the precipitation; and more preferably, precipitating at a temperature condition of 0-5 DEG C, to obtain the precipitation; and more preferably, washing the precipitation to remove the impurities with 6-12 times of the absolute ethyl alcohol in volume, as to obtain the nucleic acid nanocarrier drug.

Further, the step of loading the drug ingredient in the covalent linkage mode includes: preparing drug ingredient solution of the dihydroartemisinin, cisplatin, oxaliplatin, flavone or vincristine; enabling the drug ingredient solution to react with the G-exocyclic amino of the nucleic acid nanoparticles under a mediating effect of the formaldehyde, to obtain a reaction system; and purifying the reaction system, to obtain the nucleic acid nanocarrier drug; preferably, the reaction step includes: mixing the drug ingredient solution with a paraformaldehyde solution and the nucleic acid nanoparticles, and reacting in a dark condition, to obtain the reaction system; herein the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

Further, the preparation method further includes a step of preparing the nucleic acid nanoparticles, the step includes: self-assembling a single strand corresponding to the above nucleic acid structureal domain, to obtain the nucleic acid structureal domain; preferably, after the nucleic acid structureal domain is obtained, the preparation method further includes: loading the above bioactive substance on the nucleic acid structureal domain in the physical linkage mode and/or the covalent linkage mode, to obtain the nucleic acid nanoparticles.

Further, in a process of loading the bioactive substance in the covalent linkage mode, the loading is performed through solvent covalent linkage, linker covalent linkage or click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; preferably, the click-linkage is that a bioactive substance precursor and the nucleic acid structural domain is modified by alkynyl or azide simultaneously, and then linked through a clickreaction.

Further, the bioactive substance is linked with the nucleic acid structureal domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structureal domain is selected from a G-exocyclic amino, a 2'-hydroxyl, an A-amino or a 2'-hydroxyl.

According to a third aspect of the disclosure, a pharmaceutical composition is further provided, and the pharmaceutical composition includes any one of the above nucleic acid nanocarrier drugs.

According to a fourth aspect of the disclosure, an application of the above nucleic acid nanocarrier drug in preparing a drug for treating a tumor is further provided, and a drug ingredient thereof is dihydroartemisinin, vincristine, oxaliplatin or cisplatin.

Further, the drug ingredient is the dihydroartemisinin, and the tumor is any one or more of a liver cancer, an ovarian cancer, a lung cancer, a pancreatic cancer, osteosarcoma and cerebral medulloblastoma; or the drug ingredient is the vincristine, and the tumor is acute lymphocytic leukemia, chronic lymphocytic leukemia, Hodgkin's lymphoma, lymphosarcoma, Ewing's sarcoma, neuroblastoma, reticulocyte sarcoma, small cell carcinoma, a digestive tract cancer, a liver cancer, melanoma and multiple myeloma and a breast cancer; or the drug ingredient is the oxaliplatin, and the tumor is any one or more of a colorectal cancer, an ovarian cancer, a gastric cancer, non-Hodgkin's lymphoma, non-small cell carcinoma, and head and neck tumors; or the drug ingredient is the cisplatin, and the tumor is any one or more of an ovarian cancer, a prostate cancer, a testicular cancer, a lung cancer, a nasopharyngeal cancer, an esophageal cancer, malignant lymphoma, head and neck squamous cell carcinoma, a thyroid cancer, and osteosarcoma.

According to a fifth aspect of the disclosure, an application of the above nucleic acid nanocarrier drug in preparing a drug for treating tumors, cardiovascular and cerebrovascular diseases, retinopathy caused by diabetes and capillary fragility or allergies is further provided, herein a drug ingredient is flavone.

Further, the tumor is the ovarian cancer.

According to a sixth aspect of the disclosure, a method for preventing and/or treating a tumor is further provided, the method includes: providing any one of the above nucleic acid nanocarrier drugs or pharmaceutical compositions, herein a drug ingredient is dihydroartemisinin, vincristine, oxaliplatin or cisplatin; and administering an effective dose of the above nucleic acid nanocarrier drug or pharmaceutical composition to a tumor patient.

Further, the drug ingredient is the dihydroartemisinin, and the tumor is any one or more of a liver cancer, an ovarian cancer, a lung cancer, a pancreatic cancer, osteosarcoma and cerebral medulloblastoma; or the drug ingredient is the vincristine, and the tumor is acute lymphocytic leukemia, chronic lymphocytic leukemia, Hodgkin's lymphoma, lymphosarcoma, Ewing's sarcoma, neuroblastoma, reticulocyte sarcoma, small cell carcinoma, a digestive tract cancer, a liver cancer, melanoma and multiple myeloma and a breast cancer; or the drug ingredient is the oxaliplatin, and the tumor is any one or more of a colorectal cancer, an ovarian cancer, a gastric cancer, non-Hodgkin's lymphoma, non-small cell carcinoma, and head and neck tumors; or the drug ingredient is the cisplatin, and the tumor is any one or more of an ovarian cancer, a prostate cancer, a testicular cancer, a lung cancer, a nasopharyngeal cancer, an esophageal cancer, malignant lymphoma, head and neck squamous cell carcinoma, a thyroid cancer, and osteosarcoma.

According to a seventh aspect of the disclosure, a method for preventing and/or treating tumors, cardiovascular and cerebrovascular diseases, retinopathy caused by diabetes and capillary fragility or allergies is further provided, the method includes: providing any one of the above nucleic acid nanocarrier drugs or pharmaceutical compositions, herein a drug ingredient is flavones; the effective dose of the above flavone-containing drug or pharmaceutical composition which is administered to patients with tumors, cardiovascular and cerebrovascular diseases, retinopathy caused by diabetes and capillary fragility or allergies.

Further, the tumor is the ovarian cancer.

The nucleic acid nanocarrier drug provided by the disclosure includes the nucleic acid nanoparticles and the drug ingredient, and the drug is ingredient located on the nucleic acid nanoparticles in the physical linkage mode and/or the covalent linkage mode. The drug ingredient is dihydroartemisinin, cisplatin, oxaliplatin, flavone or vincristine. The nucleic acid nanoparticles, through including the three sequences provided by the disclosure or the variant sequences thereof, not only may be self-assembled to form the nucleic acid structureal domain, but also may be served as a carrier to link the dihydroartemisinin, cisplatin, oxaliplatin, flavone or vincristine at the arbitrary 5'-end and/or 3'-end of the three strands, or enable these drug ingredients to be stably inserted between the strands of the nucleic acid structureal domain. The disclosure is capable of, through loading the small molecular drug, such as the cisplatin, the oxaliplatin, the flavone or the vincristine on the nucleic acid nanoparticles, using internal hydrophobicity, external hydrophilicity and a base stacking effect of the nucleic acid nanoparticles to have a "coating effect" on the dihydroartemisinin, and the dihydroartemisinin is not dissolved within a certain period of time through the coating effect or the covalent linkage, so the delivery stability is improved. In addition, while the nucleic acid structureal domain is modified by the target head, it may have the better targeting property, and may deliver the drug ingredient stably, the reliability is very high; at the same time, it may reduce a chance of the drug ingredient in contact with non-target cells or tissues, toxic side effects are reduced.

## Brief Description of the Drawings

Drawings of the description for constituting a part of the disclosure are used to provide further understanding of the disclosure, exemplary embodiments of the disclosure and descriptions thereof are used to explain the disclosure, and do not constitute improper limitation to the disclosure. In the drawings:

Fig. 1 shows an electrophoresis detection result of RNA nanoparticles formed by self-assembly in Embodiment 1 of the disclosure.

Fig. 2 shows an electrophoresis detection result of DNA nanoparticles formed by self-assembly in Embodiment 1 of the disclosure.

Fig. 3 shows a 2% agarose gel electrophoresis detection result of 7 groups of short-sequence RNA nanoparticles formed by self-assembly in Embodiment 2 of the disclosure.

Fig. 4 shows a 4% agarose gel electrophoresis detection result of 7 groups of short-sequence RNA nanoparticles formed by self-assembly in Embodiment 2 of the disclosure.

Fig. 5 shows a 2% agarose gel electrophoresis detection result of 7 groups of conventional sequence RNA nanoparticles formed by self-assembly in Embodiment 3 of the disclosure.

Fig. 6 shows a 4% agarose gel electrophoresis detection result of 7 groups of conventional sequence RNA nanoparticles formed by self-assembly in Embodiment 3 of the disclosure.

Fig. 7 shows a 2% agarose gel electrophoresis detection result of 7 groups of conventional sequence DNA nanoparticles formed by self-assembly in Embodiment 4 of the disclosure.

Fig. 8 shows a 4% agarose gel electrophoresis detection result of 7 groups of conventional sequence DNA nanoparticles formed by self-assembly in Embodiment 4 of the disclosure.

Fig. 9 shows a 2% agarose gel electrophoresis detection result of 8-th and 9-th groups of DNA nanoparticles formed by self-assembly in Embodiment 4 of the disclosure.

Fig. 10 shows a transmission electron microscope picture of conventional sequence DNA nanoparticles D-7 formed by self-assembly in Embodiment 4 of the disclosure.

Fig. 11 shows a standard curve of a dihydroartemisinin absorbance in a DNA nanoparticle loading rate detection process in Embodiment 5 of the disclosure.

Fig. 12 shows an electrophoresis detection result of DNAh-Bio-Cy5-DHA nanoparticles after being incubated in serum for different times in Embodiment 7 of the disclosure.

Fig. 13 is a comparison diagram of proliferation inhibition situations of DHA, DNAh-Bio-Cy5 (targeted fluorescent carrier), and Cisplatin to LN-229 cells in Embodiment 8 of the disclosure.

Fig. 14 is a comparison diagram of proliferation inhibition situations of DNAh-Bio-Cy5-DHA (targeted drug) and Cisplatin to the LN-229 cells.

Fig. 15 shows a standard curve of a vincristine absorbance in a DNA nanoparticle loading rate detection process in Embodiment 9 of the disclosure.

Fig. 16 shows an electrophoresis detection result of DNAh-Biotin-Cy5-vincristine nanoparticles after being incubated in serum for different times in Embodiment 11 of the disclosure.

Fig. 17a and Fig. 17b show detection results of different drugs for inhibiting proliferation of HepG2 cells in Embodiment 12, herein Fig. 17a is a comparison diagram of proliferation inhibition situations of small molecular drug vincristine, DNAh-Bio-Cy5 and small molecular drug cisplatin to the HepG2 cells, and Fig. 17b is a comparison diagram of proliferation inhibition situations of DNAh-Bio-Cy5-vincristine (targeted drug) and small molecular drug cisplatin to the HepG2 cells.

Fig. 18 shows a standard curve of a flavone absorbance in a DNA nanoparticle loading rate detection process in Embodiment 13 of the disclosure.

Fig. 19 shows an electrophoresis detection result of DNAh-Bio-EGFRapt-Cy5-flavone nanoparticles after being incubated in serum for different times in Embodiment 15 of the disclosure.

Fig. 20a to Fig. 20d show detection results of DNAh-Bio-EGFRapt-Cy5-flavone nanoparticles for inhibiting proliferation of Hela cells in Embodiment 16, herein, Fig. 20a is a proliferation inhibition situation of DNAh-Bio-EGFRapt-Cy5-flavone (targeted drug) to the Hela cells, Fig. 20b is a proliferation inhibition situation of the small molecular drug flavone to the Hela cells, Fig. 20c is a proliferation inhibition situation of DNAh-Bio-EGFRapt-Cy5 (targeted fluorescent carrier) to the Hela cells, and Fig. 20d is a proliferation inhibition situation of a blank control of DMSO to the Hela cells.

Fig. 21 shows a standard curve of an oxaliplatin absorbance in a DNA nanoparticle loading rate detection process in Embodiment 17 of the disclosure.

Fig. 22 shows an electrophoresis detection result of DNAh-Biotin-EGFRapt-Cy5-oxaliplatin nanoparticles after being incubated in serum for different times in Embodiment 19 of the disclosure.

Fig. 23a to Fig. 23d show detection results of DNAh-Bio-EGFRapt-Cy5-oxaliplatin nanoparticles for inhibiting proliferation of SGC-7901 cells in Embodiment 20, herein, Fig. 23a is a proliferation inhibition situation of DNAh-Bio-EGFRapt-Cy5-oxaliplatin (targeted drug) to the SGC-7901 cells, Fig. 23b is a proliferation inhibition situation of the small molecular drug oxaliplatin to the SGC-7901 cells, Fig. 23c is a proliferation inhibition situation of DNAh-Bio-EGFRapt-Cy5 (targeted fluorescent carrier) to the SGC-7901 cells, and Fig. 23d is a proliferation inhibition situation of a blank control of DMSO to the SGC-7901 cells.

Fig. 24 shows a standard curve of a cisplatin absorbance in a loading rate detection process in Embodiment 21 of the disclosure.

Fig. 25 shows microscopic observation results of binding and internalization of RNAh-Biotin-quasar670 nanoparticles and RNAh-Biotin-quasar670-DDP nanoparticles with SKOV3 cells in Embodiment 22 of the disclosure.

Fig. 26 shows an electrophoresis detection result of RNAh-Biotin-quasar670-DDP nanoparticles, after being incubated in serum for different times, under a Coomassie Blue program in Embodiment 24 of the disclosure.

Fig. 27 shows an electrophoresis detection result of RNAh-Biotin-quasar670-DDP nanoparticles, after being incubated in serum for different times, under a Stain Free Gel program in Embodiment 24 of the disclosure.

Fig. 28 shows detection results of small molecular drug cisplatin and RNAh-Biotin-quasar670-DDP nanoparticles for inhibiting a proliferation situation of SKOV3 cells in Embodiment 25 of the disclosure.

Fig. 29 shows a detection result of a fluorescence targeting carrier RNAh-Bio-FAM for inhibiting the proliferation situation of the SKOV3 cells in Embodiment 25 of the disclosure.

Fig. 30 shows non-denaturing PAGE gel electrophoresis detection results of 7 groups of extension fragment deformation + core short-sequence RNA self-assembly products in Embodiment 26 of the disclosure.

Fig. 31 shows a solubility curve of RNA nanoparticles R-15 in Embodiment 26 of the disclosure.

Fig. 32 shows a solubility curve of RNA nanoparticles R-16 in Embodiment 26 of the disclosure.

Fig. 33 shows a solubility curve of RNA nanoparticles R-17 in Embodiment 26 of the disclosure.

Fig. 34 shows a solubility curve of RNA nanoparticles R-18 in Embodiment 26 of the disclosure.

Fig. 35 shows a solubility curve of RNA nanoparticles R-19 in Embodiment 26 of the disclosure.

Fig. 36 shows a solubility curve of RNA nanoparticles R-20 in Embodiment 26 of the disclosure.

Fig. 37 shows a solubility curve of RNA nanoparticles R-21 in Embodiment 26 of the disclosure.

Fig. 38 shows non-denaturing PAGE gel electrophoresis detection results of 7 groups of extension fragment deformation + core short-sequence DNA self-assembly products in Embodiment 27 of the disclosure.

Fig. 39 shows a solubility curve of DNA nanoparticles D-8 in Embodiment 27 of the disclosure.

Fig. 40 shows a solubility curve of DNA nanoparticles D-9 in Embodiment 27 of the disclosure.

Fig. 41 shows a solubility curve of DNA nanoparticles D-10 in Embodiment 27 of the disclosure.

Fig. 42 shows a solubility curve of DNA nanoparticles D-11 in Embodiment 27 of the disclosure.

Fig. 43 shows a solubility curve of DNA nanoparticles D-12 in Embodiment 27 of the disclosure.

Fig. 44 shows a solubility curve of DNA nanoparticles D-13 in Embodiment 27 of the disclosure.

Fig. 45 shows a solubility curve of DNA nanoparticles D-14 in Embodiment 27 of the disclosure.

Fig. 46 shows an electrophoresis detection result of RNA nanoparticles R-15 after being incubated in serum for different times in Embodiment 28 of the disclosure.

Fig. 47 shows an electrophoresis detection result of RNA nanoparticles R-16 after being incubated in serum for different times in Embodiment 28 of the disclosure.

Fig. 48 shows an electrophoresis detection result of RNA nanoparticles R-17 after being incubated in serum for different times in Embodiment 28 of the disclosure.

Fig. 49 shows an electrophoresis detection result of RNA nanoparticles R-18 after being incubated in serum for different times in Embodiment 28 of the disclosure.

Fig. 50 shows an electrophoresis detection result of RNA nanoparticles R-19 after being incubated in serum for different times in Embodiment 28 of the disclosure.

Fig. 51 shows an electrophoresis detection result of RNA nanoparticles R-20 after being incubated in serum for different times in Embodiment 28 of the disclosure.

Fig. 52 shows an electrophoresis detection result of RNA nanoparticles R-21 after being incubated in serum for different times in Embodiment 28 of the disclosure.

Fig. 53 shows an electrophoresis detection result of DNA nanoparticles D-8 after being incubated in serum for different times in Embodiment 29 of the disclosure.

Fig. 54 shows an electrophoresis detection result of DNA nanoparticles D-9 after being incubated in serum for different times in Embodiment 29 of the disclosure.

Fig. 55 shows an electrophoresis detection result of DNA nanoparticles D-10 after being incubated in serum for different times in Embodiment 29 of the disclosure.

Fig. 56 shows an electrophoresis detection result of DNA nanoparticles D-11 after being incubated in serum for different times in Embodiment 29 of the disclosure.

Fig. 57 shows an electrophoresis detection result of DNA nanoparticles D-12 after being incubated in serum for different times in Embodiment 29 of the disclosure.

Fig. 58 shows an electrophoresis detection result of DNA nanoparticles D-13 after being incubated in serum for different times in Embodiment 29 of the disclosure.

Fig. 59 shows an electrophoresis detection result of DNA nanoparticles D-14 after being incubated in serum for different times in Embodiment 29 of the disclosure.

Fig. 60a, Fig. 60b, Fig. 60c, Fig. 60d, Fig. 60e, Fig. 60f, Fig. 60g, and Fig. 60h respectively show cell survival rate curves corresponding to DMSO and original drug doxorubicin, D-8 and D-8-doxorubicin, D-9 and D-9-doxorubicin, D-10 and D-10-doxorubicin, D-11 and D-11-doxorubicin, D-12 and D-12-doxorubicin, D-13 and D-13-doxorubicin, and D-14 and D-14-doxorubicin in Embodiment 32 of the disclosure.

Fig. 33 shows a standard curve of a daunorubicin absorbance used in a loading rate detection process of Embodiment 16.

## Detailed Description of the Embodiments

It is to be noted that embodiments in the disclosure and features in the embodiments may be combined with each other under a condition without conflicting. The disclosure is described in detail below with reference to the embodiments.

Term explanation:

Blank carrier: refers to a blank nucleic acid nanoparticle carrier without containing any bioactive substances, such as an RNAh or a DNAh.

Targeting carrier: refers to a nucleic acid nanoparticle carrier which contains a target head but does not contain a fluorescent substance, such as a Biotin-RNAh or a Biotin-DNAh.

Fluorescent carrier: refers to a nucleic acid nanoparticle carrier which contains the fluorescent substance but does not contain the target head, such as a Cy5-RNAh or a Cy5-DNAh.

Targeted fluorescent carrier: refers to a nucleic acid nanoparticle carrier containing the target head and the fluorescent substance, such as a Biotin-Cy5-RNAh or a Biotin-Cy5-DNAh.

Targeted drug: refers to a nucleic acid nanoparticle carrier containing the target head, the fluorescent substance and a chemical drug, such as a RNAh-Biotin-quasar670-DHA or a DNAh-Biotin-quasar670-DHA, herein the DHA represents dihydroartemisinin.

It is to be noted that there is no special format for a naming rule of each carrier or bioactive substance in the disclosure, and front and rear positions thereof in the expression do not mean that it is at a 5'end or a 3'end of the RNAh or the DNAh, but only mean that the bioactive substance is contained.

As mentioned in the background, although there are a variety of drug carriers for improving drug delivery efficiency in the prior art, it is still difficult to solve the problem of the limited clinical applications of the drugs. In order to improve this situation, the inventor of the present application researches on all existing materials which may be used as the drug carriers, and deeply investigates and analyzes the various carriers in aspects, such as cell/tissue targeting of the carrier, stability in a delivery process, activity and efficiency of entering target cells, drug release ability after reaching the target cells and toxicity to cells, it is discovered that an emerging nanostructure formed by self-assembly of DNA and/or RNA molecules, such as a self-assembled DNA dendrimersmay significantly prevent DNA from degradation by a nuclease, and have a very important application value in the fields of gene therapy and biomedicine.

Through analyzing the existing reported nanoparticles formed by the self-assembly of DNA and RNA, it is discovered that, compared with the relatively rigid DNA nanoparticles, the RNA nanoparticles have larger flexibility and stronger tension because there are a large number of stem-loop structures within or between molecules, so it has more advantages in an aspect as a candidate drug carrier. However, the RNA nanoparticles in a natural state are relatively poor in stability, and the existing improvements based on application aspects of the RNA nanocarriers are mostly focused on improving the stability and reliability thereof. Although research results at present provide the possibility of loading the drug to a certain extent, they are more focused on researching the possibility and effectiveness of loading nucleic acid drugs, especially a siRNA drug or a miRNA drug and the like. There are few reports at present on whether non-nucleic acid drugs are equally effective. In addition, the existing self-assembled nanoparticles, especially the self-assembled nanoparticles used as carriers, are self-assembled with a RNA strand, and a very few is self-assembled in a mode of a RNA strand and DNA strand combination, but no self-assembly is achieved with a DNA strand only.

In order to provide a new RNA nanoparticle carrier with good reliability and self-assembly, the existing RNA nanoparticles are compared and improved by the applicant, a series of new RNA nanoparticles are developed, and in view of improving applicability and reducing cost, the self-assembly is further tried to be performed with the DNA strand only, it is unexpectedly discovered that after being changed, these DNA single strands may not only self-assemble into the DNA nanoparticles, but also have the same excellent performance as the RNA nanoparticles. In addition, the self-assembly of the DNA nanoparticles also has advantages of low price and easy operation. After being verified by experiments, both the RNA nanoparticles and the DNA nanoparticles improved by the inventor may be loaded with various drugs, and may exist stably in serum; and further verified by the experiments, it may carry the drugs into the cells, and the carrier alone is non-toxic to the cells. However, the drug-carried carrier may have alleviating and therapeutic effects to corresponding diseases.

On the basis of the above research result, the applicant provides a technical scheme of the disclosure. The disclosure provides a nucleic acid nanocarrier drug, the drug includes nucleic acid nanoparticles and a drug ingredient, the drug ingredient is loaded on the nucleic acid nanoparticles, and the drug ingredient is dihydroartemisinin, cisplatin, oxaliplatin, flavone or vincristine; and the nucleic acid nanoparticle includes a nucleic acid structureal domain, the nucleic acid structureal domain includes a sequence a, a sequence b and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein the sequence a1 is SEQ ID NO:1: 5'-CCAGCGUUCC-3' or SEQ ID NO:2: 5'-CCAGCGTTCC-3'; the sequence b1 is SEQ ID NO:3: 5'-GGUUCGCCG-3' or SEQ ID NO:4: 5'-GGTTCGCCG-3'; and the sequence c1 is SEQ ID NO:5: 5'-CGGCCAUAGCGG-3' or SEQ ID NO:6: 5'-CGGCCATAGCGG-3'.

The nucleic acid nanocarrier drug provided by the disclosure includes the nucleic acid nanoparticles and the drug ingredient, the drug ingredient is located on the nucleic acid nanoparticles in the physical linkage mode and/or the covalent linkage mode, and the drug ingredient is the dihydroartemisinin, cisplatin, oxaliplatin, flavone or vincristine. The nucleic acid nanoparticles, through including the above three sequences or the variant sequences thereof, not only may be self-assembled to form the nucleic acid structureal domain, but also may be served as a carrier to link the dihydroartemisinin, cisplatin, oxaliplatin, flavone or vincristine at the arbitrary 5'-end and/or 3'-end of the three strands, or enable these drug ingredients to be stably inserted between the strands of the nucleic acid structureal domain. The disclosure is capable of, through loading the small molecular drug, such as the dihydroartemisinin, the cisplatin, the oxaliplatin, the flavone or the vincristine on the nucleic acid nanoparticles, using internal hydrophobicity, external hydrophilicity and a base stacking effect of the nucleic acid nanoparticles to have a "coating effect" on the dihydroartemisinin, and the dihydroartemisinin is not dissolved within a certain period of time through the coating effect or the covalent linkage, so the delivery stability is improved. In addition, while the nucleic acid structureal domain is modified by the target head, it may have the better targeting property, and may deliver the drug ingredient stably, the reliability is very high; at the same time, it may reduce a chance of the drug ingredient in contact with non-target cells or tissues, toxic side effects are reduced.

The above self-assembly refers to a technology that basic structural units spontaneously form an ordered structure. In a process of the self-assembly, the basic structural units spontaneously organize or aggregate into a stable structure with a certain regular geometric appearance under an interaction based on a non-covalent bond. The self-assembly process is not a simple superposition of weak interaction forces (herein the "weak interaction forces" refer to a hydrogen bond, a Van der Waals force, an electrostatic force, a hydrophobic action force and the like) between a large number of atoms, ions or molecules, but a tight and orderly whole formed by simultaneously spontaneously parallel connection and aggregation between a plurality of individuals, and is an overall complicated synergistic effect.

The production of the self-assembly requires two conditions: self-assembly power and guiding effect. The self-assembly power refers to the synergistic effect of the weak interaction forces between the molecules, and it provides energy for molecular self-assembly. The guiding effect of the self-assembly refers to complementary of the molecules in space, namely the production of the self-assembly needs to meet requirements of molecular rearrangement in direction and size of the space.

A DNA nanotechnology is a bottom-up molecular self-assembly mode, a stable structure is spontaneously formed using a molecular structure as a starting point on the basis of physical and chemical properties of the nucleic acid nanoparticles, and a strict nucleic acid base pairing principle is followed. Multiple DNA fragments are linked together in vitro in a correct sequence, a sub-assembly structure is established through the base complementary pairing principle, and finally a complicated multi-level structure is formed. Unlike the DNA, the structure of the RNA may exceed limitation of double-helix. The RNA may form a series of different base pairs, and at least two hydrogen bonds are formed between the base pairs. The different bases may be divided into two types, including a standard Waston-Crick base pair type and a non-Waston-Crick base pair type, so that the RNA forms a large number and multiple types of cyclic structure modules, and these modules are basic units for forming a folded RNA three-level structure. The RNA nanotechnology may make use of these natural existing 3D modules and predictable interactions thereof, herein, many RNA structures with biological activity may have an atomic-level resolution, such as a ribosome, various ribozymes and a natural RNA

aptamer existing in a riboswitch. A superior feature of the RNA nanotechnology is that a structure which is comparable in size and complexity to a natural RNA substance may be designed. A unique assembly property of the RNA in a natural RNA complex may also be utilized.

The above nucleic acid nanoparticles of the disclosure include three sequences shown in sequences SEQ ID NO:1, SEQ ID NO:3 and SEQ ID NO:5 or variant sequences thereof, or include three sequences shown in sequences SEQ ID NO:2, SEQ ID NO:4 and SEQ ID NO:6 or variant sequences thereof, and all of the sequences are subject to an ability to form the nucleic acid nanoparticles through the self-assembly, the specific variant sequence may be obtained on the basis of the sequences of the SEQ ID NO:1, the SEQ ID NO:2, the SEQ ID NO:3, the SEQ ID NO:4, the SEQ ID NO:5 and the SEQ ID NO:6 by rationally selecting a variant site and a variant type thereof, or obtained by extending a suitable fragment.

The nanoparticles formed by the self-assembly of the SEQ ID NO:1, the SEQ ID NO:3 and the SEQ ID NO:5 are RNA nanoparticles, and the nanoparticles formed by the self-assembly of the SEQ ID NO:2, the SEQ ID NO:4 and the SEQ ID NO:6 are DNA nanoparticles. In a preferred embodiment, the above nucleic acid nanoparticles are the RNA nanoparticles, and at least one of the sequence a, the sequence b and the sequence c includes a sequence obtained by insertion, deletion or substitution of at least one base, a specific position and a base type of the variant sequence in the RNA nanoparticles may be modified into the nanoparticles of improving a drug loading amount or improving stability according to the needs under a precondition of achieving the self-assembly.

In order to make the prepared nucleic acid nanoparticles have the relatively higher stability, when the base insertion, deletion or substitution is performed on the sequences shown in the above SEQ ID NO:1/2, SEQ ID NO:3/4 and SEQ ID NO:5/6, it may be performed on bases in some specific positions of the above sequences. On the one hand, the variant sequence is the same as the original sequence, and may be self-assembled into the nanoparticles, and on the other hand, the variation retains at least 50%, 55%, 60%, 65%, 70%, 75%, 80%, 85% 90% or 95% of identity with the original sequence, so that it has the same drug-loading features and similar stability as the nanoparticles formed by the self-assembly of the above sequences, the dihydroartemisinin may be loaded and delivered well.

In a preferred embodiment, the above base insertion, deletion or substitution is occurred: (1) at 1, 2, 4 or 5-th base starting from a 5 '-end of the sequence a shown in the SEQ ID NO:1 or 2; and/or (2) between 8-th and 10-th bases starting from the 5 '-end of the sequence a shown in the SEQ ID NO:1 or 2; and/or (3) between 1-th and 3-th bases starting from a 5 '-end of the sequence b shown in the SEQ ID NO:3 or 4; and/or (4) between 6-th and 9-th bases starting from the 5 '-end of the sequence b shown in the SEQ ID NO:3 or 4; and/or (5) between 1-th and 4-th bases starting from a 5 '-end of the sequence c shown in the SEQ ID NO:5 or 6; and/or (6) between 9-th and 12-th bases starting from the 5 '-end of the sequence c shown in the SEQ ID NO:5 or 6.

In the above preferred embodiment, the defined base position in which the variation happens is a non-classical Watson-Crick paired base position or a bulged unpaired base position in the nanostructure formed by the sequences shown in the SEQ ID NO:1, SEQ ID NO:2, SEQ ID NO:3, SEQ ID NO:4, SEQ ID NO:5 and SEQ ID NO:6, thus the formation of these bulges or loop structures is not affected, thereby the flexibility and tension of the nanostructure formed by the above sequences are maintained, and it is helpful to maintain the stability thereof as a carrier.

In order to further improve the stability of the above nucleic acid nanoparticles, and improve the stability of the drug after drug ingredient loading, in a preferred embodiment, the sequence a, the sequence b and the sequence c are self-assembled into a structure shown in Formula (1):

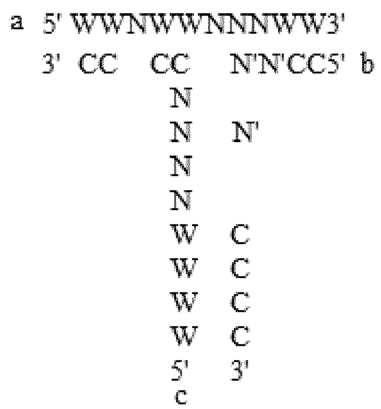


Formula (1)

herein, W-C represents Watson-Crick pairing, N and N' represent non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one

of U, T, A, C or G, the second N' is U or T, and the third N' is C; and in the sequence c, a sequence NNNN along a direction from the 5'-end to the 3'-end is CAUA or CATA.

In the above preferred embodiment, the sequences a, b and c are self-assembled to form the nucleic acid structureal domain shown in Formula (1), herein, except the non-Watson-Crick paired bases defined by N and N', the bases in the rest positions all form classical Watson-Crick pairing, and the bases of the above Watson-Crick pairing all choose G-C or C-G base pairs. Because an action force of hydrogen bonds between the G-C or C-G base pairs is greater than an action force of hydrogen bonds between the A-U/T or U/T-A base pairs, the nucleic acid nanostructure is more stable. Rather than the bulges or loop structures formed by the non-Watson-Crick paired bases, the greater tension is brought to the nucleic acid nanocarrier, so that it has the stronger adaptation to a micro-environmental change, thus the stability of the nucleic acid nanoparticles is higher.

In the nanoparticles in the above structure of Formula (1), the specific sequence formation of the sequence a, the sequence b and the sequence c is not specially limited, as long as the above structure may be formed. In view of the self-assembly of the nucleic acid sequence, in order to further improve the efficiency of the self-assembly of the above three sequences into the nanoparticles in the above structure of Formula (1), when the Watson-Crick paired bases are selected, the selection of the bases in the different positions is best to follow the following principles: (1) the sequence a, the sequence b and the sequence c, when as a single sequence, it is not complementary-paired by itself to form a secondary structure; and (2) the sequence a, the sequence b and the sequence c, one end of arbitrary two sequences is complementary-paired to form a double-strand, and the other end is not complementary-paired, to form a Y-type or T-type structure. The above principle of the base selection is to most efficiently enable two ends of any one strand to be respectively complementary-paired with two ends of the other two strands, thereby the self-assembly efficiency is improved. Certainly, in addition to the Y-type or T-type structure, it may also be other deformation modes such as a quadrangle rather than a trigeminal shape, as long as it meets the principle that one end of arbitrary two sequences is complementary-paired to form the double-strand, and the other end is not complementary-paired.

In the nanoparticles in the above structure of Formula (1), in the non-Watson-Crick paired bases, the fourth N starting from the 5'-end in the sequence a and the first N' starting from the 5'-end paired in the sequence b may be non-Watson-Crick paired U-U, or may be T, A, C or G improved for following the Watson-Crick pairing principle. A binding force between the strands is relatively improved by the Watson-Crick pairing, the stability is improved, and the nanoparticles are endowed with the larger softness and flexibility by the non-Watson-Crick pairing, in the face of the micro-environmental change, it is also helpful to improve the stability of the nanoparticles.

In a preferred embodiment, the sequence a, the sequence b and the sequence c are any one of the following groups: (1) sequence a (SEQ ID NO:7): 5'-GGAGCGUUGG-3', sequence b (SEQ ID NO:8): 5'-CCUUCGCCG-3', sequence c (SEQ ID NO:9): 5'-CGGCCAUAGCCC-3'; (2) sequence a (SEQ ID NO:10): 5'-GCAGCGUUCG-3', sequence b (SEQ ID NO:11): 5'-CGUUCGCCG-3', sequence c (SEQ ID NO:12): 5'-CGGCCAUAGCGC-3'; (3) sequence a (SEQ ID NO:13): 5'-CGAGCGUUGC-3', sequence b (SEQ ID NO:14): 5'-GCUUCGCCG-3', sequence c (SEQ ID NO:15): 5'-CGGCCAUAGCCG-3'; (4) sequence a (SEQ ID NO:16): 5'-GGAGCGUUGG-3', sequence b (SEQ ID NO:17): 5'-CCUUCGGGG-3', sequence c (SEQ ID NO:18): 5'-CCCCCAUAGCCC-3'; (5) sequence a (SEQ ID NO:19): 5'-GCAGCGUUCG-3', sequence b (SEQ ID NO:20): 5'-CGUUCGGCG-3', sequence c (SEQ ID NO:21): 5'-CGCCCAUAGCGC-3'; (6) sequence a (SEQ ID NO:22): 5'-GCAGCGUUCG-3', sequence b (SEQ ID NO:23): 5'-CGUUCGGCC-3', sequence c (SEQ ID NO:24): 5'-GGCCCAUAGCGC-3'; (7) sequence a (SEQ ID NO:25): 5'-CGAGCGUUGC-3', sequence b (SEQ ID NO:26): 5'-GCUUCGGCG-3', sequence c (SEQ ID NO:27): 5'-CGCCCAUAGCCG-3'; (8) sequence a (SEQ ID NO:28): 5'-GGAGCGTTGG-3', sequence b (SEQ ID NO:29): 5'-CCTTCGCCG-3', sequence c (SEQ ID NO:30): 5'-CGGCCATAGCCC-3'; (9) sequence a (SEQ ID NO:31): 5'-GCAGCGTTCG-3', sequence b (SEQ ID NO:32): 5'-CGTTCGCCG-3', sequence c (SEQ ID NO:33): 5'-CGGCCATAGCGC-3'; (10) sequence a (SEQ ID NO:34): 5'-CGAGCGTTGC-3', sequence b (SEQ ID NO:35): 5'-GCTTCGCCG-3', sequence c (SEQ ID NO:36): 5'-CGGCCATAGCCG-3'; (11) sequence a (SEQ ID NO:37): 5'-GGAGCGTTGG-3', sequence b (SEQ ID NO:38): 5'-CCTTCGGGG-3', sequence c (SEQ ID NO:39): 5'-CCCCCATAGCCC-3'; (12) sequence a (SEQ ID NO:40): 5'-GCAGCGTTCG-3', sequence b (SEQ ID NO:41): 5'-CGTTCGGCG-3', sequence c (SEQ ID NO:42): 5'-CGCCCATAGCGC-3'; (13) sequence a (SEQ ID NO:43): 5'-GCAGCGTTCG-3', sequence b (SEQ ID NO:44): 5'-CGTTCGGCC-3', sequence c (SEQ ID NO:45): 5'-GGCCCATAGCGC-3'; (14) sequence a (SEQ ID NO:46): 5'-CGAGCGTTGC-3', sequence b (SEQ ID NO:47): 5'-GCTTCGGCG-3', sequence c (SEQ ID NO:48): 5'-CGCCCATAGCCG-3'; and (15) sequence a (SEQ ID NO: 175): 5'-CGAGCGTTCC -3', sequence b (SEQ ID NO: 176): 5'-GGTTCGCCG -3', sequence c (SEQ ID NO: 177): 5'-CGGCCATAGCCG-3'.

The nucleic acid nanoparticles formed by the self-assembly of fifteen groups of the above sequences not only have the higher stability, but also have the higher self-assembly efficiency.

The nucleic acid nanoparticles mentioned above may not only be self-assembled for forming, but also have the ability to carry or load the drugs of the dihydroartemisinin, the cisplatin, the oxaliplatin, the flavone and the vincristine. According to the different positions of G-C or C-G base pairs in the above nucleic acid nanoparticles, the amounts of the loaded drug ingredients are also different.

In order to enable the above nucleic acid structureal domain to load more drug ingredients and other bioactive

substances (the introduction of the bioactive substance is described below), in a preferred embodiment, in the above nucleic acid structural domain, a first extension fragment is further included, the first extension fragment is an extension fragment of Watson-Crick pairing, and the first extension fragment is positioned at the 5'-end and/or the 3'-end of any one sequence of the sequence a, the sequence b or the sequence c. a certain matching relation is required between the carrier and the loaded substance, when a molecular weight of the carrier is too small and a molecular weight of the loaded substance is too large, in view of mechanics, carrying or delivering capacity of the carrier to the loaded substance is relatively reduced. Therefore, based on the above nucleic acid nanostructure, through adding the first extension fragment at the 5'-end and/or 3'-end of any one sequence of the sequence a, the sequence b and the sequence c, the carrier matched with the size of the loaded substance may be acquired.

A specific length of the above first extension fragment may be determined according to the size of the substance to be loaded. In a preferred embodiment, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; (9): b-strand 3'-end: 5'-CCA-3', c-strand 5'-end: 5'-TGG-3'; (10): a-strand 5'-end: 5'-GCGGCGAGCGGCGA-3' (SEQ ID NO:162), c-strand 3'-end: 5'-UCGCCGCUCGCCGC-3' (SEQ ID NO:163); (11): a-strand 3'-end: 5'-GGCCGGAGGCCGG-3' (SEQ ID NO:164), b-strand 5'-end: 5'-CCGGCCUCCGGCC-3' (SEQ ID NO:165); (12): b-strand 3'-end: 5'-CCAGCCGCC-3' (SEQ ID NO:166), c-strand 5'-end: 5'-GGCGGCAGG-3' (SEQ ID NO:167); (13): a-strand 5'-end: 5'-GCGGCGAGCGGCGA-3' (SEQ ID NO:168), c-strand 3'-end: 5'-TCGCCGCTCGCCGC-3' (SEQ ID NO:169); and (14): a-strand 3'-end: 5'-GGCCGGAGGCCGG-3' (SEQ ID NO:170), b-strand 5'-end: 5'-CCGGCCTCCGGCC-3' (SEQ ID NO:171).

The above first extension fragment not only increases a length of any one or more of the three sequences for forming the nucleic acid nanostructure, but also the first extension fragment formed by the GC base further improves the stability of the formed nanoparticles. In addition, the first extension fragment formed by the above sequences also enables the sequence a, the sequence b and the sequence c to maintain the higher self-assembly activity and efficiency.

In view of the size of the formed nucleic acid nanoparticles and the stability thereof while delivered as a drug delivery carrier in vivo, it is necessary not to be filtered out by kidneys before reaching the target cells while the drugs may be delivered. In a preferred embodiment, the nucleic acid structural domain further includes a second extension fragment, the second extension fragment is positioned at the 5'-end and/or the 3'-end of any one sequence of the sequence a, the sequence b, or the sequence c, and the second extension fragment is an extension fragment of Watson-Crick pairing; more preferably, the second extension fragment is an extension sequence of a CG base pair; and further preferably, the second extension fragment is an extension sequence of 1-10 CG base pairs. The second extension fragment is the extension fragment further added on the basis of the first extension fragment.

In a preferred embodiment, the above nucleic acid structureal domain further includes at least one group of the following second extension fragments: a first group: a-strand 5'-end: 5'-CGCGCG-3', c-strand 3'-end: 5'-CGCGCG-3'; a second group: a-strand 3'-end: 5'-CGCCGC-3', b-strand 5'-end: 5'-GCGGCG-3'; and a third group: b-strand 3'-end: 5'-GGCGGC-3', c-strand 5'-end: 5'-GCCGCC-3'. Such a second extension fragment enables the nanoparticles not to have immunogenicity, and there is no such a situation that the secondary structure in each strand is folded and linked by itself..

It is to be noted that the above first extension fragment and/or second extension fragment may also be separated by an unpaired base pair.

In order to enable the above nucleic acid nanoparticles to load the bioactive substance (the introduction of the bioactive substance is described below) with the larger molecular weight, increase the drug loading amount and maintain the necessary stability, in a preferred embodiment, the second extension fragment is an extension sequence containing both CG base pair and AT/AU base pair, and preferably the second extension fragment is an extension sequence of 2-50 base pairs. Here, "/" in the "AT/AU base" is a relation of or, specifically, the second extension fragment is an extension sequence containing both CG base pair and AT base pair, or the second extension fragment is an extension sequence containing both CG base pair and AU base pair.

More specifically, the sequences a, b and c after the above second extension fragment is added may be the following sequences respectively:

The sequence a is (SEQ ID NO:49):

5'-CGCGCGAAAAAACGCGCGAAAAAACGCGCGCCCACCAGCGMMCCGGGCGCGCGAAAAAACGCGCGAAAAAACGCGCG-3'.

The sequence b is (SEQ ID NO:50):

5'-CGCGCGMMMMMMCGCGCGMMMMMMCGCGCGCCCGGMMCGCCGCCAGCC GCCMMMMMMGCCGCCMMMMMMGCCGCC-3'.

The sequence c is (SEQ ID NO:51):

5'-GGCGGCAAAAAAGGCGGCAAAAAAGGCGGCAGGCGGCAMAGCGGMGGGCG CGCGMMMMMMCGCGCGMMMMMMCGCGCG-3'.

M in the above sequence a, sequence b and sequence c is U or T, while the M is the T, a synthetic cost of the above sequences is greatly reduced.

In practical applications, specific setting positions of the extension sequences of the above CG base pair and AT/AU base pair may be rationally adjusted according to actual needs. In a more preferred embodiment, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Specifically, positions of the extension fragment CGCGCG and the extension fragment CGCCGC in the sequence a as shown in the above SEQ ID NO:49 are interchanged with a position of the extension fragment AAAAAA, positions of the extension fragment GCGGCG and the extension fragment GGCGGC in the sequence b shown in the above SEQ ID NO:50 are interchanged with a position of the extension fragment TTTTTT, the extension fragment GCCGCC in the sequence c shown in the above SEQ ID NO:51 is interchanged with the extension fragment AAAAAA, and the extension fragment CGCCGC is interchanged with the extension fragment TTTTTT at the same time. The nucleic acid nanoparticles formed by the self-assembly of the above sequences are suitable for loading chemical drugs in indole molecular structures (indole drug molecules are preferably linked with base A).

In the past few years, three major challenges of the RNA as a widely used construction material includes: 1) sensitivity to RNA enzymatic degradation; 2) sensitivity to dissociation after systemic injection; and 3) toxicity and adverse immune response. At present, the three challenges are overcome to a large extent already: 1) 2'-fluoro(2'-F) or 2'-O-methyl(2'-OMe) modification of a ribose-OH group may make the RNA chemically stable in serum; 2) some natural existing linking sequence motifs are thermodynamically stable, and may keep the overall RNA nanoparticles to be integral at an ultra-low concentration; and 3) the immunogenicity of the RNA nanoparticles is sequence and shape-dependent, and may be adjusted, so that the RNA nanoparticles stimulate generation of inflammatory cytokines, or the RNA nanoparticles have non-immunogenicity and non-toxicity when administered by repeated intravenous injection of 30 mg/kg.

Therefore, in order to further reduce the sensitivity of the above nucleic acid nanoparticles to the RNA enzymatic degradation, and improve the stability in the delivery process, in a preferred embodiment, a base, a ribose and a phosphate in the sequence a, the sequence b and the sequence c have at least one modifiable site, and any one of the modifiable sites is modified by any one of the following modification adaptors: -F, a methyl, an amino, a disulfide, a carbonyl, a carboxyl, a sulfhydryl and a formyl; and preferably, the base C or U in the sequence a, the sequence b and the sequence c has 2'-F modification. While the modification adaptor is the sulfhydryl, it belongs to a thio modification, modification degree is weakr, and a cost is low.

The above drug ingredients of the dihydroartemisinin, the cisplatin, the oxaliplatin, the flavone and the vincristine may be loaded in the physical linkage mode and/or the covalent linkage mode. When the drug ingredient is linked with the nucleic acid structureal domain by the physical insertion mode and the covalent linkage mode simultaneously, the physical insertion is usually inserted between the GC base pairs, the number of the preferred insertion sites is based on the different numbers of the GC base pairs on the nucleic acid structural domain, and the insertion is performed according to the ratio of 1 to 100:1. When the covalent linkage mode is used for linkage, the above drug usually chemically reacts with a G-exocyclic amino to form the covalent linkage. More preferably, a molar ratio between the drug ingredient and the nucleic acid nanoparticles is 2 to 300:1, preferably 2 to 290:1, more preferably 2 to 29:1, further preferably 10 to 50:1, and most preferably 15 to 25:1.

In the nucleic acid nanocarrier drug provided in the disclosure, the nucleic acid nanoparticles are served as a drug ingredient delivery carrier. In addition, according to different drug purposes, in a preferred embodiment, the above nucleic acid nanoparticles also include the bioactive substance, and the bioactive substance is linked with the nucleic acid structureal domain. The bioactive substance is one of more of a target head, a fluorescein, an interfering nucleic acid siRNA, a miRNA, a ribozyme, a riboswitch, an aptamer, a RNA antibody, a protein, a peptide, a flavonoid, a glucose, a natural salicylic acid, a monoclonal antibody, a vitamin, phenols, a lecithin and a small molecular drug except the dihydroartemisinin.

In order to improve loading efficiency and delivering efficiency of the nucleic acid nanoparticles to the loaded bioactive substance, a relative molecular weight of the nucleic acid structural domain and a total relative molecular weight of the drug and the bioactive substance should preferably have a certain matching relation. In a preferred embodiment, the relative molecular weight of the nucleic acid structureal domain is marked as N1, and the total relative molecular weight of the drug and the bioactive substance is marked as N2, N1/ N2≥1:1.

According to the different types of the bioactive substances loaded specifically, performance optimization effects thereof on the nucleic acid nanoparticles of the disclosure are not the same. For example, when the bioactive substance is a biotin or a folic acid, the function thereof is to make the nucleic acid nanoparticles have a targeting property, for example, specifically targeted to cancer cells. When the bioactive substance is the fluorescein, the function thereof is to make the nucleic acid nanoparticles have a luminous tracking effect, for example, one or more of AM, CY3, CY5 and Quasar670. When the bioactive substance is some siRNA, miRNA, protein, peptide, RNA antibody and small molecular drug except the dihydroartemisinin, the cisplatin, the oxaliplatin, the flavone and the vincristine, according to the different biological functions, the nucleic acid nanocarrier drug may be made into a new product with a specific therapeutic effect, such as a drug with more excellent performance. In addition, according to the different types of the bioactive substances loaded specifically, the DNA nanoparticles and the RNA nanoparticles are specifically preferably used, and may be rationally selected according to the actual needs. For example, while the bioactive substance is the drug, the DNA nanoparticles and the RNA nanoparticles are preferably used for loading, and there is no special requirement for a length of the single strand of the nanoparticles formed by assembly.

In a preferred embodiment, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid structural domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is the folic acid or the biotin, the fluorescein is any one or more of FAM, CY5 and CY3, and the above anti-miRNA is anti-miR-21.

The above target head may be linked on any one sequence of the sequences a, b and c in a mode of linker covalent linkage, the available linker is selected from a disulfide bond, a p-phenylazide, bromopropyne or a PEG. Here, the "on any one sequence" refers to on a base in any one position of any one sequence of the sequences a, b and c, and it is more convenient and widely used to be linked at the 5'-end or 3'-end. Folic acid modification may be in a physical insertion mode linkage or a physical insertion + covalent linkage.

The above fluorescein may be a commonly used fluorescein, and preferably any one or more of FAM, CY5 and CY3.

The above miRNA may be a miRNA with a cancer suppression effect, or may be an anti-miRNA which may suppress a corresponding disease, and it may be rationally selected in practical applications according to medical needs. The above anti-miRNA may be synthesized at any one or more positions of the 3'-end of the above sequence a, the 5'-end and 3'-end of the sequence c. When the anti-miRNA is synthesized in the above three positions, the anti-miRNA has a relatively stronger suppression effect on the corresponding miRNA.

It is anti-miR-21 preferably, the miR-21 participates in initiation and progression of multiple types of cancers, and is a main oncogene for invasion and metastasis. The anti-miR-21 may effectively regulate a wide range of target genes at the same time, and is beneficial to solve a problem of cancer heterogeneity. Therefore, in the above preferred nucleic acid nanoparticles, the target head, such as the folic acid or the biotin, may be specifically targeted to the cancer cells, and after being linked and internalized with the cancer cells, the anti-miR-21 is complemented with a miR-21 base in very high affinity and specificity, thereby the expression of the oncogenic miR-21 is effectively reduced. Therefore, according to the actual needs, the above anti-miR-21 may be synthesized at any one or more positions of the 3'-end of the above sequence a, the 5'-end and the 3'-end of the sequence c. When the anti-miR-21 is synthesized in the above three positions, the anti-miR-21 has a relatively stronger suppression effect on the miR-21.

When the above bioactive substance capable of loading is other small molecular drugs except the dihydroartemisinin, the cisplatin, the oxaliplatin, the flavone and the vincristine, according to types of diseases which may be treated by the different drugs, the drugs include but not limited to drugs for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

When the above bioactive substance capable of loading is other small molecular drugs except the dihydroartemisinin, the cisplatin, the oxaliplatin, the flavone and the vincristine, according to a difference of molecular structures of the drugs or a difference of characteristic groups which it has, the drug includes but not limited to a drug containing any one or more of the following groups: an amino group, a hydroxyl group, a carboxyl group, a mercapto group, a benzene ring group and an acetamido group.

In a preferred embodiment, the above protein is one or more of antibodies or aptamers of superoxide dismutase (SOD), survivin, human telomerase reverse transcriptase (hTERT), epidermal growth factor receptor (EGFR) and prostate-specific membrane antigen (PSMA); the above vitamin is L-VC and/or esterified VC; and the above phenols are tea polyphenols and/or grape polyphenols.

In a preferred embodiment, a particle size of the nucleic acid nanoparticles is 1-100 nm, preferably 5-50 nm; more preferably 10-30 nm; and further preferably 10-15 nm. The size is appropriate within this range, it may not only enter a cell membrane through cell phagocytosis mediated by a cell surface receptor, but also avoid non-specific cell penetration so as to be filtered and removed by the kidneys. Therefore, the favorable particle size is helpful to improve pharmacokinetics, pharmacodynamics, biological distribution and toxicological distribution.

According to a second aspect of the disclosure, a preparation method for the above nucleic acid nanocarrier drug is further provided, and the preparation method includes the following operations: any one of the above nucleic acid nanoparticles is provided; a drug ingredient is loaded on the nucleic acid nanoparticles in the physical linkage mode and/or the covalent linkage mode, to obtain a drug containing dihydroartemisinin, herein a drug ingredient is dihydroartemisinin, cisplatin, oxaliplatin, flavone or vincristine.

When the physical linkage mode is used, the drug ingredient may be usually formed and inserted between GC base pairs in a physical insertion form. When the covalent linkage mode is used for linkage, the drug ingredient usually chemically reacts with a G-exocyclic amino to form the covalent linkage. The nucleic acid nanocarrier drug prepared by the above method may have the better targeting property after it is modified by the target head, the dihydroartemisinin may be stably delivered, and the reliability is very high.

In a preferred embodiment, the step of loading the drug ingredient in the physical linkage mode includes: enabling the drug ingredient, the nucleic acid nanoparticles and a first solvent to be mixed and stirring, to obtain a premixed system; and precipitating the premixed system, to obtain the nucleic acid nanocarrier drug. Specific dosages of the drug ingredient and the nucleic acid nanoparticles may be adjusted according to a change of the loading amount, this may be understood by those skilled in the art, and it is not repeatedly described here.

In order to improve the efficiency and the stability of the physical linkage, an amount of the drug ingredient added per liter of the first solvent is preferably 0.1 to 1 g. Preferably, the first solvent is selected from one or more of DCM, DCC, DMAP, Py, DMSO, PBS and glacial acetic acid. Preferably, the step of precipitating the premixed system, to obtain the nucleic acid nanocarrier drug includes: precipitating the premixed system, to obtain a precipitation; and washing the precipitation to remove impurities, to obtain the nucleic acid nanocarrier drug. More preferably, the premixed system is mixed with absolute ethyl alcohol, and precipitated at a temperature condition lower than 10 DEG C, to obtain the precipitation, further preferably, the precipitation is obtained by precipitating at a temperature condition of 0-5 DEG C. More preferably, the precipitation is washed to remove the impurities with 6-12 times of the absolute ethyl alcohol in volume, as to obtain the nucleic acid nanocarrier drug.

In a preferred embodiment, the step of loading the drug ingredient in the covalent linkage mode includes: drug ingredient solution of dihydroartemisinin, cisplatin, oxaliplatin, flavone or vincristine is prepared; the drug ingredient solution reacts with the G-exocyclic amino of the nucleic acid nanoparticles under a mediating effect of the formaldehyde, to obtain a reaction system; and the reaction system is purified, to obtain the nucleic acid nanocarrier drug.

Through a formaldehyde-mediated form, the following reaction may happen to the dihydroartemisinin:

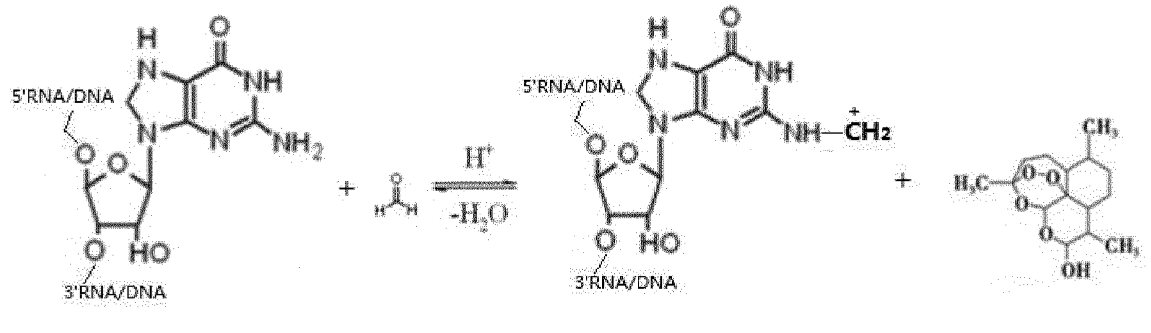

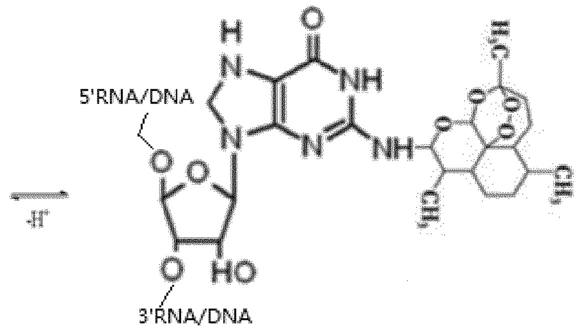


Through the formaldehyde-mediated form, the following reaction may happen to the vincristine:

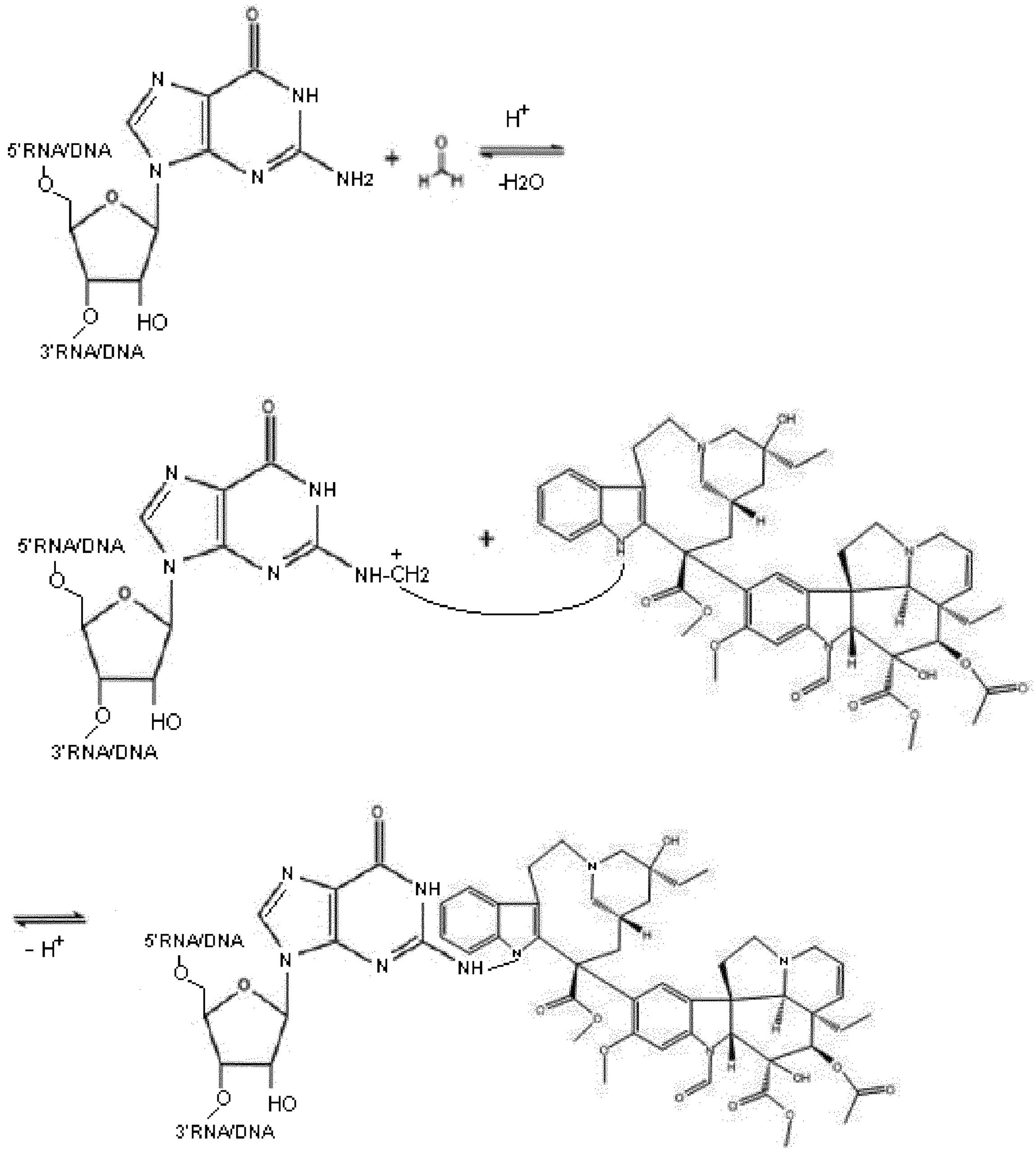


Through the formaldehyde-mediated form, the following reaction may happen to the flavone:

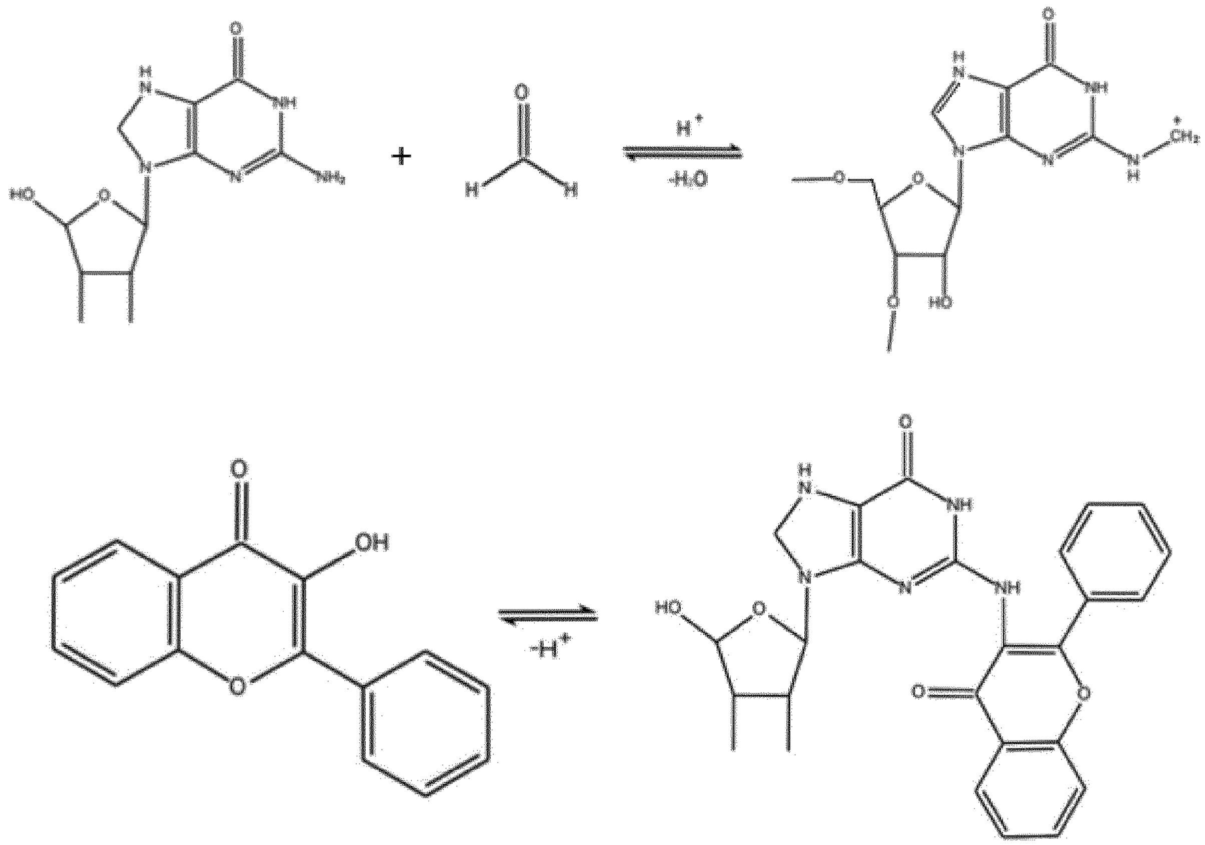


Through the formaldehyde-mediated form, the following reaction may happen to the oxaliplatin:

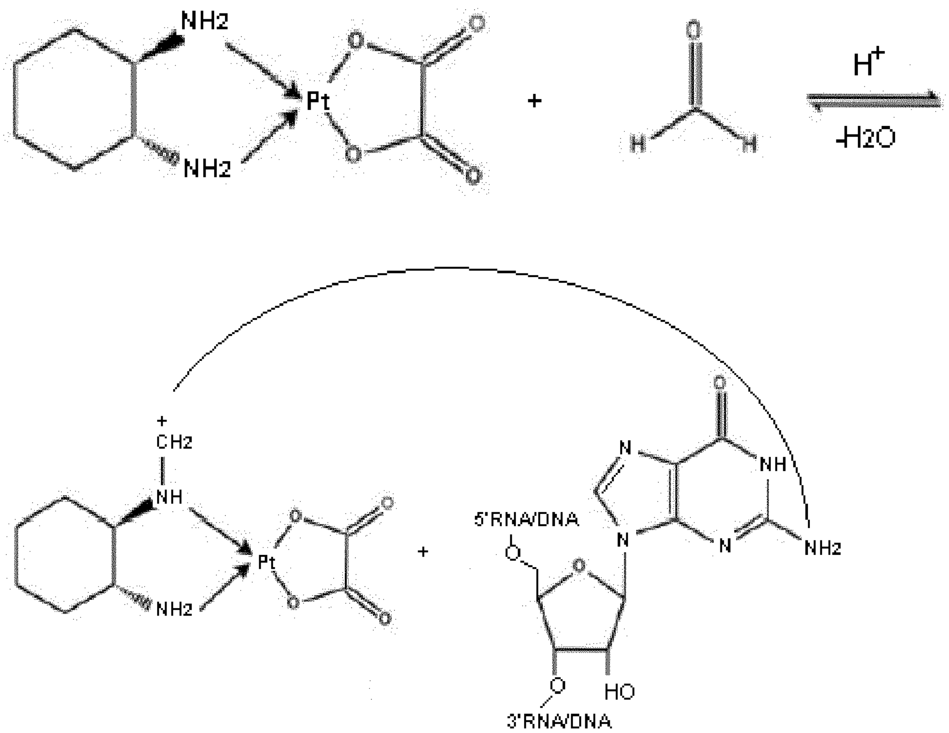

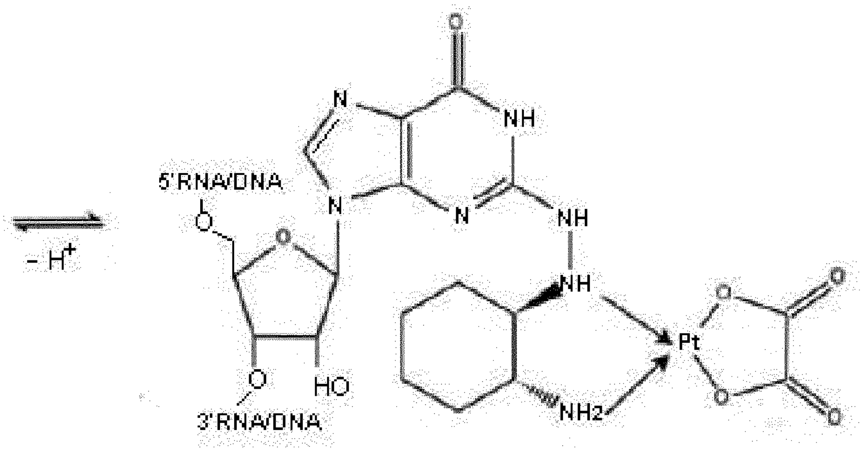


Through the formaldehyde-mediated form, the following reaction may happen to the cisplatin:

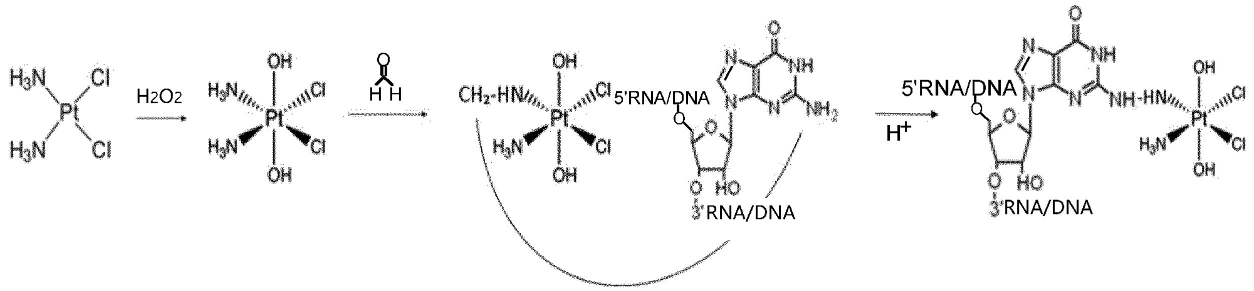


Preferably, the above reaction step includes: the drug ingredient solution is mixed with paraformaldehyde solution and the nucleic acid nanoparticles, and it is reacted in a dark condition, to obtain the reaction system. The paraformaldehyde solution may release small formaldehyde molecules to participate in the above chemical reaction. In order to improve the reaction efficiency, the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

In the above preparation method, the nucleic acid nanoparticles may be prepared in a mode of self-assembly, for example: (1) enabling the RNA or DNA single strands a, b and c to be simultaneously mixed and dissolved in DEPC water or TMS buffer solution; (2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min; (3) enabling a product to be loaded on 8% (m/v) of non-denaturing PAGE gel and placed in TBM buffer solution under a condition of 4 DEG C, purifying a complex by 100 v of electrophoresis; and (4) cutting a target band and eluting in RNA/DNA elution buffer solution at 37 DEG C, after that, precipitating overnight in ethanol, volatilizing under reduced pressure and low temperature, to obtain a self-assembly product, namely the nucleic acid structural domain, and then acquiring the nucleic acid nanoparticles.

According to actual application needs, in order to make the above nucleic acid nanocarrier drug have other functions, in a preferred embodiment, after the nucleic acid structureal domain is obtained, the preparation method further includes: enabling the bioactive substance as mentioned above to be loaded on the nucleic acid structural domain in the modes of physical linkage and/or covalent linkage, to obtain the nucleic acid nanoparticles. The loading mode of the bioactive substance may also be the physical linkage and/or the covalent linkage. The covalent linkage mode includes but not limited to the solvent covalent linkage, the linker covalent linkage or the click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; and preferably, the click-linkage is that alkynyl or azide modification is simultaneously performed on a bioactive substance precursor and the nucleic acid structural domain, and then they are linked through the click-linkage.

It is to be noted that the above classification does not mean that there is only one linkage mode to link a certain bioactive substance with the nucleic acid nanocarrier. However, some bioactive substances may be linked with the nucleic acid nanocarrier in the physical insertion mode, or may be linked with the nucleic acid nanocarrier in the physical

insertion and covalent linkage modes, or may be linked by using the click-linkage mode at the same time. However, for a certain specific bioactive substance, there may be only one linkage mode, or there may be multiple linkage modes, but it may be that the efficiency of certain linkage has an advantageous practical value.

In the above linkage modes, while the different drugs are linked with the nucleic acid structural domain in the physical insertion mode, insertion linkage sites and numbers are also different slightly. For example, while anthracycline and acridine drugs are inserted, they are usually inserted between the GC base pairs, the preferred number of the insertion sites is based on the different numbers of the GC base pairs in the nucleic acid structural domain, and the insertion is performed in a ratio of 1 to 100:1. While a naphthylamide drug is inserted, it is usually inserted between the AA base pairs, the preferred number of the insertion sites is based on the different numbers of the AA base pairs in the nucleic acid structural domain, and pyridocarbazoles are inserted according to the different numbers of the AA base pairs in a ratio of 1 to 200:1.

Specifically, according to the different types of the bioactive substances, the lengths of the sequences a, b and c for forming the nucleic acid structural domain in the nucleic acid nanoparticles and the number of GC complementary base pairs thereof, the physical insertion may be performed by rationally selecting a molar ratio of the bioactive substance and the nucleic acid structural domain.

In a preferred embodiment, while the bioactive substance is linked with the nucleic acid structural domain in the physical insertion mode and the covalent linkage mode, a molar ratio of the bioactive substance linked in the physical insertion mode and the drug linked in the covalent linkage mode is 1 to 200:1. The linkage mode is suitable for the anthracycline and acridine drugs. The proportion of the drugs linked in the above different linkage modes is not limited to the above range, as long as it may meet a requirement of high-efficient loading, there is a non-toxic effect on the cells, and the effective release of the drug is achieved after reaching a target.

While a bioactive substance precursor and the nucleic acid structural domain are simultaneously modified by an alkynyl or an azide, and linked in the click-linkage mode, the different click-linkages are selected according to changes of the different structures of the drugs. In addition, along with the different structures of the bioactive substances, the linkage positions may also be changed correspondingly, this may be understood by those skilled in the art.

In a preferred embodiment, while the bioactive substance is linked with the nucleic acid structural domain in the click-linkage mode, a site, for performing the alkynyl or azide modification, of the bioactive substance precursor is selected from a hydroxyl, a carboxyl, a mercapto or an amino, and a site, for performing the alkynyl or azide modification, of the nucleic acid structural domain is selected from the amino, an imino or the hydroxyl.

According to a third aspect of the disclosure, a pharmaceutical composition is further provided, the pharmaceutical composition includes any one of the above nucleic acid nanocarrier drug. Specifically, a suitable combination drug or ingredient may be selected according to actual needs to form the pharmaceutical composition which has the combined efficacy or may improve the performance of the drug in a certain aspect (such as stability).

According to a fourth aspect of the disclosure, an application of any one of the above nucleic acid nanocarrier drugs in preparing a drug for treating a tumor is further provided, and a drug ingredient thereof is dihydroartemisinin, vincristine, oxaliplatin or cisplatin. Further, the drug ingredient is the dihydroartemisinin, and the tumor is any one or more of a liver cancer, an ovarian cancer, a lung cancer, a pancreatic cancer, osteosarcoma and cerebral medulloblastoma; or the drug ingredient is the vincristine, and the tumor is acute lymphocytic leukemia, chronic lymphocytic leukemia, Hodgkin's lymphoma, lymphosarcoma, Ewing's sarcoma, neuroblastoma, reticulocyte sarcoma, small cell carcinoma, a digestive tract cancer, a liver cancer, melanoma and multiple myeloma and a breast cancer; or the drug ingredient is the oxaliplatin, and the tumor is any one or more of a colorectal cancer, an ovarian cancer, a gastric cancer, non-Hodgkin's lymphoma, non-small cell carcinoma, and head and neck tumors; or the drug ingredient is the cisplatin, and the tumor is any one or more of an ovarian cancer, a prostate cancer, a testicular cancer, a lung cancer, a nasopharyngeal cancer, an esophageal cancer, malignant lymphoma, head and neck squamous cell carcinoma, a thyroid cancer, and osteosarcoma. For specific applications, a new drug may be obtained by improving the drug itself on the basis of the drug of the disclosure, or it is prepared into a preparation in a suitable dosage form by using the drug of the disclosure as a main active ingredient.

According to a fifth aspect of the disclosure, an application of the above nucleic acid nanocarrier drug in preparing a drug for treating tumors, cardiovascular and cerebrovascular diseases, retinopathy caused by diabetes and capillary fragility or allergies is further provided, herein a drug ingredient is flavone. Further, the tumor is the ovarian cancer.

According to a sixth aspect of the disclosure, a method for preventing and/or treating a tumor is further provided, the method includes: providing any one of the above nucleic acid nanocarrier drugs or pharmaceutical compositions, herein a drug ingredient is dihydroartemisinin, vincristine, oxaliplatin or cisplatin; and administering an effective dose of the above nucleic acid nanocarrier drug or pharmaceutical composition to a tumor patient. Further, the drug ingredient is the dihydroartemisinin, and the tumor is any one or more of a liver cancer, an ovarian cancer, a lung cancer, a pancreatic cancer, osteosarcoma and cerebral medulloblastoma; or the drug ingredient is the vincristine, and the tumor is acute lymphocytic leukemia, chronic lymphocytic leukemia, Hodgkin's lymphoma, lymphosarcoma, Ewing's sarcoma, neuroblastoma, reticulocyte sarcoma, small cell carcinoma, a digestive tract cancer, a liver cancer, melanoma and multiple

myeloma and a breast cancer; or the drug ingredient is the oxaliplatin, and the tumor is any one or more of a colorectal cancer, an ovarian cancer, a gastric cancer, non-Hodgkin's lymphoma, non-small cell carcinoma, and head and neck tumors; or the drug ingredient is the cisplatin, and the tumor is any one or more of an ovarian cancer, a prostate cancer, a testicular cancer, a lung cancer, a nasopharyngeal cancer, an esophageal cancer, malignant lymphoma, head and neck squamous cell carcinoma, a thyroid cancer, and osteosarcoma.

The effective dosage herein includes a prophylactically effective dosage and/or a therapeutically effective dosage. The therapeutically effective dosage refers to a dosage that is effective to achieve a desired therapeutic result, such as a reduction of the Alzheimer's disease, within a necessary dosage and time period. In a specific implementation mode, the dosage may be adjusted to provide the optimal therapeutic response dosage, and the therapeutically effective dosage may be varied according to the following factors: a disease state, an age, a gender, and a weight of an individual and an ability of a preparation which causes a desired response in the individual. The meaning of the therapeutically effective dosage also includes a dosage of which beneficial effects of treatment exceed its toxic or harmful effects. The prophylactically effective dosage refers to a dosage that is effective to achieve the desired preventive result, such as preventing or inhibiting the occurrence of the Alzheimer's disease, within the necessary dosage and time period. The prophylactically effective dosage may be determined according to the above description of the therapeutically effective dosage. For any specific subjects, the specific dosage may be adjusted along with time according to individual needs and the professional judgment of an administering person.

According to a seventh aspect of the disclosure, a method for preventing and/or treating tumors, cardiovascular and cerebrovascular diseases, retinopathy caused by diabetes and capillary fragility or allergies is further provided, the method includes: providing any one of the above nucleic acid nanocarrier drugs or pharmaceutical compositions, herein a drug ingredient is: the effective dose of the above flavone-containing drug or pharmaceutical composition which is administered to patients with tumors, cardiovascular and cerebrovascular diseases, retinopathy caused by diabetes and capillary fragility or allergies. Further, the tumor is the ovarian cancer.

It is to be noted that while the above nucleic acid structureal domain is linked with the drug, the nucleic acid structureal domain is water-soluble, and most of the drugs have poor water solubility. After it is linked with the nucleic acid structureal domain, the water solubility is improved. When the above drugs are anthracyclines, these drugs are covalent-linked with the nucleic acid structureal domain through a -NH bond (under a suitable pH value condition, the activity of the -NH group is hundreds of times greater than the activity of other groups which may be covalent-linked with the drugs) on a nucleotide guanosine, thereby the nucleic acid structural domain for loading the drugs is formed. Therefore, according to a size of a specific drug molecule and the number of the GC base pairs on the sequence a, the sequence b and the sequence c in the specifically designed nucleic acid structural domain, while linked, a linking reaction is performed theoretically according to 1.1-1.3 times of a supersaturated linking amount, and at most 35 to 45 drugs may be linked in one nucleic acid structural domain. While the above drugs are other structures, the loading amount is related to an occupation situation (including but not limited to a molecular structure, a morphology, a shape and a molecular weight) of the specific drug. Therefore, a linkage condition of an activity site of the drug and the -NH bond on the nucleotide guanosine of the nucleic acid structural domain is relatively harsh, and it may also be loaded but it is relatively difficult to cause a situation of excessive linkage.

It is to be noted that the nucleic acid nanoparticles formed by the self-assembly of the sequences or sequence variations provided by the disclosure may also be used as basic structural units, and may be further polymerized to form multimers, such as a dimer, a trimer, a tetramer, a pentamer, a hexamer or a heptamer, according to the actual application needs.

The beneficial effects of the disclosure are further described below in combination with specific embodiments.

Assembly of nucleic acid nanoparticles

Embodiment 1

I. RNA and DNA nanoparticle carrier:

(1) Three polynucleotide base sequences for assembling RNA nanoparticles are specifically shown in Table 1:

Table 1:

| Name | Sequence sense (5'-3') | Base number | Molecular weight (MW) | Chemical modification | Fluore scence mark | Special modification | Total molecular weight |
|---|---|---|---|---|---|---|---|
| a-strand ( SEQ ID NO:52) | cGcGcGcccAc cAGcGuuccG GGcGccGc | 29 | 9678.7 | C/U base 2'F modification | / | 5'Biotin | 29524.9 |
| b-strand ( SEQ ID NO:53) | GcGGcGcccG GuucGccGccA GGcGGc | 27 | 9116.8 | C/U base 2'F modification | / | 5'Biotin | |
| c-strand ( SEQ ID NO:54) | GccGccAGGc GGccAuAGcG GuGGGcGcG cG | 31 | 10729.4 | C/U base 2'F modification | 5'CY3 | | |

(2) Three polynucleotide base sequences of DNA nanoparticles

DNA uses the same sequence as the above RNA, except that U is replaced by T. Herein, a molecular weight of the a-strand is 8802.66, a molecular weight of the b-strand is 8280.33, and a molecular weight of the c-strand is 9605.2.

The strands a, b and c of the above RNA nanoparticles and DNA nanoparticles are all commissioned to be synthesized by Sangon Bioengineering (Shanghai) Co., Ltd.

II. Self-assembly experiment operations:

(1) according to a molar ratio of 1:1:1, enabling the RNA or DNA single strands a, b and c to be simultaneously mixed and dissolved in DEPC treated water or TMS buffer solution;

(2) heating mixed solution to 80/95°C (herein a RNA assembly temperature is 80°C, and a DNA assembly temperature is 95°C), after keeping for 5 min, slowly cooling to a room temperature at a rate of 2°C/min;

(3) enabling a product to be loaded on 8% (m/v) of non-denaturing PAGE gel and placed in TBM buffer solution under a condition of 4°C, purifying a complex by 100 v of electrophoresis;

(4) cutting a target band and eluting in RNA/DNA elution buffer solution at 37°C, after that, precipitating overnight in ethanol, volatilizing under reduced pressure and low temperature, to obtain a self-assembly product; and

(5) electrophoresis analysis detection and laser scanning observation.

III. Self-assembly experiment result

Electrophoresis detection result

An electrophoresis detection result of the RNA self-assembly product is shown in Fig. 1. In Fig. 1, lanes 1 to 3 from left to right are successively: the a-strand, the b-strand, and the RNA self-assembly product. It may be seen from the figure that although the RNA self-assembly product is slightly diffused, it may be apparently seen that it is a single band. In addition, because the molecular weight is a molecular weight after assembly, it is larger than a single-stranded molecular weight, therefore a band position is behind the a-strand and the b-strand, and the actual situation is consistent with a theory, it is proved that a stable composite structure is formed between the above RNA single strands by the self-assembly, and RNA nanoparticles are formed.

An electrophoresis detection result of the DNA self-assembly product is shown in Fig. 2. In Fig. 2, lanes 1 to 3 from left to right are successively: the a-strand, the b-strand, and the DNA self-assembly product. It may be seen from the figure that a band of the DNA self-assembly product is bright and clear, and is a single band, it is proved that a stable

composite structure is formed between the above DNA single strands by the self-assembly, and DNA nanoparticles are formed.

In the embodiment, it is verified by gel electrophoresis that the sequences a, b and c including the RNA core sequences SEQ ID NO:1, SEQ ID NO:3 and SEQ ID NO:5 may be successfully self-assembled into the RNA nanoparticles. The sequences a, b and c including the DNA core sequences SEQ ID NO:2, SEQ ID NO:4 and SEQ ID NO:6 may also be successfully self-assembled into the DNA nanoparticles.

In addition to the core sequences which form a nucleic acid structural domain, the sequences a, b and c of the above RNA nanoparticles and DNA nanoparticles also have various extension sequences (including a drug loading linking sequence) which promote a loading function of the nucleic acid structural domain and a target head or a fluorescein which is linked with the nucleic acid structural domain. It may be seen that the presence of substances other than these core sequences does not affect the formation of the nucleic acid structural domain and the successful self-assembly of the nucleic acid nanoparticles. The self-assembled nucleic acid nanoparticles may have a targeting property under the guidance of the target head, and the fluorescein may make the nucleic acid nanoparticles have visibility and traceability.

Embodiment 2

I. 7 groups of short-sequence RNA nanoparticle carriers:

(1) 7 groups of three polynucleotide base sequences for forming RNA nanoparticles are shown in Table 2 to Table 8:

Table 2: R-1:

| Name | Sequence sense (5'-3') | Base number | Chemical modification | Molecular weight | Total molecular weight |
|---|---|---|---|---|---|
| a-strand (SEQ ID NO: 55) | **GG**AGcGuu**GG** | 10 | c/u base 2'F modification | 3262.9 | 9828.7 |
| b-strand (SEQ ID NO: 56) | **cc**uuc**GCCG** | 9 | c/u base 2'F modification | 2780.6 | |
| c-strand (SEQ ID NO: 57) | **cGGc**cAuAGc**cc** | 12 | c/u base 2'F modification | 3785.2 | |

Table 3: R-2:

| Name | Sequence sense (5'-3') | Base number | Chemical modification | Molecular weight | Total molecular weight |
|---|---|---|---|---|---|
| a-strand (SEQ ID NO: 58) | **Gc**AGcGuu**cG** | 10 | c/u base 2'F modification | 3186.7 | 9829.4 |
| b-strand (SEQ ID NO: 59) | **cG**uuc**GccG** | 9 | c/u base 2'F modification | 2820.2 | |
| c-strand (SEQ ID NO: 60) | **cGGc**cAuAGc**Gc** | 12 | c/u base 2'F modification | 3822.5 | |

Table 4: R-3:

| Name | Sequence sense (5'-3') | Base number | Chemical modification | Molecular weight | Total molecular weight |
|---|---|---|---|---|---|
| a-strand (SEQ ID NO: 61) | **cG**AGcGuu**Gc** | 10 | c/u base 2'F modification | 3187.5 | 9829.9 |
| b-strand (SEQ ID NO: 62) | **Gc**uuc**GccG** | 9 | c/u base 2'F modification | 2819.2 | |
| c-strand (SEQ ID NO: 63) | **cGGc**cAuAGc**cG** | 12 | c/u base 2'F modification | 3823.2 | |

Table 5: R-4:

| Name | Sequence sense (5'-3') | Base number | Chemical modification | Molecular weight | Total molecular weight |
|---|---|---|---|---|---|
| a-strand (SEQ ID NO: 64) | **GG**AGcGuu**GG** | 10 | c/u base 2'F modification | 3263.7 | 9830.9 |
| b-strand (SEQ ID NO : 65) | **cc**uuc**GGGG** | 9 | c/u base 2'F modification | 2858.2 | |
| c-strand (SEQ ID NO: 66) | **cccc**cAuAGc**cc** | 12 | c/u base 2'F modification | 3709.0 | |

Table 6: R-5:

| Name | Sequence sense (5'-3') | Base number | Chemical modification | Molecular weight | Total molecular weight |
|---|---|---|---|---|---|
| a-strand (SEQ ID NO: 67) | **Gc**AGcGuu**cG** | 10 | c/u base 2'F modification | 3187.5 | 9830.2 |
| b-strand (SEQ ID NO: 68) | **cG**uuc**GGcG** | 9 | c/u base 2'F modification | 2857.5 | |
| c-strand (SEQ ID NO: 69) | **cGcc**cAuAGc**Gc** | 12 | c/u base 2'F modification | 3785.2 | |

Table 7: R-6:

| Name | Sequence sense (5'-3') | Base number | Chemical modification | Molecular weight | Total molecular weight |
|---|---|---|---|---|---|
| a-strand (SEQ ID NO: 70) | **Gc**AGcGuu**cG** | 10 | c/u base 2'F modification | 3187.2 | 9830.5 |
| b-strand (SEQ ID NO: 71) | **cG**uuc**GGcc** | 9 | c/u base 2'F modification | 2820.4 | |
| c-strand (SEQ ID NO: 72) | **GGcc**cAuAGc**Gc** | 12 | c/u base 2'F modification | 3822.9 | |

Table 8: R-7:

| Name | Sequence sense (5'-3') | Base number | Chemical modification | Molecular weight | Total molecular weight |
|---|---|---|---|---|---|
| a-strand (SEQ ID NO: 73) | **cG**AGcGuu**Gc** | 10 | c/u base 2'F modification | 3187.6 | 9830.7 |
| b-strand (SEQ ID NO: 74) | **Gc**uuc**GGcG** | 9 | c/u base 2'F modification | 2857.8 | |
| c-strand (SEQ ID NO: 75) | **cGcc**cAuAGc**cG** | 12 | c/u base 2'F modification | 3785.3 | |

The single strands of the above 7 groups of the short-sequence RNA nanoparticle carriers are all commissioned to be synthesized by Sangon Bioengineering (Shanghai) Co., Ltd.

II. Self-assembly experiment operations:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, volatilizing under reduced pressure and low temperature, to obtain a short-sequence RNA self-assembly product;

(5) electrophoresis analysis detection and laser scanning observation; and

(6) electric potential detection.

III. Self-assembly experiment result

(1) Electrophoresis detection result

A 2% agarose gel electrophoresis diagram of the 7 groups of the short-sequence RNA self-assembly products is shown in Fig. 3. From left to right, lanes 1 to 7 in Fig. 3 are successively: short-sequences R-1, R-2, R-3, R-4, R-5, R-6 and R-7.

A 4% agarose gel electrophoresis diagram of the 7 groups of the short-sequence RNA self-assembly products is shown in Fig. 4. From left to right, lanes 1 to 7 in Fig. 4 are successively: short-sequences R-1, R-2, R-3, R-4, R-5, R-6 and R-7.

It may be clearly seen from results of Fig. 3 and Fig. 4 that bands of the R-2, R-3, R-5 and R-7 in the 7 groups of the short-sequence self-assembly products are bright and clear, although the R-1, R-4 and R-6 are relatively diffused, it may still be seen as a single band, it is indicated that the 7 groups of the short-sequences may be self-assembled better into a RNA nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears; setting a software detection parameter;

and then clicking Ok to complete the settings, after a measurement dialog box appears, clicking Start to start.

Measurement result: potential detection results of the 7 groups of the short-sequence RNA nanoparticles are as follows.

Table 9:

| Sample name | Detection times | Potential ZP (mV) |
|---|---|---|
| R-1 | 1 | -33.7 |
| | 2 | -35.6 |
| | 3 | -34.6 |
| Experiment result | -34.65 mV. (±0.95mV) | |

Table 10:

| Sample name | Detection times | Potential ZP (mV) |
|---|---|---|
| R-2 | 1 | -37.9 |
| | 2 | -37.3 |
| | 3 | -36.8 |
| Experiment result | -37.35 mV. (±0.55mV) | |

Table 11:

| Sample name | Detection times | Potential ZP (mV) |
|---|---|---|
| R-3 | 1 | -31.5 |
| | 2 | -33 |
| | 3 | -32.7 |
| Experiment result | -32.425 mV. (±0.75mV) | |

Table 12:

| Sample name | Detection times | Potential ZP (mV) |
|---|---|---|
| R-4 | 1 | -35.40 |
| | 2 | -36.00 |
| | 3 | -35.50 |
| Experiment result | -35.7 mV. (±0.3mV) | |

Table 13:

| Sample name | Detection times | Potential ZP (mV) |
|---|---|---|
| R-5 | 1 | -34.00 |
| | 2 | -33.30 |
| | 3 | -34.90 |
| Experiment result | -34.1 mV. (±0.8mV) | |

Table 14:

| Sample name | Detection times | Potential ZP (mV) |
|---|---|---|
| R-6 | 1 | -33.10 |
| | 2 | -36.10 |
| | 3 | -35.60 |
| Experiment result | -34.6 mV. (±1.5mV) | |

Table 15:

| Sample name | Detection times | Potential ZP (mV) |
|---|---|---|
| R-7 | 1 | -35.60 |
| | 2 | -34.80 |
| | 3 | -34.00 |
| Experiment result | -34.80 mV. ±0.8mV | |

It may be seen from the above potential detection data that the 7 groups of the short-sequence RNA self-assembly products all have good stability, and it is further indicated that the nanoparticles formed by the self-assembly of each short-sequence RNA have a relatively stable self-assembly structure.

It is indicated from the embodiment that: different combinations of the core sequences a, b and c may form the RNA nanoparticles with the nucleic acid structural domain through the self-assembly, and the structure is stable. It may be seen on the basis of Embodiment 1 that the RNA nanoparticles may also be successfully assembled by adding various functional extension fragments or a linkage target head, a fluorescein and the like on the basis of these different core sequence combinations, and have functions such as drug loading, cell targeting, visibility and traceability.

In order to further verify these performances, the extension fragment is added on the basis of Embodiment 2, and it is specifically described in Embodiment 3. On the basis of the DNA core sequence corresponding to the RNA core sequence of Embodiment 2, the extension fragment is added, and the target head is connected or unconnected at the same time, it is specifically described in Embodiment 4.

Embodiment 3

I. 7 groups of conventional-sequence RNA nanoparticle carriers:

(1) 7 groups of three polynucleotide base sequences for forming RNA nanoparticles are shown in Table 16 to Table 22:

Table 16: R-8

| Name | Sequence sense (5'-3') | Base number | Chemical modification | Molecular weight | Total molecular weight |
|---|---|---|---|---|---|
| a (SEQ ID NO: 76) | cGcGcGcccA**GG**AGcGu u**GG**cGGGcGGcG | 29 | C/U 2'F modification | 9462.78 | 28084.13 |
| b (SEQ ID NO : 77) | cGccGcccG**cc**uuc**GccG** ccAGccGcc | 27 | C/U 2'F modification | 8522.18 | |
| c (SEQ ID NO: 78) | GGcGGcAGG**cGGc**cAu AGc**cc**uGGGcGcGcG | 31 | C/U 2'F modification | 10099.17 | |

Table 17: R-9

| Name | Sequence sense (5'-3') | Base number | Chemical modification | Molecular weight | Total molecular weight |
|---|---|---|---|---|---|
| a (SEQ ID NO: 79) | cGcGcGcccA**Gc**AGcGuuc**G**cGGGcGGcG | 29 | C/U 2'F modification | 9386.73 | 28084.13 |
| b (SEQ ID NO: 80) | cGccGcccG**cG**uuc**GccG**ccAGccGcc | 27 | C/U 2'F modification | 8560.20 | |
| c (SEQ ID NO: 81) | GGcGGcAGG**cGGc**cAuAGc**Gc**uGGGcGcGcG | 31 | C/U 2'F modification | 10137.20 | |

Table 18: R-10

| Name | Sequence sense (5'-3') | Base number | Chemical modification | Molecular weight | Total molecular weight |
|---|---|---|---|---|---|
| a (SEQ ID NO: 82) | cGcGcGcccA**cG**AGcGuu**Gc**GGGGcGGcG | 29 | C/U 2'F modification | 9424.75 | 28084.13 |
| b (SEQ ID NO: 83) | cGccGcccc**Gc**uuc**GccG**ccAGccGcc | 27 | C/U 2'F modification | 8522.18 | |
| c (SEQ ID NO: 84) | GGcGGcAGG**cGGc**cAuAGc**cG**uGGGcGcGcG | 31 | C/U 2'F modification | 10137.20 | |

Table 19: R-11

| Name | Sequence sense (5'-3') | Base number | Chemical modification | Molecular weight | Total molecular weight |
|---|---|---|---|---|---|
| a (SEQ ID NO: 85) | cGcGcGcccA**GG**AGCGuu**GG**cccGcGGcG | 29 | C/U 2'F modification | 9386.73 | 28084.13 |
| b (SEQ ID NO: 86) | cGccGcGGG**cc**uuc**GGGG**ccAGccGcc | 27 | C/U 2'F modification | 8674.28 | |
| c (SEQ ID NO: 87) | GGcGGcAGG**cccc**cAuAGc**cc**uGGGcGcGcG | 31 | C/U 2'F modification | 10023.12 | |

Table 20: R-12

| Name | Sequence sense (5'-3') | Base number | Chemical modification | Molecular weight | Total molecular weight |
|---|---|---|---|---|---|
| a (SEQ ID NO: 88) | cGcGcGcccA**Gc**AGcG uu**cG**ccccGccGc | 29 | C/U 2'F modification | 9234.62 | 28084.18 |
| b (SEQ ID NO: 89) | GcGGcGGGG**cG**uuc**G** **GcG**GcAGGcGGc | 27 | C/U 2'F modification | 8864.41 | |
| c (SEQ ID NO: 90) | GccGccAGc**cGcc**cAu AGc**Gc**uGGGcGcGcG | 31 | C/U 2'F modification | 9985.10 | |

Table 21: R-13

| Name | Sequence sense (5'-3') | Base number | Chemical modification | Molecular weight | Total molecular weight |
|---|---|---|---|---|---|
| a (SEQ ID NO: 91) | cGcGcGcccA**Gc**AGcGu u**cG**GGGcGccGc | 29 | C/U 2'F modification | 9348.70 | |
| b (SEQ ID NO: 92) | GcGGcGcccc**cG**uuc**GG** **cc**GGcAGGcGGc | 28 | C/U 2'F modification | 9057.52 | 28736.53 |
| c (SEQ ID NO: 93) | GccGccAGcc**GGcc**cAu AGc**Gc**uGGGcGcGcG | 32 | C/U 2'F modification | 10330.31 | |

Table 22: R-14 (uGAcAGAuAAGGAAccuGcudTdT in the following a-strand is survivin siRNA)

| Name | Sequence sense (5'-3') | Base number | Chemical modification | Molecular weight | Total molecular weight |
|---|---|---|---|---|---|
| a (SEQ ID NO: 94) | cGcGcG**cG**AGcGuu**G** **c**AAuGAcAGAuAAGG AAccuGcudTdT | 39 | C/U 2'F modification | 12901.91 | 31832.42 |
| b (SEQ ID NO: 95) | GGcAGGuuccuuAucu GucAAA**Gc**uuc**GGcG** GcAGc | 36 | C/U 2'F modification | 11555.97 | |
| c (SEQ ID NO: 96) | GcAGc**cGcc**cAuAGc**c** **G**cGcGcG | 23 | C/U 2'F modification | 7374.54 | |

The single strands of the above 7 groups of conventional-sequence RNA nanoparticle carriers are all commissioned to be synthesized by Suzhou Gima Company, herein the sequence a, the sequence b and the sequence c in R-8 to R-14 are respectively extended RNA oligonucleotide sequences formed by adding the extension fragments on the basis of the sequence a, the sequence b and the sequence c in R-1 to R-7, a targeting module fragment is not extended, and C/U base 2'F modification (resistance to enzyme digestion and stability are enhanced) is performed. In addition, a siRNA nucleic acid interference therapeutic fragment of a survivin is modified in the above RNA nanoparticle R-14, specifically a sense strand (see an underlined part of the a-strand) of Survivin siRNA is extended at a-strand 3'-end, and an antisense strand (see an underlined part of the b-strand) is extended and connected at b-strand 5'-end, so base

pair complementary is formed.

II. Self-assembly experiment operations:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, volatilizing under reduced pressure and low temperature;

(5) electrophoresis analysis detection and laser scanning observation;

(6) electric potential detection.

III. Self-assembly experiment result

(1) Electrophoresis detection result

A 2% agarose gel electrophoresis diagram of the 7 groups of the conventional-sequence RNA self-assembly products is shown in Fig. 5. From left to right, lanes 1 to 7 in Fig. 5 are successively: conventional-sequence RNA self-assembly products R-8, R-9, R-10, R-11, R-12, R-13 and R-14.

A 4% agarose gel electrophoresis diagram of the 7 groups of the conventional-sequence RNA self-assembly products is shown in Fig. 6. From left to right, lanes 1 to 7 in Fig. 6 are successively: conventional-sequence RNA self-assembly products R-8, R-9, R-10, R-11, R-12, R-13 and R-14.

It may be clearly seen from results of Fig. 5 and Fig. 6 that bands of the 7 groups of the conventional-sequence self-assembly products are bright and clear single bands, it is indicated that the 7 groups of the conventional-sequences may be self-assembled into a nanostructure. Herein after a Survivin siRNA nucleic acid interference therapeutic fragment is modified in the conventional-sequence RNA self-assembly product R-14, it still has the stable self-assembly structure, it is also indicated that the nucleic acid nanoparticles of the disclosure may load a nucleic acid drug, and have a delivery carrier function of the nucleic acid drug.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 7 groups of the conventional-sequence RNA nanoparticles are shown below.

Table 23:

| Sample name | Detection times | Potential ZP (mV) |
|---|---|---|
| R-8 | 1 | -18.00 |
| | 2 | -16.10 |
| | 3 | -18.20 |
| Experiment result | -17.43 mV (±1.33mV) | |

Table 24:

| Sample name | Detection times | Potential ZP (mV) |
|---|---|---|
| R-9 | 1 | -16.90 |
| | 2 | -17.50 |
| | 3 | -20.20 |
| Experiment result | -18.20 mV (±1.3mV) | |

Table 25:

| Sample name | Detection times | Potential ZP (mV) |
|---|---|---|
| R-10 | 1 | -10.40 |
| | 2 | -11.80 |
| | 3 | -10.40 |
| Experiment result | -10.87 mV (±0.47mV) | |

Table 26:

| Sample name | Detection times | Potential ZP (mV) |
|---|---|---|
| R-11 | 1 | -28.30 |
| | 2 | -26.00 |
| | 3 | -33.10 |
| Experiment result | -29.13mV (±3.13mV) | |

Table 27:

| Sample name | Detection times | Potential ZP (mV) |
|---|---|---|
| R-12 | 1 | -7.59 |
| | 2 | -7.80 |
| | 3 | -17.20 |
| Experiment result | -10.86 mV (±3.27mV) | |

Table 28:

| Sample name | Detection times | Potential ZP (mV) |
|---|---|---|
| R-13 | 1 | -9.64 |
| | 2 | -15.60 |
| | 3 | -21.10 |
| Experiment result | -15.45 mV (±5.81 mV) | |

Table 29:

| Sample name | Detection times | Potential ZP (mV) |
|---|---|---|
| R-14 | 1 | -21.40 |
| | 2 | -21.20 |
| | 3 | -28.00 |
| Experiment result | -23.53 mV. ±2.33mV | |

It may be seen from the above potential detection data that the 7 groups of the conventional-sequence RNA self-assembly products all have good stability, and it is further indicated that the nanoparticles formed by the self-assembly of each conventional-sequence RNA have a relatively stable self-assembly structure.

It is indicated from the embodiment that: on the basis of different combinations of the RNA core sequences, the RNA nanoparticles with the stable structure may also be successfully self-assembled by adding the extension fragments. At the same time, the added extension fragments make the RNA nanoparticles have superior drug loading performance (specifically described in Embodiment 5 and Embodiment 7).

Embodiment 4

I. 7 groups of conventional-sequence DNA nanoparticle carriers:

(1) 7 groups of three polynucleotide base sequences for forming DNA nanoparticles:

An EGFRapt target head or a PSMAapt(A9L) target head is extended in a part of a-strands in the table:

EGFRapt (SEQ ID NO:97): GCCTTAGTAACGTGCTTTGATGTCGATTCGACAGGAGGC;

PSMAapt (A9L, SEQ ID NO: 98):

GGGCCGAAAAAGACCTGACTTCTATACTAAGTCTACGTCCC.

Table 30: D-1

| Name | Sequence sense (5'-3') | Base number | Chemical modification | Molecular weight | Total molecular weight |
|---|---|---|---|---|---|
| a (SEQ ID NO: 99) | CGCGCGCCCAGGAGCGTTGGCGGGCGGCG<u>GCCTTAGTAACGTGCTTTGATGTCGATTCGACAGGAGGC</u> | 68 | 3 bases before 5'-end and 3'-end, thio modification | 21214.63 | 39092.09 |
| b (SEQ ID NO: 100) | CGCCGCCCGCCTTCGCCGCCAGCCGCC | 27 | 3 bases before 5'-end and 3'-end, thio modification | 8176.24 | |
| c(SEQ ID NO: 101) | GGCGGCAGGCGGCCATAGCCCTGGGCGCGCG | 31 | 3 bases before 5'-end and 3'-end, thio modification | 9701.22 | |

Table 31: D-2

| Name | Sequence sense (5'-3') | Base number | Chemical modification | Molecular weight | Total molecular weight |
|---|---|---|---|---|---|
| a (SEQ ID NO: 102) | CGCGCGCCCAGCAGCGTT CGCGGGCGGCGGCCTTAG TAACGTGCTTTGATGTCGAT TCGACAGGAGGC | 68 | 3 bases before 5'-end and 3'-end, thio modification | 21134.59 | 39092.11 |
| b (SEQ ID NO: 103) | CGCCGCCCGCGTTCGCCG CCAGCCGCC | 27 | 3 bases before 5'-end and 3'-end, thio modification | 8216.27 | |
| c (SEQ ID NO: 104) | GGCGGCAGGCGGCCATAG CGCTGGGCGCGCG | 31 | 3 bases before 5'-end and 3'-end, thio modification | 9741.25 | |

Table 32: D-3

| Name | Sequence sense (5'-3') | Base number | Chemical modification | Molecular weight | Total molecular weight |
|---|---|---|---|---|---|
| a (SEQ ID NO: 105) | CGCGCGCCCACGAGCGT TGCGGGGCGGCGGCCTT AGTAACGTGCTTTGATGT CGATTCGACAGGAGGC | 68 | 3 bases before 5'-end and 3'-end, thio modification | 21174.60 | 39092.09 |
| b (SEQ ID NO: 106) | CGCCGCCCCGCTTCGCC GCCAGCCGCC | 27 | 3 bases before 5'-end and 3'-end, thio modification | 8176.24 | |
| c (SEQ ID NO: 107) | GGCGGCAGGCGGCCATA GCCGTGGGCGCGCG | 31 | 3 bases before 5'-end and 3'-end, thio modification | 9741.25 | |

Table 33: D-4

| Name | Sequence sense (5'-3') | Base number | Chemical modification | Molecular weight | Total molecular weight |
|---|---|---|---|---|---|
| a (SEQ ID NO: 108) | CGCGCGCCCAGGAGCGTT GGCCCGCGGCGTGGGCC GAAAAAGACCTGACTTCTA TACTAAGTCTACGTCCC | 71 | 3 bases before 5'-end and 3'-end, thio modification | 21923.12 | 39780.63 |
| b (SEQ ID NO: 109) | CGCCGCGGGCCTTCGGGG CCAGCCGCC | 27 | 3 bases before 5'-end and 3'-end, thio modification | 8236.34 | |

(continued)

| Name | Sequence sense (5'-3') | Base number | Chemical modification | Molecular weight | Total molecular weight |
|---|---|---|---|---|---|
| c (SEQ ID NO: 110) | GGCGGCAGGCCCCCATAG CCCTGGGCGCGCG | 31 | 3 bases before 5'-end and 3'-end, thio modification | 9621.17 | |

Table 34: D-5

| Name | Sequence sense (5'-3') | Base number | Chemical modification | Molecular weight | Total molecular weight |
|---|---|---|---|---|---|
| a (SEQ ID NO: 111) | CGCGCGCCCAGCAGCGTT CGCCCCGCCGCTGGGCC GAAAAAGACCTGACTTCTA TACTAAGTCTACGTCCC | 71 | 53 bases before 5'-end and 3'-end, thio modification | 21763.03 | 39880.64 |
| b (SEQ ID NO: 112) | GCGGCGGGGCGTTCGGC GGCAGGCGGC | 27 | 3 bases before 5'-end and 3'-end, thio modification | 8536.46 | |
| c (SEQ ID NO: 113) | GCCGCCAGCCGCCCATAG CGCTGGGCGCGCG | 31 | 3 bases before 5'-end and 3'-end, thio modification | 9581.15 | |

Table 35: D-6

| Name | Sequence sense (5'-3') | Base number | Chemical modification | Molecular weight | Total molecular weight |
|---|---|---|---|---|---|
| a (SEQ ID NO: 114) | CGCGCGCCCAGCAGCG TTCGGGGCGCCGC | 29 | 3 bases before 5'-end and 3'-end, thio modification | 8978.76 | 27594.69 |
| b (SEQ ID NO: 115) | GCGGCGCCCCGTTCGG CCGGCAGGCGGC | 28 | 3 bases before 5'-end and 3'-end, thio modification | 8705.57 | |
| c (SEQ ID NO: 116) | GCCGCCAGCCGGCCCA TAGCGCTGGGCGCGCG | 32 | 3 bases before 5'-end and 3'-end, thio modification | 9910.36 | |

Table 36: D-7

| Name | Sequence sense (5'-3') | Base number | Chemical modification | Molecular weight | Total molecular weight |
|---|---|---|---|---|---|
| a (SEQ ID NO: 117) | CGCGCGCCCACGAGCG TTGCGGGCGCCGC | 29 | 3 bases before 5'-end and 3'-end, thio modification | 8978.76 | 26976.30 |
| b (SEQ ID NO: 118) | GCGGCGCCCGCTTCGG CGGCAGGCGGC | 27 | 3 bases before 5'-end and 3'-end, thio modification | 8416.39 | |
| c (SEQ ID NO: 119) | GCCGCCAGCCGCCCAT AGCCGTGGGCGCGCG | 31 | 3 bases before 5'-end and 3'-end, thio modification | 9581.15 | |

Single strands of the above 7 groups of the conventional-sequence DNA nanoparticles are all commissioned to be synthesized by Suzhou Hongxun Company, herein:

D-1 is the conventional-sequence DNA nanoparticles formed after adding an extension sequence containing the EGFRapt target head (see an underlined part) on the basis of the above core sequences (8) (sequence a: 5'-GGAGCGTTGG-3', sequence b: 5'-CCTTCGCCG-3', and sequence c: 5'-CGGCCATAGCCC-3');

D-2 is the conventional-sequence DNA nanoparticles formed after adding an extension sequence containing the EGFRapt target head (see an underlined part) on the basis of the above core sequences (9) (sequence a: 5'-GGAGCGTTGG-3', sequence b: 5'-CCTTCGCCG-3', and sequence c: 5'-CGGCCATAGCCC-3');

D-3 is the conventional-sequence DNA nanoparticles formed after adding an extension sequence containing the EGFRapt target head (see an underlined part) on the basis of the above core sequences (10) (sequence a: 5'-GGAGCGTTGG-3', sequence b: 5'-CCTTCGCCG-3', and sequence c: 5'-CGGCCATAGCCC-3');

D-4 is the conventional-sequence DNA nanoparticles formed after adding an extension sequence containing the PSMAapt target head (see an underlined part) on the basis of the above core sequences (11) (sequence a: 5'-GGAGCGTTGG-3', sequence b: 5'-CCTTCGCCG-3', and sequence c: 5'-CGGCCATAGCCC-3');

D-5 is the conventional-sequence DNA nanoparticles formed after adding an extension sequence containing the PSMAapt target head (see an underlined part) on the basis of the above core sequences (12) (sequence a: 5'-GGAGCGTTGG-3', sequence b: 5'-CCTTCGCCG-3', and sequence c: 5'-CGGCCATAGCCC-3');

D-6 is the conventional-sequence DNA nanoparticles formed after adding an extension sequence without containing a target head structure on the basis of the above core sequences (13) (sequence a: 5'-GGAGCGTTGG-3', sequence b: 5'-CCTTCGCCG-3', and sequence c: 5'-CGGCCATAGCCC-3'); and

D-7 is the conventional-sequence DNA nanoparticles formed after adding an extension sequence without containing a target head structure on the basis of the above core sequences (14) (sequence a: 5'-GGAGCGTTGG-3', sequence b: 5'-CCTTCGCCG-3', and sequence c: 5'-CGGCCATAGCCC-3').

In addition, a single-stranded sequence used to form the 8-th group of the DNA nanoparticles and a single-stranded sequence used to form the 9-th group of the DNA nanoparticles are also synthesized.

Herein, the 8-th group is the conventional sequence DNA nanoparticles formed after the extension sequence containing the EGFRapt target head (see a bolded part) is added on the basis of the core sequence (15) described above. A specific sequence is as follows:

a-strand (SEQ ID NO:172): 5'-CGCGCGCCCACGAGCGTTCCGGGCGCGCCTTAGTAACGTGCTTTGATGTCGATTC GACAGGAGGC-3', thio modification is respectively performed on the first three bases at the 5'-end and the last three bases at the 3'-end, and the 5'-end is linked to Biotin, the bolded part is the EGFRapt sequence;

b-strand (SEQ ID NO:173): 5'-GCGCCCGGTTCGCCGCCAGCCGCCGC-3', the thio modification is respectively performed on the first three bases at the 5'-end and the last three bases at the 3'-end; and

c-strand (SEQ ID NO:174): 5'-GCGGCGGCAGGCGGCCATAGCCGTGGGCGCGCG-3', the thio modification is respectively performed on the first three bases at the 5'-end and the last three bases at the 3'-end, and the 3'-end is linked to the Cy5 fluorescence labeling.

Herein, the 9-th group is the DNA nanoparticles formed after the extension sequence is added on the basis of the core sequence (15) described above. A specific sequence is as follows:

a-strand (SEQ ID NO:178): 5'-CGCGCGCGCCCACGAGCGTTCCGGGCGCCGCCGC-3', the thio modification is respectively performed on the first three bases at the 5'-end and the last three bases at the 3'-end, and the 5'-end is linked to the Biotin;

b-strand (SEQ ID NO:179): 5'-GCGGCGGCGCCCGGTTCGCCGCCAGCCGCCGCC-3', the thio modification is respectively performed on the first three bases at the 5'-end and the last three bases at the 3'-end; and

c-strand (SEQ ID NO:180): 5'-GGCGGCGGCAGGCGGCCATAGCCGTGGGCGCGCGCG-3', the thio modification is respectively performed on the first three bases at the 5'-end and the last three bases at the 3'-end, and the 5'-end is linked to the Cy5 fluorescence labeling.

II. Self-assembly experiment operations:

(1) According to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in DNA elution buffer solution at 37 DEG C, after that, precipitating overnight in ethanol, volatilizing under reduced pressure and low temperature, to obtain a conventional-sequence DNA self-assembly product;

(5) electrophoresis analysis detection and laser scanning observation;

(6) electric potential detection;

(7) particle size measurement; and

(8) observing by a transmission electron microscope.

III. Self-assembly experiment result

(1) Electrophoresis detection result

A 2% agarose gel electrophoresis diagram of the 7 groups of the conventional-sequence DNA self-assembly products is shown in Fig. 7. From left to right, lanes 1 to 7 in Fig. 7 are successively: conventional-sequence DNA self-assembly products D-1, D-2, D-3, D-4, D-5, D-6 and D-7.

A 4% agarose gel electrophoresis diagram of the 7 groups of the conventional-sequence DNA self-assembly products is shown in Fig. 8. From left to right, lanes 1 to 7 in Fig. 8 are successively: conventional-sequence DNA self-assembly products D-1, D-2, D-3, D-4, D-5, D-6 and D-7.

A 2% agarose gel electrophoresis diagram of the 8-th and 9-th groups of the sequence DNA self-assembly products is shown in Fig. 9. From right to left, lanes in Fig. 9 are successively: a a-strand single-strand in the 8-th group,

and DNA self-assembly products D-8 and D-9.

It may be clearly seen from results of Fig. 7, Fig. 8 and Fig. 9 that bands of the 7 groups of the conventional-sequence DNA self-assembly products are bright and clear, it is indicated that the 7 groups of the conventional-sequence DNA strands are all self-assembled to form stable nanoparticle structures. Herein two groups of the self-assembly structures D-6 and D-7 carry the EGFRapt or PSMAapt target head, and the molecular weights are slightly low, positions of bands thereof are apparently higher than other bands, actual and theoretical conditions are completely consistent, so the stability of the self-assembly structures is further proved.

It is indicated from the embodiment: on the base of combinations of these different DNA core sequences, while various functional extension fragments are added or the target head is linked at the same time, the DNA nanoparticles may also be successfully assembled, and also have functions such as drug loading, cell targeting, visibility and traceability (specifically described in Embodiment 6 and Embodiment 8).

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 3 groups of the conventional-sequence DNA nanoparticles are shown below.

Table 37:

| Sample name | Detection times | Potential ZP (mV) |
|---|---|---|
| D-2 | 1 | -36.00 |
| | 2 | -35.80 |
| | 3 | -37.60 |
| Experiment result | -36.47 mV (±0.67mV) | |

Table 38:

| Sample name | Detection times | Potential ZP (mV) |
|---|---|---|
| D-6 | 1 | -31.70 |
| | 2 | -32.10 |
| | 3 | -31.90 |
| Experiment result | -31.90 mV (±0.2mV) | |

Table 39:

| Sample name | Detection times | Potential ZP (mV) |
|---|---|---|
| D-7 | 1 | -32.10 |
| | 2 | -31.70 |
| | 3 | -32.80 |
| Experiment result | -32.20 mV (±0.0.5mV) | |

It may be seen from the above potential detection data that the 3 groups of the conventional-sequence RNA self-assembly products have good stability, it is further indicated that the nanoparticles formed by the self-assembly of each conventional-sequence RNA have a relatively stable self-assembly structure.

(3) Particle size measurement

1. Preparing a potential sample (conventional-sequence DNA self-assembly product D-7) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, while a measurement dialog box appears, clicking Start to start, a DLS measurement value result of a hydrodynamic size of the self-assembly product D-7 is as follows:

Table 40:

| Sample name | Detection times | Particle size Z-Ave (d.nm) |
|---|---|---|
| 1 | 1 | 10.76 |
| | 2 | 13.9 |
| | 3 | 10.36 |
| Experiment result | 12.33 d.nm (±1.57 d.nm) | |

(4) Observed result of transmission electron microscope

The above conventional-sequence DNA self-assembly product D-7 is irradiated by a transmission electron microscope, and operations are as follows:

1. taking a drop of a sample and suspending on 400 meshes of a carbon-coated film copper grid, keeping for 1 min at a room temperature;

2. absorbing liquid by filter paper;

3. staining for 1 min by 2% uranyl acetate;

4. absorbing by the filter paper, and drying at the room temperature; and

5. observing and taking a picture by a transmission electron microscope JEM-1400 at 120 kv.

The result is as shown in Fig. 10, it may be apparently seen from the figure that the above conventional-sequence DNA self-assembly product D-7 is an integral structure, and it may be clearly seen that it has a T-type structure.

Embodiment 5

Dihydroartemisinin loading experiment

Chemical loading:

I. Experiment material and experiment method

1. Experiment materials and reagents:

(1) The DNA nucleic acid nanoparticles D-9 (hereinafter referred to as DNAh nanoparticles) formed by the self-assembly of the 9-th group of the DNA sequences in Embodiment 4 are used for loading. Specific information is shown in Embodiment 4.
A preparation method for the DNAh nanoparticles is similar to Embodiment 1.

(2) DEPC water: Biyuntian Company.

(3) PBS buffer solution: cellgro.

(4) 4% paraformaldehyde

(5) Dihydroartemisinin.

(6) Chloroform: Beijing University of Chemical Technology.

(7) Anhydrous ethanol: Beijing University of Chemical Technology.

2. Experiment method:

(1) Accurately weighing the dihydroartemisinin (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the DNAh nanoparticles (1 mg, 33.84 nmol), and reacting for 72 hours at 4 DEG C under a dark condition.

(2) Taking 10 μL of reaction solution and diluting for 10 times, using 50 μM of the dihydroartemisinin aqueous solution and 310 ng/μL of the DNAh nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), evaporating a solvent under reduced pressure and lower temperature, to obtain a blue solid product of dihydroartemisinin-DNAh particles

(4) Loading rate calculation:

1. preparing a known concentration of dihydroartemisinin-PBS standard solution: 2 μM, 4 μM, 6 μM, 8 μM, and 10 μM, 100 μl each;

2. enabling dihydroartemisinin-DNAh particles to be dissolved in 100 μl of the PBS;

3. putting the standard solution and the dihydroartemisinin-DNAh particles into a PCR plate, heating at 85 DEG C for 5 min, and then cooling to the room temperature;

4. measuring an absorbance of the dihydroartemisinin at 260 nm by a microplate reader, drawing a standard curve (as shown in Fig. 11), and calculating the molar concentration of the dihydroartemisinin in the loaded product;

5. measuring an absorbance of DNA at 260 nm by a spectrophotometer, to obtain the mass concentration of the DNAh particles contained in each sample; and

6. according to the dihydroartemisinin molar concentration and the mass concentration of the DNAh particles obtained by measuring, calculating a loading rate, the obtained loading rate of the dihydroartemisinin-DNAh is about 15, it represents that about 15 dihydroartemisinin molecules may be loaded on each DNAh nanoparticle carrier.

In addition, on the basis of the above DNAh nanoparticles loading the dihydroartemisinin, other small molecular drugs may be further loaded for the second time according to the same method as that of loading the dihydroartemisinin. For example, a folic acid is further loaded in the disclosure, to obtain DNA nanoparticles co-loaded with two small molecular drugs of the dihydroartemisinin and folic acid, and the loading rates of the two drugs may be detected by referring to the above method (values are not shown).

It is indicated from Embodiment 5 that the DNA nanoparticles with the extension fragment, the target head and

the fluorescein have a function of loaded drugs, the small molecular drug dihydroartemisinin may achieve the loading in a mode of covalent linkage (paraformaldehyde-solvent covalence), and may also achieve the co-loading with other small molecular drugs.

Embodiment 6

Cell binding ability of dihydroartemisinin drug-loaded DNA nanoparticles detected by flow cytometry

I. Cell information (see Table 41 below)

Table 41:

| Cell line | Cell type | Medium |
|---|---|---|
| LN-229 | Brain&Nerves | DMEM+5%FBS |

II. Samples to be tested

Dihydroartemisinin targeted drug: DNAh-Biotin-Cy5-DHA (loaded according to the loading method of the DNA nanoparticles in Embodiment 5).
Targeted fluorescent carrier: DNAh-Bio-Cy5 (the DNA nanoparticles provided in Embodiment 5).

III. Instrument, device and related reagent information

1) 5 ml FACS tube (BD, 352052)

2) Conventional cell culture medium and consumables

3) Folic acid and biotin-free medium (provided by Baiyao Zhida Nano-biotechnology Co., Ltd)

4) Fetal bovine serum FBS (ExCell Bio., FND500)

5) 24-well cell culture plate

6) Cell counter (Inno-Alliance Biotech, Countstar;)

7) CO2 incubator (Thermo Scientific, Model 3100 Series)

8) Biological safety cabinet (Thermo Scientific, Model 1300 Series A2)

9) Inverted microscope (Olympus, CKX41SF)

10) Centrifuge (Eppendorf, 5810R)

11) Flow cytometer (BD, BD Accuri C6)

IV. Experiment method:

1) Adjusting a cell state to a logarithmic growth phase, changing the medium to a medium without biotin and folic acid, and incubating overnight in the incubator at 37 DEG C;
2) dissolving a substance to be tested and preparing stock solution of the substance to be tested;
3) digesting and collecting single-cell suspension and counting, adjusting a cell density to 2×105/mL, and planting 1 mL/well into the 24-well plate;

4) adding the substance to be tested to the corresponding cell wells, herein a final concentration is 2.37 μM, shaking and mixing uniformly;
5) placing the cell plate in the incubator at 37 DEG C and incubating for 2 hours;
6) after the incubation, trypsinizing and collecting the cell suspension;
7) collect a cell precipitation by centrifugation, and washing twice with the PBS;
8) finally, resuspending the cell precipitation with 200 μL of the PBS, and performing a flow cytometry on-machine detection, herein, detection channels of the fluorescent carrier and dihydroartemisinin targeted drug: excitation light wavelength: 650 nm, and emission light channel: 670 nm; and
9) data analysis. An analysis result is shown in Table 42.

Table 42:

| Cell line | Test sample | Binding rate (%) |
|---|---|---|
| LN-229 | DNAh-Biotin-Cy5-DHA | 100 |
| | DNAh-Bio- Cy5 | 100 |
| | Blank control (medium only) | 0.07 |

It may be seen from Table 42 that the DNAh nanoparticles carrying the target head and the small molecular drug dihydroartemisinin have a high binding rate to the cells, and it may be clearly seen that it may efficiently bind and internalize with brain medulloblastoma cells LN-229. It may be seen that the dihydroartemisinin targeted drug DNAh-Biotin-Cy5-DHA has an application prospect in the treatment of brain medulloblastoma.

Embodiment 7

Stability detection of DNAh-Bio-Cy5-DHA nanoparticles in serum

I. Experiment material, reagent and device

1. Experiment material:

Sample to be tested: DNAh-Biotin-Cy5-DHA, herein a concentration is 1.8 mg/ml.

2. Experiment reagent:

6xDNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).
PowerPac Basic (Bio-rad), Mini PROTEAN Tetra Cell (Bio-rad), orbital shaker (TS-3D), and Tanon (Tanon 3500).

II. Experiment method

(1) Taking 2 μL of the DNAh-Biotin-Cy5-DHA nanoparticles, diluting with 4 μL of the RPMI 1640 medium containing 50% FBS, herein the concentration may reach 100 μg/ml after dilution, diluting by 5 tubes respectively, and placing the diluted sample at 37 DEG C of a water bath for different times (0, 10 min, 1 h, 12 h, and 36 h).

(2) Taking the treated sample 10μL and uniformly mixing with the 6xDNA Loading Buffer, operating on ice, and making a label.

(3) Taking 8% Native PAGE, applying a piece of the gel to the nanoparticle samples with the different incubation times, herein a loading amount is 12 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 12, herein 1 represents 0 min, 2 represents 10 min, 3 represents 1 h, 4 represents 12 h, and 5 represents 36 h. The target band of the DNAh-Bio-Cy5-DHA nanoparticles is about 200 bp. It may be seen from Fig. 12 that the DNAh-Bio-Cy5-DHA nanoparticles are basically stable after being incubated at 37 DEG C within 12 hours, and slight drug release or degradation occur after being incubated for more than 12 hours.

Embodiment 8

Cytotoxicity of DNAh-Biotin-Cy5-DHA nanoparticles in LN-229 cells

I. Experiment material and method

1. Cell information (see Table 43)

Table 43:

| Cell line | Cell type | Medium |
|---|---|---|
| LN-229 | Brain&Nerves | DMEM+5%FBS |

2. Samples to be tested (see Table 44)

Table 44:

| Name | Carrier mass (mg) | Carrier molecular weight | Solvent | Concent ration | Drug molecular weight | Volum e (μl) | Preserv ation |
|---|---|---|---|---|---|---|---|
| DNAh-Biotin-Cy5-DHA | 0.8 | 33342 | PBS | 48.47m M | 284.34 | 23 | -20 DEG C |
| DHA | 0.5 | - | DMSO | 80mM | 284.34 | 22 | -20 |
| (dihydroarte misinin) | | | | | | | DEG C |
| DNAh-Biotin-Cy5 | 0.317 | 33342 | PBS | 98.82μ M | - | 242.8 | -20 DEG C |

3. Reagents, consumables and devices

EnVision multi-label microplate detector, PerkinElmer, 2104-0010A;
Cell counter, Inno-Alliance Biotech, Countstar;
CO2 incubator, Thermo Scientific, Model 3100 Series;
Biological safety cabinet, Thermo Scientific, Model 1300 Series A2;
Inverted microscope, Olympus, CKX41SF;
Refrigerator, SIEMENS, KK25E76TI;
Cell culture conventional medium and consumables
Folic acid-free and biotin-free medium (provided by Baiyao Zhida Nano-biotechnology Co., Ltd)
Fetal Bovine Serum FBS (ExCell Bio., Cat# FND500)
CellTiter-Glo Luminescent Cell Viability Assay (Promega, Cat# G7573)
96-well black wall transparent flat bottom cell culture plate (Corning, Cat# 3340)

4. Quality control reference drug (see Table 45)

Table 45:

| Name | Molecular weight | Article number | Package | Supplier | Concent ration | Preservat ion |
|---|---|---|---|---|---|---|
| Cisplatin | 300.05 | E021881 AA | 50mg in 50mL | Hospira Australia Pty Ltd | 3.33 mM | RT |

II. Experiment method:

1) Harvesting cells in a logarithmic growth phase, taking a small amount and staining with trypan blue for counting to ensure that the cell viability reaches more than 80%;
2) adjusting the cell density to 2.22 × 104 /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 46 below;

Table 46:

| Test sample | Concentration gradient |
|---|---|
| DHA (dihydroartemisinin) | 400μM, 126.4μM, 40μM, 12.6μM, 4μM, 1.26μM, 400nM, 126nM, 40nM |
| DNAh-Biotin-Cy5-DHA | 400μM, 126.4μM, 40μM, 12.6μM, 4μM, 1.26μM, 400nM, 126nM, 40nM |
| DNAh-Bio- Cy5 | 0.99μM, 313nM, 99nM, 31.3nM, 9.9nM, 3.13nM, 0.99nM |
| Cisplatin | 100μM, 31.6μM, 10μM, 3.16μM, 1μM, 316nM, 100nM, 31.6nM, 10nM |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and then equilibrating at the room temperature for 30 minutes;
9) adding 50μL/well of the CellTiter 96® AQueous One Solution reagent to the cell culture plate;
10) placing the cell culture plate in the incubator at 37 DEG C and continuously incubating for 72 hours;
11) using the microplate reader to read a OD490 value of each well in the cell plate; and
12) data processing and analysis.

GraphPad Prism 5.0 software is used to process data graphically. In order to calculate the IC50, "S"-shaped non-linear regression analysis is performed on the data to match a suitable dosage-effect curve. A calculation formula of the cell survival rate is as follows, the IC50 may be automatically calculated in the GraphPad Prism 5.0.

Cell survival rate (%) = (OD test well-OD blank control) / (OD negative control-OD blank control) x 100%.

III. Experiment result (see Table 47, Fig. 13 and 14)

Table 47:

| Cell line | Test sample | IC50 (μM) |
|---|---|---|
| LN-229 | DHA (dihydroartemisinin) | 20.15 |

(continued)

| Cell line | Test sample | IC50 ($\mu$M) |
|---|---|---|
| | DNAh-Bio-Cy5-DHA | 67.95 |
| | DNAh-Bio-Cy5 | >0.99 |
| | Cisplatin | 12.52 |

It may be seen from Table 47 and Fig 13 and 14 that for the LN-229 cell line, compared with the simple DNAh targeted fluorescent carrier, the small molecular drug dihydroartemisinin (DHA) and DNAh drug-loaded particles DNAh-Bio-Cy5-DHA are both toxic to the LN-229 cells.

Embodiment 9

Vincristine loading experiment

(I) Chemical loading:

I. Experiment material and experiment method

1. Experiment materials and reagents:

(1) The DNA nucleic acid nanoparticles D-9 (molecular weight: 33342) formed by the self-assembly of the 9-th group of the DNA sequences in Embodiment 4 are used for loading. Specific information is shown in Embodiment 4.

(2) DEPC water: Biyuntian Company.

(3) PBS buffer solution: cellgro.

(4) 4% paraformaldehyde

(5) Vincristine.

(6) Chloroform: Beijing University of Chemical Technology.

(7) Anhydrous ethanol: Beijing University of Chemical Technology.

2. Experiment method:

(1) Accurately weighing the vincristine (1.354$\mu$mol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the nucleic acid nanoparticles D-8 (33.84 nmol), and reacting for 72 hours at 4 DEG C under a dark condition.

(2) Taking 10 $\mu$L of reaction solution and diluting for 10 times, using 50 $\mu$M of the vincristine aqueous solution and 310 ng/$\mu$L of the nucleic acid nanoparticles D-8 as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), evaporating a solvent under reduced pressure and lower temperature, to obtain a solid product of vincristine-DNAh particles

(4) Vincristine-DNAh loading rate measurement:

1. preparing a known concentration of vincristine-absolute ethyl alcohol standard solution: 20 uM, 40 uM, 60 uM, 80 uM, and 100 uM, 100 μl each;

2. enabling vincristine-DNAh particles to be dissolved in 100 μl of the PBS;

3. putting the standard solution and the vincristine-DNAh particles into a PCR plate, heating at 85 DEG C for 5 min, and then cooling to the room temperature;

4. measuring an absorbance of the vincristine at 297 nm by using a NanoDrop micro spectrophotometer, drawing a standard curve (as shown in Fig. 15), to obtain a pseudo-recursive curve function formula and a value R, and calculating the molar concentration of the vincristine in the loaded product according to the function formula;

5. measuring a DNA absorbance of the loaded product at 260 nm by using the NanoDrop micro spectrophotometer, to obtain the mass concentration of the DNAh particles thereof, and converting into the molar concentration according to the molecular weight; and

6. according to the vincristine molar concentration and the mass concentration of the vincristine-DNAh particles obtained by measuring, calculating a loading rate.

A calculating process is specifically as follows.
The vincristine molar concentration in the loaded product is as follows:
(297nm vincristine absorbance -0.003)/0.002=(0.364-0.003)/0.002=180.50 μM.
The DNAh molar concentration in the loaded product is as follows:
Mass concentration/molecular weight of DNAh particles=732.4/(33342x10e3)=21.97 μM.
Loading rate=vincristine molar concentration in loaded product/DNAh molar concentration in loaded product=180.50 μM/21.97 μM=8.2.
An average value is taken, it is obtained that the loading rate of vincristine-DNAh particles is 8.2, it means that about 8.2 vincristine may be loaded on each DNA nanoparticle carrier.
In addition, on the basis of the DNAh nanoparticles loading the vincristine, other small molecular drugs may be further loaded for the second time according to the same method as that of loading the vincristine. For example, a folic acid is further loaded in the disclosure, to obtain DNA nanoparticles co-loaded with two small molecular drugs of the vincristine and folic acid, and the loading rates of the two drugs may be detected by referring to the above method (values are not shown).
It is indicated from Embodiment 9 that the DNA nanoparticles with the extension fragment, the target head and the fluorescein have a function of loaded drugs, the small molecular drug vincristine may achieve the loading in a mode of covalent linkage (paraformaldehyde-solvent covalence), and may also achieve the co-loading with other small molecular drugs.

Embodiment 10

Cell binding ability of vincristine drug-loaded DNA nanoparticles detected by flow cytometry

I. Cell information (see Table 48 below)

Table 48:

| Cell line | Medium | Culture condition |
|---|---|---|
| HepG-2 | (MEM+0.01 Mm NEAA) +10% FBS | 37 DEG C, 5% $CO_2$, saturated humidity |

II. Samples to be tested

Vincristine targeted drug: DNAh-Biotin-Cy5-vincristine (loaded product of the DNA nanoparticles in Embodiment 9).

Targeted fluorescent carrier: DNAh-Biotin-Cy5 (namely the nanoparticles D-9).

III. Instrument, device and related reagent information (see Table 49 and Table 50)

Table 49:

| Name | Brand | Article number / Model |
|---|---|---|
| 24-well cell culture plate | Corning | 3526 |
| Centrifuge | Eppendorf | 5810R |
| $CO_2$ incubator | Thermo | 3100 |
| Microplate shaker | QILINBEIER | QB-9001 |
| Inverted microscope | Olympus | CKX41SF |
| Multifunctional microplate reader | Bio Tek | Synergy H1 |
| Flow cytometer | BD | Accuri C6 |
| Cell counter | Inno-Alliance Biotech | Countstar |
| Biological safety cabinet | Thermo | 1300A2 |

Table 50:

| Name | Brand | Article number |
|---|---|---|
| MEM+0.01 mM NEAA medium(Folic acid and biotin-free medium) | provided by Baiyao Zhida Nano-biotechnology Co., Ltd | YS4150-500 |
| Fetal bovine serum FBS | ExCell Bio | FND500 |
| GlutaMax | Gibco | 35050-061 |
| Trypsin-EDTA digestive juice (0.25%) | Solarbio | T1320-100ml |

IV. Experiment method:

1) Adjusting a cell state to a logarithmic growth phase, changing the medium to a medium without biotin and folic acid, and incubating overnight in the incubator at 37 DEG C;

2) digesting and collecting single-cell suspension and counting, adjusting a cell density to 2x105/mL, and planting 1 mL/well into the 24-well plate;

3) dissolving a substance to be tested and preparing stock solution of the substance to be tested;

4) respectively adding the substance to be tested to the corresponding cell wells, herein a final concentration is 1.98 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 200 μL of the PBS, and performing a flow cytometry on-machine detection (using 650 nm of an excitation wavelength and 670 nm of an emission wavelength of Cy5); and

9) data analysis, an analysis result is shown in Table 51.

Table 51:

| Cell line | Test sample | Binding rate (%) |
|---|---|---|
| HepG-2 | DNAh-Bio-Cy5-vincristine | 97.3 |
| | DNAh-Bio-Cy5 | 96.7 |
| | Blank control (PBS) | 0 |

It may be seen from Table 51 that, compared with the PBS blank control, after the DNAh-Bio-Cy5 and DNAh-Bio-Cy5-vincristine at a working concentration of 1.98 μM are incubated with the HepG-2 cells for 2 h, a proportion of the number of the Cy5 positive binding cells detected by using the excitation and emission wavelengths of the Cy5 accounting for the number of the cells in the FSC-SSC gate is 96.7% and 97.3% respectively. It may be seen that the DNA nucleic acid nanoparticles carrying the target head and the small molecular drug vincristine have a high binding rate after being incubated with the HepG-2 cells, may efficiently bind and internalize with the HepG-2 cells in the human liver cancer cell line, and have an application prospect for the treatment of the liver cancer.

Embodiment 11

Stability detection of DNAh-Bio-Cy5-vincristine nanoparticles in serum

I. Experiment material, reagent and device

1. Experiment material:

Sample to be tested: DNAh-Biotin-Cy5-vincristine, herein a concentration is 1000μg/ml.

2. Experiment reagent:

6xDNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 2 μL of the DNAh drug-loaded nanoparticles, diluting with 4 μL of the 50% FBS 1640 and 14μL of the RPMI 1640, herein the concentration may reach 100 μg/ml after dilution, diluting by 5 tubes respectively, and placing the diluted sample at 37 DEG C of a water bath for different times (0, 10 min, 1 h, 12 h, and 36 h).

(2) Taking 10 μl of the treated sample and uniformly mixing with 2 μl of the 6xDNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 16, herein 1 represents 0 min, 2 represents 10 min, 3 represents 1 h, 4 represents 12 h, and 5 represents 36 h. The target band of the DNAh-Bio-Cy5-vincristine nanoparticles is about 200 bp. It may be seen from Fig. 16 that the DNAh-Bio-Cy5-vincristine nanoparticles are basically stable after being incubated at 37 DEG C for 36 h.

Embodiment 12

Cytotoxicity of DNAh-Biotin-Cy5-vincristine nanoparticles in HepG-2 cells

I. Experiment material and method

1. Cell information (see Table 52)

Table 52:

| Cell line | Medium | Culture condition |
|---|---|---|
| HepG-2 | (MEM+0.01 Mm NEAA) +10%FBS | 37 DEG C, 5%$CO_2$, 95% humidity |

2. Samples to be tested and quality control reference substances (see Table 53)

Table 53:

| Name | Molecular weight | Mass (mg) | Solvent | Concentration | Treatment time (hrs) | Preservation condition |
|---|---|---|---|---|---|---|
| DNAh-Biotin-Cy5-vincristine | 34166.96 | 0.15 | PBS | 7.9mM | 96 | -20 DEG C |
| Vincristine | 824.96 | 1.4 | DMSO | 20 mM | 96 | -20 DEG C |
| DNAh-Biotin-Cy5 | 33342 | 0.8 | PBS | 98.82μM | 96 | -20 DEG C |
| Cisplatin | 300.05 | / | / | 3.33 mM | / | Room temperature |

3. Consumables and devices (see Table 54):

Table 54:

| Name | Brand | Article number / Model |
|---|---|---|
| 96-well plate | Corning | 3340 |
| Centrifuge | Gineek | LD5-2B |
| $CO_2$incubator | Thermo | 3100 |
| Microplate shaker | QILINBEIER | QB-9001 |
| Inverted microscope | Olympus | IX53 |
| EnVision Multi-label microplate detector | PerkinElmer | 2104-0010A |
| Cell counter | Inno-Alliance Biotech | Countstar |

(continued)

| Name | Brand | Article number / Model |
|---|---|---|
| Biological safety cabinet | Thermo | 1300 A2 |

4. Reagents (see Table 55):

Table 55:

| Name | Brand | Article number |
|---|---|---|
| MEM+0.01 mM NEAA medium (without folic acid and biotin) | provided by Baiyao Zhida Nano-biotechnology Co., Ltd | YS4150-500 |
| FBS | Cegrogen | A0500-3018 |
| GlutaMax | Gibco | 35050-061 |
| PBS | ExCell Bio | FND500 |
| DMSO | Solarbio | D8371 |
| Trypsin-EDTA digestive juice (0.25%) | Solarbio | T1320-100ml |
| CellTiter 96® AQueous One Solution | Promega | G7573 |

II. Experiment method:

1) Harvesting cells in a logarithmic growth phase, taking a small amount and staining with trypan blue for counting to ensure that the cell viability reaches more than 80%;
2) adjusting the cell density to 2.22 × 104 /mL with a growth medium without folic acid and biotin;
3) planting 90 μL/well of cell suspension into the 96-well plate, herein the number of cells per well in the plate is 2000;
4) placing the planted cell plate in the incubator at 37 DEG C and incubating overnight under conditions of 5% CO2 and 95% humidity;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 56 below;

Table 56:

| Test sample | Concentration gradient |
|---|---|
| Small molecular drug | 100μM, 31.6μM, 10μM, 3.16μM, 1μM, 316nM, 100nM, 31.6nM, 10nM, 0 (medium) |
| DNAh-Bio-Cy5-small molecular drug | 100μM, 31.6μM, 10μM, 3.16μM, 1μM, 316nM, 100nM, 31.6nM, 10nM, 0 (medium) |
| DNAh-Bio-Cy5 | 4.9μM, 1.55μM, 490.5nM, 158nM, 155.2nM, 49nM, 15.5nM, 4.9nM,1.55 nM, 0 (medium) |
| Cisplatin | 100μM, 31.6μM, 10μM, 3.16μM, 1μM, 316nM, 100nM, 31.6nM, 10nM, 0 (medium) |

7) placing the cell culture plate in the incubator and continuously incubating for 96 hours;
8) placing the CellTiter-Glo reagent and the drug treatment cell culture plate at the room temperature and equilibrating for 30 minutes;
9) adding 50 μL of the CellTiter-Glo reagent to each well;
10) shaking in the orbital shaker for 2 minutes so that the cells are adequately lysed;
11) placing the cell culture plate at the room temperature and equilibrating for 10 minutes, reading a chemiluminescence value by using EnVision; and

12) data processing and analysis.

GraphPad Prism 5.0 software is used to process data graphically. Nonlinear S-curve regression is used to fit the data to obtain a dosage-effect curve, and an IC50 value is calculated from this.

Cell survival rate (%) = (Lumdrug to be tested-Lummedium control)/(Lumcell control-Lummedium control)×100%.

Lumcell control-Lummedium control is set to 100%, and a LumMedium control value is set to 0%.

III. Experiment result (see Table 57 and 58, Fig. 17a to 17b)

Table 57:

| Cell line | Test sample | $IC_{50}$ (μM) |
|---|---|---|
| HepG2 | Vincristine | 1.68 |
| | DNAh-Bio-Cy5-vincristine | 5.96 |
| | DNAh-Bio- Cy5 | >4.9 |
| | Cisplatin | 3.99 |

Table 58:

| Cell line | Test sample | Inhibition rate of cell proliferation at highest concentration itself (%) |
|---|---|---|
| HepG2 | Vincristine | 70.71% |
| | DNAh-Bio-Cy5-vincristine | 68.32% |
| | DNAh-Bio-Cy5 | 3.05% |
| | Cisplatin | 99.36% |

It may be seen from Table 57 and 58 and Fig. 17a and 17b that for the HepG2 cell line, compared with the simple DNAh targeted fluorescent carrier, the small molecular drug vincristine and DNAh drug-loaded particles DNAh-Bio-Cy5-vincristine are both toxic to the HepG2 cells. In addition, at the highest administration concentration of 100 μM, the small molecular drug vincristine and the DNAh-Bio-Cy5-vincristine have the similar inhibition rates to the HepG2 cell proliferation, which are 70.71% and 68.32%, respectively.

In addition, it may be seen from Fig. 17a and Table 58, the simple DNAh targeted fluorescent carrier DNAh-Bio-Cy5 is not toxic to the cells at the highest administration concentration of 100 μM (the inhibition rate is only 3.05%).

Embodiment 13

Flavone loading experiment

(I) Chemical loading:

I. Experiment material and experiment method

1. Experiment materials and reagents:

(1) The nucleic acid nanoparticles (molecular weight: 29550): similar to the RNA nanoparticles in Embodiment 1, a difference is that the fluorescence labeling on the c-strand is Cy5.

(2) Flavone (CAS: 525-82-6, molecular formula: C15H10O2, and molecular weight: 222.24).

(3) solvent solution such as insertion buffer, chloroform, and ethyl alcohol.

2. Experiment method:

(1) Accurately weighing the flavone (200 um);

(2) accurately weighing the RNAh (0.1 mg, 1eq2um);

(3) preparing the insertion buffer (0.1M sodium acetate, 0.05M NaCl, 0.1M MgCl2) and taking (1ml);

(4) firstly dissolving the RNAh particles in the insertion buffer, stirring and mixing uniformly, then putting the flavone in it, and continuously stirring at the room temperature for 6 hours in the dark;

(5) then using a Saphadex G50 spin column to remove the free flavone from the system (or extracting the reaction solution with the chloroform (2xvolumex2)), and then adding the absolute ethyl alcohol (10×volume) to precipitate at 4 degrees for 4 hours;

(6) washing with a small amount of the absolute ethyl alcohol (1ml×), and evaporating to dry under low temperature and reduced pressure to obtain flavone-RNAh nanoparticles. (7) Loading rate calculation:

1. preparing a known concentration of flavone-methyl alcohol standard solution: 2 μM, 4 μM, 6 μM, 8 μM, and 10 μM, 100 μl each;

2. enabling flavone-RNAh particles to be dissolved in 100 μl of the PBS;

3. putting the standard solution and the flavone-RNAh particles into a PCR plate, heating at 85 DEG C for 5 min, and then cooling to the room temperature;

4. measuring an absorbance of the flavone at 283 nm by a microplate reader, drawing a standard curve, and calculating the molar concentration of the flavone in the loaded product;

5. measuring an absorbance of RNA at 260 nm by a spectrophotometer, to obtain the mass concentration of the RNAh particles contained in each sample; and

6. according to the flavone molar concentration and the mass concentration of the RNAh particles obtained by measuring, calculating a loading rate.

A standard curve of the RNA nucleic acid nanoparticles loading the flavone is shown in Fig. 18, and a specific calculation process is as follows:

CRNAh-1 = 46.0 ug/ml, MRNAh ≈30000, 100μl; C flavone -1 = 7.6 μM, 100μl;

CRNAh-2 = 39.0 ug/ml, $M_{RNAh}$ ≈30000, 100μl; C flavone -1 = 6.92 μM, 100μl;

$$\text{N-2}=\frac{6.72\times 10\text{-}6\times 100\times 10\text{-}6}{0.0390\times 100\times 10\text{-}6/30000}=5.3$$

$$\text{N-1}=\frac{7.6\times 10\text{-}6\times 100\times 10\text{-}6}{0.0460\times 100\times 10\text{-}6/30000}=4.98$$

An average value thereof is taken, it is obtained that the loading rate of the flavone-RNAh nucleic acid nanoparticles is about 5, and it represents that about 5 flavone molecules may be loaded on each nucleic acid nanoparticle

carrier.

In addition, on the basis of the RNA nanoparticles loading the flavone, other small molecular drugs may be further loaded for the second time according to the same method as that of loading the flavone. For example, a folic acid is further loaded in the disclosure, to obtain RNA nanoparticles co-loaded with two small molecular drugs of the flavone and folic acid, and the loading rates of the two drugs may be detected by referring to the above method (values are not shown).

It is indicated from Embodiment 13 that the RNA nanoparticles (in Embodiment 1) with the extension fragment, the target head and the fluorescein have a function of loaded drugs, the small molecular drug flavone may achieve the loading in a mode of covalent linkage (paraformaldehyde-solvent covalence).

Embodiment 14

Cell binding ability of flavone drug-loaded DNA nanoparticles detected by flow cytometry experiment

I. Cell information

Hela (source: ATCC, and article number: CCL-2), a medium is DMEM + 10% FBS, and culture conditions are 37 DEG C, 5% CO2, and saturated humidity.

II. Substances to be tested

Targeted fluorescent carrier: DNAh-Bio-EGFRapt-Cy5 (namely, nanoparticles D-8 formed by the self-assembly of the 8-th group of the sequences in Embodiment 4).

Targeted drug: DNAh-Bio-EGFRapt-Cy5-flavone (loaded according to the loading method of the RNA nanoparticles in Embodiment 13).

III. Devices and consumables (see Table 59)

Table 59:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents (see Table 60)

Table 60:

| Name | Brand | Article number |
|---|---|---|
| DMEM medium (without folic acid and biotin) | provided by Baiyao Zhida Nano-biotechnology Co., Ltd | YS1200-500 |
| Fetal bovine serum FBS | Cegrogen | A0500-3018 |
| GlutaMax | Gibco | 35050-061 |
| Trypsin-EDTA digestive juice (0.25%) | Solarbio | T1320-100ml |

V. Experiment method:

1) Adjusting a cell state to a logarithmic growth phase, changing the medium to a medium without biotin and folic acid, and incubating overnight in the incubator at 37 DEG C;

2) digesting and collecting single-cell suspension and counting, adjusting a cell density to 2×105/mL, and planting 1 mL/well into the 24-well plate;

3) dissolving a substance to be tested and preparing stock solution of the substance to be tested;

4) respectively adding the substance to be tested to the corresponding cell wells, herein a final concentration is 0.2 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection;

9) fluorescent carrier or flavone detection channels: excitation light wavelength: 488 nm, and emission light wavelength: 560 nm; and

10) data analysis.

VI. Experiment result (see Table 61)

Table 61:

| Cell line | Test sample | Binding rate (%) |
|---|---|---|
| Hela | DNAh-Bio-EGFRapt-Cy5-flavone | 99.99 |
| | DNAh-Bio-EGFRapt-Cy5 | 100 |
| | Blank control (cell-only medium) | 0.18 |

It may be seen from Table 61 that the flavone targeted drug DNAh-Bio-EGFRapt-Cy5-flavone may bind to the Hela cells, and the binding rate is 99.99%, which is almost 100%; and the targeted fluorescent carrier DNAh-Bio-EGFRapt-Cy5 may also bind to the Hela cells, the binding rate is 100%, and the binding rates of two parties are both extremely high.

Embodiment 15

Stability detection of DNAh-Bio-Cy5-flavone nanoparticles in serum

I. Experiment material, reagent and device

1. Experiment material:

DNAh-Bio-EGFRapt-Cy5-flavone, herein a concentration is 9764.811 μg/ml.

2. Experiment reagent:

6xDNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 2 μL of the DNAh-Bio-EGFRapt-Cy5-flavone nanoparticles, diluting with the medium containing 19.5μL of 50% FBS 1640 + 76.1μL of RPMI 1640, herein the concentration may reach 200 μg/ml after dilution, diluting by 5 tubes respectively, and placing the diluted sample at 37 DEG C of a water bath for different times (0, 10 min, 1 h, 12 h, and 36 h).

(2) Taking 5 μl of the treated sample and uniformly mixing with the 6xDNA Loading Buffer, operating on ice, and making a label.

(3) Taking 8% Native PAGE, applying a piece of the gel to the nanoparticle samples with the different incubation times, herein a loading amount is 12 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 19, herein 1 represents 0 min, 2 represents 10 min, 3 represents 1 h, 4 represents 12 h, and 5 represents 36 h. The target band of the DNAh-Bio-EGFRapt-Cy5-flavone nanoparticles is below 200 bp. It may be seen from Fig. 19 that the DNAh-Bio-EGFRapt-Cy5-flavone nanoparticles are basically stable after being incubated at 37 DEG C within 0-1 h; and partial degradation of the sample occurs after being incubated for 12 h and 36 h.

Embodiment 16

Cytotoxicity of DNAh-Bio-EGFRapt-Cy5-flavone nanoparticles in Hela cells

I. Experiment material

1. Cell information (see Table 62)

Table 62:

| Name | Source | Medium | Culture condition |
|---|---|---|---|
| Hela | ATCC | DMEM, 10%FBS | 37°C, 5%$CO_2$, saturated humidity |

2. Samples to be tested (see Table 63):

| Name | Carrier mass (mg) | Carrier molecular weight | Drug loaded mass (mg) | Drug molecular weight | Character | Preservation condition |
|---|---|---|---|---|---|---|
| DNAh-Bio-EGFR apt-Cy5-flavone | 0.375 | 39493 | 0.026 | 222.24 | Blue liquid | -20 DEG C |
| Flavone | - | - | ~5mg | 222.24 | Blue solid | -20 DEG C |
| DNAh-Bio-EG FRapt-Cy5 | 1 | 39493 | - | - | Blue liquid | -20 DEG C |

3. Consumables and devices (see Table 64):

Table 64:

| Name | Brand | Article number / Model |
|---|---|---|
| 96-well plate | Corning | 3599 |
| Centrifuge | Gineek | LD5-2B |
| $CO_2$incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 65):

Table 65:

| Name | Brand | Article number |
|---|---|---|
| DMEM medium (without folic acid and biotin) | provided by Baiyao Zhida Nano-biotechnology Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to 2.22 × 104 /mL with a growth medium;
3) planting 90 μL/well of cell suspension into the 96-well plate, herein the number of cells per well in the plate is 5000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action

concentration is shown in Table 66 below;

Table 66:

| Test sample | Concentration gradient |
|---|---|
| Flavone | 200μM, 63.2μM, 20μM, 6.32μM, 2μM, 632nM, 200nM, 63.2nM, 20nM, 0 (10%PBS) |
| DNAh-Bio-EGFRapt-Cy5-flavone | 249μM, 78.8μM, 24.9μM, 7.88μM, 2.49μM, 788nM, 249nM, 78.8nM, 24.9nM, 0 (10%PBS) |
| DNAh-Bio-EGFRapt-Cy5 | 2μM, 632nM, 200nM, 63.2nM, 20nM, 6.32nM, 2nM, 0 (10%PBS) |
| DMSO | 1%, 0.5%, 0.25%, 0.125%, 0.06%, 0.03%, 0 |

7) placing the cell culture plate in the incubator and continuously incubating for 72 hours;
8) placing the CellTiter 96® AQueous One Solution reagent at the room temperature and thawing for 90 minutes or thawing in a water bath at 37 DEG C, placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a OD490 value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula of the survival rate is as follows, the IC50 may be automatically calculated in GraphPad Prism 5.0.

Cell survival rate (%) = (OD test well-OD blank control) / (OD negative control-OD blank control) × 100%.

III. Experiment result (see Table 67, Fig. 20a to 20d)

Table 67:

| Cell line | Test sample | $IC_{50}$ (μM) |
|---|---|---|
| Hela | Flavone | 12.59 |
| | DNAh-Bio-EGFRapt-Cy5-flavone | 23.56 |
| | DNAh-Bio-EGFRapt-Cy5 | >2 |
| | DMSO | >1% |

It may be seen from Table 67 and Fig. 20a, 20b, 20c, and 20d that for the Hela cell line, compared with the simple targeted fluorescent carrier DNAh-Bio-EGFRapt-Cy5, the small molecular drug flavone and DNAh drug-loaded particles DNAh- Bio-EGFRapt-Cy5-flavone are both toxic to the HeLa cells, and the IC50 drug concentration of DNAh drug-loaded particles DNAh-Bio-EGFRapt-Cy5-flavone is greater than the IC50 drug concentration of the small molecular drug flavone. The IC50 of flavone and DNAh-Bio-EGFRapt-Cy5-flavone acting on the Hela cells is 12.59 μM and 23.56 μM, and the IC50 of the targeted fluorescent carrier DNAh-Bio-EGFRapt-Cy5 and DMSO acting on the Hela cells is >0.2μM and > 1%, respectively.

Embodiment 17

Oxaliplatin loading experiment

(I) Chemical loading:

I. Experiment material and experiment method

1. Experiment materials and reagents:

(1) The DNA nucleic acid nanoparticles D-8 formed by the self-assembly of the 8-th group of the DNA sequences in Embodiment 4 are used for loading. Specific information is shown in Embodiment 4.

(2) DEPC water: Biyuntian Company.

(3) PBS buffer solution: cellgro.

(4) 4% paraformaldehyde

(5) Oxaliplatin.

(6) Chloroform: Beijing University of Chemical Technology.

(7) Anhydrous ethanol: Beijing University of Chemical Technology.

2. Experiment method:

(1) Accurately weighing the oxaliplatin (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the nucleic acid nanoparticles D-8 (33.84 nmol), and reacting for 72 hours at 4 DEG C under a dark condition.

(2) Taking 10 μL of reaction solution and diluting for 10 times, using 50 μM of the oxaliplatin aqueous solution and 310 ng/μL of the nucleic acid nanoparticles D-8 as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), evaporating a solvent under reduced pressure and lower temperature, to obtain a solid product of oxaliplatin-DNAh particles

(4) Oxaliplatin-DNAh loading rate calculation:

1. preparing a known concentration of oxaliplatin-methyl alcohol standard solution: 2 μM, 4 μM, 6 μM, 8 μM, and 10 μM, 100 μl each;

2. enabling oxaliplatin-DNAh particles to be dissolved in 100 μl of the PBS;

3. putting the standard solution and the oxaliplatin-DNAh particles into a PCR plate, heating at 85 DEG C for 5 min, and then cooling to the room temperature;

4. measuring an absorbance of the oxaliplatin at 210 nm by using a microplate reader, drawing a standard curve

(as shown in Fig. 21), and calculating the molar concentration of the oxaliplatin in the loaded product;

5. measuring an absorbance of DNA at 260 nm by using a spectrophotometer, to obtain the mass concentration of the DNAh particles contained in each sample; and

6. according to the oxaliplatin molar concentration and the mass concentration of the oxaliplatin-DNAh particles obtained by measuring, calculating a loading rate.

A calculation process is specifically as follows:

CRNAh-1 = 1048 μg/ml, $M_{RNAh}$ ≈30000, 100μl; C oxaliplatin -1 = 60.2 μM, 100μl;

CRNAh-2 = 12.39 μg/ml, $M_{RNAh}$ ≈30000, 100μl; C oxaliplatin -2 = 102.7 μM, 100μl;

$$N\text{-}1 = \frac{60.2 \times 10\text{-}6 \times 100 \times 10\text{-}6}{0.0457 \times 100 \times 10\text{-}6/30000} = 39.6,$$

$$N\text{-}2 = \frac{102.7 \times 10\text{-}6 \times 100 \times 10\text{-}6}{0.0539 \times 100 \times 10\text{-}6/30000} = 57.1.$$

An average value thereof is taken, it is obtained that the loading rate of the oxaliplatin-DNAh particles is about 48, it represents that about 48 oxaliplatin may be loaded on each DNA nanoparticle carrier.

In addition, on the basis of the DNA nanoparticles loading the oxaliplatin, other small molecular drugs may be further loaded for the second time according to the same method as that of loading the oxaliplatin. For example, a folic acid is further loaded in the disclosure, to obtain DNA nanoparticles co-loaded with two small molecular drugs of the oxaliplatin and folic acid, and the loading rates of the two drugs may be detected by referring to the above method (values are not shown).

It is indicated from Embodiment 17 that the DNA nanoparticles with the extension fragment, the target head and the fluorescein have a function of loaded drugs, the small molecular drug oxaliplatin may achieve the loading in a mode of covalent linkage (paraformaldehyde-solvent covalence), and may also achieve the co-loading with other small molecular drugs.

Embodiment 18

Cell binding ability of oxaliplatin drug-loaded DNA nanoparticles detected by flow cytometry

I. Cell information (see Table 68 below)

Table 68:

| Cell line | Source | Medium | Culture condition |
|---|---|---|---|
| SGC-7901 | Bolise Co. | DMEM + 10% FBS | 37 DEG C, 5% $CO_2$, saturated humidity |

II. Samples to be tested

Oxaliplatin targeted drug: DNAh-Biotin- EGFRapt-Cy5-oxaliplatin (loaded product of the DNA nanoparticles in Embodiment 17).

Targeted fluorescent carrier: DNAh-Bio-EGFRapt-Cy5.

III. Instrument, device and related reagent information (see Table 69 and Table 70)

Table 69:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

Table 70:

| Name | Brand | Article number |
|---|---|---|
| DMEM medium (Folic acid and biotin-free medium) | provided by Baiyao Zhida Nano-biotechnology Co., Ltd | YS4150-500 |
| Fetal bovine serum FBS | Cegrogen | A0500-3018 |
| GlutaMax | Gibco | 35050-061 |
| Trypsin-EDTA digestive juice (0.25%) | Solarbio | T1320-100ml |

IV. Experiment method:

1) Adjusting a cell state to a logarithmic growth phase, changing the medium to a medium without biotin and folic acid, and incubating overnight in the incubator at 37 DEG C;

2) digesting and collecting single-cell suspension and counting, adjusting a cell density to 2x105/mL, and planting 1 mL/well into the 24-well plate;

3) dissolving a substance to be tested and preparing stock solution of the substance to be tested;

4) respectively adding the substance to be tested to the corresponding cell wells, herein a final concentration is 2 μM, shaking and mixing uniformly;

5) placing the cell plate in the incubator at 37 DEG C and incubating for 16 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection; and

9) data analysis, an analysis result is shown in Table 71.

Table 71:

| Cell line | Test sample | Binding rate (%) |
|---|---|---|
| SGC-7901 | DNAh-Bio-EFGRapt-Cy5-oxaliplatin | 99.97 |
| | DNAh-Bio-EFGRapt-Cy5 | 99.99 |
| | Blank control (medium only) | 0.34 |

It may be seen from Table 71 that compared with the blank control, the DNA nucleic acid nanoparticles carrying the target head and the small molecular drug oxaliplatin have a high binding rate after being incubated with the SGC-7901 cells, it shows that it may efficiently bind and internalize with the SGC-7901 cells in the human gastric cancer cell line, and has an application prospect for the treatment of the gastric cancer.

Embodiment 19

Stability detection of DNAh-Bio-EGFRapt-Cy5-oxaliplatin nanoparticles in serum

I. Experiment material, reagent and device

1. Experiment material:

Sample to be tested: DNAh-Bio-EGFRapt-Cy5-oxaliplatin, herein a concentration is 10 mg/ml.

2. Experiment reagent:

6xDNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 3 μL of the DNAh drug-loaded nanoparticles, diluting with 146.9μL of the RPMI 1640 medium containing 10% of serum, herein the concentration may reach 197.5 μg/ml after dilution, diluting by 5 tubes respectively, and placing the diluted sample at 37 DEG C of a water bath for different times (0, 10 min, 1 h, 12 h, and 36 h).

(2) Taking 20 μl of the treated sample and uniformly mixing with 4 μl of the 6xDNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 22, herein 1 represents 0 min, 2 represents 10 min, 3 represents 1 h, 4 represents 12 h, and 5 represents 36 h. The target band of the DNAh-Bio-EGFRapt-Cy5-oxaliplatin nanoparticles is about 200 bp. It may be seen from Fig. 22 that the DNAh-Bio-EGFRapt-Cy5-oxaliplatin nanoparticles are basically stable after being incubated at 37 DEG C for 36 h (slight drug release or degradation occurs after 36 h).

Embodiment 20

Cytotoxicity of DNAh-Biotin-EGFRapt-Cy5-oxaliplatin nanoparticles in SGC-7901 cells

I. Experiment material and method

1. Cell information (see Table 72)

Table 72:

| Cell line | Source | Medium | Culture condition |
|---|---|---|---|
| SGC-7901 | Bolise Co. | DMEM + 10% FBS | 37 DEG C, 5%$CO_2$, saturated humidity |

2. Samples to be tested (see Table 73)

Table 73:

| Name | Carrier mass (mg) | Carrier molecular weight | Drug loaded mass (mg) | Drug molecular weight | Character | Preservation condition |
|---|---|---|---|---|---|---|
| DNAh-Biotin-EGFR apt-Cy5-oxaliplatin | 0.9 | 39493 | 0.250 | 497.49 | Blue solid | -20 DEG C |
| Oxaliplatin | - | - | 2 | 497.49 | White powder | -20 DEG C |
| DNAh-Biotin-EGFR apt-Cy5 | 1.25*3 | 39485.9 | - | - | Blue solid | -20 DEG C |

3. Consumables and devices (see Table 74):

Table 74:

| Name | Brand | Article number / Model |
|---|---|---|
| 96-well plate | Corning | 3599 |
| Centrifuge | Gineek | LD5-2B |
| $CO_2$incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 75):

Table 75:

| Name | Brand | Article number |
|---|---|---|
| DMEM medium | provided by Baiyao Zhida | YS4150-500 |

(continued)

| Name | Brand | Article number |
|---|---|---|
| (without folic acid and biotin) | Nano-biotechnology Co., Ltd | |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to 2.22 × 104 /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 76 below;

Table 76:

| Test sample | Concentration gradient |
|---|---|
| Small molecular drug | 100μM, 31.6μM, 10μM, 3.16μM, 1μM, 316nM, 100nM, 31.6nM, 10nM, 0 (10%PBS) |
| DNAh-Bio-EGFRapt-Cy5-small molecular drug | 350μM, 111μM, 35μM, 11.1μM, 3.5μM, 1.11μM, 350nM, 111nM, 35nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a OD490 value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula of the survival rate is as follows, the IC50 may be automatically calculated in GraphPad Prism 5.0.

Cell survival rate (%) = (OD test well-OD blank control) / (OD negative control-OD blank control) x 100%.

III. Experiment result (see Table 77, Fig. 23a to 23d)

Table 77:

| Cell line | Test sample | $IC_{50}$ (μM) |
|---|---|---|
| SGC-7901 | Oxaliplatin | 55.81 |
| | DNAh-Bio-EGFRapt-Cy5-oxaliplatin | 16.35 |
| | DNAh-Bio-EGFRapt-Cy5 | >0.316 |
| | DMSO | >1% |

It may seen from Table 11 and Fig. 23a, 23b, 23c, and 23d that for the SGC-7901 cell line, compared with the simple DNAh targeted fluorescent carrier, the small molecular drug oxaliplatin and DNAh drug-loaded particles DNAh-Bio-EGFRapt-Cy5-oxaliplatin are both toxic to the SGC-7901 cells. In addition, compared with the small molecular drug oxaliplatin, the IC50 of DNAh-Bio-EGFRapt-Cy5-oxaliplatin is reduced by at least 2/3, it shows that the toxicity of the oxaliplatin targeted drug to the SGC-7901 cells is stronger. Accordingly, an amount required to obtain the same inhibitory effect is greatly reduced.

Embodiment 21

Cisplatin (DDP) loading experiment

(I) Chemical loading:

I. Experiment material and experiment method

1. Experiment materials and reagents:

(1) Nucleic acid nanoparticles (a molecular weight is 28752): similar to the RNA nanoparticles in Embodiment 1, and a difference is that the fluorescence labeling on the c-strand is Cy5.

(2) DEPC water: Biyuntian Company.

(3) PBS buffer solution: cellgro.

(4) 4% paraformaldehyde

(5) Cisplatin.

(6) Chloroform: Beijing University of Chemical Technology.

(7) Anhydrous ethanol: Beijing University of Chemical Technology.

2. Experiment method:

(1) Accurately weighing the cisplatin (0.79 mg, 1.354 μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the RNA nanoparticles (1 mg, 33.84 nmol), and reacting for 72 hours at 4 DEG C under a dark condition.

(2) Taking 10 μL of reaction solution and diluting for 10 times, using 50 μM of the cisplatin aqueous solution and 310 ng/μL of the RNA nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), and evaporating a solvent under reduced pressure and lower temperature, to obtain a dark red solid product.

(4) Loading rate calculation:

1. Firstly dissolving 20 mM of the cisplatin in the DMSO, and preparing a known concentration of cisplatin-PBS standard solution: 1 mM, 2 mM, 4 mM, and 8 mM, 100 μl each;

2. enabling cisplatin-RNAh particles to be dissolved in 100 μl of the PBS;

3. putting the standard solution and the cisplatin-RNAh particles into a PCR plate, heating at 95 DEG C for 5 min, and then cooling to the room temperature;

4. because the maximum light absorption of the cisplatin (301 nm) and the detection wavelength of the DNA (260 nm) are very approximate, they are mutually overlapped to affect each other. Therefore, the RNAh particles and cisplatin in the loaded product after the heat treatment should be separated by using centrifugal ultrafiltration (3K MWCO), after that, the RNAh concentration and the cisplatin concentration are measured respectively;

5. measuring an absorbance of the cisplatin at 301 nm with a NanoDrop spectrophotometer, drawing a standard curve (see Fig. 24), to obtain a function formula of a pseudo-recursive curve and a value R. Calculating the molar concentration of the cisplatin in the loaded product according to the function formula;

6. measuring a RNA absorbance of the loaded product at 260 nm with the NanoDrop spectrophotometer to obtain the mass concentration of the RNAh particles thereof, and converting it to the molar concentration according to the molecular weight; and

7. loading rate = molar concentration of loaded product cisplatin / molar concentration of loaded product RNAh.

A calculation process is specifically as follows:
The molar concentration of the cisplatin in the loaded product is as follows:
(301 nm cisplatin absorbance-0.003)/0.034=(0.079-0.003)/0.034=2.24mM
The molar concentration of the RNAh in the loaded product is as follows:

Mass concentration of RNAh particles /molecular weight =1.804/(28752×10e3)=0.063mM; and

Loading rate=molar concentration of cisplatin in loaded product/molar concentration of RNAh in loaded product=2.24mM/0.063mM=35.6

It shows that the loading rate of the RNAh-cisplatin is about 35.6, and represents that about 35.6 cisplatin molecules may be loaded on each nucleic acid nanoparticle carrier.
In addition, on the basis of the RNA nanoparticles loading the cisplatin, other small molecular drugs may be further loaded for the second time according to the same method as that of loading the cisplatin. For example, a folic acid is further loaded in the disclosure, to obtain RNA nanoparticles co-loaded with two small molecular drugs of the cisplatin and folic acid, and the loading rates of the two drugs may be detected by referring to the above method (values are not shown).
It is indicated from Embodiment 21 that the RNA nanoparticles with the extension fragment, the target head and the fluorescein have a function of loaded drugs, the small molecular drug cisplatin may achieve the loading in a mode of covalent linkage (paraformaldehyde-solvent covalence), and may also achieve the co-loading with other small molecular drugs.

Embodiment 22

Cell binding ability of cisplatin drug-loaded RNA nanoparticles detected by confocal microscopy experiment

I. Experiment material and experiment method:

1. Samples to be tested as shown in Table 78:

Table 78:

| Nanoparticles | | MW | Dissolution reagent |
|---|---|---|---|
| 1 | RNAh-Biotin-quasar670, RNAh-Bio-670 for short | 29552.6 | PBS |
| 2 | RNAh-Biotin-quasar670-DDP, RNAh-Bio-670-DDP for short | 35352.4 | PBS |
| Note: the RNAh-Bio-670 in the table is served as a control, and refers to the nanoparticles formed by performing the Biotin modification at the 5'-end of the a-strand and b-strand prepared according to the self-assembly method in Embodiment 1, and performing the quasar670 fluorescein modification at the 3'-end of the c-strand, and the RNAh-Bio-670-DDP refers to the nanoparticles formed after further loading the cisplatin (loaded according to the chemical method in Embodiment 21). | | | |

2. Experiment reagents used and sources thereof are as follows:

RPMI-1640 medium (Gibco, C11875500BT-500mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Trypsin-EDTA (Stemcell, 07901-500mL); DMSO (Sigma, D5879-1L); Prolong Gold Antifade Mountant (Thermo, P36941-2 mL); and DAPI (Yeasen, 36308ES11-4 mL).

3. Experiment instruments used are as follows:

Inverted Microscope (Olympus BX53, U-RFL-T); BD Falcon (Corning, 354118); and Cytospin (TXD3).

4. Experiment method:

(1) Placing SKOV3 cells (acute leukemia cell line) in a RPMI1640+10%FBS+1%PS medium, and culturing under a condition of 37 DEG C and 5% CO2.

(2) Collecting the cells, washing with the PBS, and adding to the 48-well plate in 1x105 cells per well.

(3) Incubating the cells with 200 nM and 400 nM of the RNAh-Bio-670 and RNAh-Bio-670-DDP nanoparticles at 37 DEG C and 5% CO2 for 2 h and 4 h.

(4) After the SKOV3 cells are washed with the PBS, adding the cells to a glass slide by a centrifugal picture method, treating with the Prolong Gold Antifade Mountant, and keeping overnight at a room temperature.

(5) Staining with DAPI for 5 min at the room temperature, and then sealing the glass slide.

(6) Using DAPI and FAM channels of the laser scanning confocal microscope to evaluate the cell binding and internalization, taking pictures under the microscope and saving.

II. Experiment result

An experiment result is shown in Fig. 25. It may be seen from Fig. 25 that the results of cell binding and internalization experiments show that the RNAh-Bio-670 and RNAh-Bio-670-DDP nanoparticles may bind and internalize

with the cells because they both carry the target head-Biotin. Because the small molecular drug cisplatin itself is orange-yellow or yellow, after the SKOV3 cells are incubated with the RNAh-Bio-670-DDP nanoparticles, the SKOV3 cells are stained red by the cisplatin carried by the nanoparticles, and the color is deepened along with the increase of the concentration and time. This result shows that the cisplatin-containing drug RNAh-Bio-670-DDP nanoparticles have a strong ability to bind and internalize with the SKOV3 cells.

Embodiment 23

Cell binding ability of drug-loaded RNA nanoparticles detected by flow cytometer

I. Samples to be tested

Targeted drug: RNAh-Biotin-Cy5-DDP, herein a preparation method of the RNAh-Biotin-Cy5 is the same as that of the RNAh-Biotin-quasar670, and a difference is that the fluorescent substance is replaced by the Cy5 from the quasar670. The RNAh-Biotin-Cy5-DDP is the nanoparticles formed by further loading the THP on the RNAh-Biotin-Cy5 (loaded according to the method in Embodiment 5).

II. Experiment cells and culture conditions (SKOV3 cells, the details are the same as the above confocal microscopy experiment in Embodiment 6, and it is not repeatedly described here)

III. Fluorescence detection

Conditions of a fluorescence detection are as follows:

Excitation light is 640 nm, emission light is 675 nm, a detection height is 7 mm, measured value/data point=10, detection speed: normal, and extension: 100 ms.

IV. Detection result (see Table 79)

Table 79:

| Cell line | Test sample | Binding rate (%) | | Binding rate (%) | |
|---|---|---|---|---|---|
| | | Treatment time 1h | | Treatment time 2h | |
| | | 0.2μM | 0.4μM | 0.2μM | 0.4μM |
| SKOV3 | RNAh-Biotin-Cy5-DDP | 99.76% | 99.76% | 99.77% | 99.86% |
| | Blank control (medium only) | 0.08% | 0.08% | 0.24% | 0.24% |

It may be seen from Table 79 that the binding rate of RNAh-Biotin-Cy5-DDP nanoparticles and SKOV3 cells may be as high as 99.7% or more. Compared with the blank control containing the medium only, the RNA drug-loaded particles have a strong ability to bind and internalize with the SKOV3 cells.

Embodiment 24

Stability detection of cisplatin-containing drug loaded on nucleic acid nanoparticles in serum

I. Experiment material and experiment method

1. Samples to be tested: RNAh-Bio-670-DDP nanoparticles prepared in Embodiment 21 dissolved in PBS solution.

2. Experiment reagents:

RPMI-1640 medium (Gibco, C11875500BT-500 mL); Fetal bovine serum (FBS) (ExCell Bio, FNA500-500 mL); Penicillin/Streptomycin (PS) (Gibco, 15140 -122-100 mL); PBS buffer solution (Gibco, C20012500BT-500 mL); Novex™ Tris-Glycine Native Sample Buffer (2X) (Invitrogen, LC2673-20 mL); Novex™ 8% Tris-Glycine Mini Gels (Invitrogen , XP00080BOX-1.0 mm); Tris-Glycine Native Running buffer (10x) (Life science, LC2672-500 mL); and G250 staining

solution (Beyotime, P0017-250 mL).

3. Experiment instrument:

Spectrophotometer (Thermo, ND2000C); Mini Gel Tank (Invitrogen, PS0301); and Imaging System (Bio-Rad, ChemiDoc MP).

4. Experiment method:

(1) Taking 10 μL of the 10 μM RNAh-Bio-670-DDP nanoparticles and placing in 90 μL of a RPMI 1640 medium containing 10% serum and incubating.

(2) After being incubated at 37 DEG C for 10 min, 1 h, 12 h, and 36 h, respectively taking samples.

(3) After using NanoDrop for quantification, taking 200 ng of the RNA nanoparticles, adding the same volume of Tris-Glycine SDS sample buffer solution (2X), and adequately mixing uniformly.

(4) Taking a piece of Novex™ 8% Tris-Glycine Mini gel, loading the samples in order, setting a program at 200 V, 30 min, and starting electrophoresis.

(5) After the electrophoresis is finished, performing G250 staining, placing on a horizontal shaker for 30 min, taking pictures and imaging.

II. Experiment result

Table 80: quantitative result and loading volume

| Sample | RNAh-Bio-670-DDP (ng/μL) | 200ng RNAh-Bio-670-DDP (μL) | Buffer solution (μL) |
|---|---|---|---|
| 0 | 89.7 | 2.2 | 2.2 |
| 10 min | 91.6 | 2.2 | 2.2 |
| 1 h | 89.0 | 2.2 | 2.2 |
| 12 h | 88.6 | 2.3 | 2.3 |
| 36 h | 89.4 | 2.2 | 2.2 |

The electrophoresis detection results are shown in Fig. 26 and Fig. 27. Herein, Fig. 26 shows the electrophoresis result of 8% non-denaturing gel (Coomassie Blue program), and Fig. 27 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Bio-670-DDP nanoparticles, it is indicated that it is relatively stable in the 1640 medium with the 10% FBS without significant degradation.

Embodiment 25

Cytotoxicity research of RNAh-Bio-670-DDP nanoparticles in SKOV3 cells

I. Experiment material and experiment method

1. Experiment material:

Samples to be tested: small molecular drug cisplatin and RNAh-Bio-670-DDP nanoparticles.

Drug concentration preparation:

Preparing a freshly prepared reagent into a corresponding volume container, and adding PBS to be quantified to 10 μM.

Preparing serial dilution solvents with a culture medium, from 10 μM to 3.33 μM, 1.11 μM, 0.370 μM, 0.124 μM, 0.041 μM, 0.014 μM, 0.0046 μM, 0.0015 μM successively.

Transferring 50 μl of the solution to each well to obtain the final concentrations of 5 μM, 1.667 μM, 0.556 μM, 0.185 μM, 0.062 μM, 0.021 μM, 0.0069 μM, and 0.0023 μM, respectively.

2. Experiment reagent:

RPMI-1640 medium (Gibco, C11875500BT-500 mL); Fetal bovine serum (FBS) (ExCell Bio, FNA500-500 mL); Penicillin/Streptomycin (PS) (Gibco, 15140-122-100 mL); PBS buffer solution (Gibco, C20012500BT-500 mL); Trypsin-EDTA (Stemcell, 07901-500 mL); DMSO (Sigma, D5879-1 L); and CellTiter-Glo Luminescent Cell Viability Assay kit (CTG) (Promega, G7572-100 mL).

3. Experiment instrument:

Inverted Microscope (Olympus IX71, No.112A-1); 96-well Plate Reader (Molecular Devices, Flexstation 3); and Perkin Elmer Envision 2104 Multilabel Reader (No. 01-094-0002).

4. Experiment method:

1) Cell culturing and plating

Cells are cultured at 37 DEG C and 5%CO2 in a corresponding basal medium in which 10%FBS and 1%PS are respectively added. The cell density used in the experiment is above 80%. The cells are collected, and centrifuged at 1000 rpm for 4 minutes, the medium is resuspended, the cell concentration is adjusted, and it is added to the 96-well plate in a volume of 3000 cells per 50μL, and each group has 3 replicate wells.

2) Gradient drug concentration preparation and administration

After 24 hours, the compound solution is transferred to each well by 50 μL/well. Finally, the solution of which the final concentrations are: 5 μM, 1.667 μM, 0.556 μM, 0.185 μM, 0.062 μM, 0.021 μM, 0.0069 μM, and 0.0023 μM respectively is obtained;
Solvent control = DMSO
Medium (untreated) control: only cells without compound treatment
Blank control: without cells, used for instrument zero calibration

3) Cell culture after administration

The above cells after administration are cultured for 72 hours under a condition of 37 DEG C and 5% CO2.

4) Cells treated by detection kit

The well plate is brought to a room temperature in advance and stands for 30 minutes. 100μL of a CellTiter-Glo® reagent is added to each well of the well plate and mixed for 2 minutes on a shaker to promote cell lysis. The Perkin Elmer Envision 2104 Multilabel Reader is used to read values and the values are recorded.

5) Experiment data acquisition and processing

The acquired experiment data is analyzed and processed by using excel software, and GraphPad Prism 5 software is used for curve fitting analysis.

II. Experiment result:

Table 81: $IC_{50}$ value

| Cell line | Treatment time | Cisplatin $IC_{50}$ ($\mu$M) | RNAh-Bio-670-DDP $IC_{50}$ ($\mu$M) |
|---|---|---|---|
| SKOV3 | 72h | 45.73 | 1.847 |

The experiment results are shown in Table 81 and Fig. 28, it may be seen from Table 81 and Fig. 28 that the cisplatin and RNAh-Bio-670-DDP nanoparticles have an inhibitory effect on the proliferation of the SKOV3 cells, and it is unpredictable that while the IC50 drug concentration of the small molecular drug cisplatin is 45.73 $\mu$M, the IC50 drug concentration of the RNAh-Bio-670-DDP is 1.847$\mu$M, it is almost only 1/45 of the small molecular drug cisplatin. It may be seen that the RNAh-Bio-670-DDP nanoparticles have the stronger inhibitory activity on the cell proliferation, so the dosage of local drugs may be significantly reduced during the treatment, and the toxic side effects are reduced.

Furthermore, in order to determine that the carrier itself is not significantly toxic to the SKOV3 cells, the disclosure further designs a toxicity experiment of the RNAh-Bio-FAM targeted fluorescent carrier to SKOV3 cells, 10% PBS is served as a negative control, and a medium is served as a blank control, a specific result is shown in Fig. 29. It may be seen from Fig. 29 that the fluorescent carrier itself has no apparent toxicity to the SKOV3 cells.

Assembly of nucleic acid nanoparticles

Embodiment 26

I. 7 groups of extension fragment deformation + core short-sequence RNA nanoparticle carriers:

(1) 7 groups of three polynucleotide base sequences for forming extension fragment deformation + core short-sequence RNA nanoparticles:

Table 82: R-15

| Name | Sequence sense (5'-3') | Base number | Chemical modification | Molecular weight | Total molecular weight |
|---|---|---|---|---|---|
| a (SEQ ID NO: 120) | GCGGCGAGCGGCG AGGAGCGUUGGGG CCGGAGGCCGG | 37 | 3'-end is linked with a biotin (Bio); bases C and U, and 2'F modification | 12668.8 | 33713 |
| b (SEQ ID NO: 121) | CCGGCCUCCGGCC **CCUUCGGGG**CCAG CCGCC | 31 | bases C and U, and 2'F modification | 9866.8 | |
| c (SEQ ID NO: 122) | GGCGGCAGG**CCCC CAUAGCCC**UCGCCG CUCGCCGC | 35 | bases C and U, and 2'F modification | 11177.4 | |

Table 83: R-16:

| Name | Sequence sense (5'-3') | Base number | Chemical modification | Molecular weight | Total molecular weight |
|---|---|---|---|---|---|
| a (SEQ ID NO: 123) | GCGGCGAGCGGCGAGCAGCGUUCGGGCCGGAGGCCGG | 37 | 3'-end is linked with a biotin (Bio); bases C and U, and 2'F modification | 12591.6 | 33709.8 |
| b (SEQ ID NO: 124) | CCGGCCUCCGGCCCGUUCGCCGCCAGCCGCC | 31 | bases C and U, and 2'F modification | 9827.6 | |
| c (SEQ ID NO: 125) | GGCGGCAGGCGGCCAUAGCGCUCGCCGCUCGCCGC | 35 | bases C and U, and 2'F modification | 11290.6 | |

Table 84: R-17:

| Name | Sequence sense (5'-3') | Base number | Chemical modification | Molecular weight | Total molecular weight |
|---|---|---|---|---|---|
| a (SEQ ID NO: 126) | GCGGCGAGCGGCGA**GGAGCGUUGG**GGCCGGAGGCCGG | 37 | 3'-end is linked with a biotin (Bio); bases C and U, and 2'F modification | 12668.9 | 33713 |
| b (SEQ ID NO: 127) | CCGGCCUCCGGCC**CCUUCGCCG**CCAGCCGCC | 31 | bases C and U, and 2'F modification | 9790.6 | |
| c (SEQ ID NO: 128) | GGCGGCAGG**CGGCCAUAGCCC**UCGCCGCUCGCCGC | 35 | bases C and U, and 2'F modification | 11253.5 | |

Table 85: R-18:

| Name | Sequence sense (5'-3') | Base number | Chemical modification | Molecular weight | Total molecular weight |
|---|---|---|---|---|---|
| a (SEQ ID NO: 129) | GCGGCGAGCGGCGAGCAGCGUUCGGGCCGGAGGCCGG | 37 | 3'-end is linked with a biotin (Bio); bases C and U, and 2'F modification | 12591.6 | 33709.8 |
| b (SEQ ID NO: 130) | CCGGCCUCCGGCCCGUUCGGCGCCAGCCGCC | 31 | bases C and U, and 2'F modification | 9865.7 | |
| c (SEQ ID NO: 131) | GGCGGCAGGCGCCCAUAGCGCUCGCCGCUCGCCGC | 35 | bases C and U, and 2'F modification | 11252.5 | |

Table 86: R-19:

| Name | Sequence sense (5'-3') | Base number | Chemical modification | Molecular weight | Total molecular weight |
|---|---|---|---|---|---|
| a (SEQ ID NO: 132) | GCGGCGAGCGGCGAGCAGCGUUCGGGCCGGAGGCCGG | 37 | 3'-end is linked with a biotin (Bio); bases C and U, and 2'F modification | 12591.6 | 33709.8 |
| b (SEQ ID NO: 133) | CCGGCCUCCGGCCCGUUCGGCCCCAGCCGCC | 31 | bases C and U, and 2'F modification | 9827.6 | |
| c (SEQ ID NO: 134) | GGCGGCAGGGGCCCAUAGCGCUCGCCGCUCGCCGC | 35 | bases C and U, and 2'F modification | 11290.6 | |

Table 87: R-20:

| Name | Sequence sense (5'-3') | Base number | Chemical modification | Molecular weight | Total molecular weight |
|---|---|---|---|---|---|
| a (SEQ ID NO: 135) | GCGGCGAGCGGCGACGAGCGUUGCGGCCGGAGGCCGG | 37 | 3'-end is linked with a biotin (Bio); bases C and U, and 2'F modification | 12591.6 | 33709.8 |
| b (SEQ ID NO: 136) | CCGGCCUCCGGCCGCUUCGCCGCCAGCCGCC | 31 | bases C and U, and 2'F modification | 9827.6 | |
| c (SEQ ID NO: 137) | GGCGGCAGGCGGCCAUAGCCGUCGCCGCUCGCCGC | 35 | bases C and U, and 2'F modification | 11290.6 | |

Table 88: R-21:

| Name | Sequence sense (5'-3') | Base number | Chemical modification | Molecular weight | Total molecular weight |
|---|---|---|---|---|---|
| a (SEQ ID NO: 138) | GCGGCGAGCGGCGACGAGCGUUGCGGCCGGAGGCCGG | 37 | 3'-end is linked with a biotin (Bio); bases C and U, and 2'F modification | 11290.6 | 32408.8 |
| b (SEQ ID NO: 139) | CCGGCCUCCGGCCGCUUCGGCGCCAGCCGCC | 31 | bases C and U, and 2'F modification | 9865.7 | |
| c (SEQ ID NO: 140) | GGCGGCAGGCGCCCAUAGCCGUCGCCGCUCGCCGC | 35 | bases C and U, and 2'F modification | 11252.5 | |

II. Self-assembly experiment steps:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, and volatilizing under reduced pressure and low temperature; and

(5) electrophoresis analysis detection and laser scanning observation.

III. Self-assembly experiment result

(1) Electrophoresis detection

Main reagents and instruments are as follows:

Table 89:

| Reagent name | Article number | Manufacturer |
|---|---|---|
| 6xDNA Loading buffer | TSJ010 | Qingke Biotechnology Co., Ltd |
| 20bp DNA Ladder | 3420A | TAKARA |
| 10000*SolarGelRed nucleic acid dye | E1020 | solarbio |
| 8% non-denaturing PAGE get | / | Self-made |
| 1 × TBE Buffer (RNA enzyme-free) | / | Self-made |

Table 90:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Operations:

① The RNA nanoparticles are diluted with ultrapure water by using a method shown in Table 54 below.

Table 91:

| Serial number | Measured concentration (μg/mL) |
|---|---|
| R-15 | 165.937 |
| R-16 | 131.706 |
| R-17 | 144.649 |
| R-18 | 164.743 |
| R-19 | 126.377 |
| R-20 | 172.686 |
| R-21 | 169.455 |

②10μL (500ng) of the processed sample is taken and uniformly mixed with 2μL of 6xDNA Loading Buffer, it is operated on ice, and a label is made.
③A 8% non-denaturing PAGE gel is taken, a piece of the gel is applied to samples with different incubation times, 12 μL of the processed samples are all loaded, and a program is set to run the gel at 100V for 40 minutes.
④ After running the gel, dyeing is performed, it is placed on a horizontal shaker for 30 minutes, and pictures are taken for imaging.

Detection result:

Non-denaturing PAGE gel running results of the 7 groups of extension fragment deformation + core short-sequence RNA self-assembly products are shown in Fig. 16. Lanes 1 to 7 in Fig. 16 from left to right are successively: the 7 groups of extension fragment deformation + core short-sequence RNA self-assembly products R-15, R-16, R-17, R-18, R-19, R-20 and R-21.
It may be clearly seen from the results in Fig. 30 that the bands of the 7 groups of extension fragment deformation + core short-sequence RNA self-assembly products are bright and clear, it is indicated that the 7 groups of extension fragment deformation + core short-sequence RNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.
Measurement result: potential detection results of the 7 groups of the extension fragment deformation + core short-sequence RNA nanoparticles at 25 DEG C are as follows.

Table 92:

| Sample name | Detection times | Potential ZP (mV) |
|---|---|---|
| R-15 | 1 | -22.50 |
| | 2 | -23.90 |
| | 3 | -23.30 |
| Experiment result | -23.23 mV | |

Table 93:

| Sample name | Detection times | Potential ZP (mV) |
|---|---|---|
| R-16 | 1 | -21.10 |
| | 2 | -19.80 |
| | 3 | -21.90 |
| Experiment result | -20.93 mV | |

Table 94:

| Sample name | Detection times | Potential ZP (mV) |
|---|---|---|
| R-17 | 1 | -24.90 |
| | 2 | -20.90 |
| | 3 | -24.70 |
| Experiment result | -23.50 mV | |

Table 95:

| Sample name | Detection times | Potential ZP (mV) |
|---|---|---|
| R-18 | 1 | -20.80 |
| | 2 | -21.30 |
| | 3 | -21.70 |
| Experiment result | -21.27 mV | |

Table 96:

| Sample name | Detection times | Potential ZP (mV) |
|---|---|---|
| R-19 | 1 | -16.80 |
| | 2 | -16.90 |
| | 3 | -21.20 |
| Experiment result | -18.30 mV | |

Table 97:

| Sample name | Detection times | Potential ZP (mV) |
|---|---|---|
| R-20 | 1 | -16.00 |
| | 2 | -16.90 |
| | 3 | -21.20 |
| Experiment result | -17.90 mV | |

Table 98:

| Sample name | Detection times | Potential ZP (mV) |
|---|---|---|
| R-21 | 1 | -15.20 |
| | 2 | -16.70 |
| | 3 | -16.40 |
| Experiment result | -16.10 mV | |

It may be seen from the above potential detection data that: the 7 groups of extension fragment deformation + core short-sequence RNA nanoparticles all have the good stability, and it is further indicated that the nanoparticles formed by the self-assembly of each extension fragment deformation + core short-sequence RNA have a relative stable self-assembly structure.

(3) Particle size measurement

1. Preparing a potential sample (7 groups of extension fragment deformation + core short-sequence RNA) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, while a measurement dialog box appears, clicking Start to start, a DLS measurement value result of a hydrodynamic size of the 7 groups of extension fragment deformation + core short-sequence RNA is as follows:

Table 99:

| Serial number | Mean particle size (nm) |
|---|---|
| R-15 | 6.808 |
| R-16 | 6.978 |
| R-17 | 7.592 |
| R-18 | 7.520 |
| R-19 | 6.936 |
| R-20 | 7.110 |
| R-21 | 6.720 |

(4) TM value detection

A solubility curve method is used to detect TM values of the 7 groups of the extension fragment deformation + core short-sequence RNA nanoparticles, and the samples are consistent with the potential samples.

Reagents and instruments are as follows.

Table 100:

| Reagent name | Article number | Manufacturer |
|---|---|---|
| AE buffer | / | Takara |
| SYBR Green I dye | / | Self-made |

Table 101:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

①After the sample is diluted with ultrapure water, 5μg of the diluted sample is mixed with 2μL of SYBR Green I dye (diluted by 1:200), a final volume is 20μL, and the dilution concentration is as follows:

Table 102:

| Serial number | Measured concentration (μg/mL) |
|---|---|
| R-15 | 773.009 |
| R-16 | 782.098 |
| R-17 | 740.607 |
| R-18 | 806.163 |
| R-19 | 829.996 |
| R-20 | 723.082 |
| R-21 | 721.674 |

②It is incubated for 30 min in the dark at a room temperature; and
③On-machine detection is performed, a program is set to start at 20 DEG C, the temperature rises per second from 0.1 DEG C to 95 DEGC, and reading is performed every 5s.

Detection result:

The TM values of the 7 groups of extension fragment deformation + core short-sequence RNA nanoparticles are as follows, a solubility curve diagram of the R-15 is shown in Fig. 31, a solubility curve diagram of the R-16 is shown in Fig. 32, a solubility curve diagram of the R-17 is shown in Fig. 33, a solubility curve diagram of the R-18 is shown in Fig. 34, a solubility curve diagram of the R-19 is shown in Fig. 35, a solubility curve diagram of the R-20 is shown in Fig. 36, and a solubility curve diagram of the R-21 is shown in Fig. 37. Due to the particularity of the RNA samples, a temperature corresponding to 1/2 RFUmax in a range of 20 to 90 DEG C is used as a sample Tm value in this test.

Table 103:

| | TM value (DEG C) |
|---|---|
| R-15 | 57.5 DEG C |
| R-16 | 53.8 DEG C |
| R-17 | 55.2 DEG C |
| R-18 | 54.5 DEG C |
| R-19 | 54.0 DEG C |

(continued)

| | TM value (DEG C) |
|---|---|
| R-20 | 59.5 DEG C |
| R-21 | 65.0 DEG C |

The TM values of the 7 groups of extension fragment deformation + core short-sequence RNA nanoparticles are all high, it is indicated that the self-assembly products have the good structural stability.

Embodiment 27

I. 7 groups of extension fragment deformation + core short-sequence DNA nanoparticle carriers:

(1) 7 groups of three polynucleotide base sequences for forming extension fragment deformation + core short-sequence DNA nanoparticles:

Table 104: D-8:

| Name | Sequence sense (5'-3') | Base number | Chemical modification | Molecular weight | Total molecular weight |
|---|---|---|---|---|---|
| a (SEQ ID NO: 141) | GCGGCGAGCGGCGA**GGAGCGTTGG**GGCCGGAGGCCGG | 37 | 3'-end is linked with a biotin (Bio) | 12087.3 | 32081.8 |
| b (SEQ ID NO: 142) | CCGGCCTCCGGCC**CCTTCGGGG**CCAGCCGCC | 31 | | 9375.1 | |
| c (SEQ ID NO: 143) | GGCGGCAGG**CCCCCATAGCCC**TCGCCGCTCGCCGC | 35 | | 10619.4 | |

Table 105: D-9:

| Name | Sequence sense (5'-3') | Base number | Chemical modification | Molecular weight | Total molecular weight |
|---|---|---|---|---|---|
| a (SEQ ID NO: 144) | GCGGCGAGCGGCGAGCAGCGTTCGGGCCGGAGGCCGG | 37 | 3'-end is linked with a biotin (Bio) | 12000.4 | 32071.6 |
| b (SEQ ID NO: 145) | CCGGCCTCCGGCCCGTTCGCCGCCAGCCGCC | 31 | | 9333.2 | |
| c (SEQ ID NO: 146) | GGCGGCAGGCGGCCATAGCGCTCGCCGCTCGCCGC | 35 | | 10738 | |

Table 106: D-10:

| Name | Sequence sense (5'-3') | Base number | Chemical modification | Molecular weight | Total molecular weight |
|---|---|---|---|---|---|
| a (SEQ ID NO: 147) | GCGGCGAGCGGCGA**GGAGCGTTGG**GGCCGGAGGCCGG | 37 | 3'-end is linked with a biotin (Bio) | 12087.7 | 32081.6 |
| b (SEQ ID NO: 148) | CCGGCCTCCGGCC**CCTTCGCCG**CCAGCCGCC | 31 | | 9294.3 | |
| c (SEQ ID NO: 149) | GGCGGCAGG**CGGCCATAGCCC**TCGCCGCTCGCCGC | 35 | | 10699.6 | |

Table 107: D-11:

| Name | Sequence sense (5'-3') | Base number | Chemical modification | Molecular weight | Total molecular weight |
|---|---|---|---|---|---|
| a (SEQ ID NO: 150) | GCGGCGAGCGGCGAGCAGCGTTCGGGCCGGAGGCCGG | 37 | 3'-end is linked with a biotin (Bio) | 12000.4 | 32071.6 |
| b (SEQ ID NO: 151) | CCGGCCTCCGGCCCGTTCGGCGCCAGCCGCC | 31 | | 9333.2 | |
| c (SEQ ID NO: 152) | GGCGGCAGGCGCCCATAGCGCTCGCCGCTCGCCGC | 35 | | 10738 | |

Table 108: D-12:

| Name | Sequence sense (5'-3') | Base number | Chemical modification | Molecular weight | Total molecular weight |
|---|---|---|---|---|---|
| a (SEQ ID NO: 153) | GCGGCGAGCGGCGAGCAGCGTTCGGGCCGGAGGCCGG | 37 | 3'-end is linked with a biotin (Bio) | 12000.4 | 32071.6 |
| b (SEQ ID NO: 154) | CCGGCCTCCGGCCCGTTCGGCCCCAGCCGCC | 31 | | 9333.2 | |

(continued)

| Name | Sequence sense (5'-3') | Base number | Chemical modification | Molecular weight | Total molecular weight |
|---|---|---|---|---|---|
| c (SEQ ID NO: 155) | GGCGGCAGGGGCC CATAGCGCTCGCCG CTCGCCGC | 35 | | 10738 | |

Table 109: D-13:

| Name | Sequence sense (5'-3') | Base number | Chemical modification | Molecular weight | Total molecular weight |
|---|---|---|---|---|---|
| a (SEQ ID NO: 156) | GCGGCGAGCGGCGA CGAGCGTTGCGGCC GGAGGCCGG | 37 | 3'-end is linked with a biotin (Bio) | 12000.4 | 32071.6 |
| b (SEQ ID NO: 157) | CCGGCCTCCGGCCG CTTCGCCGCCAGCCG CC | 31 | | 9333.2 | |
| c (SEQ ID NO: 158) | GGCGGCAGGCGGCC ATAGCCGTCGCCGCT CGCCGC | 35 | | 10738 | |

Table 110: D-14:

| Name | Sequence sense (5'-3') | Base number | Chemical modification | Molecular weight | Total molecular weight |
|---|---|---|---|---|---|
| a (SEQ ID NO: 159) | GCGGCGAGCGGCGAC GAGCGTTGCGGCCGG AGGCCGG | 37 | 3'-end is linked with a biotin (Bio) | 12000.4 | 32071.6 |
| b (SEQ ID NO: 160) | CCGGCCTCCGGCCGC TTCGGCGCCAGCCGC C | 31 | | 9373.2 | |
| c (SEQ ID NO: 161) | GGCGGCAGGCGCCCA TAGCCGTCGCCGCTCG CCGC | 35 | | 10698 | |

II. Self-assembly experiment steps:

(1) according to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100V of electrophoresis;

(4) cutting a target band and eluting in DNA elution buffer solution at 37 DEG C, after that, precipitating overnight in ethanol, and volatilizing under reduced pressure and low temperature, to obtain the DNA self-assembly products;

(5) electrophoresis analysis detection and laser scanning observation;

(6) electric potential detection;

(7) particle size detection; and

(8) TM value detection.

III. Self-assembly experiment result

(1) Electrophoresis detection

Main reagents and instruments are as follows:

Table 111:

| Reagent name | Article number | Manufacturer |
|---|---|---|
| 6xDNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE get | / | Self-made |
| 1× TBE Buffer (RNA enzyme-free) | / | Self-made |

Table 112:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Steps:

① The DNA nanoparticles are diluted with ultrapure water by a method shown in Table 82 below.

Table 113:

| | Measured concentration (μg/mL) |
|---|---|
| D-8 | 2890.932 |
| D-9 | 2238.682 |
| D-10 | 2075.084 |

(continued)

| | Measured concentration (μg/mL) |
|---|---|
| D-11 | 3117.389 |
| D-12 | 2880.939 |
| D-13 | 2704.757 |
| D-14 | 3216.917 |

②10μL (500ng) of the processed sample is taken and uniformly mixed with 2μL of 6xDNA Loading Buffer, it is operated on ice, and a label is made.
③A 8% non-denaturing PAGE gel is taken, a piece of the gel is applied to samples with different incubation times, 12 μL of the processed samples are all loaded, and a program is set to run the gel at 100V for 40 minutes.
④ After running the gel, dyeing is performed, it is placed on a horizontal shaker for 30 minutes, and pictures are taken for imaging.

Detection result:

Non-denaturing PAGE gel running results of the 7 groups of extension fragment deformation + core short-sequence DNA self-assembly products are shown in Fig. 38. Lanes 1 to 7 in Fig. 38 from left to right are successively: the 7 groups of extension fragment deformation + core short-sequence DNA self-assembly products D-8, D-9, D-10, D-11, D-12, D-13 and D-14.
It may be clearly seen from the results in Fig. 38 that the bands of the 7 groups of extension fragment deformation + core short-sequence DNA self-assembly products are bright and clear, it is indicated that the 7 groups of extension fragment deformation + core short-sequence DNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears; setting a software detection parameter;
and then clicking Ok to complete the settings, after a measurement dialog box appears, clicking Start to start.
Measurement result: potential detection results of the 7 groups of the extension fragment deformation + core short-sequence DNA nanoparticles at 25 DEG C are as follows:

Table 114:

| Sample name | Detection times | Potential ZP (mV) |
|---|---|---|
| D-8 | 1 | -29.20 |
| | 2 | -32.90 |
| | 3 | -28.60 |
| Experiment result | -30.23 mV | |

Table 115:

| Sample name | Detection times | Potential ZP (mV) |
|---|---|---|
| D-9 | 1 | -39.20 |
| | 2 | -34.20 |
| | 3 | -31.80 |
| Experiment result | -35.07 mV | |

Table 116:

| Sample name | Detection times | Potential ZP (mV) |
|---|---|---|
| D-10 | 1 | -27.90 |
| | 2 | -28.30 |
| | 3 | -21.10 |
| Experiment result | -25.77 mV | |

Table 117:

| Sample name | Detection times | Potential ZP (mV) |
|---|---|---|
| D-11 | 1 | -29.70 |
| | 2 | -24.80 |
| | 3 | -27.80 |
| Experiment result | -27.43 mV | |

Table 118:

| Sample name | Detection times | Potential ZP (mV) |
|---|---|---|
| D-12 | 1 | -33.50 |
| | 2 | -29.80 |
| | 3 | -34.20 |
| Experiment result | -32.50 mV | |

Table 119:

| Sample name | Detection times | Potential ZP (mV) |
|---|---|---|
| D-13 | 1 | -26.80 |
| | 2 | -27.80 |
| | 3 | -26.40 |
| Experiment result | -27.00 mV | |

Table 120:

| Sample name | Detection times | Potential ZP (mV) |
|---|---|---|
| D-14 | 1 | -31.70 |
| | 2 | -32.10 |
| | 3 | -22.40 |
| Experiment result | -28.73 mV | |

It may be seen from the above potential detection data that: the 7 groups of extension fragment deformation + core short-sequence DNA nanoparticles all have the good stability, and it is further indicated that the nanoparticles formed by the self-assembly of each extension fragment deformation + core short-sequence DNA have a relative stable self-assembly structure.

(3) Particle size measurement

① Preparing a potential sample (7 groups of extension fragment deformation + core short-sequence DNA) and putting into a sample pool, opening a sample pool cover of an instrument, and placing the instrument;
② opening software, and clicking a menu, so a manual measurement parameter setting dialog box appears;
③ setting a software detection parameter; and then
④ clicking Ok to complete the settings, while a measurement dialog box appears, clicking Start to start, a DLS measurement value result of a hydrodynamic size of the 7 groups of extension fragment deformation + core short-sequence DNA is as follows:

Table 121:

| Serial number | Mean particle size (nm) |
|---|---|
| D-8 | 7.460 |
| D-9 | 7.920 |
| D-10 | 7.220 |
| D-11 | 7.472 |
| D-12 | 6.968 |
| D-13 | 7.012 |
| D-14 | 6.896 |

(4) TM value detection

A solubility curve method is used to detect TM values of the 7 groups of the extension fragment deformation + core short-sequence DNA nanoparticles, and the samples are consistent with the potential samples.

Reagents and instruments are as follows.

Table 122:

| Reagent name | Article number | Manufacturer |
|---|---|---|
| AE buffer | / | Takara |
| SYBR Green I dye | / | Self-made |

Table 123:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

①After the sample is diluted with ultrapure water, 5μg of the diluted sample is mixed with 2μL of SYBR Green I dye (diluted by 1:200), a final volume is 20μL, and the dilution concentration is as follows:

Table 124:

| Serial number | Measured concentration (μg/mL) |
|---|---|
| D-8 | 2890.932 |
| D-9 | 2238.682 |

(continued)

| Serial number | Measured concentration (μg/mL) |
|---|---|
| D-10 | 2075.084 |
| D-11 | 3117.389 |
| D-12 | 2880.939 |
| D-13 | 2704.757 |
| D-14 | 3216.917 |

②It is incubated for 30 min in the dark at a room temperature; and
③On-machine detection is performed, a program is set to start at 20 DEG C, the temperature rises per second from 0.1 DEG C to 95 DEG C, and reading is performed every 5s.

Detection result:

The TM values of the 7 groups of extension fragment deformation + core short-sequence DNA nanoparticles are as follows, a solubility curve diagram of the D-8 is shown in Fig. 39, a solubility curve diagram of the D-9 is shown in Fig. 40, a solubility curve diagram of the D-10 is shown in Fig. 41, a solubility curve diagram of the D-11 is shown in Fig. 42, a solubility curve diagram of the D-12 is shown in Fig. 43, a solubility curve diagram of the D-13 is shown in Fig. 44, and a solubility curve diagram of the D-14 is shown in Fig. 45.

Table 125:

| Serial number | TM value (DEG C) |
|---|---|
| D-8 | 48.5 |
| D-9 | 52.5 |
| D-10 | 54.5~55.0 |
| D-11 | 48.7 |
| D-12 | 51.5 |
| D-13 | 51.0 |
| D-14 | 49.2 |

It may be seen from Figs. 39 to 45 that the TM values of the 7 groups of extension fragment deformation + core short-sequence DNA nanoparticles are all high, it is indicated that the self-assembly products have the good structural stability.

**Stability** detection of nucleic acid nanoparticles in serum

Embodiment 28

A non-denaturing PAGE method is used to characterize the stability of 7 groups of extension fragment deformation + core short-sequence RNA nanoparticles in serum.
Main reagents and instruments are as follows.

Table 126:

| Reagent name | Article number | Manufacturer |
|---|---|---|
| 6xDNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |

(continued)

| Reagent name | Article number | Manufacturer |
|---|---|---|
| 1 × TBE Buffer (RNA enzyme-free) | / | Self-made |
| Serum (FBS) | / | Excel |
| RPMI 1640 | / | GBICO |

Table 127:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

① The RNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 128:

| Sample name | Sample concentration (μg/mL | Sample volume | 50%FBS-1640 / μL | 1640/ μL | Diluted concentration (μg/mL |
|---|---|---|---|---|---|
| R-15 | 773.009 | 1.3 | 4 | 14.7 | 50 μg/mL |
| R-16 | 782.098 | 1.3 | 4 | 14.7 | 50 μg/mL |
| R-17 | 740.607 | 1.4 | 4 | 14.6 | 50 μg/mL |
| R-18 | 806.163 | 1.2 | 4 | 14.8 | 50 μg/mL |
| R-19 | 829.996 | 1.2 | 4 | 14.8 | 50 μg/mL |
| R-20 | 723.082 | 1.4 | 4 | 14.6 | 50 μg/mL |
| R-21 | 721.674 | 1.4 | 4 | 14.6 | 50 μg/mL |

②10μL of the processed sample is taken and uniformly mixed with 2μL of 6xDNA Loading Buffer, it is operated on ice, and a label is made;
③a 8% non-denaturing PAGE gel is taken, a piece of the gel is applied on the samples with different incubation times, 12 μL of the processed samples are all loaded, and a program is set to run the gel at 100V for 40 min; and
④ after running the gel, dyeing is performed, it is placed on a horizontal shaker and shaken slowly for 30 min, pictures are taken for imaging.

An electrophoresis detection result of the R-15 is shown in Fig. 46, an electrophoresis detection result of the R-16 is shown in Fig. 47, an electrophoresis detection result of the R-17 is shown in Fig. 48, an electrophoresis detection result of the R-18 is shown in Fig. 49, an electrophoresis detection result of the R-19 is shown in Fig. 50, an electrophoresis detection result of the R-20 is shown in Fig. 51, and an electrophoresis detection result of the R-21 is shown in Fig. 52. In Fig. 46 to 52, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of RNA nanoparticle samples at different times, it is indicated that RNA nanoparticles R-15 to R-21 are relatively stable in a 1640 medium of 50% FBS without apparent degradation.

Embodiment 29

A non-denaturing PAGE method is used to characterize the stability of 7 groups of extension fragment deformation + core short-sequence DNA nanoparticles in serum.

Main reagents and instruments are as follows.

Table 129:

| Reagent name | Article number | Manufacturer |
|---|---|---|
| 6xDNA Loading buffer | TSJ010 | Qingke Biotechnology Co., Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1 × TBE Buffer (RNA enzyme-free) | / | Self-made |
| Serum (FBS) | / | Excel |
| RPMI 1640 | / | GBICO |

Table 130:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

① The DNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 131:

| Sample name | Sample concentration (μg/mL) | Sample volume | 50%FBS-1640 / μL | 1640/ μL | Diluted concentration (μg/mL) |
|---|---|---|---|---|---|
| D-8 | 2890.932 | 1.4 | 8 | 30.6 | 100 |
| D-9 | 2238.682 | 1.8 | 8 | 30.2 | 100 |
| D-10 | 2075.084 | 1.9 | 8 | 30.1 | 100 |
| D-11 | 3117.389 | 1.3 | 8 | 30.7 | 100 |
| D-12 | 2880.939 | 1.4 | 8 | 30.6 | 100 |
| D-13 | 2704.757 | 1.5 | 8 | 30.5 | 100 |
| D-14 | 3216.917 | 1.2 | 8 | 30.8 | 100 |

②10μL of the processed sample is taken and uniformly mixed with 2μL of 6xDNA Loading Buffer, it is operated on ice, and a label is made;
③a 8% non-denaturing PAGE gel is taken, a piece of the gel is applied on the samples with different incubation times, 6 μL of the processed samples are all loaded, and a program is set to run the gel at 100V for 40 min; and
④ after running the gel, dyeing is performed, it is placed on a horizontal shaker and shaken slowly for 30 min, pictures

are taken for imaging.

An electrophoresis detection result of the D-8 is shown in Fig. 53, an electrophoresis detection result of the D-9 is shown in Fig. 54, an electrophoresis detection result of the D-10 is shown in Fig. 55, an electrophoresis detection result of the D-11 is shown in Fig. 56, an electrophoresis detection result of the D-12 is shown in Fig. 57, an electrophoresis detection result of the D-13 is shown in Fig. 58, and an electrophoresis detection result of the D-14 is shown in Fig. 59. In Fig. 53 to 59, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of DNA nanoparticle samples at different times, it is indicated that DNA nanoparticles D-8 to D-14 are relatively stable in a 1640 medium of 50% FBS without apparent degradation.

Drug loading test of nucleic acid nanoparticle

Embodiment 30

Doxorubicin loading experiment:

According to the chemical loading method (unless otherwise specified, the method is the same as that of Embodiment 5) of Embodiment 5, the RNA nanoparticles formed by the self-assembly of the previous R-15, R-16, R-17, R-18, R-19, R-20 and R-21 in Embodiment 9, and the DNA nanoparticles formed by the self-assembly of the D-8, D-9, D-10, D-11, D-12, D-13 and D-14 in Embodiment 10 are respectively used as doxorubicin loading carriers, doxorubicin loading rates measured are respectively as follows.

The doxorubicin loading rate of RNA nanoparticles R-15 is 20.5.

The doxorubicin loading rate of RNA nanoparticles R-16 is 29.4.

The doxorubicin loading rate of RNA nanoparticles R-17 is 30.9.

The doxorubicin loading rate of RNA nanoparticles R-18 is 34.1.

The doxorubicin loading rate of RNA nanoparticles R-19 is 27.1.

The doxorubicin loading rate of RNA nanoparticles R-20 is 30.2.

The doxorubicin loading rate of RNA nanoparticles R-21 is 20.1.

The doxorubicin loading rate of DNA nanoparticles D-8 is 28.0.

The doxorubicin loading rate of DNA nanoparticles D-9 is 27.9.

The doxorubicin loading rate of DNA nanoparticles D-10 is 18.9.

The doxorubicin loading rate of DNA nanoparticles D-11 is 26.8.

The doxorubicin loading rate of DNA nanoparticles D-12 is 27.6.

The doxorubicin loading rate of DNA nanoparticles D-13 is 31.8.

The doxorubicin loading rate of DNA nanoparticles D-14 is 32.

Cell binding ability of DNA nanoparticles and carrier drugs detected by Fluorescence Activated Cell Sorter (FACS) Experiment

Embodiment 31

I. Cell information

HepG2 (from Concord Cell Bank), a medium is DMEM+10% FBS+1% double antibody (gibco, 15140-122), and culture conditions are 37 DEG C, 5% CO2 and saturated humidity.

II. Substances to be tested

Blank carrier: DNA nanoparticle carriers formed by the self-assembly of the previous D-8, D-9, D-10, D-11, D-12, D-13 and D-14 in the above Embodiment 10.

Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 12 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

III. Main devices and consumables

Table 132:

| | **Manufacturer** | **Model** |
|---|---|---|
| Biosafety cabinet | Beijing Donglian Haer Instrument Manufacturing Company | BSC-1 360 II A2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Fluorescence activated cell sorter | BD | BD FACSCalibur™ |

IV. Main reagent

Table 133:

| Reagent name | Manufacturer | Article number | Remark |
|---|---|---|---|
| DMEM (biotin-free) | Provided by Baiyao Zhida Nano-biotechnology Co., Ltd. | YS3160 | |
| 1%BSA-PBS | Self-made | - | |

V. Experiment method

1. A cell state is adjusted to a logarithmic growth phase, the medium is changed into a medium without biotin and folic acid, and it is incubated overnight in an incubator at 37 DEG C.

2. After incubation, cell suspension is trypsinized and collected, and centrifuged at 1000 rpm for 5 min, after the concentration is adjusted, a 2×105-5×105 cell/EP tube is taken and washed twice with 1 mL/tube of 1% BSA-PBS, and cells at the bottom of the tube is observed to prevent them from being absorbed.

3. A substance to be tested is dissolved, and the substance to be tested is diluted to a use concentration.

4. Cell supernatant is absorbed, 100μL of a corresponding sample is added to each tube in order, light is avoided, and it is incubated at 37 DEG C for 2 h.

5. It is washed twice with 1% BSA-PBS; and centrifuged at 1000 rpm for 5min.

6. Finally, it is precipitated with 300 μL of PBS cell resuspension, and flow cytometric on-machine detection (the blank carrier used in the present embodiment is labeled by Quasar670, and the doxorubicin in the carrier drug has its own fluorescence, so the detection may be performed through FL4-APC and FL4-APC respectively) is performed.

7. Data analysis.

VI. Experiment result

1. An experiment result is shown in the table below:

Table 134:

| Detected substance | | Experiment cell | Positive rate |
|---|---|---|---|
| PBS | | HepG2 | 2.11% |
| D-8-doxorubicin (carrier drug) | 1μM | HepG2 | 93.1% |
| | 2μM | HepG2 | 96.3% |
| D-8 (blank carrier) | 1μM | HepG2 | 96.9% |
| | 2μM | HepG2 | 98.4% |
| D-9-doxorubicin (carrier drug) | 1μM | HepG2 | 88.6% |
| | 2μM | HepG2 | 95.4% |
| D-9 (blank carrier) | 1μM | HepG2 | 96.7% |
| | 2μM | HepG2 | 98.1% |
| D-10-doxorubicin (carrier drug) | 1μM | HepG2 | 89.4% |
| | 2μM | HepG2 | 95.5% |
| D-10 (blank carrier) | 1μM | HepG2 | 97.9% |
| | 2μM | HepG2 | 98.3% |
| D-11-doxorubicin (carrier drug) | 1μM | HepG2 | 89.3% |
| | 2μM | HepG2 | 95.5% |
| D-11 (blank carrier) | 1μM | HepG2 | 97.7% |
| | 2μM | HepG2 | 97.8% |
| D-12-doxorubicin (carrier drug) | 1μM | HepG2 | 94.6% |
| | 2μM | HepG2 | 95.9% |
| D-12 (blank carrier) | 1μM | HepG2 | 96.9% |
| | 2μM | HepG2 | 97.1% |
| D-13-doxorubicin (carrier drug) | 1μM | HepG2 | 89.6% |
| | 2μM | HepG2 | 94.0% |
| D-13 (blank carrier) | 1μM | HepG2 | 97.6% |
| | 2μM | HepG2 | 98.2% |
| D-14-doxorubicin (carrier drug) | 1μM | HepG2 | 90.3% |
| | 2μM | HepG2 | 96.1% |
| D-14 (blank carrier) | 1μM | HepG2 | 97.4% |
| | 2μM | HepG2 | 98.3% |

2. Conclusion

1. After the HepG2 cells are incubated with D-8-doxorubicin (carrier drug) and D-8 (blank carrier), the binding rates are very high (93.1%-98.4%).

2. After the HepG2 cells are incubated with D-9-doxorubicin (carrier drug) and D-9 (blank carrier), the binding rates are very high (88.6%-98.1%).

3. After the HepG2 cells are incubated with D-10-doxorubicin (carrier drug) and D-10 (blank carrier), the binding rates are very high (89.4%-98.3%).

4. After the HepG2 cells are incubated with D-11-doxorubicin (carrier drug) and D-11 (blank carrier), the binding

rates are very high (89.3%-97.8%).

5. After the HepG2 cells are incubated with D-12-doxorubicin (carrier drug) and D-12 (blank carrier), the binding rates are very high (94.6%-97.1%).

6. After the HepG2 cells are incubated with D-13-doxorubicin (carrier drug) and D-13 (blank carrier), the binding rates are very high (89.6%-98.2%).

7. After the HepG2 cells are incubated with D-14-doxorubicin (carrier drug) and D-14 (blank carrier), the binding rates are very high (90.3%-98.3%).

Cytotoxicity research of DNA nanoparticles and carrier drugs in HepG2 cells

Embodiment 32

A CCK8 method is used to detect the toxicity of DNA naoparticles and carrier drugs to HepG2.

I. Main reagent

Table 135:

| Reagent name | Manufacturer | Article number |
|---|---|---|
| PBS | - | - |
| DMSO | SIGMA | D2650 |
| DMEM(biotin-free) | Provided by Baiyao Zhida Nano-biotechnology Co., Ltd. | YS3160 |
| FBS | Excell Bio | FSP500 |
| Double-antibody | gibco | 15140-122 |
| pancreatin | gibco | 25200-056 |
| CCK8 kit | Biyuntian Company | C0038 |

II. Main consumables and instruments

Table 136:

| **Name** | **Manufacturer** | **Model** |
|---|---|---|
| 96-well cell culture plate | NEST | 701001 |
| Biosafety cabinet | Beijing Donglian Haer Instrument Manufacturing Company | BSC-1360 II A2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Microplate reader | Shanghai Ouying Experimental Equipment Co., Ltd. | K3 |

III. Cell information

HepG2 (from Concord Cell Bank), a medium is DMEM+10% FBS+1% double antibody (gibco, 15140-122), and culture conditions are 37 DEG C, 5% CO2 and saturated humidity.

IV. Experiment material

1. Samples to be tested

Blank carrier: DNA nanoparticle carriers formed by the self-assembly of the previous D-8, D-9, D-10, D-11, D-12, D-13 and D-14 in Embodiment 10, respectively marked as: D-8, D-9, D-10, D-11, D-12, D-13 and D-14.

Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 10 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

Original drug doxorubicin.

DMSO.

V. Experiment procedure

1. HepG2 cells in a logarithmic growth phase are taken, trypan blue is used for staining and the cell viability is counted to be 98.3%, plating is performed with 5000 Cell/well, a volume is 100 μL/well, 8 96-well plates are paved with 57 wells per plate, and it is incubated overnight at 37 DEG C.
2. A sample to be tested is diluted according to the following table and added: the original culture medium is removed and 100 μL of a culture medium of the sample to be tested with the different concentration is added, and there are 3 replicate wells in each group.

Table 137:

| Well number | C9 | C8 | C7 | C6 | C5 | C4 | C3 | C2 | C1 |
|---|---|---|---|---|---|---|---|---|---|
| Final concentration of loaded drug | 10μM | 3.16μM | 1μM | 316nM | 100nM | 31.6nM | 10nM | 3.16nM | 1nM |
| Final concentration of blank carrier | 1μM | 316nM | 100nM | 31.6nM | 10nM | 3.16nM | 1nM | 0.316nM | 0.1nM |
| Final concentration of original drug doxorubicin | 10μM | 3.16μM | 1μM | 316nM | 100nM | 31.6nM | 10nM | 3.16nM | 1nM |
| DMSO(%) | 0.1 | 0.0316 | 0.01 | 0.00316 | 0.001 | 0.00036 | 0.0001 | 0.000036 | 0.00001 |

In the present embodiment, the loaded drug and the blank carrier are firstly prepared into 100 μM of stock solution by using the PBS, and then diluted with the complete culture medium (biotin-free DMEM). The original drug doxorubicin is firstly prepared into 100 μM of stock solution by the DMSO, and then diluted with the complete culture medium (biotin-free DMEM). The DMSO is directly diluted with the complete medium (biotin-free DMEM).

3. After the sample to be tested is added, the 96-well plate is placed in an incubator under 37 DEG C and 5% CO2 and incubated for 72 hours.

4. A kit is taken out and melted at a room temperature, 10 μL of CCK-8 solution is added to each well, or the CCK8 solution is mixed with the culture medium at a ratio of 1:9, and then added to the well in an amount of 100 μL/well.

5. It is continuously incubated in the cell incubator for 4 hours, and a length of time depends on experimental conditions such as a cell type and a cell density.

6. A microplate reader is used to measure an absorbance at 450 nm.

7. Calculation: cell viability (%)=(OD experimental group-OD blank group)×100%/(OD control group-OD blank group), IC50 is calculated by GraphPad Prism 5.0.

VI. Experiment result

Table 138:

| Cell line | Detected sample | $IC_{50}$ (μM) |
|---|---|---|
| HepG2 | Original drug doxorubicin | 0.2725 |
| | D-8-doxorubicin (loaded drug) | 0.05087 |
| | D-8 (blank carrier) | >1 |
| | D-9-doxorubicin (loaded drug) | 0.0386 |
| HepG2 | D-9 (blanking carrier) | >1 |
| | D-10-doxorubicin (loaded drug) | 0.03955 |
| | D-10 (blank carrier) | >1 |
| | D-11-doxorubicin (loaded drug) | 0.04271 |
| HepG2 | D-11 (blank carrier) | >1 |
| | D-12-doxorubicin (loaded drug) | 0.02294 |
| | D-12 (blank carrier) | >1 |
| | D-13-doxorubicin (loaded drug) | 0.03017 |
| HepG2 | D-13 (blank carrier) | >1 |
| | D-14-doxorubicin (loaded drug) | 0.03458 |
| | D-14 (blank carrier) | >1 |
| | DMSO | >0.1% |

Conclusion:

It may be seen from the above table and Fig. 60a, Fig. 60b, Fig. 60c, Fig. 60d, Fig. 60e, Fig. 60f. Fig. 60g and Fig. 60h that the IC50 of the original drug doxorubicin and the loaded drugs D-8-doxorubicin, D-9-doxorubicin, D-10-doxorubicin, D-11-doxorubicin, D-12-doxorubicin, D-13-doxorubicin and D-14-doxorubicin acting on the HepG2 cells is 0.2725 μM, 0.05087 μM, 0.0386, 0.03955, 0.04271, 0.02294, 0.03017 and 0.03458 respectively; the IC50 of the DMSO acting on the HepG2 cells is >0.1%; the IC50 of the D-8 (blank carrier), D-9 (blank carrier), D-10 (blank carrier), D-11 (blank carrier), D-12 (blank carrier), D-13 (blank carrier) and D-14 (blank carrier) acting on the HepG2 cells is >1 μM. It is indicated that for the HepG2 cell line, compared with the simple blank carriers D-8, D-9, D-10, D-11, D-12, D-13 and D-14, the original drug doxorubicin of small molecular drug and the loaded drugs D-8-doxorubicin, D-9-doxorubicin, D-10-doxorubicin, D-11-doxorubicin, D-12-doxorubicin, D-13-doxorubicin Both D-14-doxorubicin and D-14-doxorubicin are toxic to the HepG2 cells, and compared with the original drug doxorubicin, the loaded drugs D-8-doxorubicin, D-9-doxorubicin, D-10-doxorubicin, and D-11-doxorubicin, D-12-doxorubicin, D-13-doxorubicin and D-14-doxorubicin have

an apparent synergistic effect.

Embodiment 33

According to the chemical loading method (unless otherwise specified, the method is the same as that of Embodiment 5) of Embodiment 5, the DNA nanoparticles formed by the self-assembly of the previous D-10 and D-14 in the above Embodiment 27 are used as a daunorubicin loading carrier. A microplate reader is used to measure an absorbance of the daunorubicin at 492 nm, and a standard curve (as shown in Fig.61) is drawn.

Measured loading rates of the daunorubicin are respectively as follows:
The daunorubicin loading rate of the DNA nanoparticles D-10 is 24.0.

The daunorubicin loading rate of the DNA nanoparticles D-14 is 25.1.

It may be seen from the above description that the above embodiments of the disclosure achieve the following technical effects: the disclosure provides a series of nucleic acid nanoparticle carriers with thermodynamic stability, chemical stability, high loading rate and multivalent combination modules. A unique modular design of this type of the carriers is performed to obtain a core modular structure which not only maintains a naturally compatible affinity, but also has high stability and diverse combination characteristics. This structure may flexibly and efficiently integrate various functional modules, including a targeting module, an imaging and probe module, a treatment module and other composite intelligent modules, so that it may be used for targeted delivery in vivo to achieve precise diagnosis and treatment.

Through loading the small molecular drugs such as the dihydroartemisinin, cisplatin, oxaliplatin, flavonoids or vincristine on the nucleic acid nanoparticle carrier provided by the disclosure to form the nucleic acid nanocarrier drug, not only the delivery stability of the drug may be improved, but also in the case that the nucleic acid nanoparticles carry the target head, on the one hand, the drug is targed and delivered to the target cells, and the bioavailability of the drug is imporved; and on the other hand, the toxic and side effects to the non-target cells or tissues are reduced due to the targeted delivery, and the local drug concentration is reduced, thus the toxic and side effects caused by the high drug concentration are further reduced.

The above are only the preferred embodiments of the disclosure, and are not used to limit the disclosure, and various modifications and changes may be made to the disclosure by those skilled in the art. Any modifications, equivalent replacements, improvements and the like made within spirit and principle of the disclosure should be included in a scope of protection of the disclosure.

## Claims

1. A nucleic acid nanocarrier drug, wherein the drug comprises nucleic acid nanoparticles and a drug ingredient, the drug ingredient is loaded on the nucleic acid nanoparticles, and the drug ingredient is dihydroartemisinin, cisplatin, oxaliplatin, flavone or vincristine; and
the nucleic acid nanoparticles comprise a nucleic acid structureal domain, the nucleic acid structureal domain comprises a sequence a, a sequence b, and a sequence c, the sequence a comprises a sequence a1 or a sequence obtained by insertion, deletion or substitution of at least one base in the sequence a1, the sequence b comprises a sequence b1 or a sequence obtained by insertion, deletion or substitution of at least one base in the sequence b1, and the sequence c comprises a sequence c1 or a sequence obtained by insertion, deletion or substitution of at least one base in the sequence c1,
wherein the sequence a1 is SEQ ID NO:1:5'-CCAGCGUUCC-3'or SEQ ID NO:2 :5' -CCAGCGTTCC-3';
the sequence b1 is SEQ ID NO:3:5'-GGUUCGCCG-3'or SEQ ID NO:4:5'-GGTTCGCCG-3'; and
the sequence c1 is SEQ ID NO:5:5'-CGGCCAUAGCGG-3'or SEQ ID NO:6:5' -CGGCCATAGCGG-3'.

2. The drug according to claim 1, wherein when the sequence a1 is the SEQ ID NO:1, the sequence b1 is the SEQ ID NO:3, and the sequence c1 is the SEQ ID NO:5, at least one sequence of the sequence a, the second b and the sequence c comprises a sequence obtained by insertion, deletion or substitution of at least one base in the sequence a, the sequence b and/or the sequence c.

3. The drug according to claim 1 or 2, wherein the insertion, deletion or substitution of at least one base is occurred:

   (1) at 1, 2, 4 or 5-th base starting from a 5 '-end of the sequence shown in the SEQ ID NO:1 or the SEQ ID NO:2; and/or
   (2) between 8-th and 10-th bases starting from the 5 '-end of the sequence shown in the SEQ ID NO:1 or the SEQ ID NO:2; and/or
   (3) between 1-th and 3-th bases starting from a 5 '-end of the sequence shown in the SEQ ID NO:3 or the SEQ ID NO:4; and/or
   (4) between 6-th and 9-th bases starting from the 5 '-end of the sequence shown in the SEQ ID NO:3 or the SEQ ID NO:4; and/or
   (5) between 1-th and 4-th bases starting from a 5 '-end of the sequence shown in the SEQ ID NO:5 or the SEQ ID NO:6; and/or
   (6) between 9-th and 12-th bases starting from the 5 '-end of the sequence shown in the SEQ ID NO:5 or the SEQ ID NO:6.

4. The drug according to claim 1 or 2, wherein the sequence a, the sequence b and the sequence c are self-assembled into a structure shown in Formula (1):

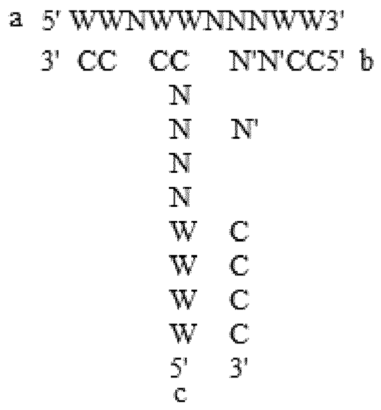

Formula (1),

wherein, W-C represents a Watson-Crick pairing, N and N' represent a non-Watson-Crick pairing, the W-C in any one position is independently selected from C-G or G-C;
in the sequence a, the first N from the 5'-end is A, the second N is G, the third N is U or T, and the fourth N is any one of U, T, A, C or G;
in the sequence b, the first N' from the 5'-end is any one of U, T, A, C or G, the second N' is U or T, and the third N' is C; and
in the sequence c, a sequence NNNN along a direction from the 5'-end to the 3'-end is CAUA or CATA;
preferably, the sequence a, the sequence b and the sequence c are any one of the following groups:

(1)

sequence a: 5'-GGAGCGUUGG-3',
sequence b: 5'-CCUUCGCCG-3',
sequence c: 5'-CGGCCAUAGCCC-3';

(2)

sequence a: 5'-GCAGCGUUCG-3',
sequence b: 5'-CGUUCGCCG-3',
sequence c: 5'-CGGCCAUAGCGC-3';

(3)

sequence a: 5'-CGAGCGUUGC-3',
sequence b: 5'-GCUUCGCCG-3',
sequence c: 5'-CGGCCAUAGCCG-3';

(4)

sequence a: 5'-GGAGCGUUGG-3',
sequence b: 5'-CCUUCGGGG-3',
sequence c: 5'-CCCCCAUAGCCC-3';

(5)

sequence a: 5'-GCAGCGUUCG-3',
sequence b: 5'-CGUUCGGCG-3',
sequence c: 5'-CGCCCAUAGCGC-3';

(6)

sequence a: 5'-GCAGCGUUCG-3',
sequence b: 5'-CGUUCGGCC-3',

sequence c: 5'-GGCCCAUAGCGC-3';

(7)

sequence a: 5'-CGAGCGUUGC-3',
sequence b: 5'-GCUUCGGCG-3',
sequence c: 5'-CGCCCAUAGCCG-3';

(8) sequence a: 5'-GGAGCGTTGG-3', sequence b: 5'-CCTTCGCCG-3', sequence c: 5'-CGGCCATAGCCC-3';
(9)

sequence a: 5'-GCAGCGTTCG-3',
sequence b: 5'-CGTTCGCCG-3',
sequence c: 5'-CGGCCATAGCGC-3';

(10)

sequence a: 5'-CGAGCGTTGC-3',
sequence b: 5'-GCTTCGCCG-3',
sequence c: 5'-CGGCCATAGCCG-3';

(11)

sequence a: 5'-GGAGCGTTGG-3',
sequence b: 5'-CCTTCGGGG-3',
sequence c: 5'-CCCCCATAGCCC-3';

(12)

sequence a: 5'-GCAGCGTTCG-3',
sequence b: 5'-CGTTCGGCG-3',
sequence c: 5'-CGCCCATAGCGC-3';

(13)

sequence a: 5'-GCAGCGTTCG-3',
sequence b: 5'-CGTTCGGCC-3',
sequence c: 5'-GGCCCATAGCGC-3';

(14)

sequence a: 5'-CGAGCGTTGC-3',
sequence b: 5'-GCTTCGGCG-3',
sequence c: 5'-CGCCCATAGCCG-3'; and

(15)

sequence a: 5'-CGAGCGTTCC -3',
sequence b: 5'-GGTTCGCCG -3',
sequence c: 5'-CGGCCATAGCCG-3'.

**5.** The drug according to claim 3, wherein in the nucleic acid structureal domain, a first extension fragment is further comprised, the first extension fragment is an extension fragment of Watson-Crick pairing, and the first extension fragment is positioned at the 5'-end and/or the 3'-end of any one sequence of the sequence a, the sequence b or the sequence c;
preferably, the first extension fragment is at least selected from any one of the following groups:

(1): a-strand 5'-end: 5'-CCCA-3', c-strand 3'-end: 5'-UGGG-3';

(2): a-strand 3'-end: 5'-GGG-3', b-strand 5'-end: 5'-CCC-3';
(3): b-strand 3'-end: 5'-CCA-3', c-strand 5'-end: 5'-UGG-3';
(4): a-strand 5'-end: 5'-CCCG-3', c-strand 3'-end: 5'-CGGG-3';
(5): a-strand 5'-end: 5'-CCCC-3', c-strand 3'-end: 5'-GGGG-3';
(6): b-strand 3'-end: 5'-CCC-3', c-strand 5'-end: 5'-GGG-3';
(7): b-strand 3'-end: 5'-CCG-3', c-strand 5'-end: 5'-CGG-3';
(8): a-strand 5'-end: 5'-CCCA-3', c-strand 3'-end: 5'-TGGG-3'; and
(9): b-strand 3'-end: 5'-CCA-3', c-strand 5'-end: 5'-TGG-3'.

**6.** The drug according to any one of claims 1 to 5, wherein the nucleic acid structureal domain further comprises a second extension fragment, the second extension fragment is positioned at the 5'-end and/or the 3'-end of any one sequence of the sequence a, the sequence b or the sequence c, and the second extension fragment is an extension fragment of Watson-Crick pairing;
preferably, the second extension fragment is an extension sequence of a CG base pair;
more preferably, the second extension fragment is an extension sequence of 1-10 CG base pairs;
preferably, the nucleic acid structureal domain further comprises at least one group of the following second extension fragments:

a first group: a-strand 5'-end: 5'-CGCGCG-3', c-strand 3'-end: 5'-CGCGCG-3';
a second group: a-strand 3'-end: 5'-CGCCGC-3', b-strand 5'-end: 5'-GCGGCG-3'; and
a third group: b-strand 3'-end: 5'-GGCGGC-3', c-strand 5'-end: 5'-GCCGCC-3'.

**7.** The drug according to claim 6, wherein the second extension fragment is an extension sequence containing both CG base pair and AT/AU base pair, and preferably the second extension fragment is an extension sequence of 2-50 base pairs;
preferably, the second extension fragment is an extension sequence in which sequences of 2-8 continuous CG base pairs and sequences of 2-8 continuous AT/AU base pairs are alternately arranged; or
preferably, the second extension fragment is an extension sequence in which a sequence of 1 CG base pair and a sequence of 1 AT/AU base pair are alternately arranged.

**8.** The drug according to any one of claims 1 to 7, wherein a base, a ribose and a phosphate in the sequence a, the sequence b and the sequence c have at least one modifiable site, and any one of the modifiable sites is modified by any one of the following modificationadaptors: -F, a methyl, an amino, a disulfide, a carbonyl, a carboxyl, a sulfhydryl and a formyl; and
preferably, the base C or U in the sequence a, the sequence b and the sequence c has 2'-F modification.

**9.** The drug according to any one of claims 1 to 8, wherein the drug ingredient is loaded on the nucleic acid nanoparticles in a physical linkage mode and/or a covalent linkage mode, and a molar ratio between the drug ingredient and the nucleic acid nanoparticles is 2-300:1, preferably 10-50:1, and more preferably 15-25:1.

**10.** The drug according to any one of claims 1 to 9, wherein the nucleic acid nanoparticles further comprise a bioactive substance, the bioactive substance is linked with the nucleic acid structureal domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenols, a lecithin, and a small molecular drug except the dihydroartemisinin, the cisplatin, the oxaliplatin, the flavone and the vincristine.

**11.** The drug according to claim 10, wherein a relative molecular weight of the nucleic acid structureal domain is marked as N1, and a total relative molecular weight of the drug ingredient and the bioactive substance is marked as N2, N1/N2≥1:1; and
preferably, the bioactive substance is one or more of the target head, the fluorescein and the miRNA,
wherein the target head is positioned on any one sequence of the sequence a , the sequence b and the sequence c, preferably the 5'-end or the 3'-end of any one sequence of the sequence a, the sequence b and the sequence c, or inserted between GC bonds of the nucleic acid structureal domain,
the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and
preferably, the target head is a folic acid or a biotin, the fluorescein is any one or more of FAM, CY5 and CY3, and

the anti-miRNA is anti-miR-21.

**12.** The drug according to claim 10, wherein the small molecular drug except the dihydroartemisinin, the cisplatin, the oxaliplatin, the flavone and the vincristine is a drug containing any one or more of the following groups: an amino group, a hydroxyl group, a carboxyl group, a mercapto group, a benzene ring group and an acetamido group; and preferably, the protein is one or more of SOD, survivin, hTERT, EGFR and PSMA; the vitamin is L-Vc and/or esterified Vc; and the phenols is a tea polyphenols and/or a grape polyphenols.

**13.** The drug according to claim 1, wherein a particle size of the nucleic acid nanoparticles is 1-100 nm, preferably 5-50 nm; more preferably 10-30 nm; and further preferably 10-15 nm.

**14.** A method for preparing a nucleic acid nanocarrier drug, wherein the method comprises the following operations:

providing nucleic acid nanoparticles in the drug as claimed in any one of claims 1 to 13; and
loading the drug ingredient on the nucleic acid nanoparticles in a physical linkage mode and/or a covalent linkage mode, to obtain the nucleic acid nanocarrier drug, wherein the drug ingredient is dihydroartemisinin, cisplatin, oxaliplatin, flavone or vincristine.

**15.** The method according to claim 14, wherein the step of loading the drug ingredient in the physical linkage mode comprises:

mixing and stirring the drug ingredient, the nucleic acid nanoparticles and a first solvent to obtain a premixed system; and
precipitating the premixed system, to obtain the nucleic acid nanocarrier drug;
preferably, the first solvent is selected from one or more of DCM, DCC, DMAP, Py, DMSO, PBS and glacial acetic acid;
preferably, the step of precipitating the premixed system to obtain the nucleic acid nanocarrier drug comprises:

precipitating the premixed system, to obtain a precipitation; and
washing the precipitation to remove impurities, as to obtain the nucleic acid nanocarrier drug;
more preferably, mixing the premixed system with absolute ethyl alcohol, and precipitating at a temperature condition lower than 10 DEG C, to obtain the precipitation; and further preferably, precipitating at a temperature condition of 0-5 DEG C, to obtain the precipitation; and
more preferably, washing the precipitation to remove the impurities with 6-12 times of the absolute ethyl alcohol in volume, as to obtain the nucleic acid nanocarrier drug.

**16.** The method according to claim 14, wherein the step of loading the drug ingredient in the covalent linkage mode comprises:

preparing drug ingredient solution of the dihydroartemisinin, cisplatin, oxaliplatin, flavone or vincristine;
enabling the drug ingredient solution to react with the G-exocyclic amino of the nucleic acid nanoparticles under a mediating effect of the formaldehyde, to obtain a reaction system; and
purifying the reaction system, to obtain the nucleic acid nanocarrier drug;
preferably, the reaction step comprises:
mixing the drug ingredient solution with a paraformaldehyde solution and the nucleic acid nanoparticles, and reacting in a dark condition, to obtain the reaction system; wherein the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

**17.** The method according to any one of claims 14 to 16, wherein the preparation method further comprises a step of preparing the nucleic acid nanoparticles, the step comprises: self-assembling a single strand corresponding to the nucleic acid structureal domain in the drug according to any one of claims 1 to 13, to obtain the nucleic acid structureal domain;
preferably, after the nucleic acid structureal domain is obtained, the preparation method further comprises: loading the bioactive substance in the drug as claimed in any one of claims 10 to 12 on the nucleic acid structureal domain in the physical linkage mode and/or the covalent linkage mode, to obtain the nucleic acid nanoparticles.

**18.** The method according to claim 17, wherein in a process of loading the bioactive substance in the covalent linkage mode, the loading is performed through solvent covalent linkage, linker covalent linkage or click-linkage;
preferably, a third solvent used in the solvent covalent linkage is served as a linkage medium, and the third solvent is selected from one or more of paraformaldehyde, DCM, DCC, DMAP, Py, DMSO, PBS and glacial acetic acid;
preferably, the linker is selected from a disulfide bond, a p-phenylazide, bromopropyne or PEG;
preferably, the click-linkage is that a bioactive substance precursor and the nucleic acid structural domain is modified by alkynyl or azide simultaneously, and then linked through a clickreaction; and
preferably, the bioactive substance is linked with the nucleic acid structureal domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structureal domain is selected from a G-exocyclic amino, a 2'-hydroxyl, an A-amino or a 2'-hydroxyl.

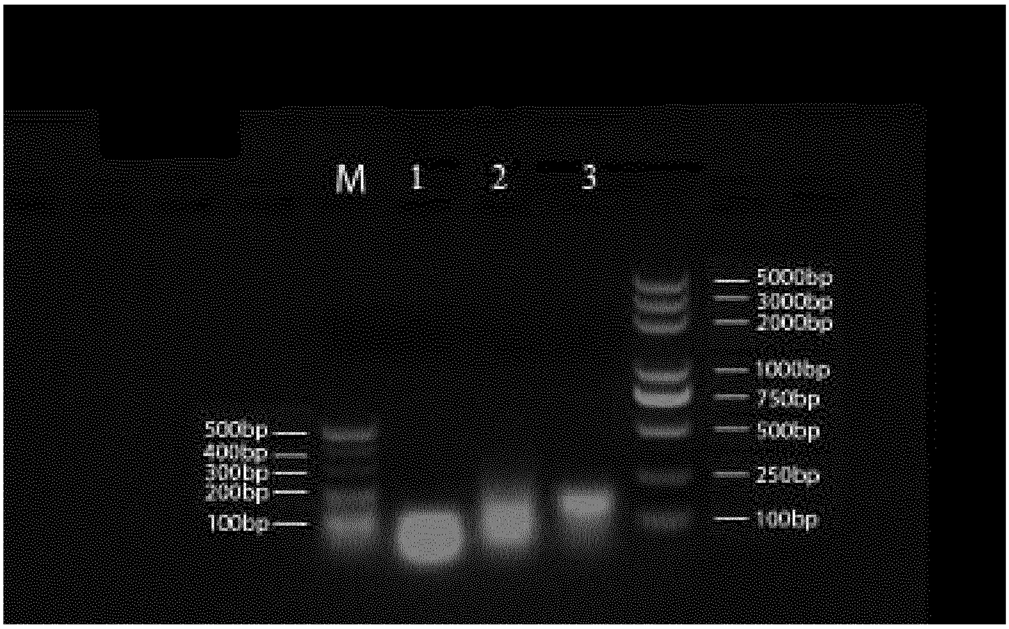
M 1 2 3
5000bp
3000bp
2000bp
1000bp
750bp
500bp
250bp
100bp
500bp
400bp
300bp
200bp
100bp

**Fig.1**

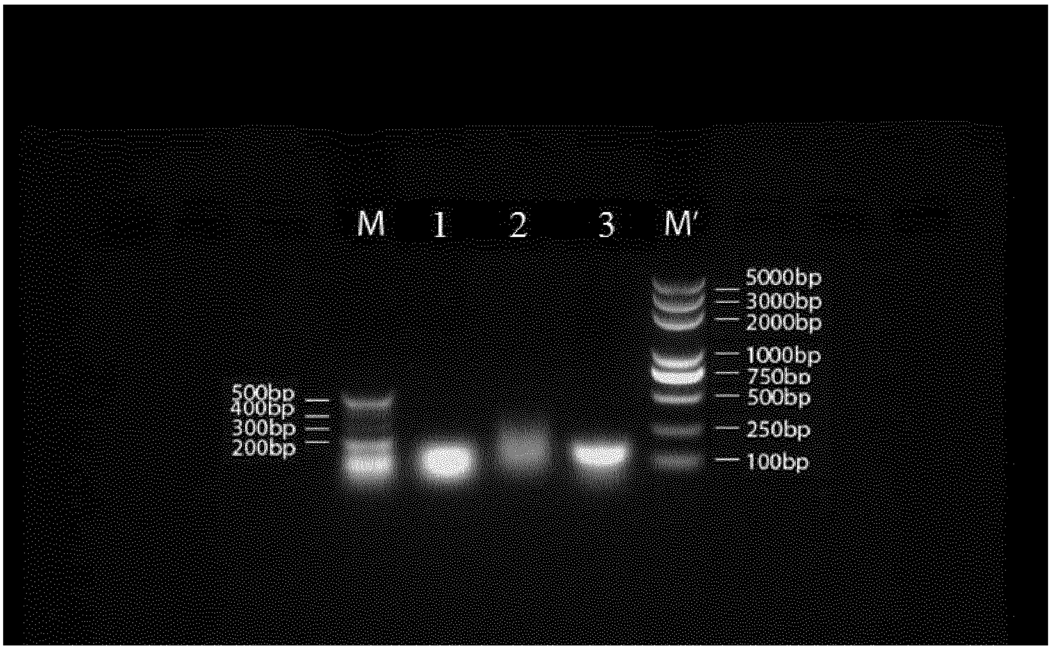
M 1 2 3 M′
5000bp
3000bp
2000bp
1000bp
750bp
500bp
250bp
100bp
500bp
400bp
300bp
200bp

**Fig.2**

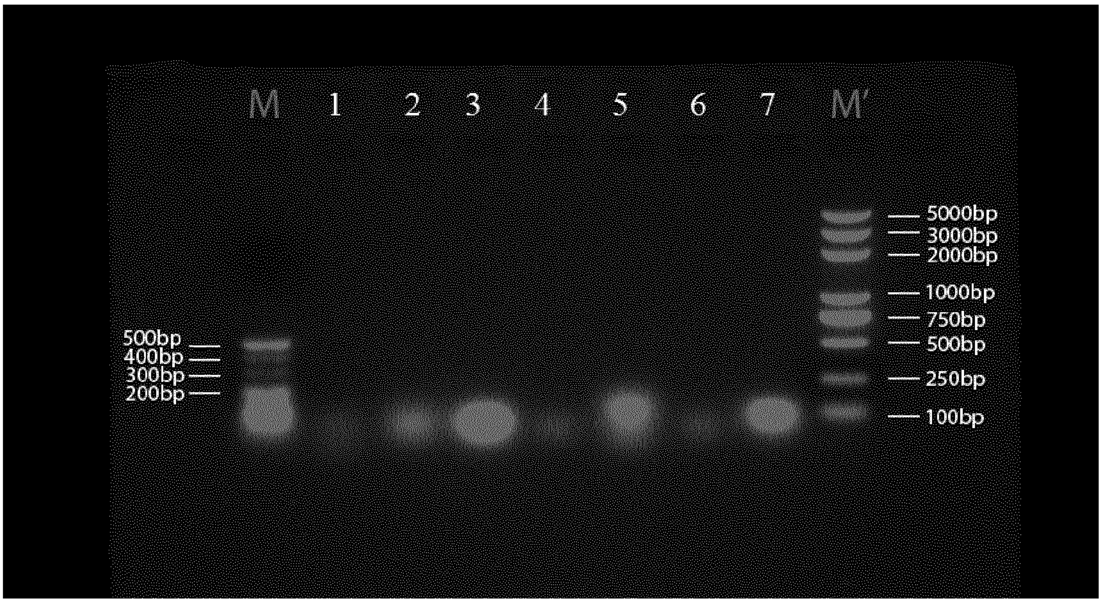
M
1
2
3
4
5
6
7
M′
5000bp
3000bp
2000bp
1000bp
750bp
500bp
250bp
100bp
500bp
400bp
300bp
200bp

Fig.3

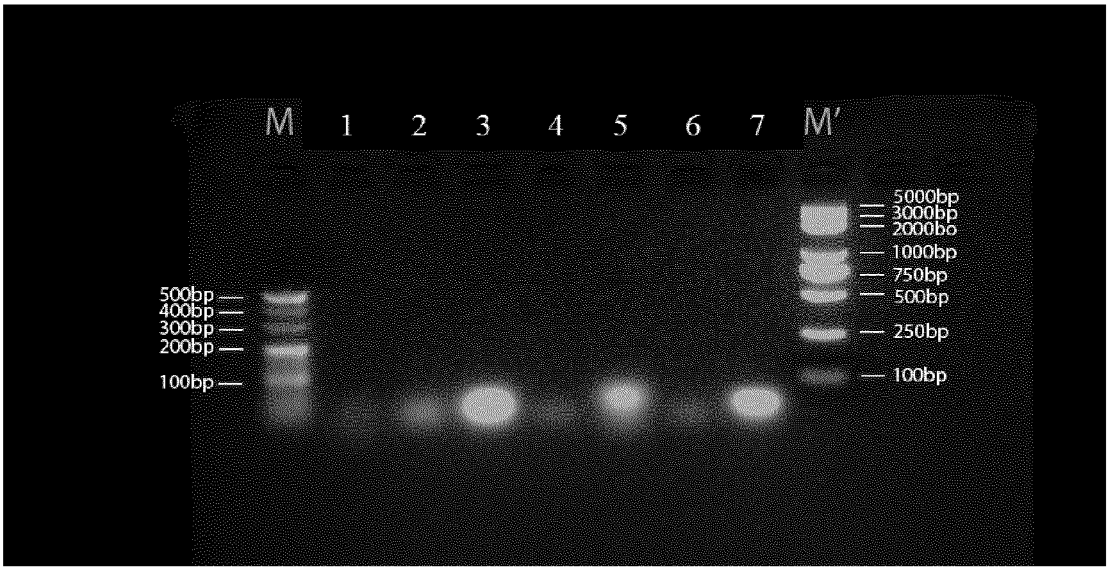
M
1
2
3
4
5
6
7
M′
5000bp
3000bp
2000bo
1000bp
750bp
500bp
250bp
100bp
500bp
400bp
300bp
200bp
100bp

Fig.4

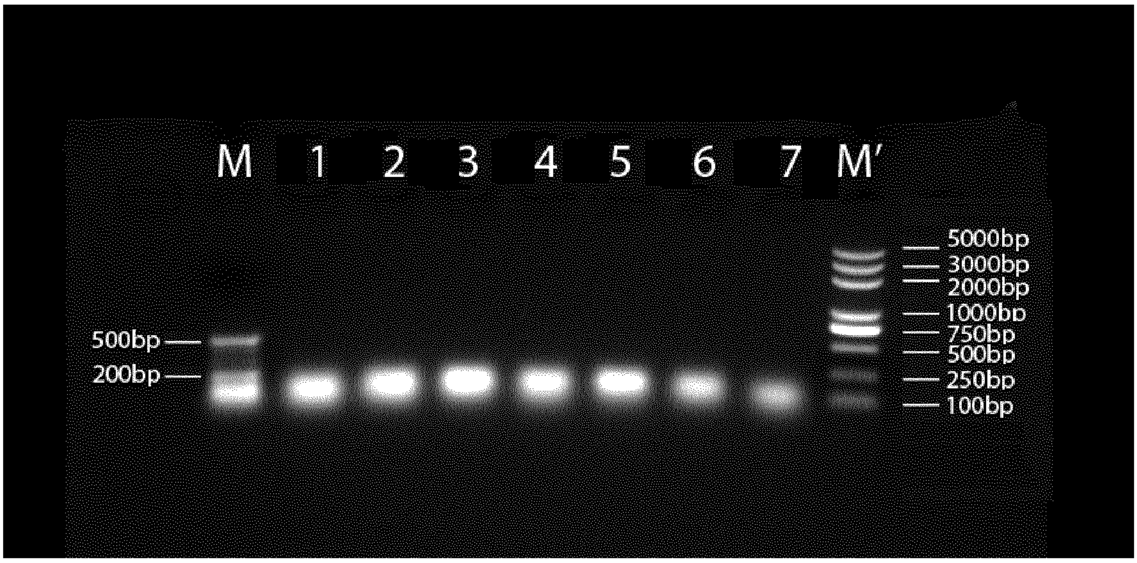
M
1
2
3
4
5
6
7
M′
500bp
200bp
5000bp
3000bp
2000bp
1000bp
750bp
500bp
250bp
100bp

Fig.5

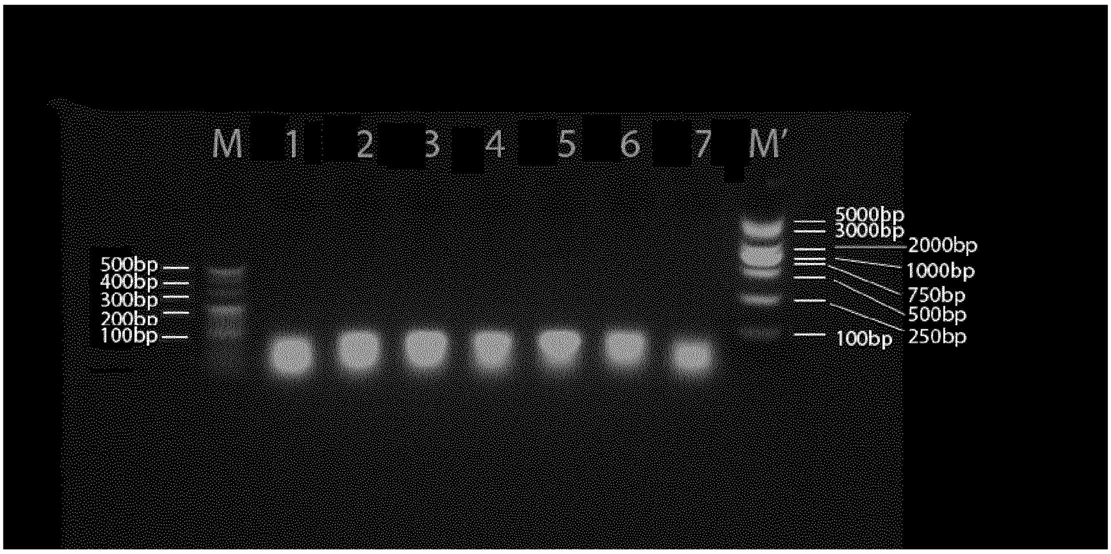
M
1
2
3
4
5
6
7
M′
500bp
400bp
300bp
200bp
100bp
5000bp
3000bp
2000bp
1000bp
750bp
500bp
250bp
100bp

Fig.6

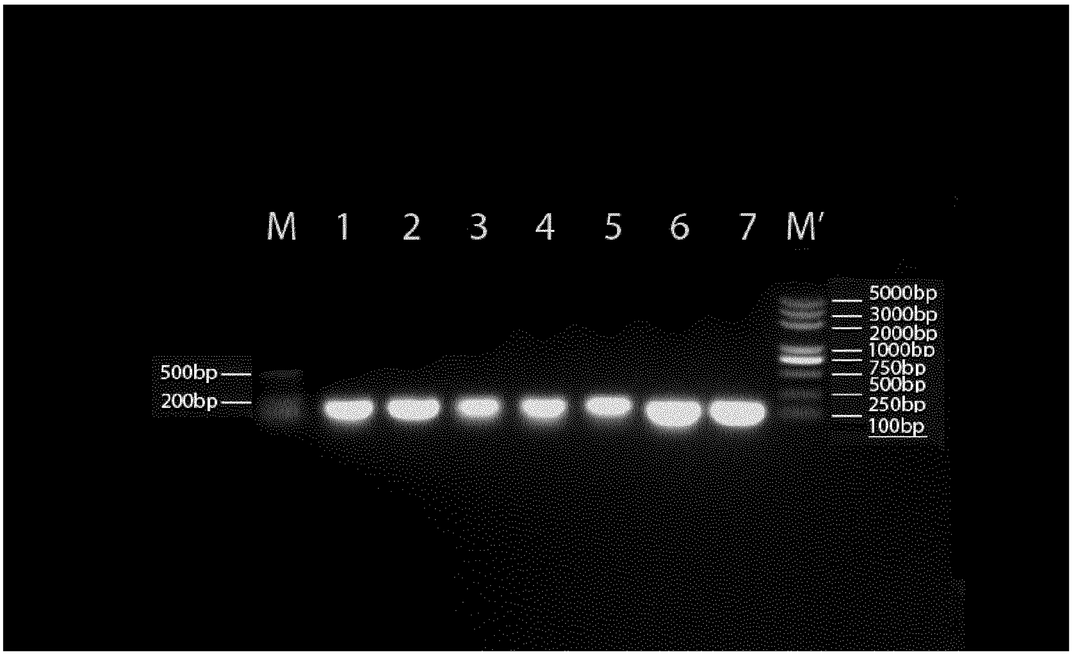
M 1 2 3 4 5 6 7 M'
500bp
200bp
5000bp
3000bp
2000bp
1000bp
750bp
500bp
250bp
100bp

Fig.7

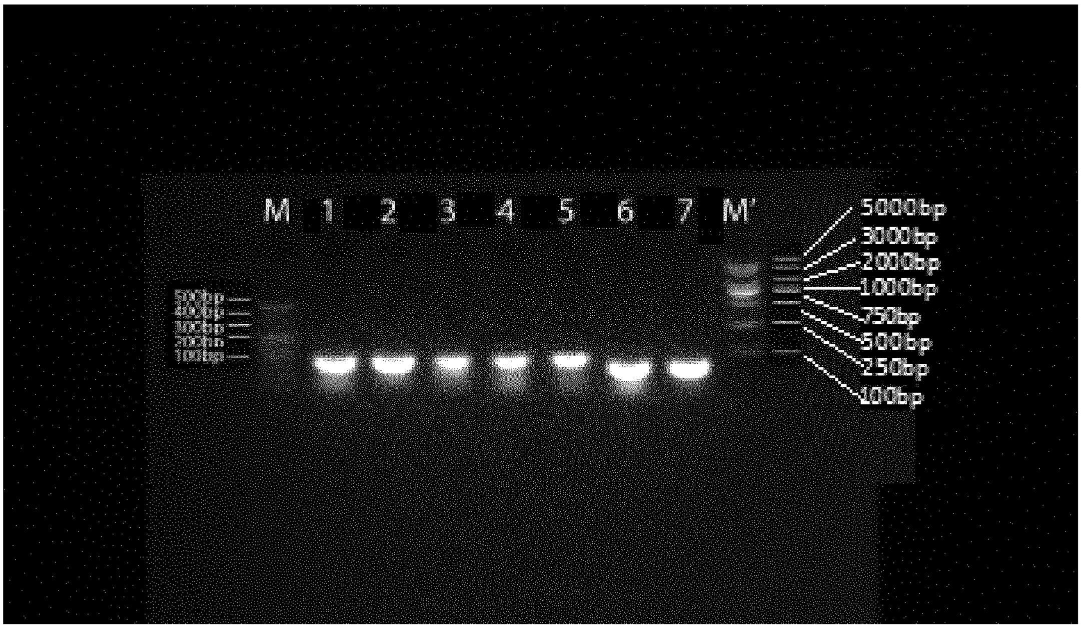
M 1 2 3 4 5 6 7 M'
5000bp
3000bp
2000bp
1000bp
750bp
500bp
250bp
100bp

Fig.8

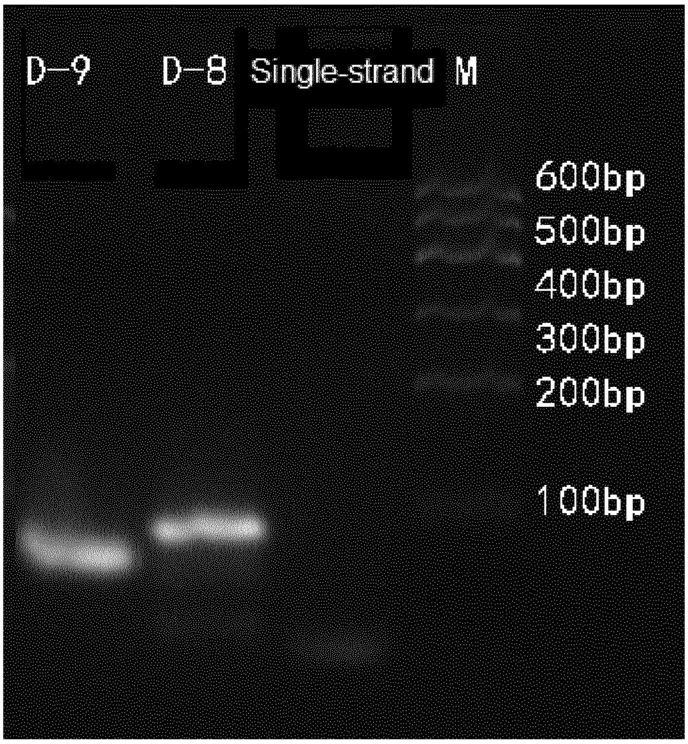


Fig.9

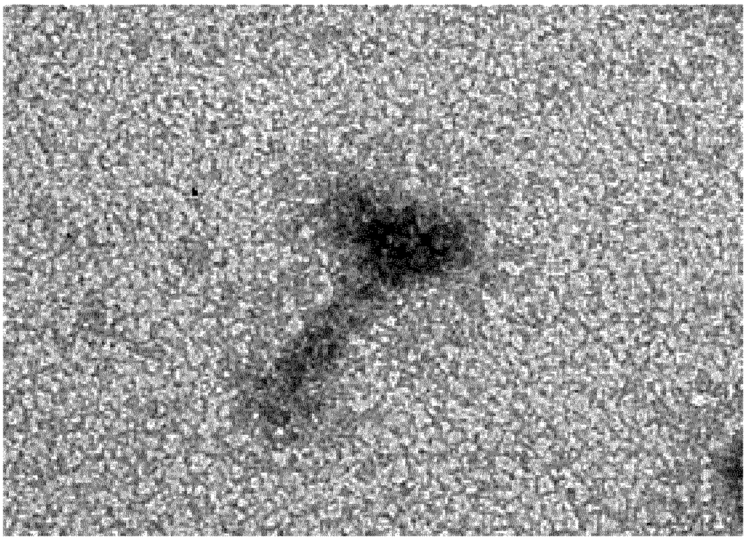

Fig. 10

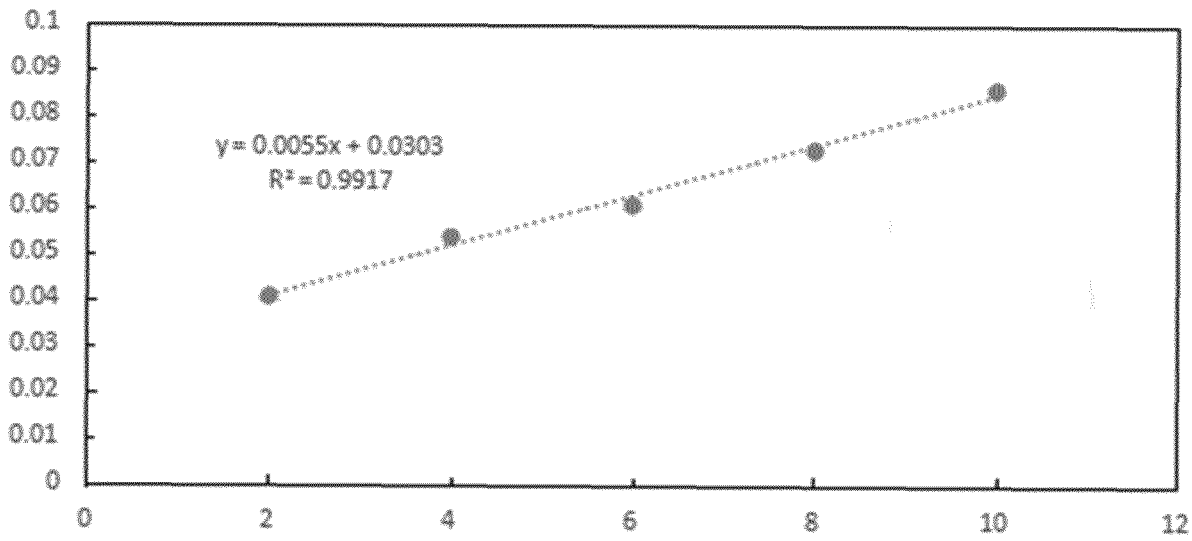
y = 0.0055x + 0.0303
R² = 0.9917
0.1
0.09
0.08
0.07
0.06
0.05
0.04
0.03
0.02
0.01
0
0
2
4
6
8
10
12

Fig. 11

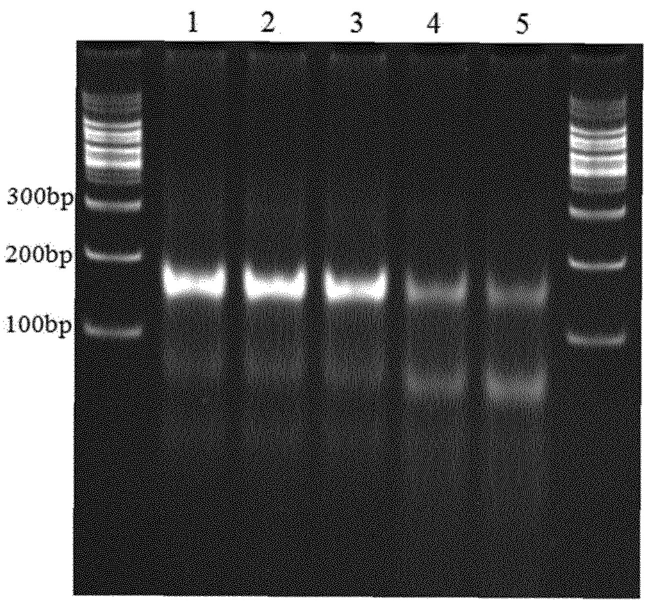
1
2
3
4
5
300bp
200bp
100bp

Fig. 12

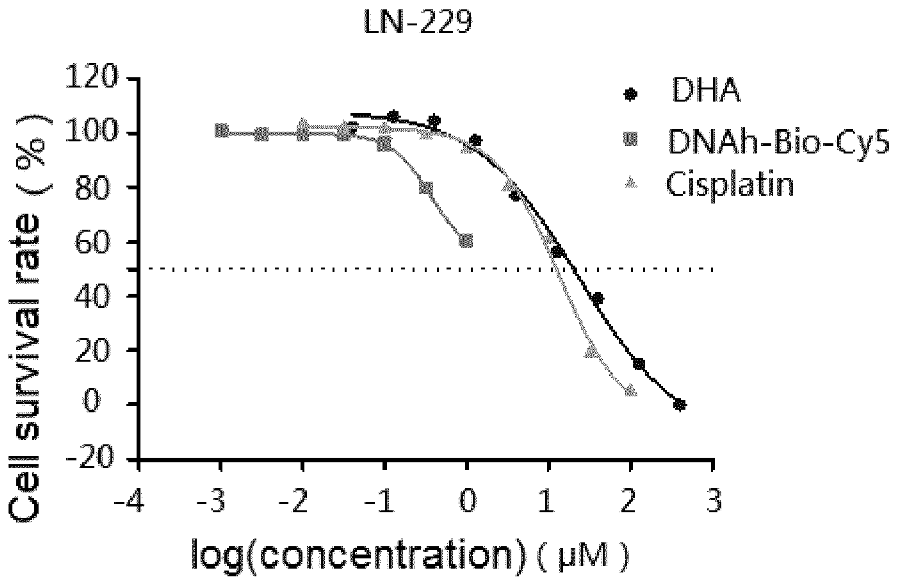
LN-229
Cell survival rate（%）
log(concentration)（μM）
DHA
DNAh-Bio-Cy5
Cisplatin
120
100
80
60
40
20
0
-20
-4
-3
-2
-1
0
1
2
3

**Fig. 13**

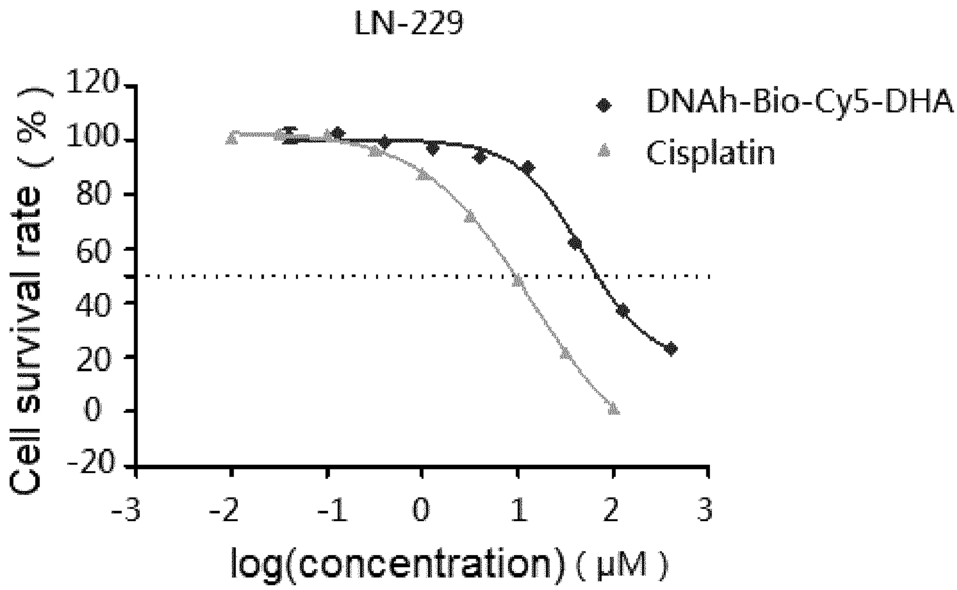
LN-229
Cell survival rate（%）
log(concentration)（μM）
DNAh-Bio-Cy5-DHA
Cisplatin
120
100
80
60
40
20
0
-20
-3
-2
-1
0
1
2
3

**Fig. 14**

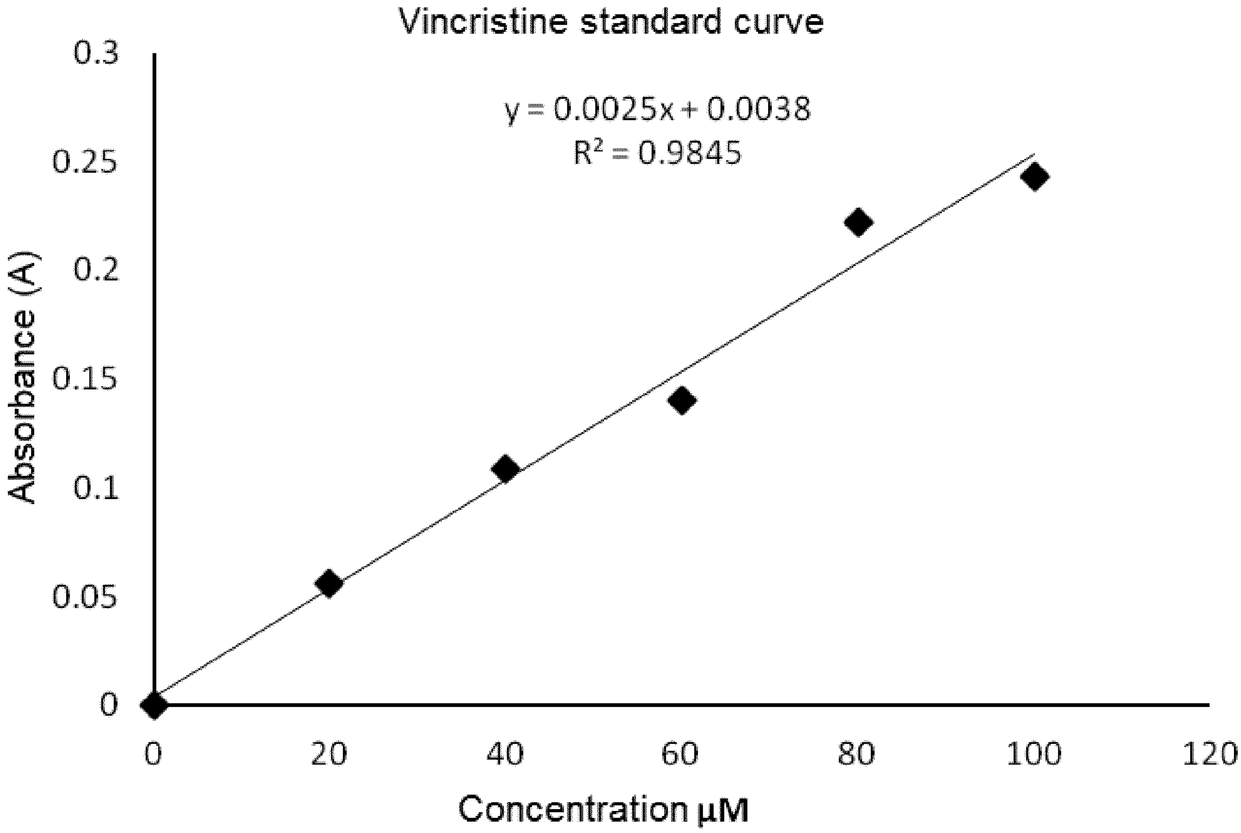
Vincristine standard curve
y = 0.0025x + 0.0038
R² = 0.9845
Absorbance (A)
0.3
0.25
0.2
0.15
0.1
0.05
0
0
20
40
60
80
100
120
Concentration µM

Fig. 15

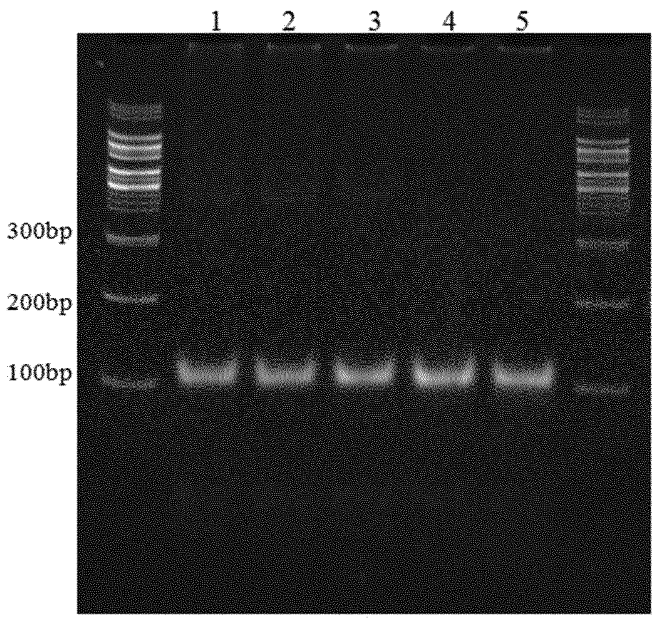
1
2
3
4
5
300bp
200bp
100bp

Fig. 16

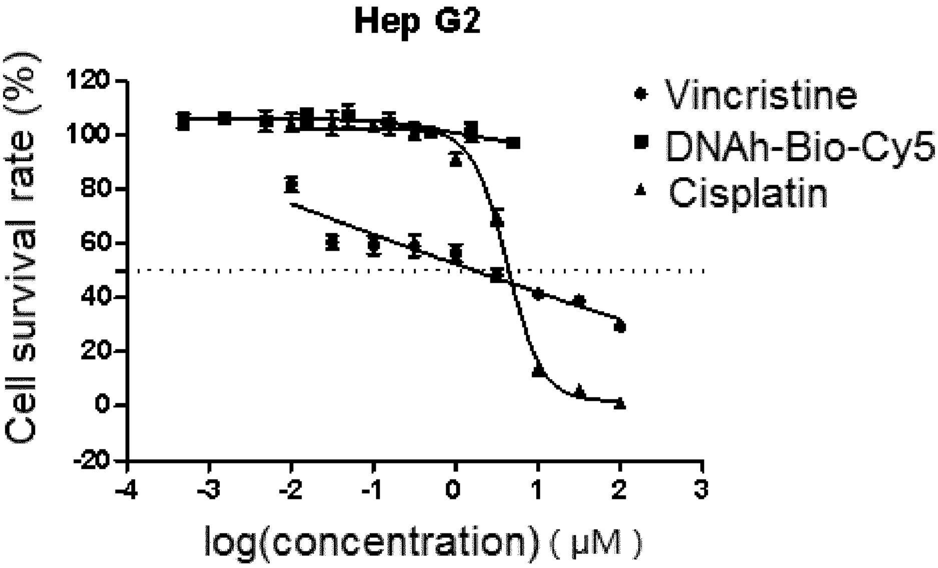


Fig. 17a

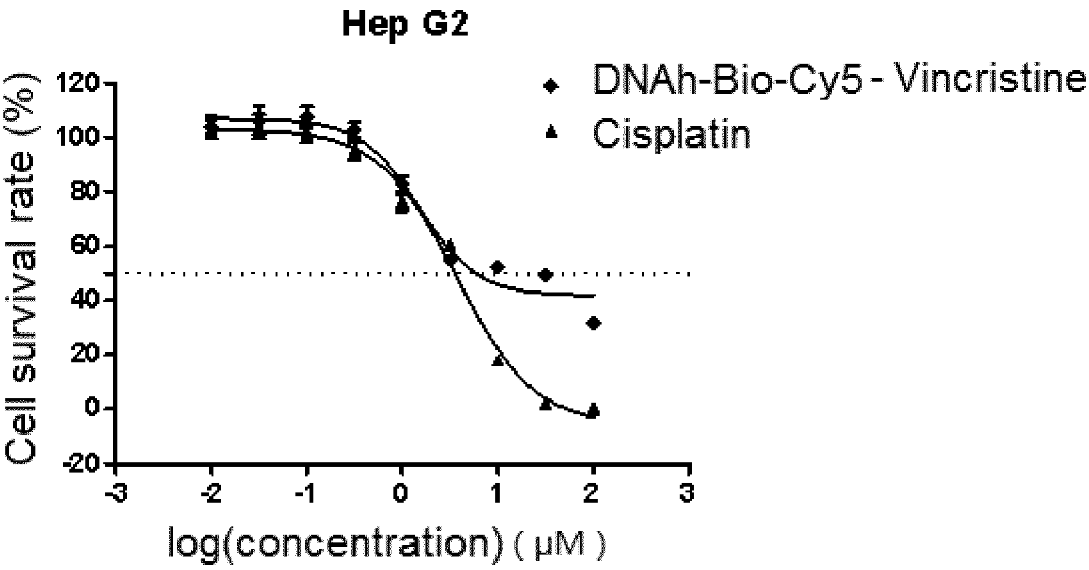


Fig. 17b

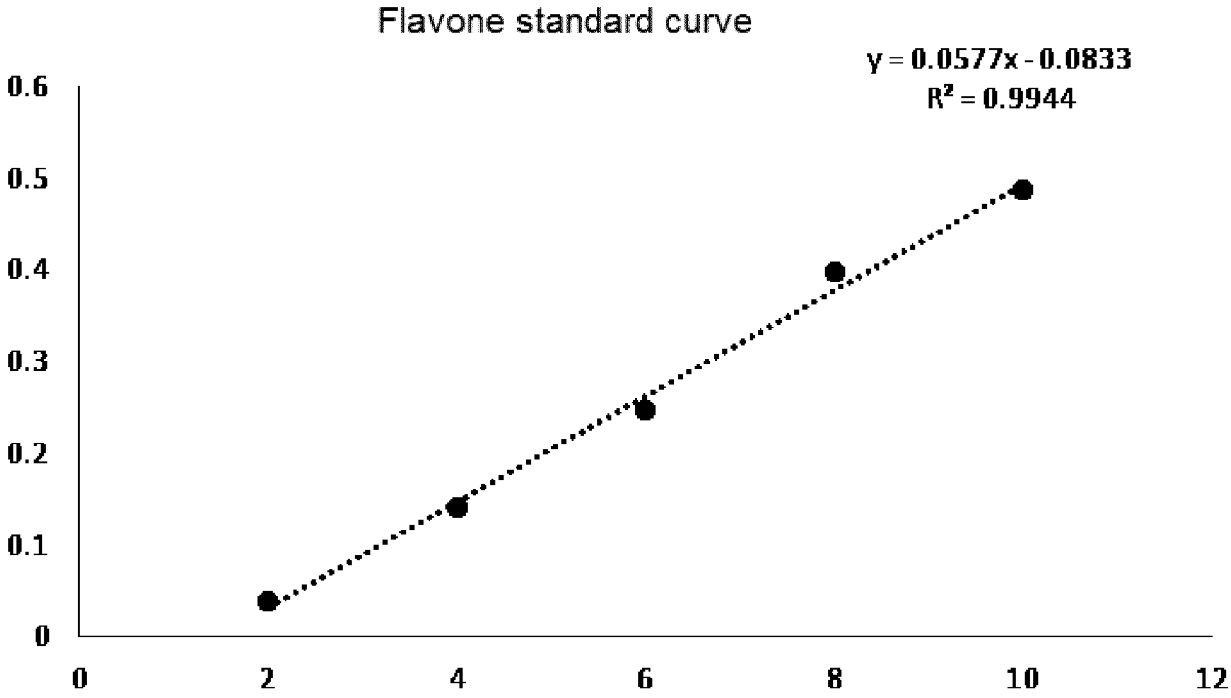
Flavone standard curve
y = 0.0577x - 0.0833
R² = 0.9944
0.6
0.5
0.4
0.3
0.2
0.1
0
0
2
4
6
8
10
12

Fig. 18

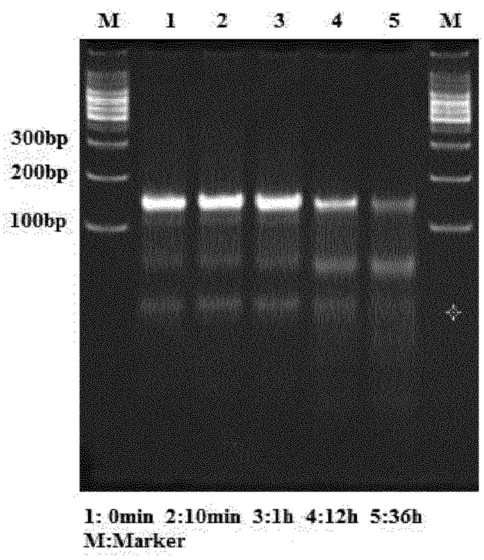
M 1 2 3 4 5 M
300bp
200bp
100bp
1: 0min 2:10min 3:1h 4:12h 5:36h
M:Marker

Fig. 19

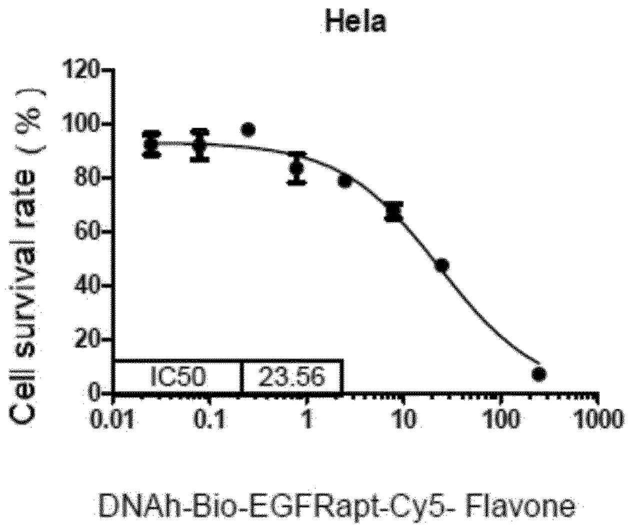


Fig. 20a

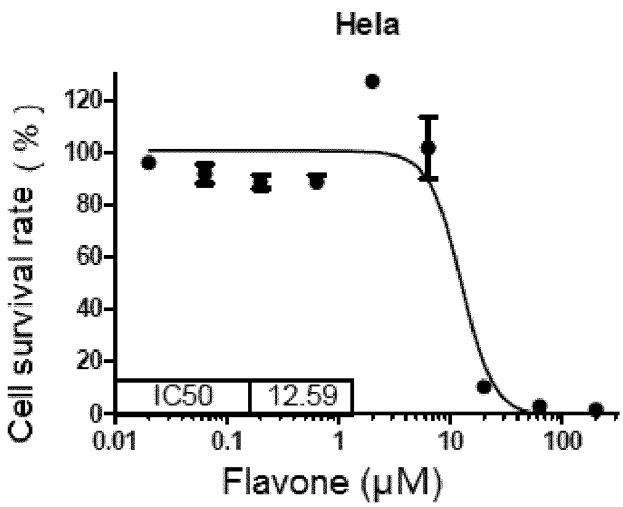


Fig. 20b

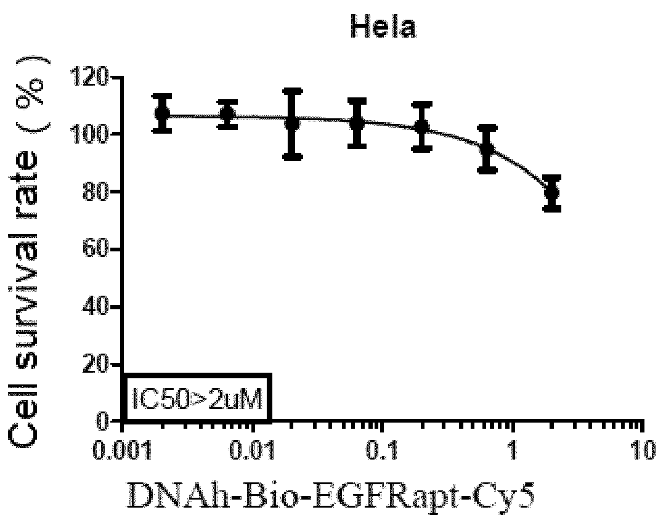

Fig. 20c

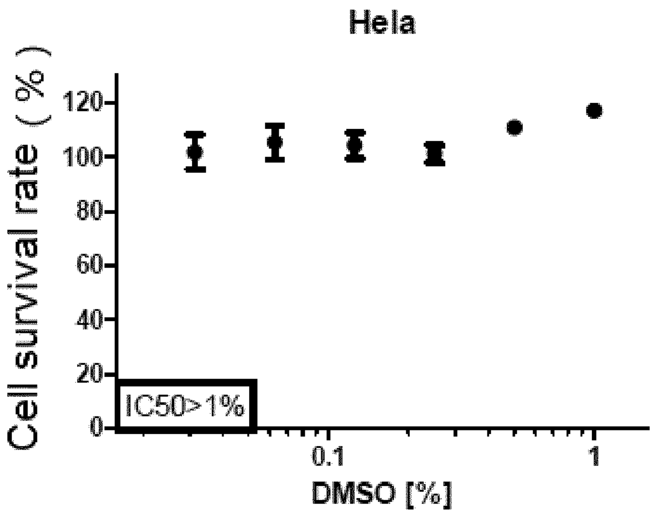


Fig. 20d

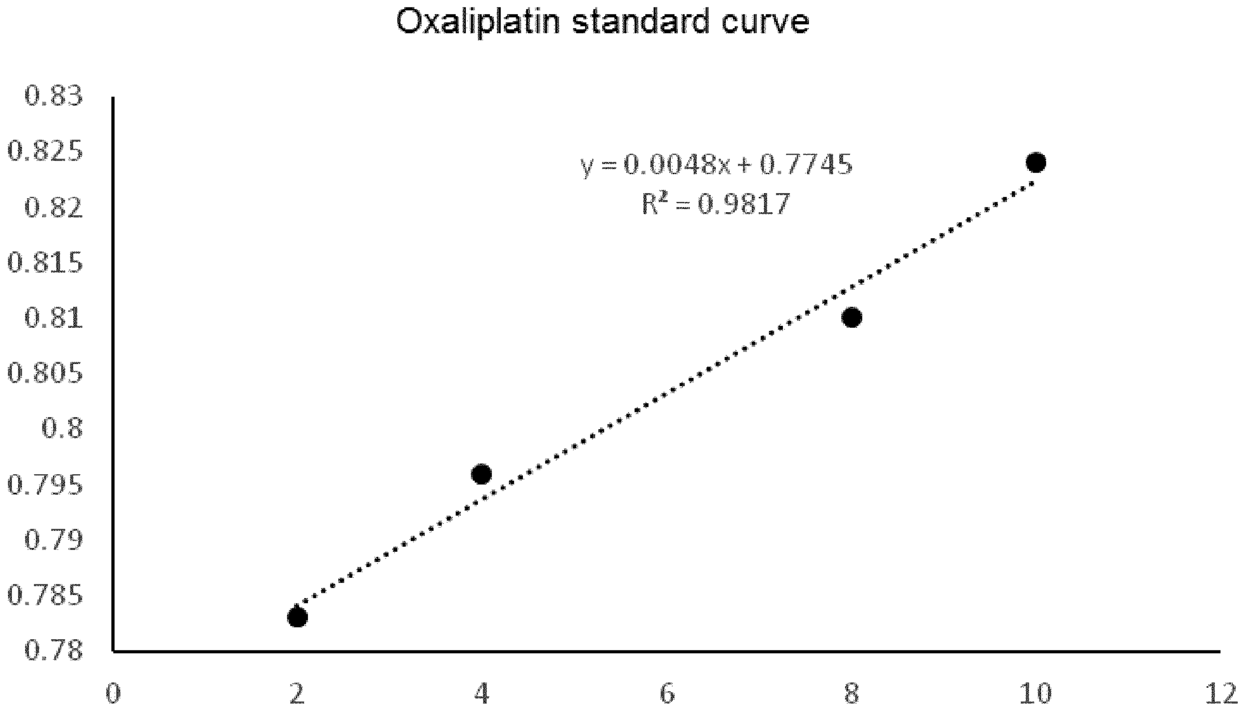


Fig. 21

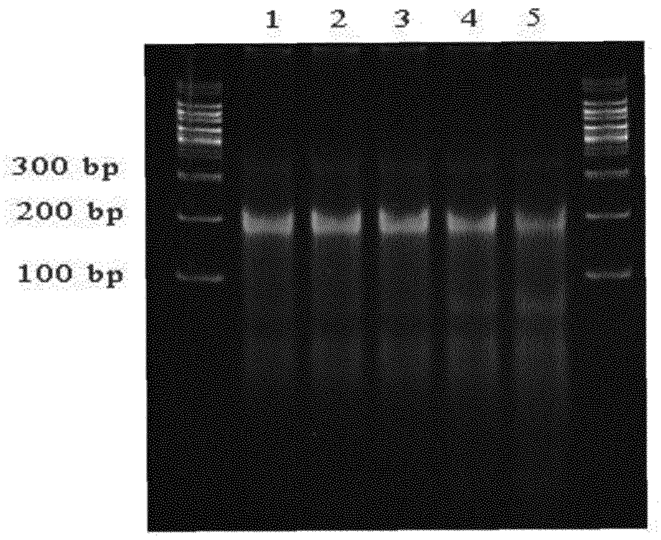


Fig. 22

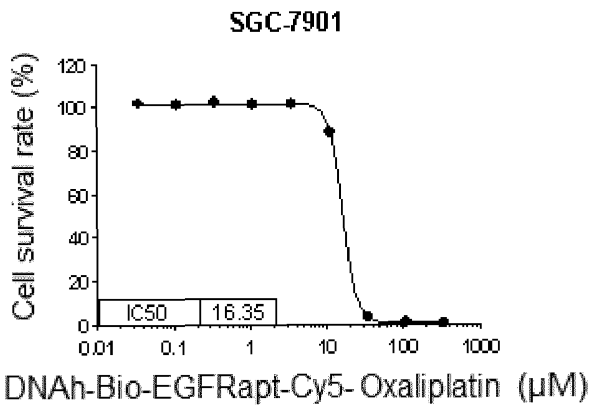


Fig. 23a

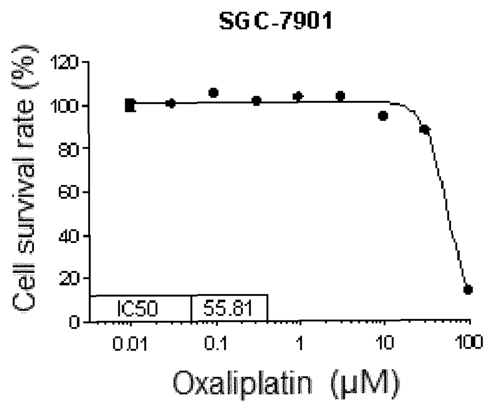


Fig. 23b

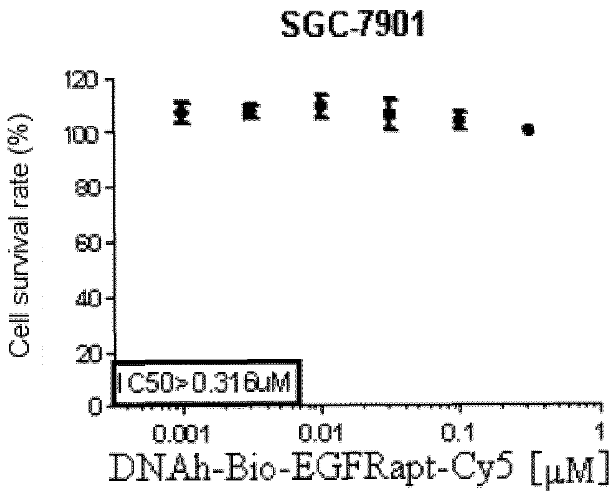


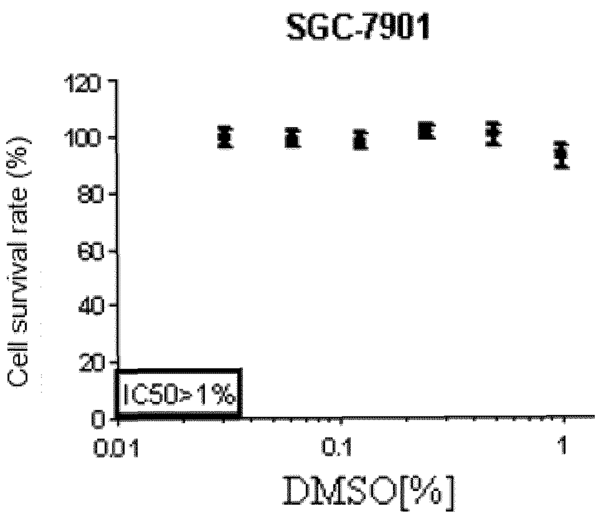

Fig. 23c

Fig. 23d

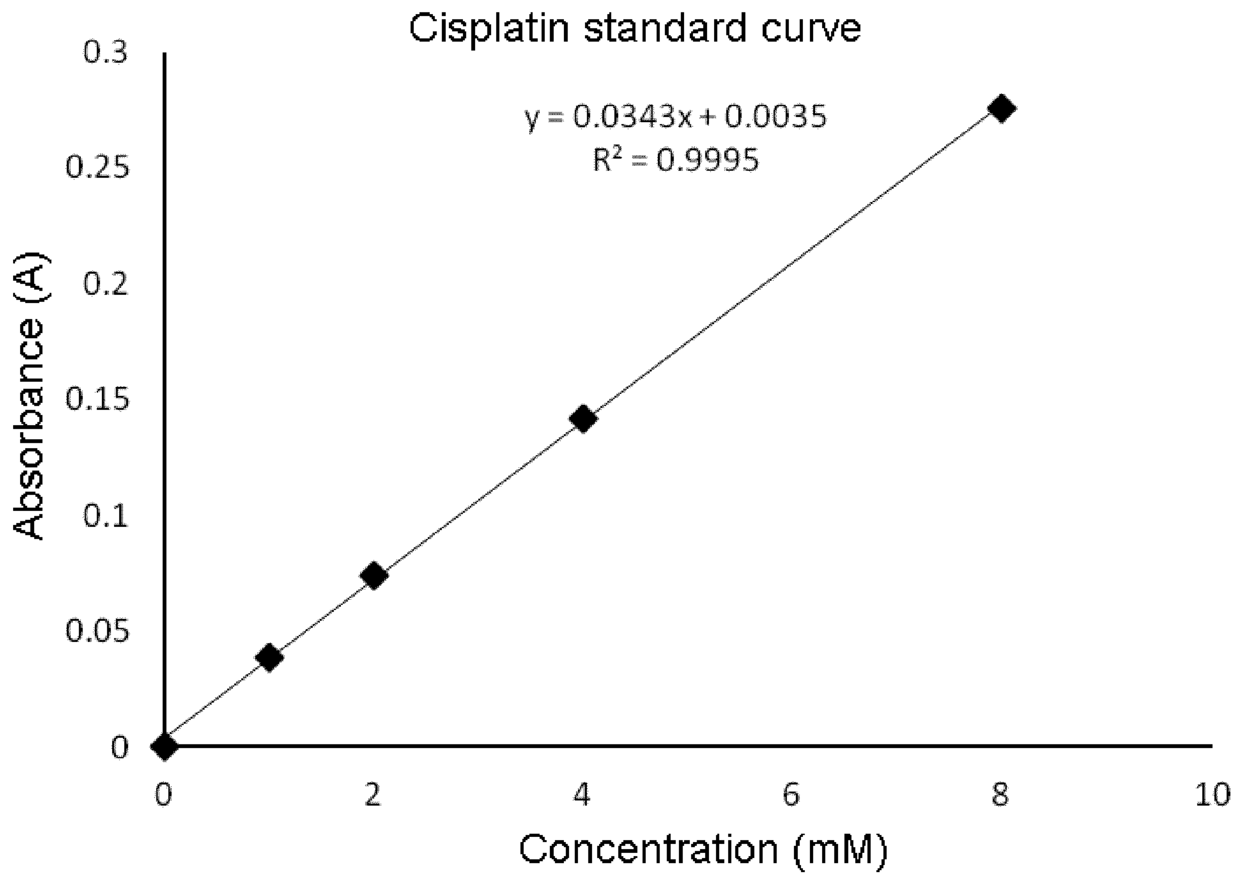


Fig. 24

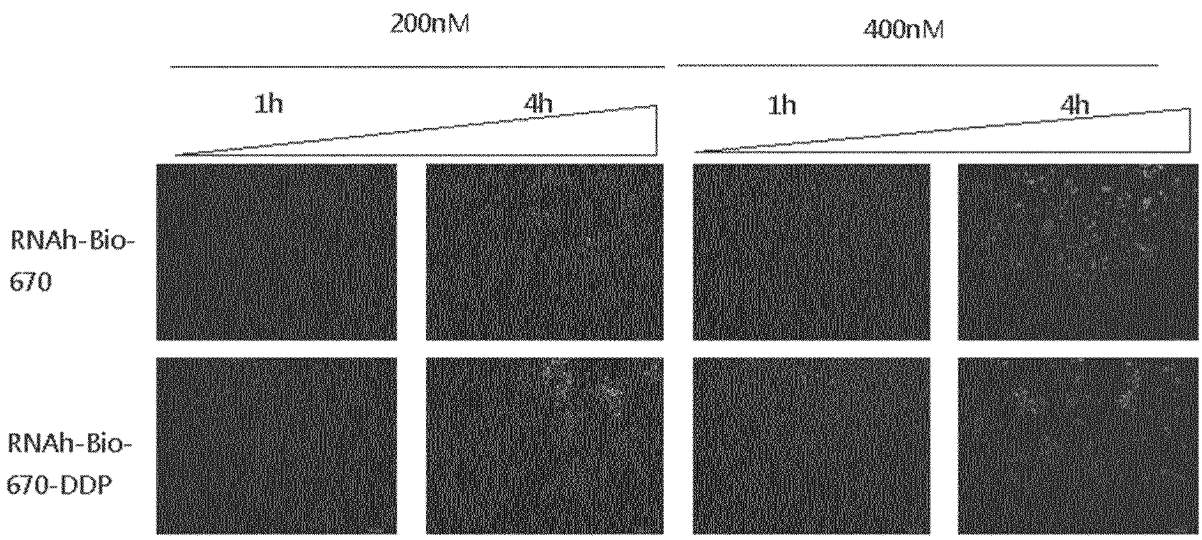


Fig. 25

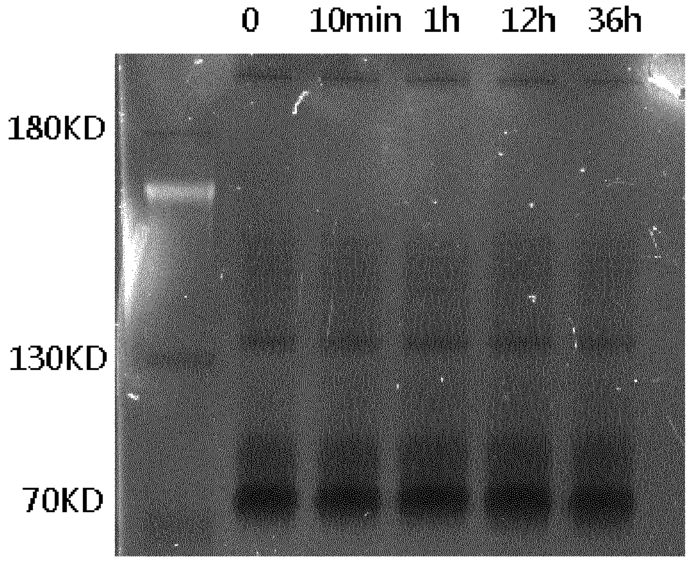
0
10min
1h
12h
36h
180KD
130KD
70KD

Fig. 26

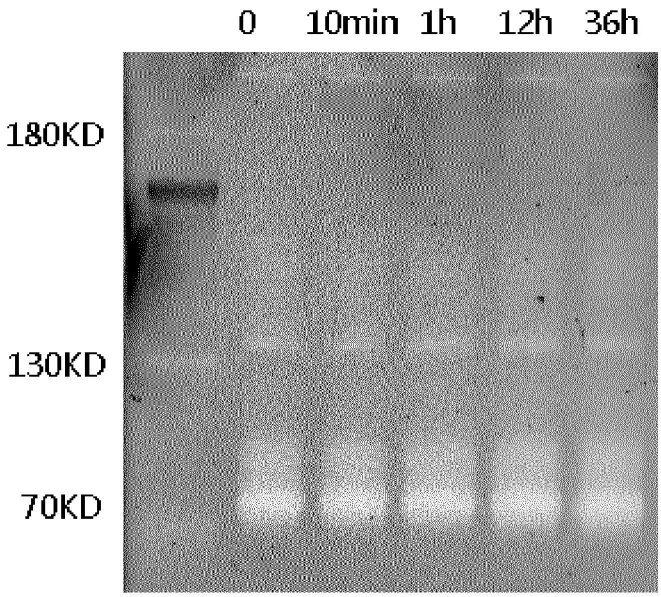
0
10min
1h
12h
36h
180KD
130KD
70KD

Fig. 27

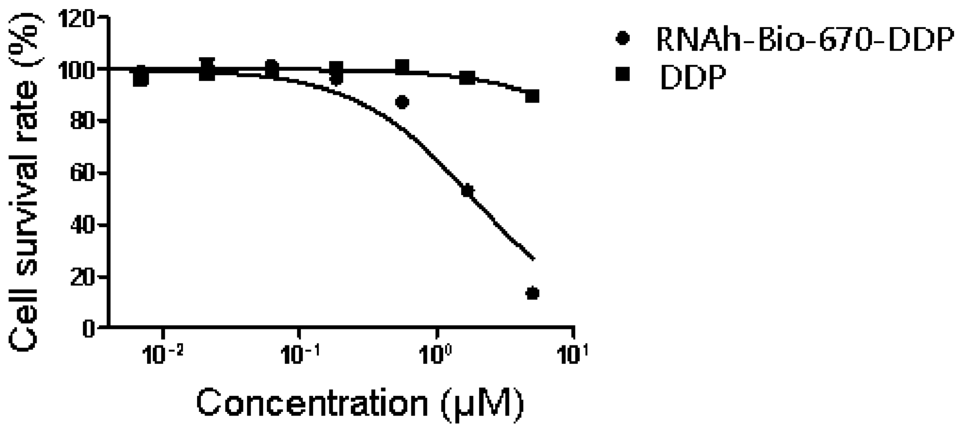


Fig. 28

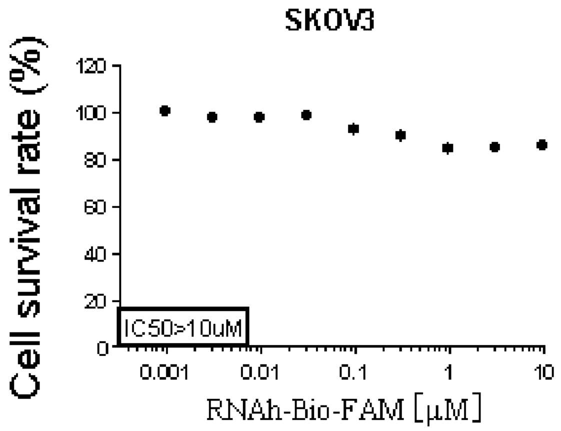


Fig. 29

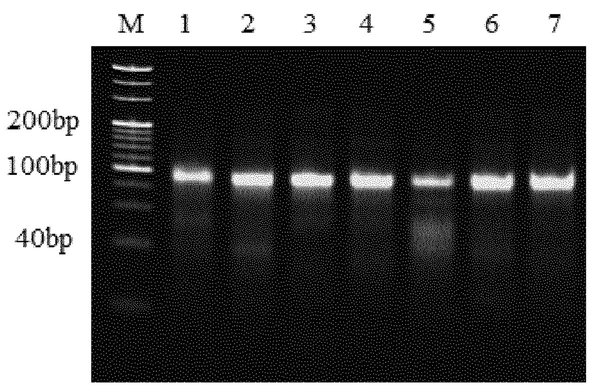


Fig. 30

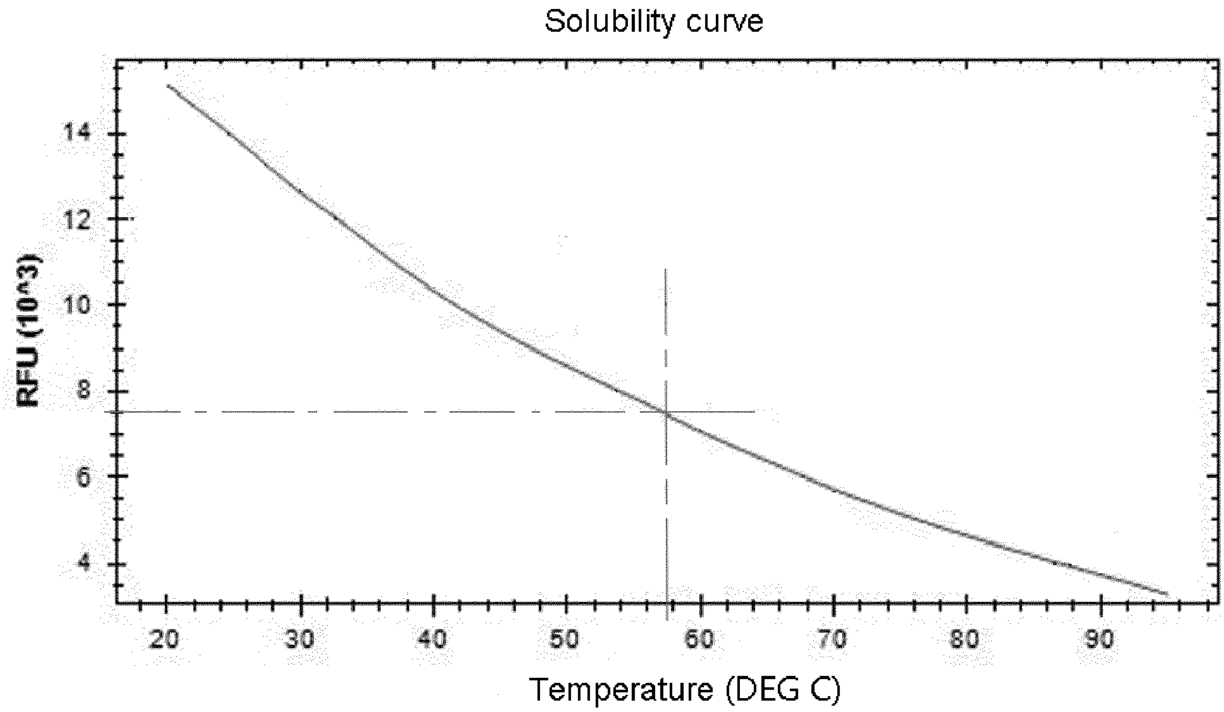
Solubility curve
RFU (10^3)
14
12
10
8
6
4
20
30
40
50
60
70
80
90
Temperature (DEG C)

Fig. 31

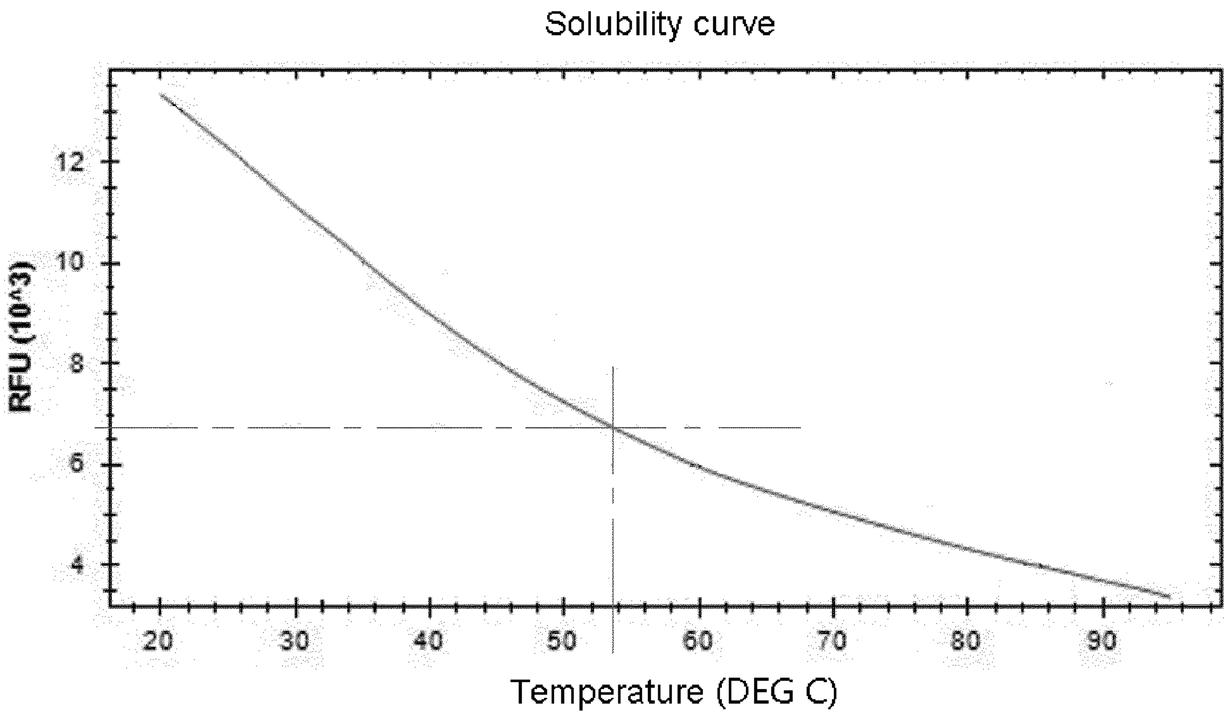
Solubility curve
RFU (10^3)
12
10
8
6
4
20
30
40
50
60
70
80
90
Temperature (DEG C)

Fig. 32

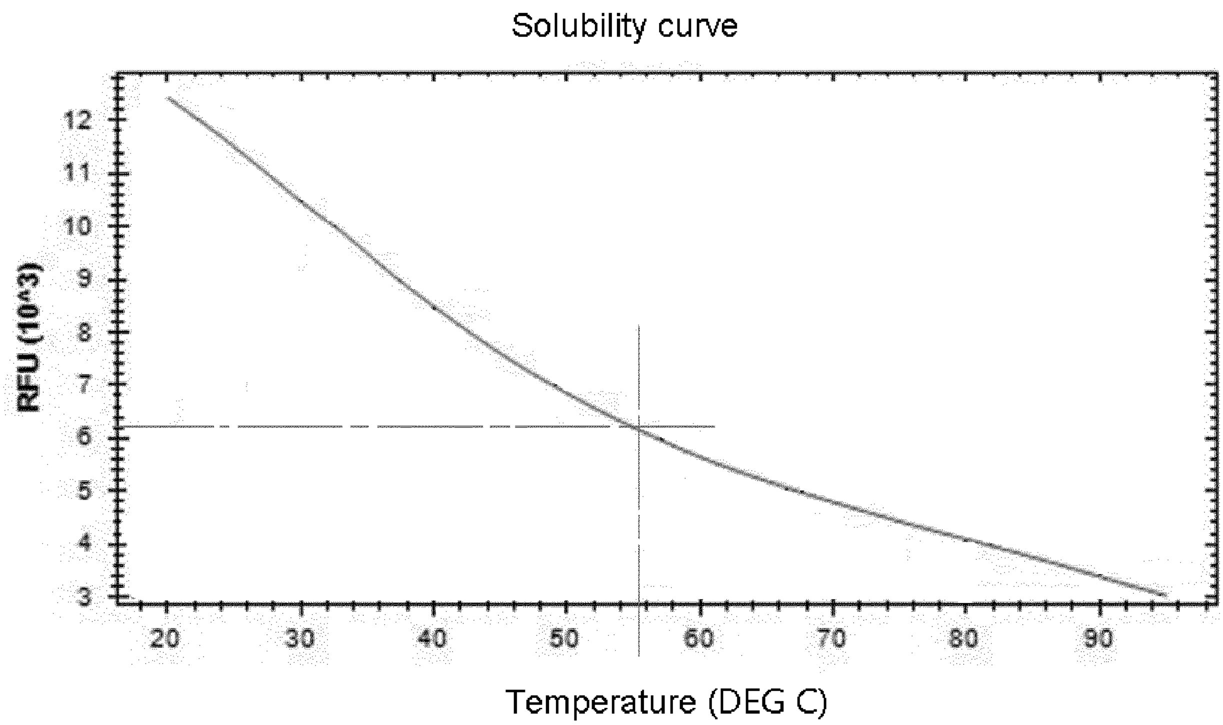

Solubility curve
RFU (10^3)
12
11
10
9
8
7
6
5
4
3
20
30
40
50
60
70
80
90
Temperature (DEG C)


**Fig. 33**

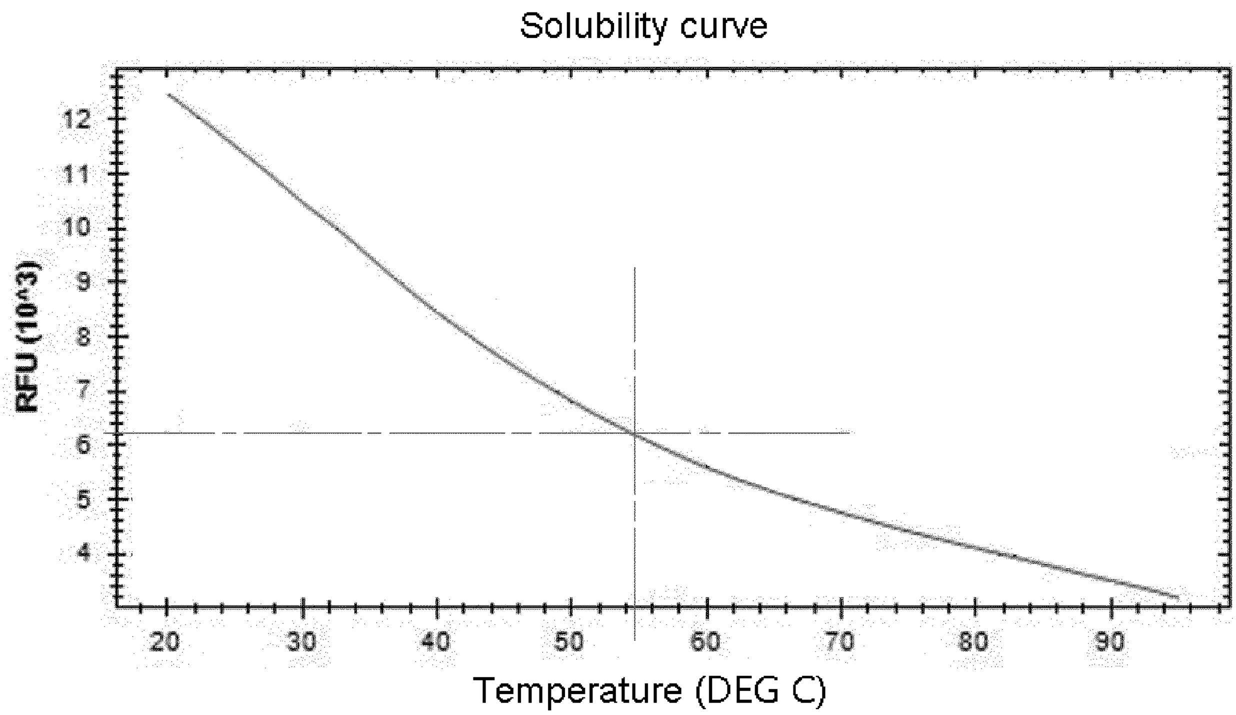

Solubility curve
RFU (10^3)
12
11
10
9
8
7
6
5
4
20
30
40
50
60
70
80
90
Temperature (DEG C)


**Fig. 34**

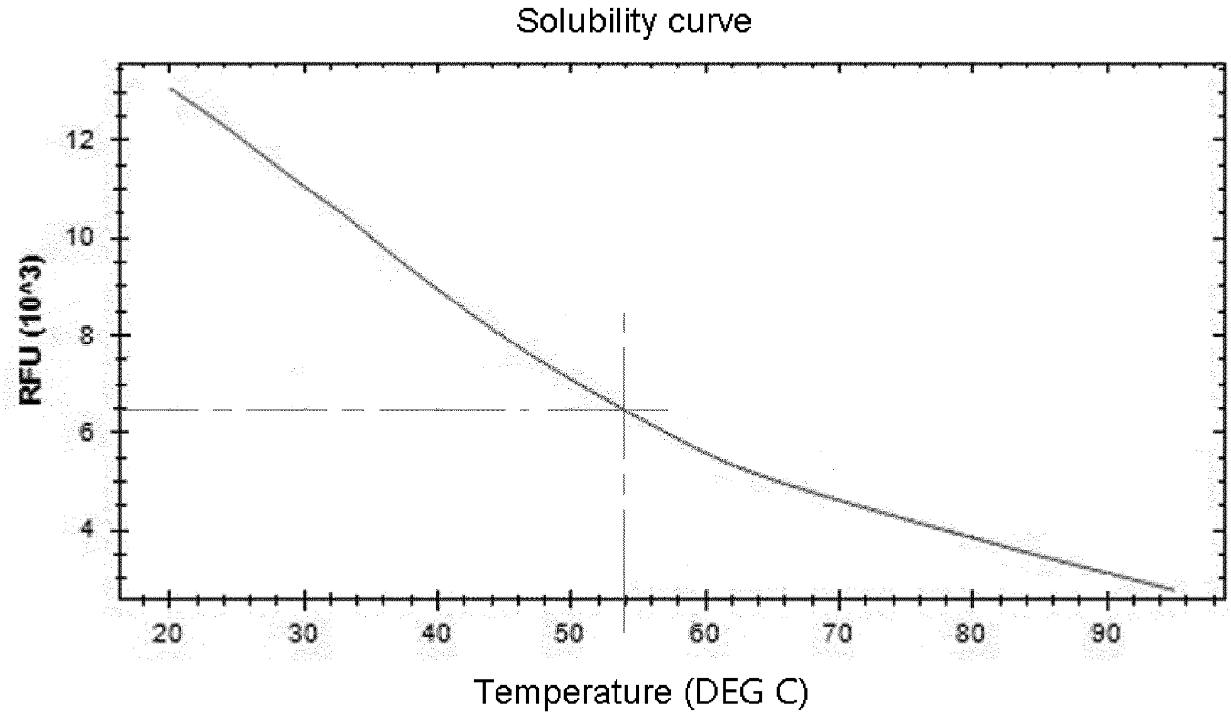
Solubility curve
RFU (10^3)
12
10
8
6
4
20
30
40
50
60
70
80
90
Temperature (DEG C)

**Fig. 35**

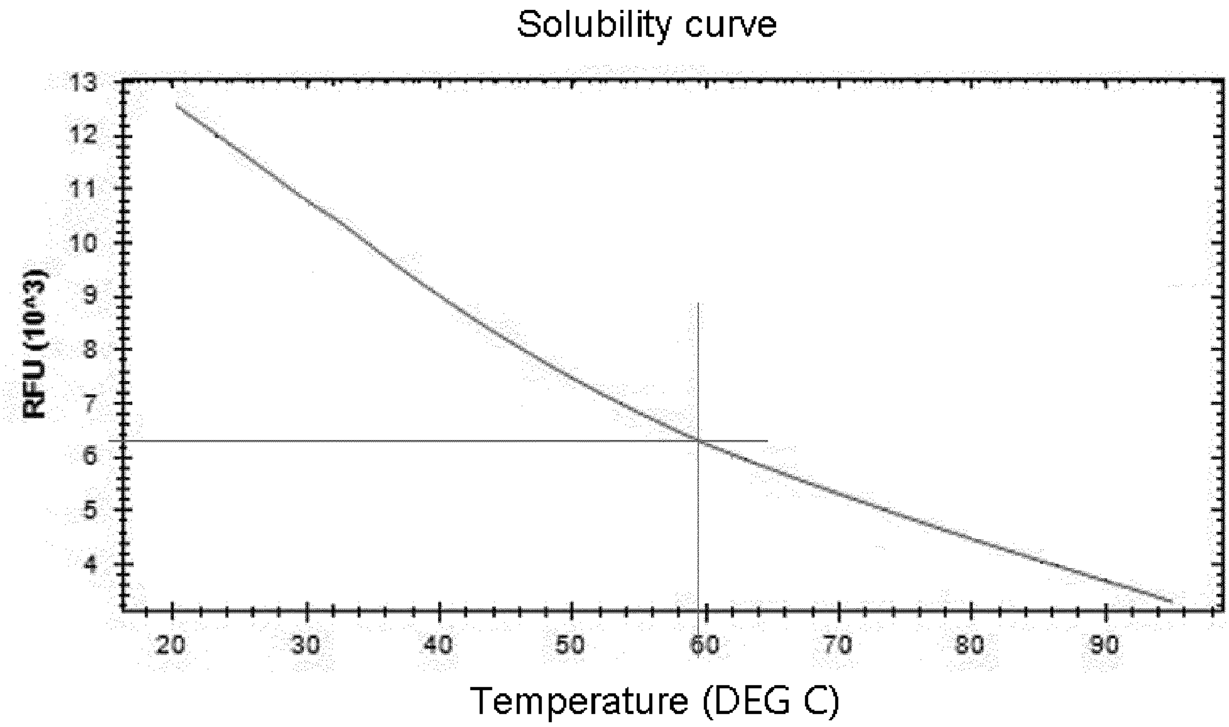
Solubility curve
RFU (10^3)
13
12
11
10
9
8
7
6
5
4
20
30
40
50
60
70
80
90
Temperature (DEG C)

**Fig. 36**

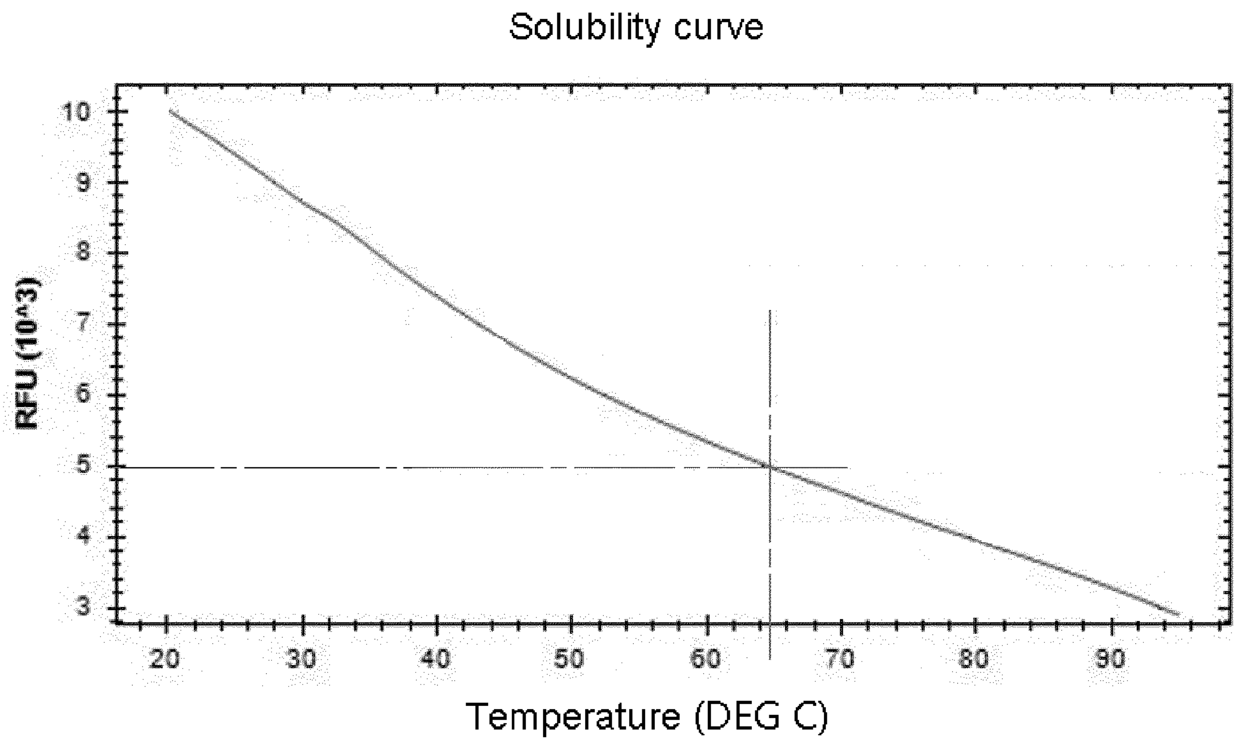
Solubility curve
RFU (10^3)
10
9
8
7
6
5
4
3
20
30
40
50
60
70
80
90
Temperature (DEG C)

Fig. 37

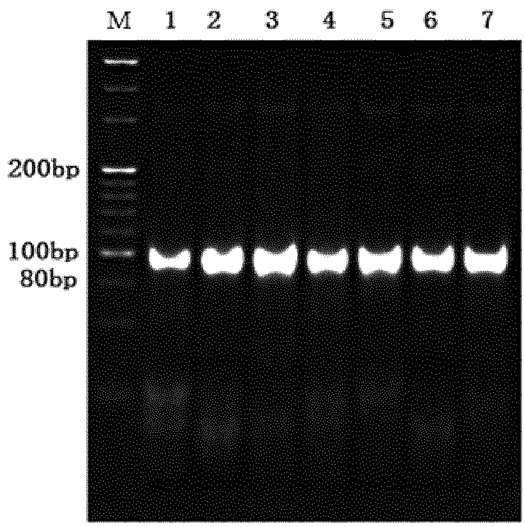
M 1 2 3 4 5 6 7
200bp
100bp
80bp

Fig. 38

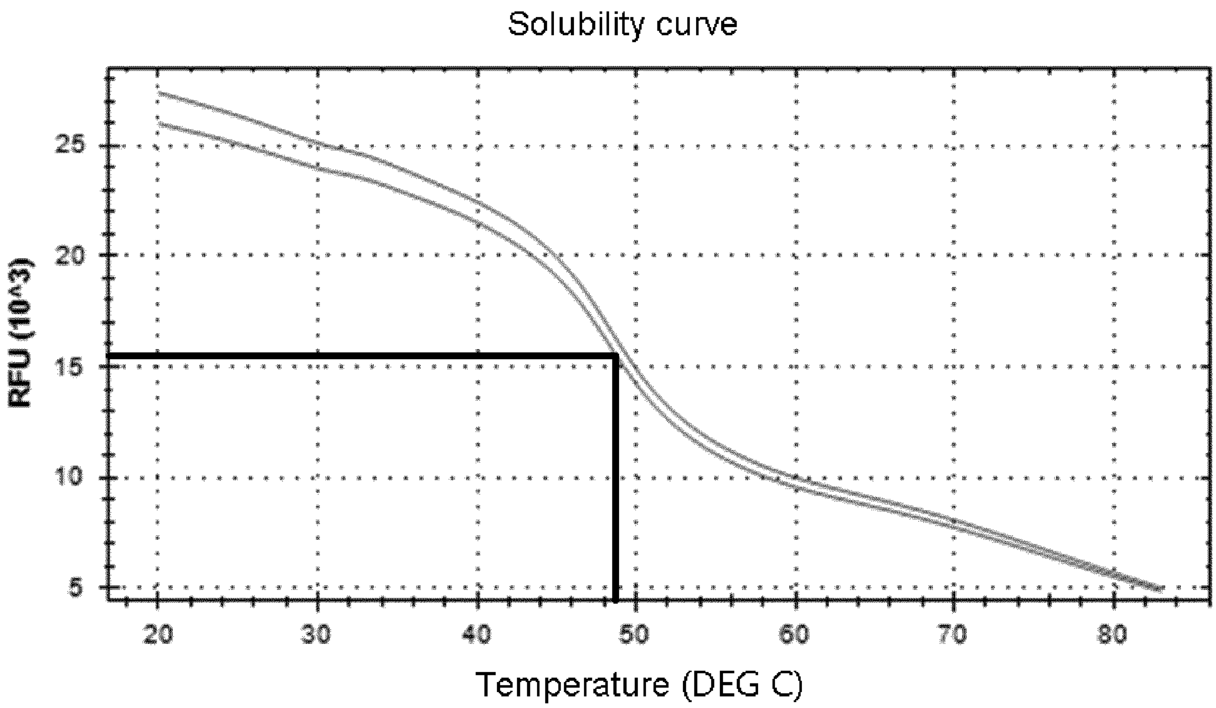
Solubility curve
RFU (10^3)
25
20
15
10
5
20
30
40
50
60
70
80
Temperature (DEG C)

Fig. 39

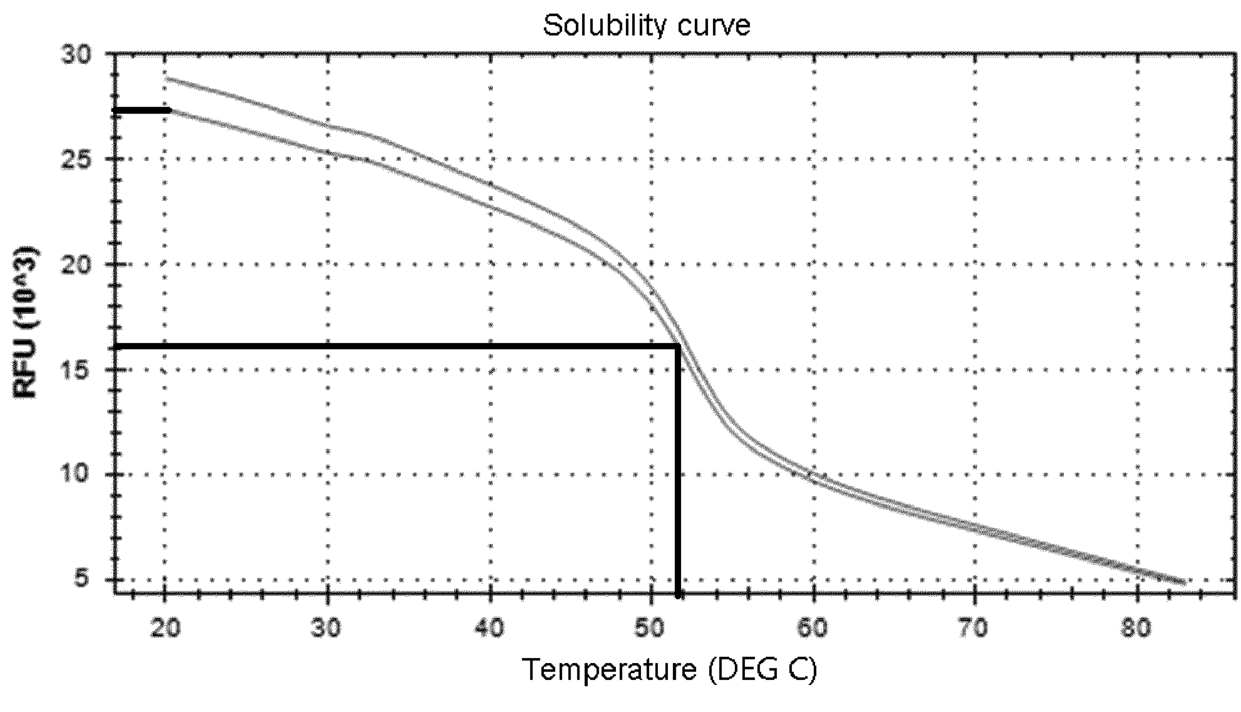
Solubility curve
RFU (10^3)
30
25
20
15
10
5
20
30
40
50
60
70
80
Temperature (DEG C)

Fig. 40

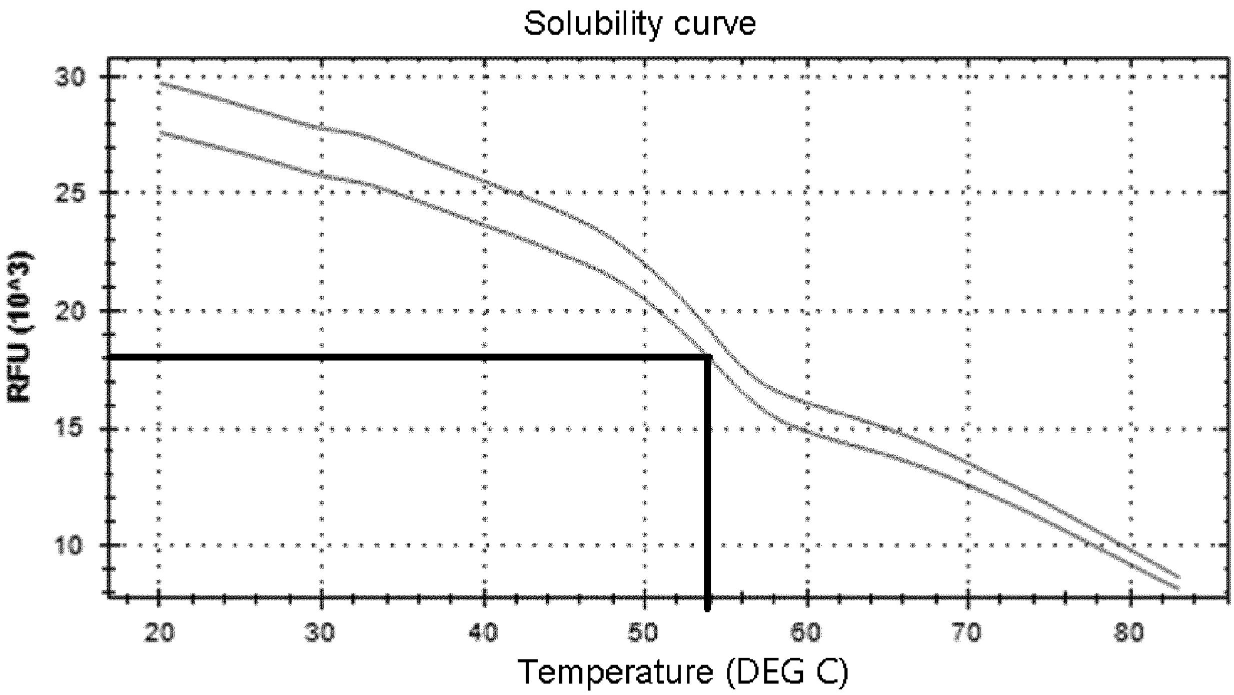
Solubility curve
RFU (10^3)
30
25
20
15
10
20
30
40
50
60
70
80
Temperature (DEG C)

Fig. 41

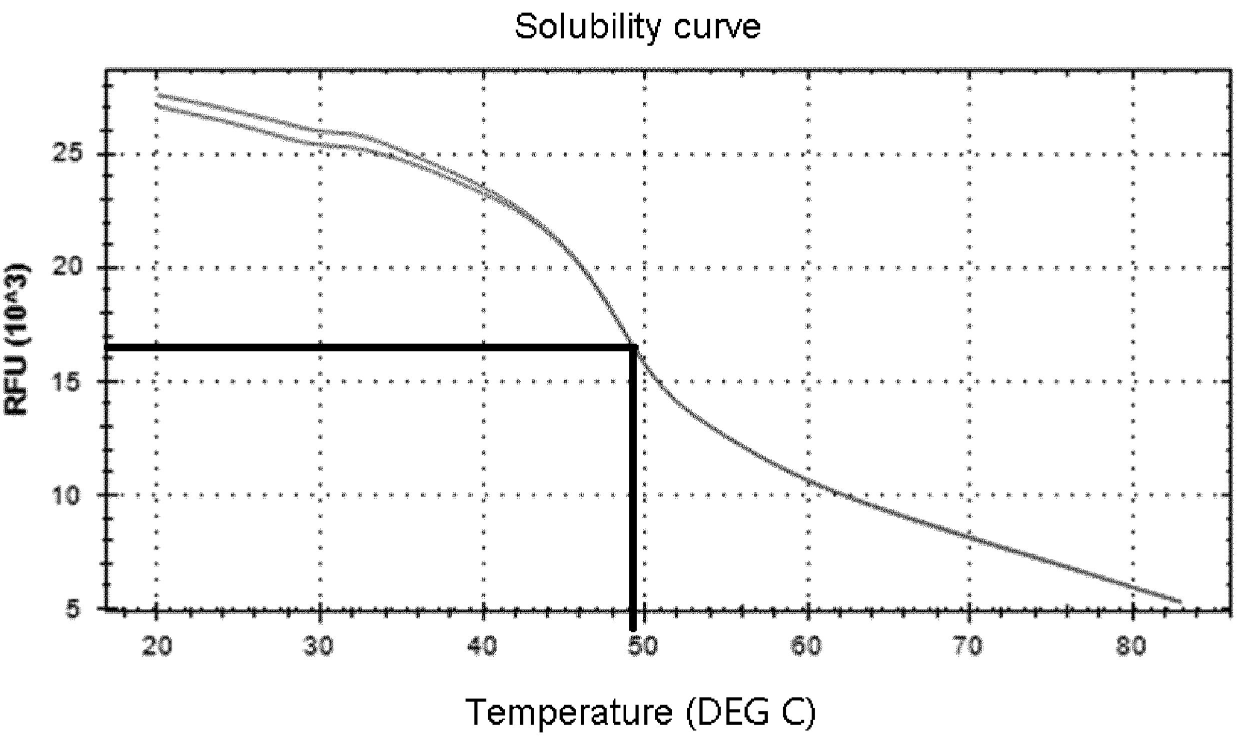
Solubility curve
RFU (10^3)
25
20
15
10
5
20
30
40
50
60
70
80
Temperature (DEG C)

Fig. 42

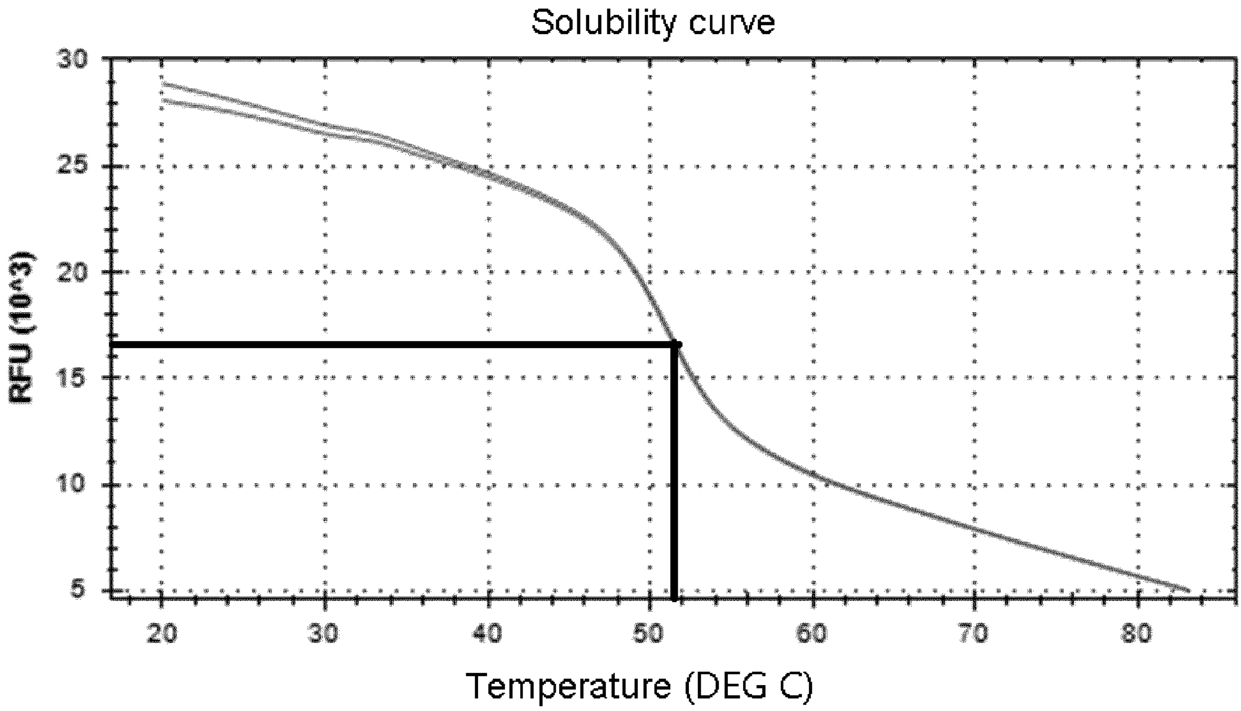
Solubility curve
RFU (10^3)
30
25
20
15
10
5
20
30
40
50
60
70
80
Temperature (DEG C)

Fig. 43

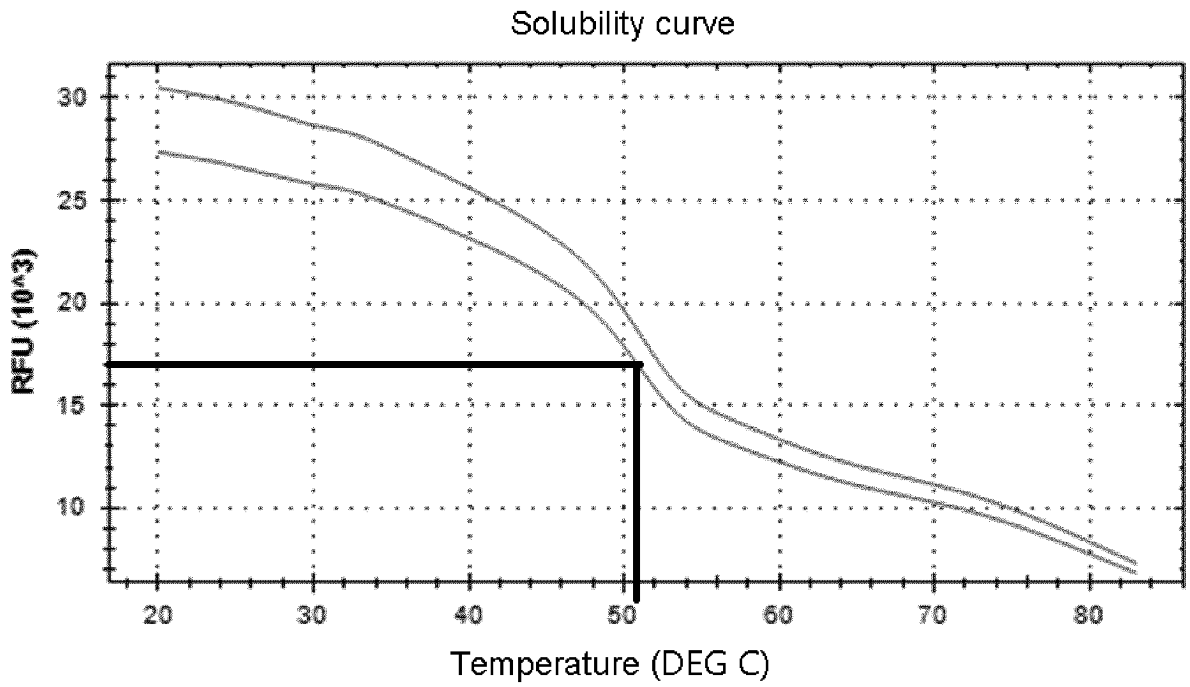
Solubility curve
RFU (10^3)
30
25
20
15
10
20
30
40
50
60
70
80
Temperature (DEG C)

Fig. 44

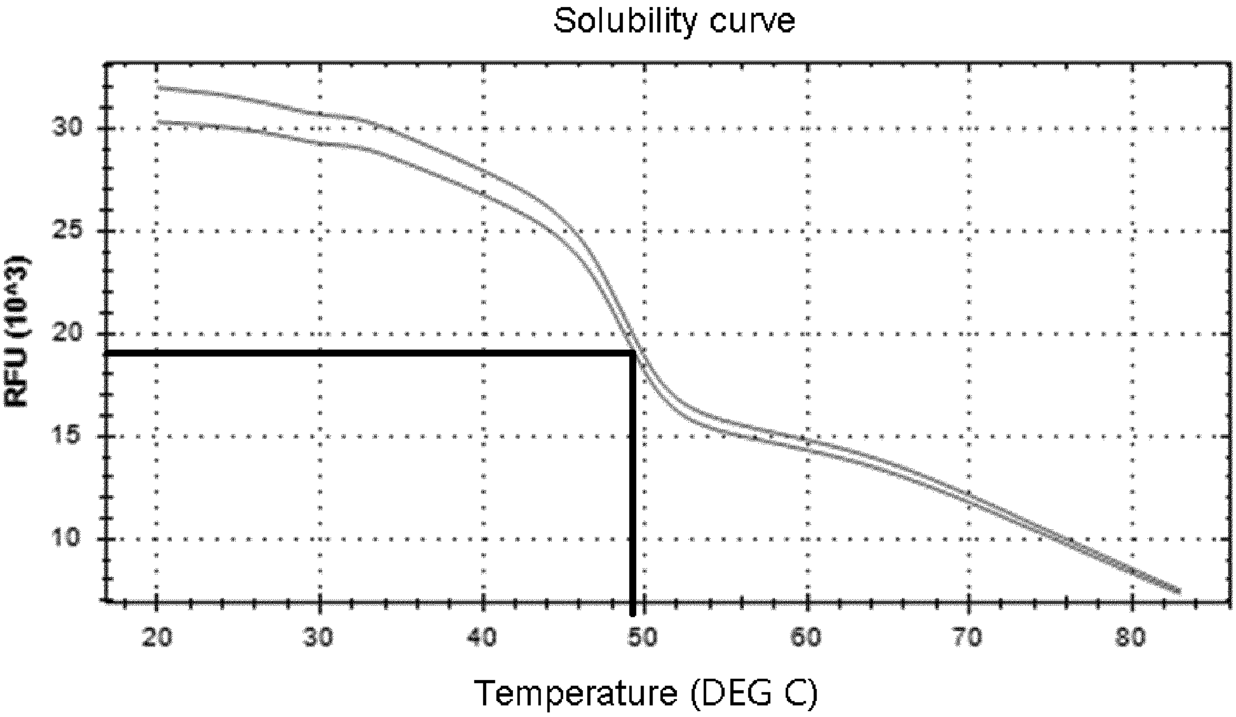
Solubility curve
30
25
20
15
10
RFU (10^3)
20
30
40
50
60
70
80
Temperature (DEG C)

Fig. 45

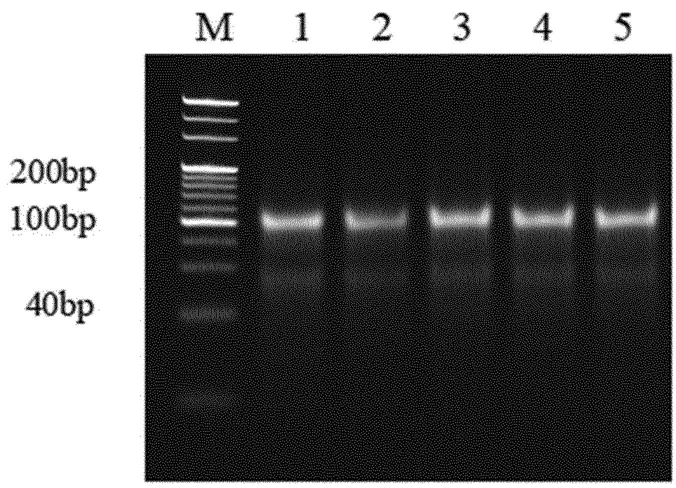
M 1 2 3 4 5
200bp
100bp
40bp

Fig. 46

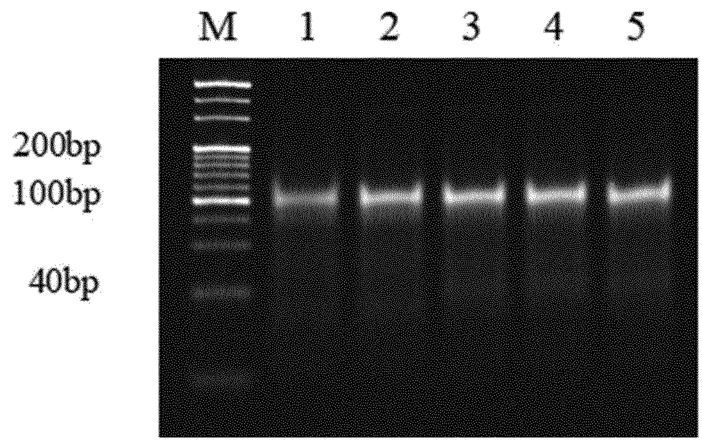


Fig. 47

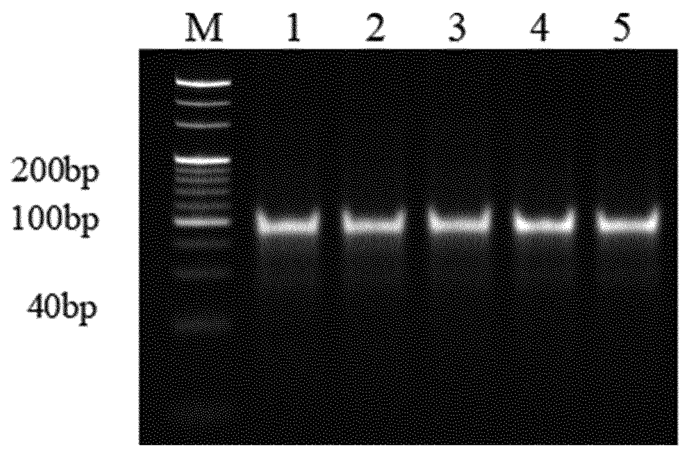


Fig. 48

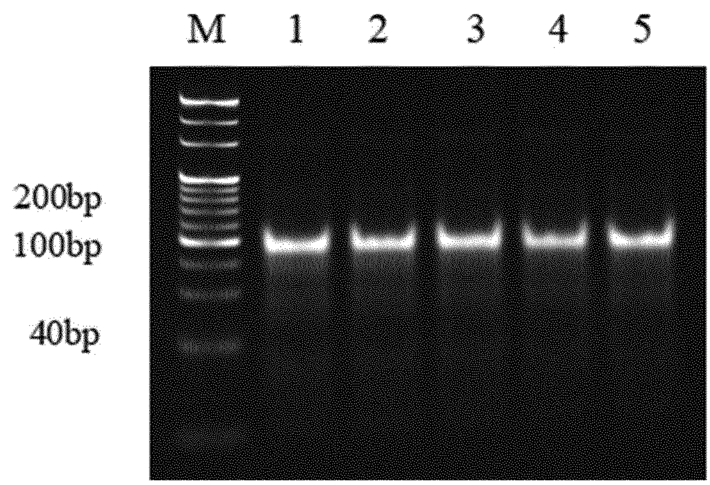


Fig. 49

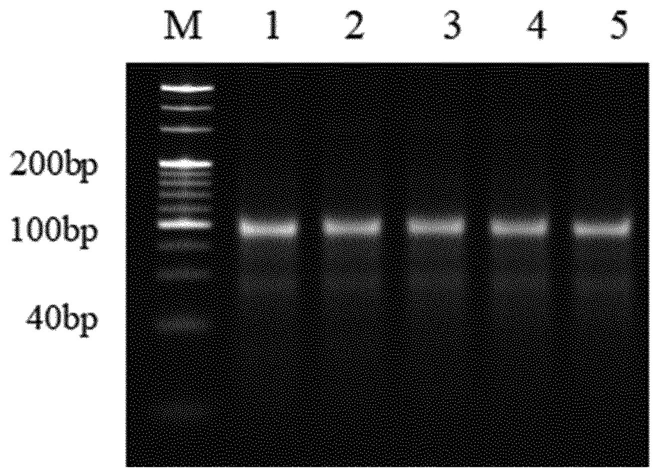


Fig. 50

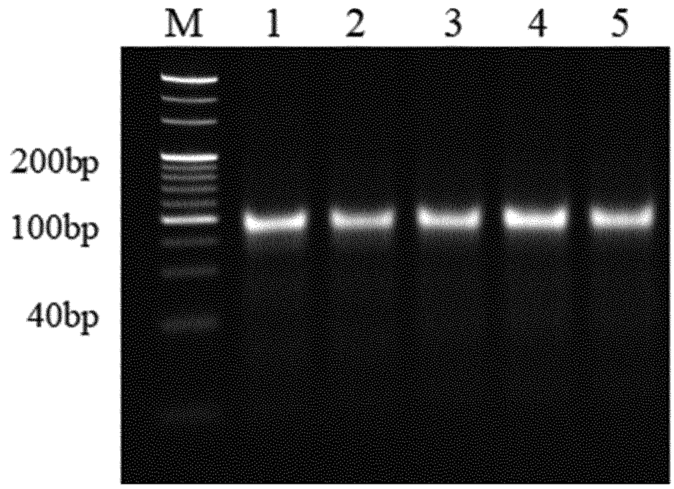


Fig. 51

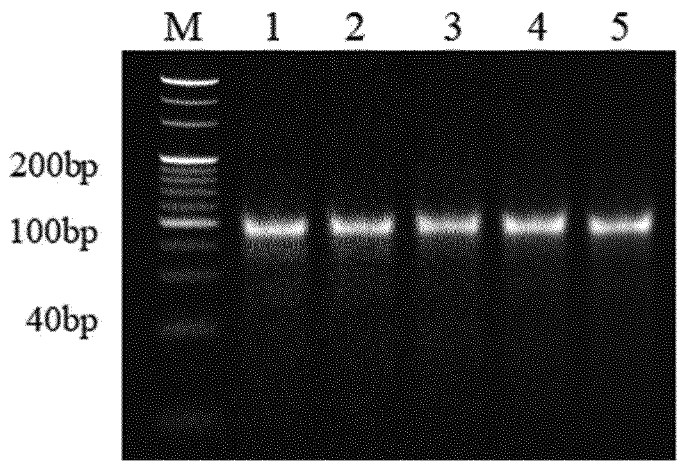


Fig. 52

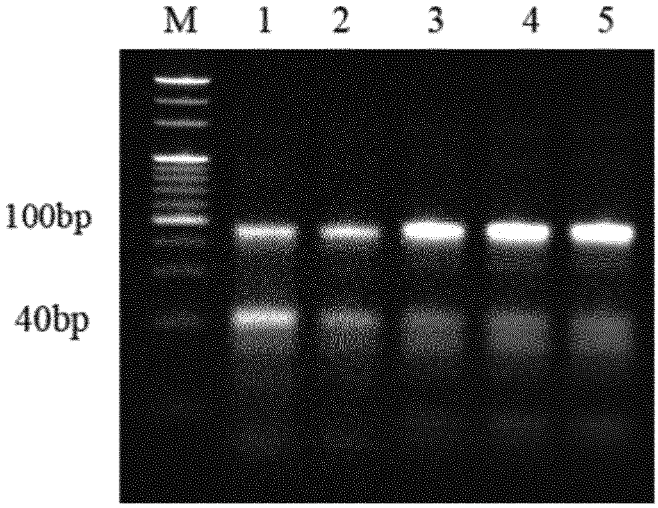


Fig. 53

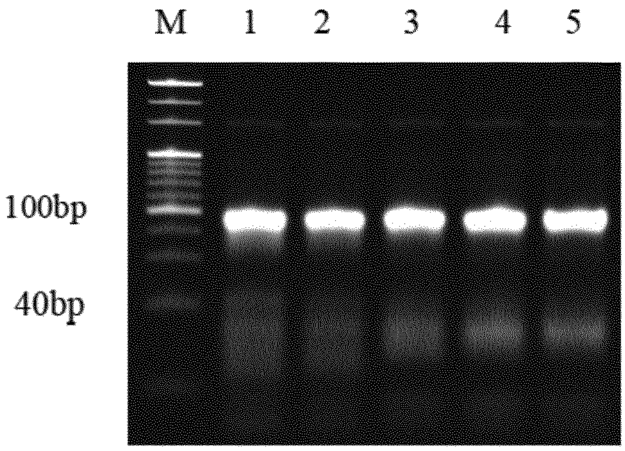


Fig. 54

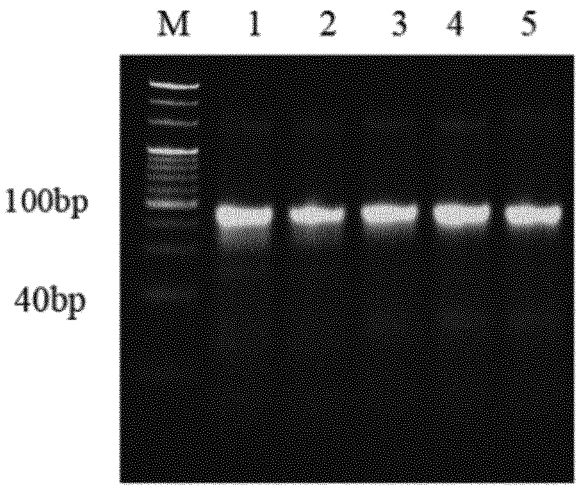


Fig. 55

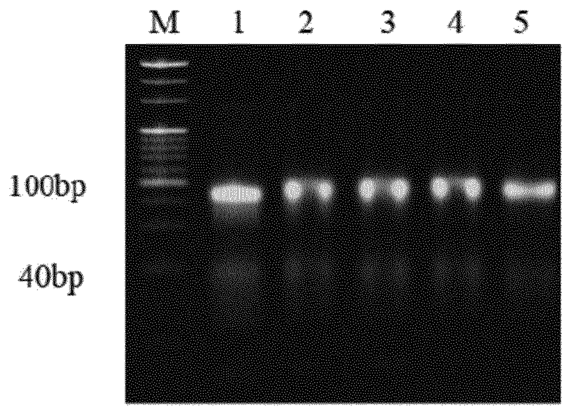


Fig. 56

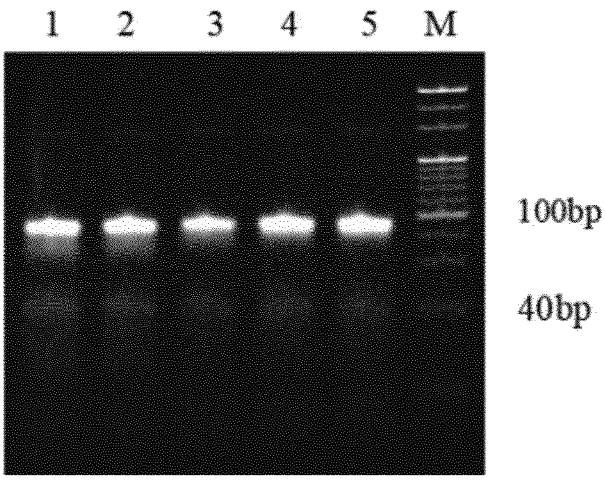


Fig. 57

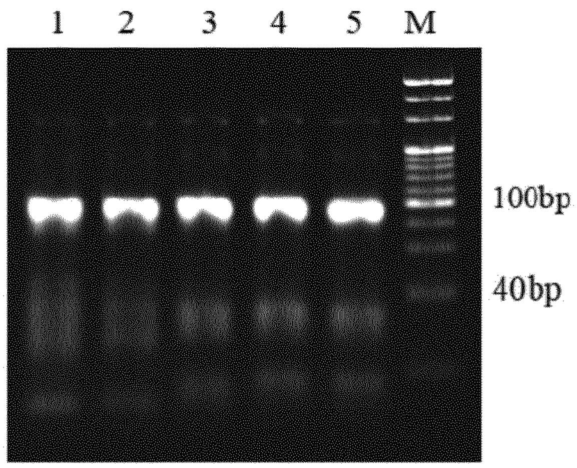


Fig. 58

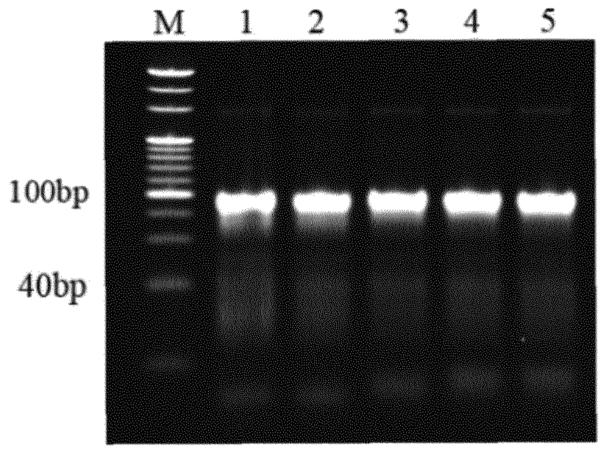


Fig. 59

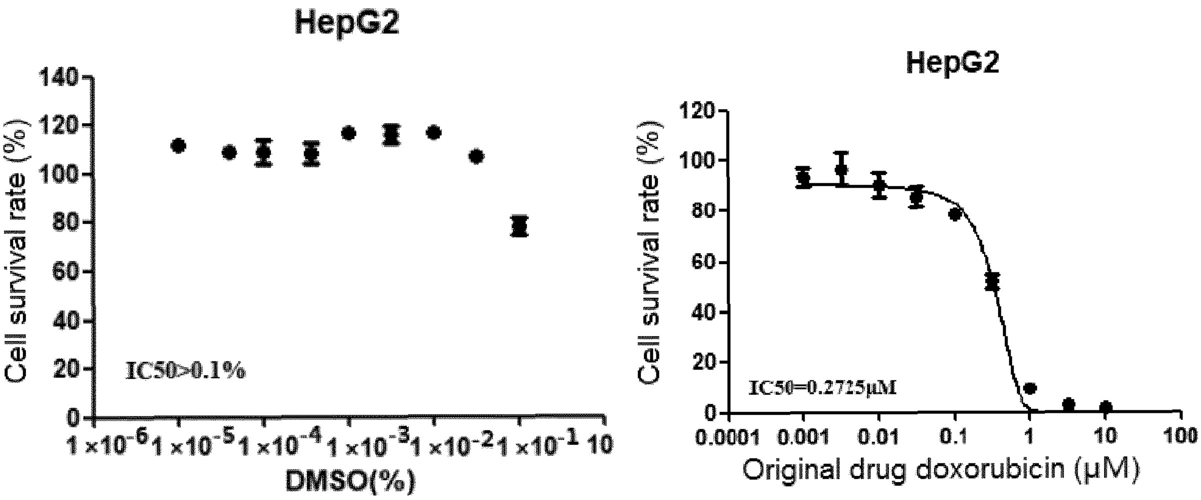


Fig. 60a

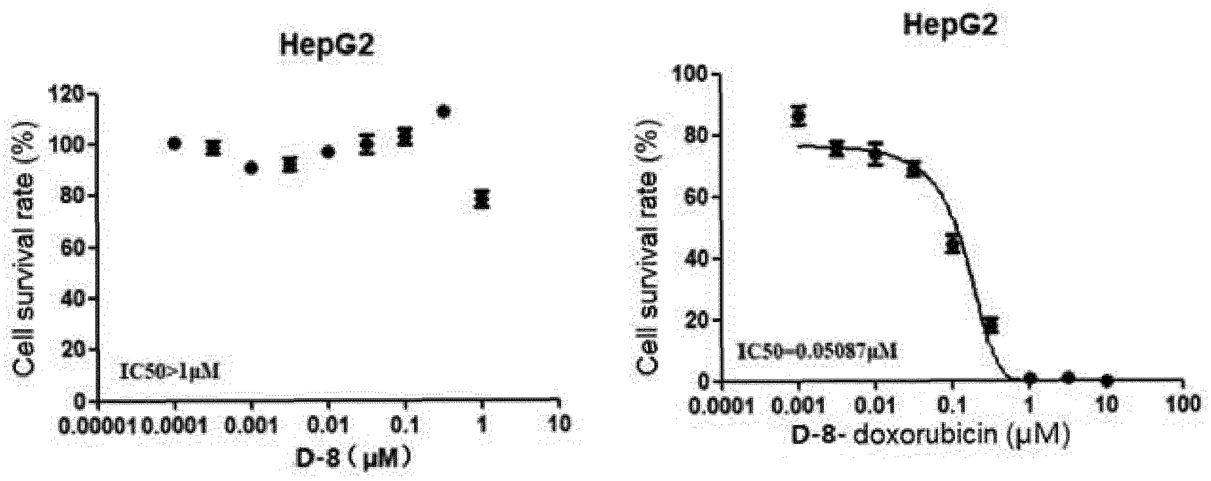


Fig. 60b

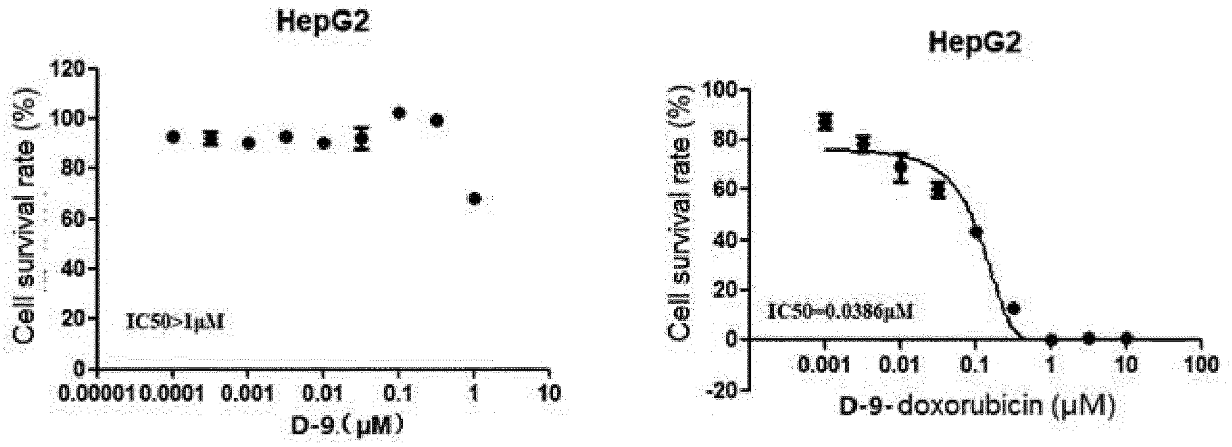


Fig. 60c

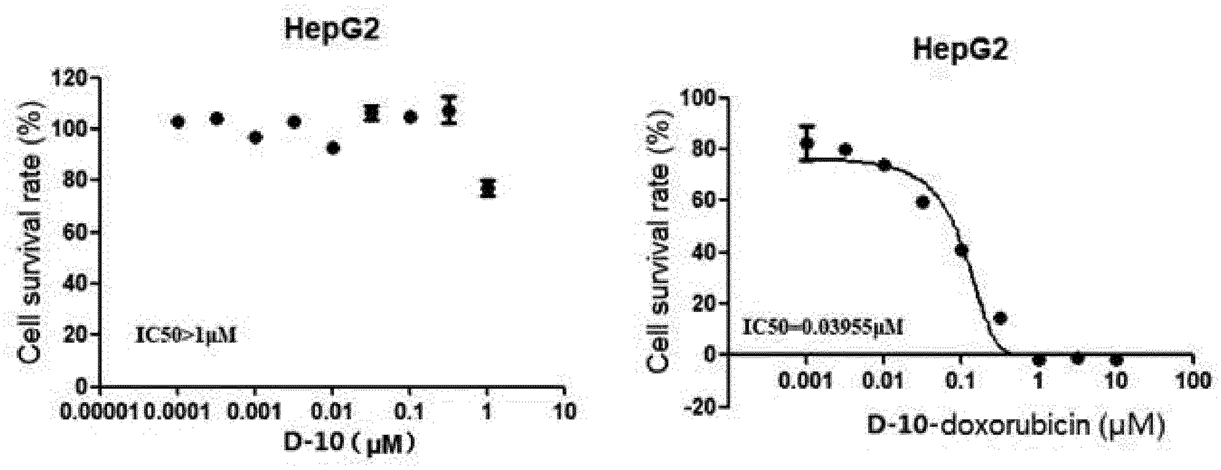


Fig. 60d

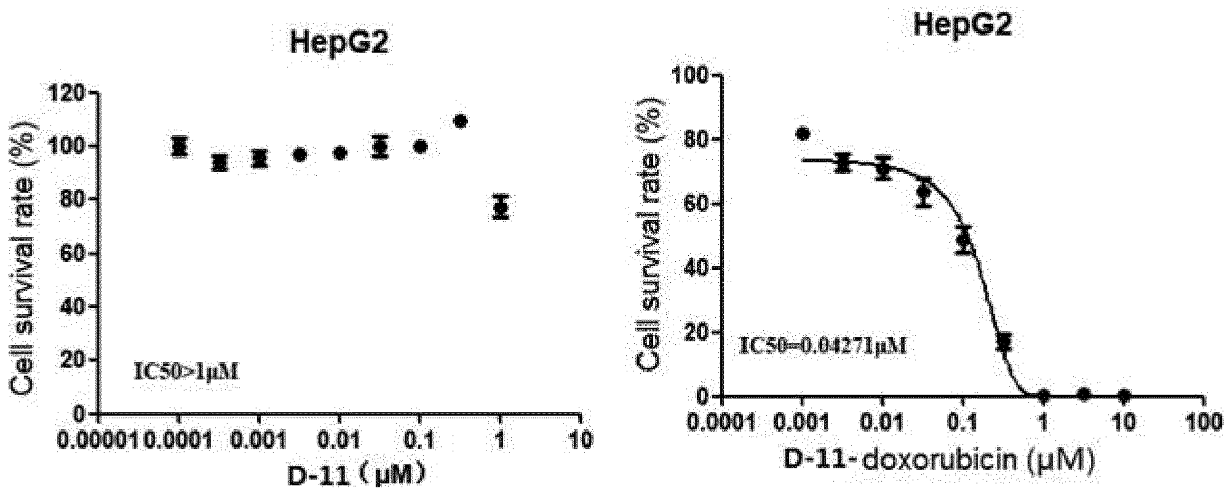


Fig. 60e

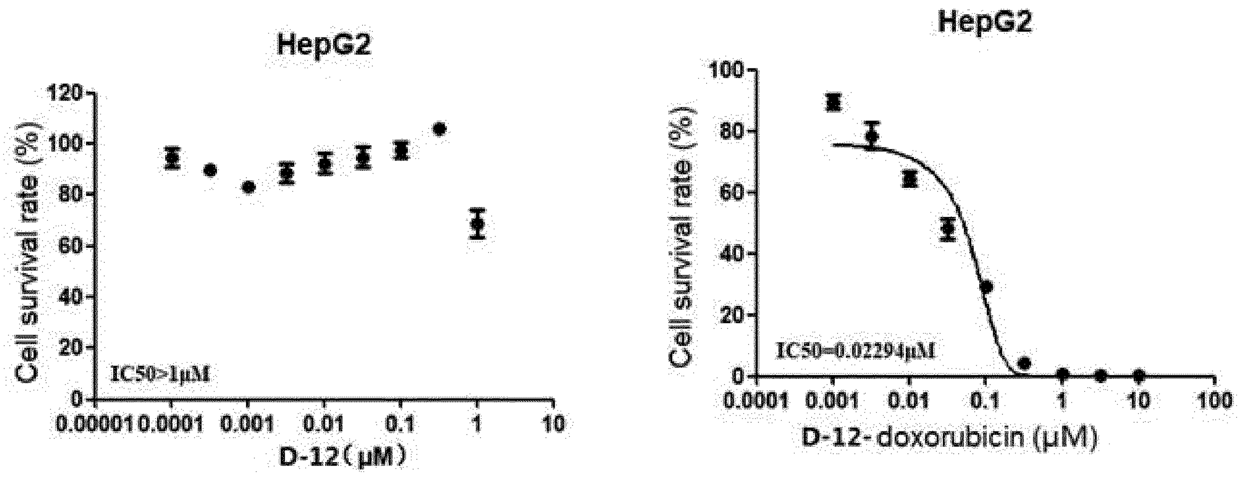


Fig. 60f

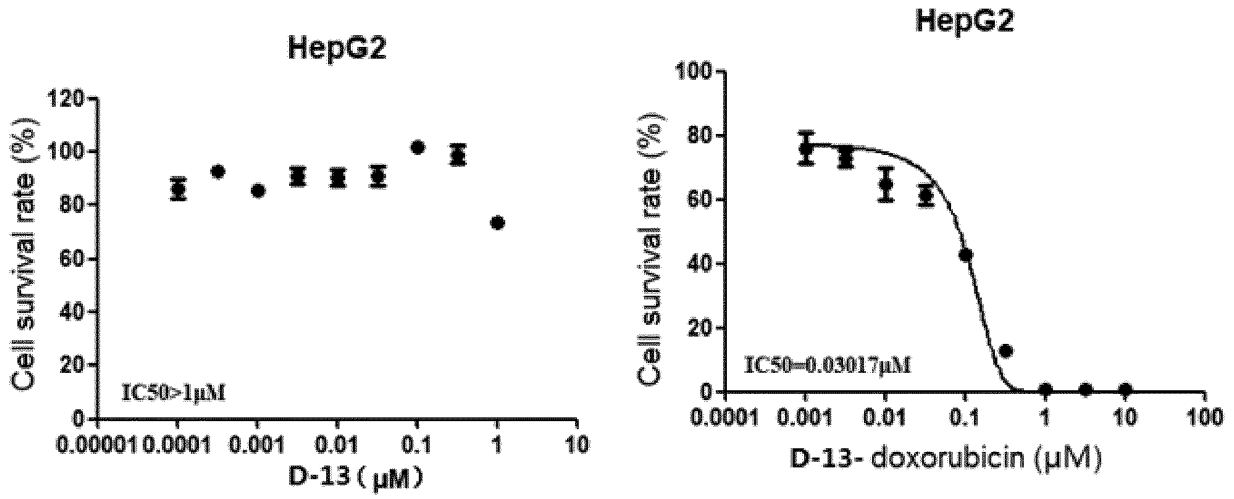


Fig. 60g

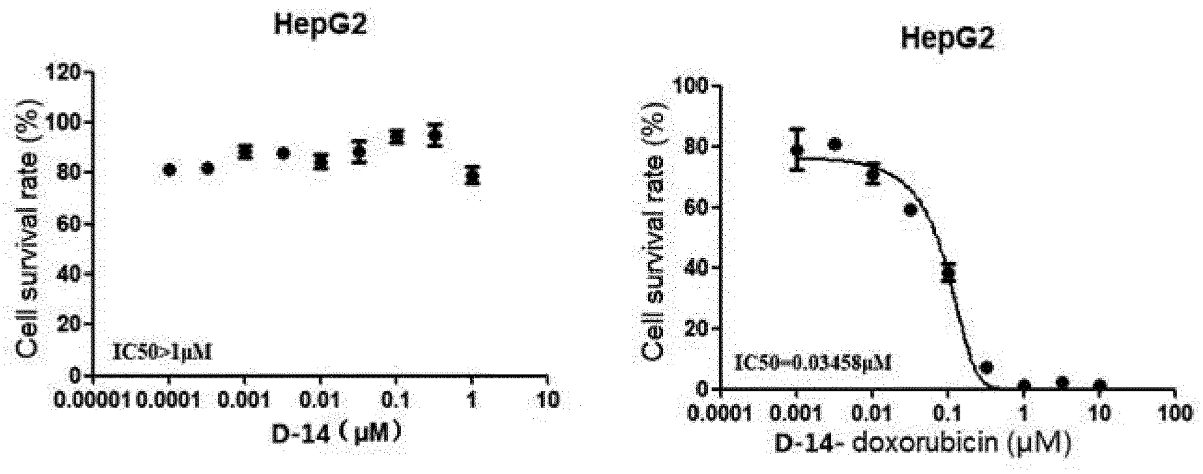


Fig. 60h

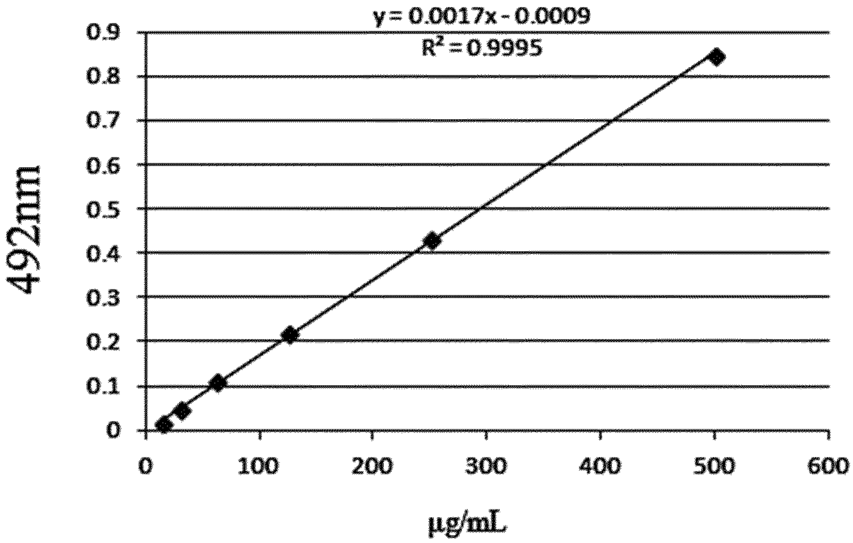
y = 0.0017x - 0.0009
R² = 0.9995
0.9
0.8
0.7
0.6
0.5
0.4
0.3
0.2
0.1
0
492nm
0
100
200
300
400
500
600
μg/mL

Fig. 61

**INTERNATIONAL SEARCH REPORT**

International application No.
**PCT/CN2019/109466**

**A. CLASSIFICATION OF SUBJECT MATTER**

C12N 15/115(2010.01)i； A61K 47/56(2017.01)i； A61K 31/713(2006.01)i； C07H 21/04(2006.01)i

According to International Patent Classification (IPC) or to both national classification and IPC

**B. FIELDS SEARCHED**

Minimum documentation searched (classification system followed by classification symbols)

C12N; A61K; C07H

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, CNKI, 万方数据资源系统, WANFANG DATA, 中国专利生物序列检索系统, CHINA PATENT BIOLOGICAL SEQUENCE SEARCH SYSTEM, PUBMED, ISI WEB OF KNOWLEDGE, EMBL, PATENTICS: 申请人/发明人, 纳米颗粒, nanoparticle, RNA scaffold, 3WJ, three-way junction, H.marismortui 5S rRNA, 双氢青蒿素, 顺铂, 奥沙利铂, 黄酮, 长春新碱, dihydroartemisin? , DHA, Vincristine, Oncovin, VCR, Cisplatin, DDP, Oxaliplatin, Flavone, Anthoxanthin, 小分子药物, 适体, aptamer, SEQ ID N0s: 1-6.

**C. DOCUMENTS CONSIDERED TO BE RELEVANT**

| Category* | Citation of document, with indication, where appropriate, of the relevant passages | Relevant to claim No. |
|---|---|---|
| X | CN 103403189 A (UNIVERSITY OF CINCINNATI) 20 November 2013 (2013-11-20) figures 10E and 10G, and description, paragraphs 0148, 0155, 0189-0197 and 0207 | 1-18 |
| X | WO 2005106035 A2 (CORNELL RESEARCH FOUNDATION, INC.) 10 November 2005 (2005-11-10) claims 1-26, and figures 4A-B and 5A-C | 1-18 |
| Y | WO 2017197009 A1 (OHIO STATE INNOVATION FOUNDATION et al.) 16 November 2017 (2017-11-16) claims 1-26, and figures 1A and 13A | 1-18 |
| Y | CN 104327141 A (SHANGHAI JIAOTONG UNIVERSITY) 04 February 2015 (2015-02-04) claims 1-11, and figure 1 | 1-18 |
| Y | CN 108602849 A (OHIO STATE INNOVATION FOUNDATION) 28 September 2018 (2018-09-28) claims 1-20, and figure 2A | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 December 2019** | **31 December 2019** |

| Name and mailing address of the ISA/CN | Authorized officer |
|---|---|
| **China National Intellectual Property Administration**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No.
PCT/CN2019/109466

C. DOCUMENTS CONSIDERED TO BE RELEVANT

| Category* | Citation of document, with indication, where appropriate, of the relevant passages | Relevant to claim No. |
|---|---|---|
| Y | BAN, N. et al. "The Complete Atomic Structure of the Large Ribosomal Subunit at 2.4 Å Resolution" *Science,* Vol. 289, No. (5481), 11 August 2000 (2000-08-11), pages 905-920 | 1-18 |
| Y | DIAMOND, J.M. et al. "Thermodynamics of Three-Way Multibranch Loops in RNA" *Biochemistry,* Vol. 40, No. (23), 18 May 2001 (2001-05-18), pages 6971-6981 | 1-18 |
| A | WO 2009054813 A1 (AGENCY FOR SCIENCE, TECHNOLOGY AND RESEARCH) 30 April 2009 (2009-04-30) entire document | 1-18 |
| A | US 2012189688 A1 (NATIONAL CHIAO TUNG UNIVERSITY) 26 July 2012 (2012-07-26) entire document | 1-18 |
| A | CN 104368004 A (NATIONAL CENTER FOR NANOSCIENCE AND TECHNOLOGY et al.) 25 February 2015 (2015-02-25) entire document | 1-18 |
| A | CN 106806895 A (SHANGHAI CANCER INSTITUTE) 09 June 2017 (2017-06-09) entire document | 1-18 |
| A | WO 2013185032 A1 (PRESIDENT AND FELLOWS OF HARVARD COLLEGE) 12 December 2013 (2013-12-12) entire document | 1-18 |

Form PCT/ISA/210 (second sheet) (January 2015)

**INTERNATIONAL SEARCH REPORT**
**Information on patent family members**

International application No.
**PCT/CN2019/109466**

| Patent document cited in search report | | | Publication date (day/month/year) | Patent family member(s) | | | Publication date (day/month/year) |
|---|---|---|---|---|---|---|---|
| CN | 103403189 | A | 20 November 2013 | US | 9297013 | B2 | 29 March 2016 |
| | | | | WO | 2012170372 | A2 | 13 December 2012 |
| | | | | WO | 2012170372 | A3 | 18 April 2013 |
| | | | | CN | 103403189 | B | 25 November 2015 |
| | | | | US | 2014179758 | A1 | 26 June 2014 |
| WO | 2005106035 | A2 | 10 November 2005 | WO | 2005106035 | A3 | 06 July 2006 |
| | | | | US | 2005282190 | A1 | 22 December 2005 |
| WO | 2017197009 | A1 | 16 November 2017 | CN | 109196103 | A | 11 January 2019 |
| | | | | US | 2019127739 | A1 | 02 May 2019 |
| CN | 104327141 | A | 04 February 2015 | CN | 104327141 | B | 04 August 2017 |
| CN | 108602849 | A | 28 September 2018 | EP | 3440090 | A4 | 27 November 2019 |
| | | | | WO | 2017176894 | A1 | 12 October 2017 |
| | | | | US | 2019024085 | A1 | 24 January 2019 |
| | | | | EP | 3440090 | A1 | 13 February 2019 |
| WO | 2009054813 | A1 | 30 April 2009 | JP | 2015007107 | A | 15 January 2015 |
| | | | | KR | 101694920 | B1 | 11 January 2017 |
| | | | | EP | 2214715 | A4 | 10 September 2014 |
| | | | | JP | 2011500799 | A | 06 January 2011 |
| | | | | ES | 2677327 | T3 | 01 August 2018 |
| | | | | JP | 6109795 | B2 | 05 April 2017 |
| | | | | US | 9248200 | B2 | 02 February 2016 |
| | | | | EP | 2214715 | B1 | 18 April 2018 |
| | | | | US | 2016106706 | A1 | 21 April 2016 |
| | | | | JP | 2017025108 | A | 02 February 2017 |
| | | | | KR | 20150030781 | A | 20 March 2015 |
| | | | | KR | 20100093048 | A | 24 August 2010 |
| | | | | KR | 101576310 | B1 | 09 December 2015 |
| | | | | CN | 101909655 | B | 10 April 2013 |
| | | | | EP | 2214715 | A1 | 11 August 2010 |
| | | | | CN | 101909655 | A | 08 December 2010 |
| | | | | US | 2011044992 | A1 | 24 February 2011 |
| | | | | JP | 5612475 | B2 | 22 October 2014 |
| US | 2012189688 | A1 | 26 July 2012 | TW | 201231088 | A | 01 August 2012 |
| | | | | TW | I464000 | B | 11 December 2014 |
| CN | 104368004 | A | 25 February 2015 | None | | | |
| CN | 106806895 | A | 09 June 2017 | None | | | |
| WO | 2013185032 | A1 | 12 December 2013 | US | 2015147276 | A1 | 28 May 2015 |
| | | | | HK | 1209021 | A1 | 24 March 2016 |
| | | | | JP | 2018172415 | A | 08 November 2018 |
| | | | | CN | 104684546 | A | 03 June 2015 |
| | | | | EP | 2858630 | A1 | 15 April 2015 |
| | | | | CA | 2876139 | A1 | 12 December 2013 |
| | | | | JP | 2015520194 | A | 16 July 2015 |
| | | | | EP | 2858630 | A4 | 24 February 2016 |

Form PCT/ISA/210 (patent family annex) (January 2015)

SEQUENCE LISTING

<110> BAI YAO ZHI DA (Beijing) NanoBio Technology Co., Ltd

<120> Nucleic acid nanocarrier drug and preparation method thereof

<130> PN148696BYZD

<150> 201811270120.8
<151> 2018-10-29

<150> 201811270171.0
<151> 2018-10-29

<150> 201811237274.7
<151> 2018-10-23

<150> 201811230635.5
<151> 2018-10-22

<150> 201811224511.6
<151> 2018-10-19

<160> 180

<170> PatentIn version 3.5

<210> 1
<211> 10
<212> DNA
<213> Artificial Sequence

<220>
<223> Synthesized

<220>
<221> misc_feature
<222> (1)..(10)
<223> a1 sequence

<400> 1
ccagcguucc 10

<210> 2
<211> 10
<212> DNA
<213> Artificial Sequence

<220>
<223> Synthesized

<220>
<221> mRNA
<222> (1)..(10)
<223> a1 sequcence

<400> 2
ccagcgttcc 10

<210> 3
<211> 10
<212> DNA
<213> Artificial Sequence

<220>
<223> Synthesized and y is not exist,just for completing the sequence listing

<220>
<221> misc_feature
<222> (1)..(9)
<223> b1 sequcence

<400> 3
gguucgccgy 10

<210> 4
<211> 10
<212> DNA
<213> Artificial Sequence

<220>
<223> Synthesized and y is not exist,just for completing the sequence listing

<220>
<221> misc_feature
<222> (1)..(9)
<223> b1 sequcence

<400> 4
ggttcgccgy 10

<210> 5
<211> 12
<212> DNA
<213> Artificial Sequence

<220>
<223> Synthesized

<220>
<221> misc_feature
<222> (1)..(12)
<223> c1 sequcence

<400> 5
cggccauagc gg 12

<210> 6
<211> 12
<212> DNA
<213> Artificial Sequence

<220>
<223> Synthesized

<220>
<221> misc_feature
<222> (1)..(12)
<223> c1 sequcence

<400> 6
cggccatagc gg 12

<210> 7
<211> 10
<212> DNA
<213> Artificial Sequence

<220>
<223> Synthesized

<220>
<221> misc_feature
<222> (1)..(10)
<223> a sequcence

<400> 7
ggagcguugg 10

<210> 8
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 8
ccuucgccgy 10

<210> 9
<211> 12
<212> DNA
<213> Artificial Sequence

<220>
<223> Synthesized

<220>
<221> misc_feature
<222> (1)..(12)
<223> c sequcence

<400> 9

cggccauagc cc 12

<210> 10
<211> 10
<212> DNA
<213> Artificial Sequence

<220>
<223> Synthesized

<220>
<221> misc_feature
<222> (1)..(10)
<223> a sequcence

<400> 10
gcagcguucg 10

<210> 11
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 11
cguucgccgy 10

<210> 12
<211> 12
<212> DNA
<213> Artificial Sequence

<220>
<223> Synthesized

<220>
<221> misc_feature
<222> (1)..(12)
<223> c sequcence

<400> 12
cggccauagc gc 12

<210> 13
<211> 10
<212> DNA
<213> Artificial Sequence

<220>
<223> Synthesized

<220>
<221> misc_feature
<222> (1)..(10)
<223> a sequcence

<400> 13
cgagcguugc 10

<210> 14
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 14
gcuucgccgy 10

<210> 15
<211> 12
<212> DNA
<213> Artificial Sequence

<220>
<223> Synthesized

<220>
<221> misc_feature
<222> (1)..(12)
<223> c sequcence

<400> 15
cggccauagc cg 12

<210> 16
<211> 10
<212> DNA
<213> Artificial Sequence

<220>
<223> Synthesized

<220>
<221> misc_feature
<222> (1)..(10)
<223> a sequcence

<400> 16
ggagcguugg 10

<210> 17
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 17
ccuucggggy 10

<210> 18
<211> 12
<212> DNA
<213> Artificial Sequence

<220>
<223> Synthesized

<220>
<221> misc_feature
<222> (1)..(12)
<223> c sequcence

<400> 18
cccccauagc cc 12

<210> 19
<211> 10
<212> DNA
<213> Artificial Sequence

<220>
<223> Synthesized

<220>
<221> misc_feature
<222> (1)..(10)
<223> a sequcence

<400> 19
gcagcguucg 10

<210> 20
<211> 10
<212> DNA

<213> Artificial Sequence

<220>
<223> Synthesized and y is not exist,just for completing the sequence listing

<220>
<221> misc_feature
<222> (1)..(9)
<223> b sequcence

<400> 20
cguucggcgy 10

<210> 21
<211> 12
<212> DNA
<213> Artificial Sequence

<220>
<223> Synthesized

<220>
<221> misc_feature
<222> (1)..(12)
<223> c sequcence

<400> 21
cgcccauagc gc 12

<210> 22
<211> 10
<212> DNA
<213> Artificial Sequence

<220>
<223> Synthesized

<220>
<221> misc_feature
<222> (1)..(10)
<223> a sequcence

<400> 22
gcagcguucg 10

<210> 23
<211> 10
<212> DNA
<213> Artificial Sequence

<220>
<223> Synthesized and y is not exist,just for completing the sequence listing

<220>

<221> misc_feature
<222> (1)..(9)
<223> b sequcence

<400> 23
cguucggccy 10

<210> 24
<211> 12
<212> DNA
<213> Artificial Sequence

<220>
<223> Synthesized

<220>
<221> misc_feature
<222> (1)..(12)
<223> c sequcence

<400> 24
ggcccauagc gc 12

<210> 25
<211> 10
<212> DNA
<213> Artificial Sequence

<220>
<223> Synthesized

<220>
<221> misc_feature
<222> (1)..(10)
<223> a sequcence

<400> 25
cgagcguugc 10

<210> 26
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 26
gcuucggcgy 10

<210> 27
<211> 12
<212> DNA
<213> Artificial Sequence

<220>
<223> Synthesized

<220>
<221> misc_feature
<222> (1)..(12)
<223> c sequcence

<400> 27
cgcccauagc cg 12

<210> 28
<211> 10
<212> DNA
<213> Artificial Sequence

<220>
<223> Synthesized

<220>
<221> misc_feature
<222> (1)..(10)
<223> a sequcence

<400> 28
ggagcgttgg 10

<210> 29
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 29
ccttcgccgy 10

<210> 30
<211> 12
<212> DNA
<213> Artificial Sequence

<220>
<223> Synthesized

<220>
<221> misc_feature
<222> (1)..(12)
<223> c sequcence

<400> 30
cggccatagc cc 12

<210> 31
<211> 10
<212> DNA
<213> Artificial Sequence

<220>
<223> Synthesized

<220>
<221> misc_feature
<222> (1)..(10)
<223> a sequcence

<400> 31
gcagcgttcg 10

<210> 32
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 32
cgttcgccgy 10

<210> 33
<211> 12
<212> DNA
<213> Artificial Sequence

<220>
<223> Synthesized

<220>
<221> misc_feature
<222> (1)..(12)
<223> c sequcence

<400> 33
cggccatagc gc 12

<210> 34
<211> 10
<212> DNA
<213> Artificial Sequence

<220>
<223> Synthesized

<220>
<221> misc_feature
<222> (1)..(10)
<223> a sequcence

<400> 34
cgagcgttgc 10

<210> 35
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 35
gcttcgccgy 10

<210> 36
<211> 12
<212> DNA
<213> Artificial Sequence

<220>
<223> Synthesized

<220>
<221> misc_feature
<222> (1)..(12)
<223> c sequcence

<400> 36
cggccatagc cg 12

<210> 37
<211> 10
<212> DNA
<213> Artificial Sequence

<220>

<223> Synthesized

<220>
<221> misc_feature
<222> (1)..(10)
<223> a sequcence

<400> 37
ggagcgttgg 10

<210> 38
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 38
ccttcggggy 10

<210> 39
<211> 12
<212> DNA
<213> Artificial Sequence

<220>
<223> Synthesized

<220>
<221> misc_feature
<222> (1)..(12)
<223> c sequcence

<400> 39
cccccatagc cc 12

<210> 40
<211> 10
<212> DNA
<213> Artificial Sequence

<220>
<223> Synthesized

<220>
<221> misc_feature
<222> (1)..(10)
<223> a sequcence

<400> 40
gcagcgttcg 10

<210> 41
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 41
cgttcggcgy 10

<210> 42
<211> 12
<212> DNA
<213> Artificial Sequence

<220>
<223> Synthesized

<220>
<221> misc_feature
<222> (1)..(12)
<223> c sequcence

<400> 42
cgcccatagc gc 12

<210> 43
<211> 10
<212> DNA
<213> Artificial Sequence

<220>
<223> Synthesized

<220>
<221> misc_feature
<222> (1)..(10)
<223> a sequcence

<400> 43
gcagcgttcg 10

<210> 44
<211> 10
<212> DNA
<213> Artificial Sequence

<220>
<223> Synthesized and y is not exist,just for completing the sequence listing

<220>
<221> misc_feature
<222> (1)..(9)
<223> b sequcence

<400> 44
cgttcggccy 10

<210> 45
<211> 12
<212> DNA
<213> Artificial Sequence

<220>
<223> Synthesized

<220>
<221> misc_feature
<222> (1)..(12)
<223> c sequcence

<400> 45
ggcccatagc gc 12

<210> 46
<211> 10
<212> DNA
<213> Artificial Sequence

<220>
<223> Synthesized

<220>
<221> misc_feature
<222> (1)..(10)
<223> a sequcence

<400> 46
cgagcgttgc 10

<210> 47
<211> 10
<212> DNA
<213> Artificial Sequence

<220>
<223> Synthesized and y is not exist,just for completing the sequence listing

<220>
<221> misc_feature

<222> (1)..(9)
<223> b sequcence

<400> 47
gcttcggcgy 10

<210> 48
<211> 12
<212> DNA
<213> Artificial Sequence

<220>
<223> Synthesized

<220>
<221> misc_feature
<222> (1)..(12)
<223> c sequcence

<400> 48
cgcccatagc cg 12

<210> 49
<211> 77
<212> DNA
<213> Artificial Sequence

<220>
<223> Synthesized

<220>
<221> misc_feature
<222> (1)..(77)
<223> a sequcence

<220>
<221> misc_feature
<222> (1)..(77)
<223> M is U or T

<400> 49
cgcgcgaaaa aacgcgcgaa aaaacgcgcg cccaccagcg mmccgggcgc gcgaaaaaac 60

gcgcgaaaaa acgcgcg 77

<210> 50
<211> 75
<212> DNA
<213> Artificial Sequence

<220>
<223> Synthesized

<220>
<221> misc_feature
<222> (1)..(75)
<223> b sequcence

<220>
<221> misc_feature
<222> (1)..(75)
<223> M is U or T

<400> 50
cgcgcgmmmm mmcgcgcgmm mmmmcgcgcg cccggmmcgc cgccagccgc cmmmmmmgcc 60

gccmmmmmmg ccgcc 75

<210> 51
<211> 78
<212> DNA
<213> Artificial Sequence

<220>
<223> Synthesized

<220>
<221> misc_feature
<222> (1)..(78)
<223> c sequcence

<220>
<221> misc_feature
<222> (1)..(78)
<223> M is U or T

<400> 51
ggcggcaaaa aaggcggcaa aaaaggcggc aggcggcama gcggmgggcg cgcgmmmmmm 60

cgcgcgmmmm mmcgcgcg 78

<210> 52
<211> 29
<212> RNA
<213> Artificial Sequence

<220>
<223> Synthesized

<220>
<221> misc_feature
<222> (1)..(29)
<223> a strand

<220>
<221> misc_feature
<222> (1)..(1)
<223> 5′ Biotin

<400> 52
cgcgcgccca ccagcguucc gggcgccgc 29

<210> 53
<211> 27
<212> RNA

<213> Artificial Sequence

<220>
<223> Synthesized

<220>
<221> misc_feature
<222> (1)..(27)
<223> b strand

<220>
<221> misc_feature
<222> (1)..(1)
<223> 5’Biotin

<400> 53
gcggcgcccg guucgccgcc aggcggc 27

<210> 54
<211> 31
<212> RNA
<213> Artificial Sequence

<220>
<223> Synthesized

<220>
<221> misc_feature
<222> (1)..(31)
<223> c strand

<220>
<221> misc_feature
<222> (1)..(1)
<223> 5’CY3

<400> 54
gccgccaggc ggccauagcg gugggcgcgc g 31

<210> 55
<211> 10
<212> RNA
<213> Artificial Sequence

<220>
<223> Synthesized

<220>
<221> misc_feature
<222> (1)..(10)
<223> a strand

<400> 55
ggagcguugg 10

<210> 56
<211> 10

<212> RNA
<213> Artificial Sequence

<220>
<223> Synthesized and y is not exist,just for completing the sequence listing

<220>
<221> misc_feature
<222> (1)..(9)
<223> b strand

<400> 56
ccuucgccgy 10

<210> 57
<211> 12
<212> RNA
<213> Artificial Sequence

<220>
<223> Synthesized

<220>
<221> misc_feature
<222> (1)..(12)
<223> c strand

<400> 57
cggccauagc cc 12

<210> 58
<211> 10
<212> RNA
<213> Artificial Sequence

<220>
<223> Synthesized

<220>
<221> misc_feature
<222> (1)..(10)
<223> a strand

<400> 58
gcagcguucg 10

<210> 59
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 59
cguucgccgy 10

<210> 60
<211> 12
<212> RNA
<213> Artificial Sequence

<220>
<223> Synthesized

<220>
<221> misc_feature
<222> (1)..(12)
<223> c strand

<400> 60
cggccauagc gc 12

<210> 61
<211> 10
<212> RNA
<213> Artificial Sequence

<220>
<223> Synthesized

<220>
<221> misc_feature
<222> (1)..(10)
<223> a strand

<400> 61
cgagcguugc 10

<210> 62
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 62
gcuucgccgy 10

<210> 63
<211> 12
<212> RNA
<213> Artificial Sequence

<220>
<223> Synthesized

<220>
<221> misc_feature
<222> (1)..(12)
<223> c strand

<400> 63
cggccauagc cg 12

<210> 64
<211> 10
<212> RNA
<213> Artificial Sequence

<220>
<223> Synthesized

<220>
<221> misc_feature
<222> (1)..(10)
<223> a strand

<400> 64
ggagcguugg 10

<210> 65
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 65
ccuucggggy 10

<210> 66
<211> 12
<212> RNA
<213> Artificial Sequence

<220>
<223> Synthesized

<220>
<221> misc_feature
<222> (1)..(12)
<223> c strand

<400> 66
cccccauagc cc 12

<210> 67
<211> 10
<212> RNA
<213> Artificial Sequence

<220>
<223> Synthesized

<220>
<221> misc_feature
<222> (1)..(10)
<223> a strand

<400> 67
gcagcguucg 10

<210> 68
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 68
cguucggcgy 10

<210> 69
<211> 12
<212> RNA
<213> Artificial Sequence

<220>
<223> Synthesized

<220>
<221> misc_feature
<222> (1)..(12)
<223> c strand

<400> 69

cgcccauagc gc 12

<210> 70
<211> 10
<212> RNA
<213> Artificial Sequence

<220>
<223> Synthesized

<220>
<221> misc_feature
<222> (1)..(10)
<223> a strand

<400> 70
gcagcguucg 10

<210> 71
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 71
cguucggccy 10

<210> 72
<211> 12
<212> RNA
<213> Artificial Sequence

<220>
<223> Synthesized

<220>
<221> misc_feature
<222> (1)..(12)
<223> c strand

<400> 72
ggcccauagc gc 12

<210> 73
<211> 10
<212> RNA
<213> Artificial Sequence

<220>
<223> Synthesized

<220>
<221> misc_feature
<222> (1)..(10)
<223> a strand

<400> 73
cgagcguugc 10

<210> 74
<211> 10
<212> RNA
<213> Artificial Sequence

<220>
<223> Synthesized and y is not exist,just for completing the sequence listing

<220>
<221> misc_feature
<222> (1)..(9)
<223> b strand

<400> 74
gcuucggcgy 10

<210> 75
<211> 12
<212> RNA
<213> Artificial Sequence

<220>
<223> Synthesized

<220>
<221> misc_feature
<222> (1)..(12)
<223> c strand

<400> 75
cgcccauagc cg 12

<210> 76
<211> 29
<212> RNA
<213> Artificial Sequence

<220>
<223> Synthesized

<220>
<221> misc_feature
<222> (1)..(29)
<223> a strand

<400> 76
cgcgcgccca ggagcguugg cgggcggcg 29

<210> 77
<211> 27
<212> RNA
<213> Artificial Sequence

<220>
<223> Synthesized

<220>
<221> misc_feature
<222> (1)..(27)
<223> b strand

<400> 77
cgccgcccgc cuucgccgcc agccgcc 27

<210> 78
<211> 31
<212> RNA
<213> Artificial Sequence

<220>
<223> Synthesized

<220>
<221> misc_feature
<222> (1)..(31)
<223> c strand

<400> 78
ggcggcaggc ggccauagcc cugggcgcgc g 31

<210> 79
<211> 29
<212> RNA
<213> Artificial Sequence

<220>
<223> Synthesized

<220>
<221> misc_feature
<222> (1)..(29)
<223> a strand

<400> 79
cgcgcgccca gcagcguucg cgggcggcg 29

<210> 80
<211> 27
<212> RNA
<213> Artificial Sequence

<220>
<223> Synthesized

<220>
<221> misc_feature
<222> (1)..(27)
<223> b strand

<400> 80
cgccgcccgc guucgccgcc agccgcc 27

<210> 81
<211> 31
<212> RNA
<213> Artificial Sequence

<220>
<223> Synthesized

<220>
<221> misc_feature
<222> (1)..(31)
<223> c strand

<400> 81
ggcggcaggc ggccauagcg cugggcgcgc g 31

<210> 82
<211> 29
<212> RNA
<213> Artificial Sequence

<220>
<223> Synthesized

<220>
<221> misc_feature
<222> (1)..(29)
<223> a strand

<400> 82
cgcgcgccca cgagcguugc ggggcggcg 29

<210> 83
<211> 27
<212> RNA
<213> Artificial Sequence

<220>
<223> Synthesized

<220>
<221> misc_feature
<222> (1)..(27)
<223> b strand

<400> 83
cgccgccccg cuucgccgcc agccgcc 27

<210> 84
<211> 31
<212> RNA
<213> Artificial Sequence

<220>
<223> Synthesized

<220>
<221> misc_feature
<222> (1)..(31)
<223> c strand

<400> 84
ggcggcaggc ggccauagcc gugggcgcgc g 31

<210> 85
<211> 29
<212> RNA
<213> Artificial Sequence

<220>
<223> Synthesized

<220>
<221> misc_feature
<222> (1)..(29)
<223> a strand

<400> 85
cgcgcgccca ggagcguugg cccgcggcg 29

<210> 86
<211> 27
<212> RNA
<213> Artificial Sequence

<220>
<223> Synthesized

<220>
<221> misc_feature
<222> (1)..(27)
<223> b strand

<400> 86
cgccgcgggc cuucggggcc agccgcc 27

<210> 87
<211> 31
<212> RNA
<213> Artificial Sequence

<220>
<223> Synthesized

<220>
<221> misc_feature
<222> (1)..(31)
<223> c strand

<400> 87
ggcggcaggc ccccauagcc cugggcgcgc g 31

<210> 88
<211> 29
<212> RNA
<213> Artificial Sequence

<220>
<223> Synthesized

<220>
<221> misc_feature
<222> (1)..(29)
<223> a strand

<400> 88
cgcgcgccca gcagcguucg ccccgccgc 29

<210> 89
<211> 27
<212> RNA
<213> Artificial Sequence

<220>
<223> Synthesized

<220>
<221> misc_feature
<222> (1)..(27)
<223> b strand

<400> 89
gcggcggggc guucggcggc aggcggc 27

<210> 90
<211> 31
<212> RNA
<213> Artificial Sequence

<220>
<223> Synthesized

<220>
<221> misc_feature
<222> (1)..(31)
<223> c strand

<400> 90
gccgccagcc gcccauagcg cugggcgcgc g 31

<210> 91
<211> 29
<212> RNA
<213> Artificial Sequence

<220>
<223> Synthesized

<220>
<221> misc_feature
<222> (1)..(29)
<223> a strand

<400> 91
cgcgcgccca gcagcguucg gggcgccgc 29

<210> 92
<211> 28
<212> RNA
<213> Artificial Sequence

<220>
<223> Synthesized

<220>
<221> misc_feature
<222> (1)..(28)
<223> b strand

<400> 92
gcggcgcccc guucggccgg caggcggc 28

<210> 93
<211> 32
<212> RNA
<213> Artificial Sequence

<220>
<223> Synthesized

<220>
<221> misc_feature
<222> (1)..(32)
<223> c strand

<400> 93
gccgccagcc ggcccauagc gcugggcgcg cg 32

<210> 94
<211> 40
<212> DNA
<213> Artificial Sequence

<220>
<223>  Synthesized

<220>
<221>  misc_feature
<222>  (1)..(40)
<223>  a strand

<400>  94
cgcgcgcgag cguugcaaug acagauaagg aaccugcutt    40

<210>  95
<211>  36
<212>  RNA
<213>  Artificial Sequence

<220>
<223>  Synthesized

<220>
<221>  misc_feature
<222>  (1)..(36)
<223>  b strand

<400>  95
ggcagguucc uuaucuguca aagcuucggc ggcagc    36

<210>  96
<211>  23
<212>  RNA
<213>  Artificial Sequence

<220>
<223>  Synthesized

<220>
<221>  misc_feature
<222>  (1)..(23)
<223>  c strand

<400>  96
gcagccgccc auagccgcgc gcg    23

<210>  97
<211>  39
<212>  DNA
<213>  Artificial Sequence

<220>
<223>  Synthesized

<220>
<221>  misc_feature
<222>  (1)..(39)
<223>  EGFRapt

<400> 97
gccttagtaa cgtgctttga tgtcgattcg acaggaggc 39

<210> 98
<211> 41
<212> DNA
<213> Artificial Sequence

<220>
<223> Synthesized

<220>
<221> misc_feature
<222> (1)..(41)
<223> PSMAapt

<400> 98
gggccgaaaa agacctgact tctatactaa gtctacgtcc c 41

<210> 99
<211> 68
<212> DNA
<213> Artificial Sequence

<220>
<223> Synthesized

<220>
<221> misc_feature
<222> (1)..(68)
<223> a strand

<400> 99
cgcgcgccca ggagcgttgg cgggcggcgg ccttagtaac gtgctttgat gtcgattcga 60

caggaggc 68

<210> 100
<211> 27
<212> DNA
<213> Artificial Sequence

<220>
<223> Synthesized

<220>
<221> misc_feature
<222> (1)..(27)
<223> b strand

<400> 100
cgccgcccgc cttcgccgcc agccgcc 27

<210> 101
<211> 31

<212> DNA
<213> Artificial Sequence

<220>
<223> Synthesized

<220>
<221> misc_feature
<222> (1)..(31)
<223> c strand

<400> 101
ggcggcaggc ggccatagcc ctgggcgcgc g 31

<210> 102
<211> 68
<212> DNA
<213> Artificial Sequence

<220>
<223> Synthesized

<220>
<221> misc_feature
<222> (1)..(68)
<223> a strand

<400> 102
cgcgcgccca gcagcgttcg cgggcggcgg ccttagtaac gtgctttgat gtcgattcga 60

caggaggc 68

<210> 103
<211> 27
<212> DNA
<213> Artificial Sequence

<220>
<223> Synthesized

<220>
<221> misc_feature
<222> (1)..(27)
<223> b strand

<400> 103
cgccgcccgc gttcgccgcc agccgcc 27

<210> 104
<211> 31
<212> DNA
<213> Artificial Sequence

<220>
<223> Synthesized

<220>
<221> misc_feature
<222> (1)..(31)
<223> c strand

<400> 104
ggcggcaggc ggccatagcg ctgggcgcgc g 31

<210> 105
<211> 68
<212> DNA
<213> Artificial Sequence

<220>
<223> Synthesized

<220>
<221> misc_feature
<222> (1)..(68)
<223> a strand

<400> 105
cgcgcgccca cgagcgttgc ggggcggcgg ccttagtaac gtgctttgat gtcgattcga 60

caggaggc 68

<210> 106
<211> 27
<212> DNA
<213> Artificial Sequence

<220>
<223> Synthesized

<220>
<221> misc_feature
<222> (1)..(27)
<223> b strand

<400> 106
cgccgccccg cttcgccgcc agccgcc 27

<210> 107
<211> 31
<212> DNA
<213> Artificial Sequence

<220>
<223> Synthesized

<220>
<221> misc_feature
<222> (1)..(31)
<223> c strand

<400> 107
ggcggcaggc ggccatagcc gtgggcgcgc g 31

<210> 108
<211> 71
<212> DNA
<213> Artificial Sequence

<220>
<223> Synthesized

<220>
<221> misc_feature
<222> (1)..(71)
<223> a strand

<400> 108
cgcgcgccca ggagcgttgg cccgcggcgt gggccgaaaa agacctgact tctatactaa 60

gtctacgtcc c 71

<210> 109
<211> 27
<212> DNA
<213> Artificial Sequence

<220>
<223> Synthesized

<220>
<221> misc_feature
<222> (1)..(27)
<223> b strand

<400> 109
cgccgcgggc cttcggggcc agccgcc 27

<210> 110
<211> 31
<212> DNA
<213> Artificial Sequence

<220>
<223> Synthesized

<220>
<221> misc_feature
<222> (1)..(31)
<223> c strand

<400> 110
ggcggcaggc ccccatagcc ctgggcgcgc g 31

<210> 111
<211> 71
<212> DNA
<213> Artificial Sequence

<220>
<223>  Synthesized

<220>
<221>  misc_feature
<222>  (1)..(71)
<223>  a strand

<400>  111
cgcgcgccca gcagcgttcg ccccgccgct gggccgaaaa agacctgact tctatactaa      60

gtctacgtcc c      71

<210>  112
<211>  27
<212>  DNA
<213>  Artificial Sequence

<220>
<223>  Synthesized

<220>
<221>  misc_feature
<222>  (1)..(27)
<223>  b strand

<400>  112
gcggcggggc gttcggcggc aggcggc      27

<210>  113
<211>  31
<212>  DNA
<213>  Artificial Sequence

<220>
<223>  Synthesized

<220>
<221>  misc_feature
<222>  (1)..(31)
<223>  c strand

<400>  113
gccgccagcc gcccatagcg ctgggcgcgc g      31

<210>  114
<211>  29
<212>  DNA
<213>  Artificial Sequence

<220>
<223>  Synthesized

<220>
<221>  misc_feature
<222>  (1)..(29)

<223> a strand

<400> 114
cgcgcgccca gcagcgttcg gggcgccgc 29

<210> 115
<211> 28
<212> DNA
<213> Artificial Sequence

<220>
<223> Synthesized

<220>
<221> misc_feature
<222> (1)..(20)
<223> b strand

<400> 115
gcggcgcccc gttcggccgg caggcggc 28

<210> 116
<211> 32
<212> DNA
<213> Artificial Sequence

<220>
<223> Synthesized

<220>
<221> misc_feature
<222> (1)..(32)
<223> c strand

<400> 116
gccgccagcc ggcccatagc gctgggcgcg cg 32

<210> 117
<211> 29
<212> DNA
<213> Artificial Sequence

<220>
<223> Synthesized

<220>
<221> misc_feature
<222> (1)..(29)
<223> a strand

<400> 117
cgcgcgccca cgagcgttgc gggcgccgc 29

<210> 118
<211> 27
<212> DNA

<213> Artificial Sequence

<220>
<223> Synthesized

<220>
<221> misc_feature
<222> (1)..(27)
<223> b strand

<400> 118
gcggcgcccg cttcggcggc aggcggc 27

<210> 119
<211> 31
<212> DNA
<213> Artificial Sequence

<220>
<223> Synthesized

<220>
<221> misc_feature
<222> (1)..(31)
<223> c strand

<400> 119
gccgccagcc gcccatagcc gtgggcgcgc g 31

<210> 120
<211> 37
<212> RNA
<213> Artificial Sequence

<220>
<223> Synthesized

<220>
<221> misc_feature
<222> (1)..(37)
<223> a strand

<400> 120
gcggcgagcg gcgaggagcg uuggggccgg aggccgg 37

<210> 121
<211> 31
<212> RNA
<213> Artificial Sequence

<220>
<223> Synthesized

<220>
<221> misc_feature
<222> (1)..(31)

<223> b strand

<400> 121
ccggccuccg gccccuucgg ggccagccgc c 31

<210> 122
<211> 35
<212> RNA
<213> Artificial Sequence

<220>
<223> Synthesized

<220>
<221> misc_feature
<222> (1)..(35)
<223> c strand

<400> 122
ggcggcaggc ccccauagcc cucgccgcuc gccgc 35

<210> 123
<211> 37
<212> RNA
<213> Artificial Sequence

<220>
<223> Synthesized

<220>
<221> misc_feature
<222> (1)..(37)
<223> a strand

<400> 123
gcggcgagcg gcgagcagcg uucgggccgg aggccgg 37

<210> 124
<211> 31
<212> RNA
<213> Artificial Sequence

<220>
<223> Synthesized

<220>
<221> misc_feature
<222> (1)..(31)
<223> b strand

<400> 124
ccggccuccg gcccguucgc cgccagccgc c 31

<210> 125
<211> 35
<212> RNA

<213> Artificial Sequence

<220>
<223> Synthesized

<220>
<221> misc_feature
<222> (1)..(35)
<223> c strand

<400> 125
ggcggcaggc ggccauagcg cucgccgcuc gccgc 35

<210> 126
<211> 37
<212> RNA
<213> Artificial Sequence

<220>
<223> Synthesized

<220>
<221> misc_feature
<222> (1)..(37)
<223> a strand

<400> 126
gcggcgagcg gcgaggagcg uuggggccgg aggccgg 37

<210> 127
<211> 31
<212> RNA
<213> Artificial Sequence

<220>
<223> Synthesized

<220>
<221> misc_feature
<222> (1)..(31)
<223> b strand

<400> 127
ccggccuccg gccccuucgc cgccagccgc c 31

<210> 128
<211> 35
<212> RNA
<213> Artificial Sequence

<220>
<223> Synthesized

<220>
<221> misc_feature
<222> (1)..(35)

<223> c strand

<400> 128
ggcggcaggc ggccauagcc cucgccgcuc gccgc 35

<210> 129
<211> 37
<212> RNA
<213> Artificial Sequence

<220>
<223> Synthesized

<220>
<221> misc_feature
<222> (1)..(37)
<223> a strand

<400> 129
gcggcgagcg gcgagcagcg uucgggccgg aggccgg 37

<210> 130
<211> 31
<212> RNA
<213> Artificial Sequence

<220>
<223> Synthesized

<220>
<221> misc_feature
<222> (1)..(31)
<223> b strand

<400> 130
ccggccuccg gcccguucgg cgccagccgc c 31

<210> 131
<211> 35
<212> RNA
<213> Artificial Sequence

<220>
<223> Synthesized

<220>
<221> misc_feature
<222> (1)..(35)
<223> c strand

<400> 131
ggcggcaggc gcccauagcg cucgccgcuc gccgc 35

<210> 132
<211> 37
<212> RNA

<213> Artificial Sequence

<220>
<223> Synthesized

<220>
<221> misc_feature
<222> (1)..(37)
<223> a strand

<400> 132
gcggcgagcg gcgagcagcg uucgggccgg aggccgg 37

<210> 133
<211> 31
<212> RNA
<213> Artificial Sequence

<220>
<223> Synthesized

<220>
<221> misc_feature
<222> (1)..(31)
<223> b strand

<400> 133
ccggccuccg gcccguucgg ccccagccgc c 31

<210> 134
<211> 35
<212> RNA
<213> Artificial Sequence

<220>
<223> Synthesized

<220>
<221> misc_feature
<222> (1)..(35)
<223> c strand

<400> 134
ggcggcaggg gcccauagcg cucgccgcuc gccgc 35

<210> 135
<211> 37
<212> RNA
<213> Artificial Sequence

<220>
<223> Synthesized

<220>
<221> misc_feature
<222> (1)..(37)

<223> a strand

<400> 135
gcggcgagcg gcgacgagcg uugcggccgg aggccgg 37

<210> 136
<211> 31
<212> RNA
<213> Artificial Sequence

<220>
<223> Synthesized

<220>
<221> misc_feature
<222> (1)..(31)
<223> b strand

<400> 136
ccggccuccg gccgcuucgc cgccagccgc c 31

<210> 137
<211> 35
<212> RNA
<213> Artificial Sequence

<220>
<223> Synthesized

<220>
<221> misc_feature
<222> (1)..(35)
<223> c strand

<400> 137
ggcggcaggc ggccauagcc gucgccgcuc gccgc 35

<210> 138
<211> 37
<212> RNA
<213> Artificial Sequence

<220>
<223> Synthesized

<220>
<221> misc_feature
<222> (1)..(37)
<223> a strand

<400> 138
gcggcgagcg gcgacgagcg uugcggccgg aggccgg 37

<210> 139
<211> 31
<212> RNA

<213> Artificial Sequence

<220>
<223> Synthesized

<220>
<221> misc_feature
<222> (1)..(31)
<223> b strand

<400> 139
ccggccuccg gccgcuucgg cgccagccgc c 31

<210> 140
<211> 35
<212> RNA
<213> Artificial Sequence

<220>
<223> Synthesized

<220>
<221> misc_feature
<222> (1)..(35)
<223> c strand

<400> 140
ggcggcaggc gcccauagcc gucgccgcuc gccgc 35

<210> 141
<211> 37
<212> DNA
<213> Artificial Sequence

<220>
<223> Synthesized

<220>
<221> misc_feature
<222> (1)..(37)
<223> a strand

<400> 141
gcggcgagcg gcgaggagcg ttggggccgg aggccgg 37

<210> 142
<211> 31
<212> DNA
<213> Artificial Sequence

<220>
<223> Synthesized

<220>
<221> misc_feature
<222> (1)..(31)

<223> b strand

<400> 142
ccggcctccg gccccttcgg ggccagccgc c 31

<210> 143
<211> 35
<212> DNA
<213> Artificial Sequence

<220>
<223> Synthesized

<220>
<221> misc_feature
<222> (1)..(35)
<223> c strand

<400> 143
ggcggcaggc ccccatagcc ctcgccgctc gccgc 35

<210> 144
<211> 37
<212> DNA
<213> Artificial Sequence

<220>
<223> Synthesized

<220>
<221> misc_feature
<222> (1)..(37)
<223> a strand

<400> 144
gcggcgagcg gcgagcagcg ttcgggccgg aggccgg 37

<210> 145
<211> 31
<212> DNA
<213> Artificial Sequence

<220>
<223> Synthesized

<220>
<221> misc_feature
<222> (1)..(31)
<223> b strand

<400> 145
ccggcctccg gcccgttcgc cgccagccgc c 31

<210> 146
<211> 35
<212> DNA

<213>  Artificial Sequence

<220>
<223>  Synthesized

<220>
<221>  misc_feature
<222>  (1)..(35)
<223>  c strand

<400>  146
ggcggcaggc ggccatagcg ctcgccgctc gccgc    35

<210>  147
<211>  37
<212>  DNA
<213>  Artificial Sequence

<220>
<223>  Synthesized

<220>
<221>  misc_feature
<222>  (1)..(37)
<223>  a strand

<400>  147
gcggcgagcg gcgaggagcg ttggggccgg aggccgg    37

<210>  148
<211>  31
<212>  DNA
<213>  Artificial Sequence

<220>
<223>  Synthesized

<220>
<221>  misc_feature
<222>  (1)..(31)
<223>  b strand

<400>  148
ccggcctccg gccccttcgc cgccagccgc c    31

<210>  149
<211>  35
<212>  DNA
<213>  Artificial Sequence

<220>
<223>  Synthesized

<220>
<221>  misc_feature
<222>  (1)..(35)

<223>  c strand

<400>  149
ggcggcaggc ggccatagcc ctcgccgctc gccgc    35

<210>  150
<211>  37
<212>  DNA
<213>  Artificial Sequence

<220>
<223>  Synthesized

<220>
<221>  misc_feature
<222>  (1)..(37)
<223>  a strand

<400>  150
gcggcgagcg gcgagcagcg ttcgggccgg aggccgg    37

<210>  151
<211>  31
<212>  DNA
<213>  Artificial Sequence

<220>
<223>  Synthesized

<220>
<221>  misc_feature
<222>  (1)..(31)
<223>  b strand

<400>  151
ccggcctccg gcccgttcgg cgccagccgc c    31

<210>  152
<211>  35
<212>  DNA
<213>  Artificial Sequence

<220>
<223>  Synthesized

<220>
<221>  misc_feature
<222>  (1)..(35)
<223>  c strand

<400>  152
ggcggcaggc gcccatagcg ctcgccgctc gccgc    35

<210>  153
<211>  37
<212>  DNA

<213> Artificial Sequence

<220>
<223> Synthesized

<220>
<221> misc_feature
<222> (1)..(37)
<223> a strand

<400> 153
gcggcgagcg gcgagcagcg ttcgggccgg aggccgg 37

<210> 154
<211> 31
<212> DNA
<213> Artificial Sequence

<220>
<223> Synthesized

<220>
<221> misc_feature
<222> (1)..(31)
<223> b strand

<400> 154
ccggcctccg gcccgttcgg ccccagccgc c 31

<210> 155
<211> 35
<212> DNA
<213> Artificial Sequence

<220>
<223> Synthesized

<220>
<221> misc_feature
<222> (1)..(35)
<223> c strand

<400> 155
ggcggcaggg gcccatagcg ctcgccgctc gccgc 35

<210> 156
<211> 37
<212> DNA
<213> Artificial Sequence

<220>
<223> Synthesized

<220>
<221> misc_feature
<222> (1)..(37)

<223> a strand

<400> 156
gcggcgagcg gcgacgagcg ttgcggccgg aggccgg 37

<210> 157
<211> 31
<212> DNA
<213> Artificial Sequence

<220>
<223> Synthesized

<220>
<221> misc_feature
<222> (1)..(31)
<223> b strand

<400> 157
ccggcctccg gccgcttcgc cgccagccgc c 31

<210> 158
<211> 35
<212> DNA
<213> Artificial Sequence

<220>
<223> Synthesized

<220>
<221> misc_feature
<222> (1)..(35)

<220>
<221> misc_feature
<222> (1)..(35)
<223> c strand

<400> 158
ggcggcaggc ggccatagcc gtcgccgctc gccgc 35

<210> 159
<211> 37
<212> DNA
<213> Artificial Sequence

<220>
<223> Synthesized

<220>
<221> misc_feature
<222> (1)..(37)
<223> a strand

<400> 159
gcggcgagcg gcgacgagcg ttgcggccgg aggccgg 37

<210> 160
<211> 31
<212> DNA
<213> Artificial Sequence

<220>
<223> Synthesized

<220>
<221> misc_feature
<222> (1)..(31)
<223> b strand

<400> 160
ccggcctccg gccgcttcgg cgccagccgc c 31

<210> 161
<211> 35
<212> DNA
<213> Artificial Sequence

<220>
<223> Synthesized

<220>
<221> misc_feature
<222> (1)..(35)
<223> c strand

<400> 161
ggcggcaggc gcccatagcc gtcgccgctc gccgc 35

<210> 162
<211> 14
<212> DNA
<213> Artificial Sequence

<220>
<223> Synthesized

<220>
<221> misc_feature
<222> (1)..(14)
<223> first elongating fragment

<400> 162
gcggcgagcg gcga 14

<210> 163
<211> 14
<212> DNA
<213> Artificial Sequence

<220>
<223> Synthesized

<220>
<221> misc_feature
<222> (1)..(14)
<223> first elongating fragment

<400> 163
ucgccgcucg ccgc 14

<210> 164
<211> 13
<212> DNA
<213> Artificial Sequence

<220>
<223> Synthesized

<220>
<221> misc_feature
<222> (1)..(13)
<223> first elongating fragment

<400> 164
ggccggaggc cgg 13

<210> 165
<211> 13
<212> DNA
<213> Artificial Sequence

<220>
<223> Synthesized

<220>
<221> misc_feature
<222> (1)..(13)
<223> first elongating fragment

<400> 165
ccggccuccg gcc 13

<210> 166
<211> 10
<212> DNA
<213> Artificial Sequence

<220>
<223> Synthesized and y is not exist,just for completing the sequence listing

<220>
<221> misc_feature
<222> (1)..(9)
<223> first elongating fragment

<400> 166
ccagccgccy 10

<210> 167
<211> 10
<212> DNA
<213> Artificial Sequence

<220>
<223> Synthesized and y is not exist,just for completing the sequence listing

<220>
<221> misc_feature
<222> (1)..(9)
<223> first elongating fragment

<400> 167
ggcggcaggy 10

<210> 168
<211> 14
<212> DNA
<213> Artificial Sequence

<220>
<223> Synthesized

<220>
<221> misc_feature
<222> (1)..(14)
<223> first elongating fragment

<400> 168
gcggcgagcg gcga 14

<210> 169
<211> 14
<212> DNA
<213> Artificial Sequence

<220>
<223> Synthesized

<220>
<221> misc_feature
<222> (1)..(14)
<223> first elongating fragment

<400> 169
tcgccgctcg ccgc 14

<210> 170
<211> 13
<212> DNA
<213> Artificial Sequence

<220>

<223> Synthesized

<220>
<221> misc_feature
<222> (1)..(13)
<223> first elongating fragment

<400> 170
ggccggaggc cgg 13

<210> 171
<211> 13
<212> DNA
<213> Artificial Sequence

<220>
<223> Synthesized

<220>
<221> misc_feature
<222> (1)..(13)
<223> first elongating fragment

<400> 171
ccggcctccg gcc 13

<210> 172
<211> 65
<212> DNA
<213> Artificial Sequence

<220>
<223> Synthesized

<220>
<221> misc_feature
<222> (1)..(65)
<223> a strand

<400> 172
cgcgcgccca cgagcgttcc gggcgcgcct tagtaacgtg ctttgatgtc gattcgacag 60

gaggc 65

<210> 173
<211> 26
<212> DNA
<213> Artificial Sequence

<220>
<223> Synthesized

<220>
<221> misc_feature
<222> (1)..(26)
<223> b strand

<400> 173
gcgcccggtt cgccgccagc cgccgc 26

<210> 174
<211> 33
<212> DNA
<213> Artificial Sequence

<220>
<223> Synthesized

<220>
<221> misc_feature
<222> (1)..(33)
<223> c strand

<400> 174
gcggcggcag gcggccatag ccgtgggcgc gcg 33

<210> 175
<211> 10
<212> DNA
<213> Artificial Sequence

<220>
<223> Synthesized

<220>
<221> misc_feature
<222> (1)..(10)
<223> a strand

<400> 175
cgagcgttcc 10

<210> 176
<211> 10
<212> DNA
<213> Artificial Sequence

<220>
<223> Synthesized and y is not exist,just for completing the sequence listing

<220>
<221> misc_feature
<222> (1)..(9)
<223> b strand

<400> 176
ggttcgccgy 10

<210> 177
<211> 12
<212> DNA

<213> Artificial Sequence

<220>
<223> Synthesized

<220>
<221> misc_feature
<222> (1)..(12)
<223> c strand

<400> 177
cggccatagc cg 12

<210> 178
<211> 34
<212> DNA
<213> Artificial Sequence

<220>
<223> Synthesized

<220>
<221> misc_feature
<222> (1)..(34)
<223> a strand

<400> 178
cgcgcgcgcc cacgagcgtt ccgggcgccg ccgc 34

<210> 179
<211> 33
<212> DNA
<213> Artificial Sequence

<220>
<223> Synthesized

<220>
<221> misc_feature
<222> (1)..(33)
<223> a strand

<220>
<221> misc_feature
<222> (1)..(33)
<223> b strand

<400> 179
gcggcggcgc ccggttcgcc gccagccgcc gcc 33

<210> 180
<211> 36
<212> DNA
<213> Artificial Sequence

<220>
<223> Synthesized

```
<220>
<221>  misc_feature
<222>  (1)..(36)
<223>  c strand

<400>  180
ggcggcggca ggcggccata gccgtgggcg cgcgcg                              36
```